(19) Europäisches Patentamt
European Patent Office
Office européen des brevets

(11) **EP 4 137 167 A1**

(12) **EUROPEAN PATENT APPLICATION**

(43) Date of publication:
**22.02.2023 Bulletin 2023/08**

(21) Application number: **22189379.5**

(22) Date of filing: **09.08.2022**

(51) International Patent Classification (IPC):
**A61L 27/26** (2006.01)     **A61L 27/52** (2006.01)

(52) Cooperative Patent Classification (CPC):
(C-Sets available)
**A61L 27/26; A61L 27/52;** A61L 2430/16     (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.08.2021 US 202163231138 P**

(71) Applicant: **RVO 2.0, Inc. D/B/A Optics Medical Aliso Viejo, CA 92656 (US)**

(72) Inventors:
• **NJIKANG, Gabriel N.**
**Orcutt, 93455 (US)**
• **LE, Alan Ngoc**
**Lake Forest, 92630 (US)**

(74) Representative: **Schlich, George Schlich**
**9 St Catherine's Road**
**Littlehampton, West Sussex BN17 5HS (GB)**

(54) **CORNEAL IMPLANTS FOR TREATING ECTATIC CORNEAL DISEASE**

(57)     A corneal device comprising a base surface and a top surface, and having a water content ranging from about 78%-92% (w/w), inclusive. The corneal device can be used to treat, for example without limitation, ectatic corneal disease by supporting corneal structure weakened by the disease and/or changing an anterior curvature of the cornea.

FIG. 6

EP 4 137 167 A1

(52) Cooperative Patent Classification (CPC): (Cont.)

C-Sets
**A61L 27/26, C08L 71/02;**
**A61L 27/26, C08L 89/06;**
**A61L 27/52, C08L 71/02;**
**A61L 27/52, C08L 89/06**

**Description**

**CROSS-REFERENCE TO RELATED APPLICATIONS**

This application claims the benefit of priority to U.S. provisional applications 63/231,138 (filed August 9, 2021). This application is incorporated herein by reference in its entirety.

**FIELD OF THE INVENTION**

The described invention relates generally to methods for treating ectatic corneal diseases and methods for treating vision deficits in ectatic corneal diseases.

**BACKGROUND OF THE INVENTION**

**Parts of the Eye**

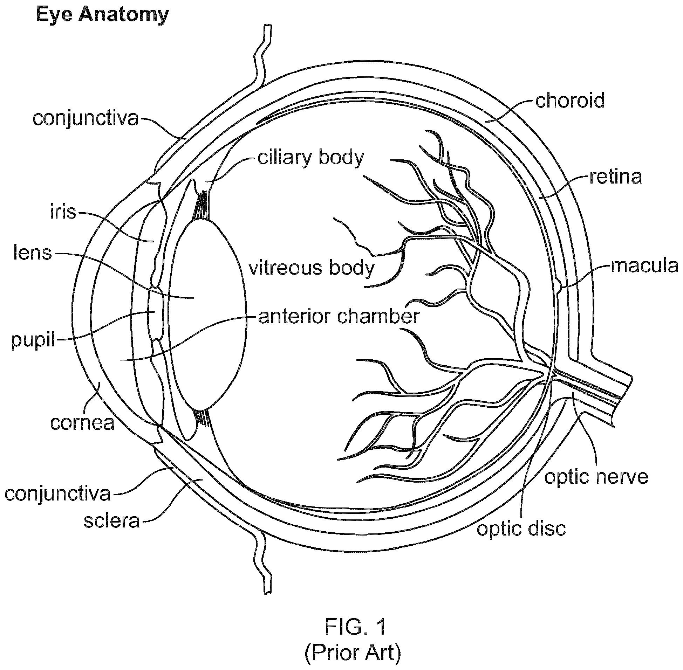

The eye is an organ which reacts to light and pressure and allows the sense of vision. **FIG. 1** is an illustration of a human eye. Allaboutvision.com/resources/anatomy.htm, Accessed March 2019). The anatomy of the eye includes a conjunctiva, an iris, a lens, a pupil, a cornea, a sclera, a ciliary body, a vitreous body, an anterior chamber, a choroid, a retina, a macula, an optic nerve, and an optic disc. The conjunctiva is a clear, thin membrane that covers part of the front surface of the eye and the inner surface of the eyelids. The iris is a thin, circular structure made of connective tissue and muscle that surrounds the pupil and regulates the amount of light that strikes the retina. The retina is a light-sensitive membrane on which light rays are focused. It is composed of several layers, including one that contains specialized cells called photoreceptors. Photoreceptor cells take light focused by the cornea and lens (a transparent, biconvex structure) and convert it into chemical and nervous signals which are transported to visual centers in the brain by way of the optic nerve. The sclera is a dense connective tissue that surrounds the cornea and forms the white part of the eye. The ciliary body connects the iris to the choroid and consists of ciliary muscle (which alters the curvature of the lens), a series of radial ciliary processes (from which the lens is suspended by ligaments), and the ciliary ring (which adjoins the choroid). The choroid is the pigmented vascular layer of the eye between the retina and the sclera. The vitreous body is a transparent, colorless, semisolid mass composed of collagen fibrils and hyaluronic acid that fills the posterior cavity of the eye between the lens and the retina. The anterior chamber is an aqueous humor-filled space inside the eye between the iris and the cornea's innermost surface. The macula is an oval-shaped pigmented area near the center of the retina. The optic disc is the raised disk on the retina at the point of entry of the optic nerve, which lacks visual receptors, thus creating a blind spot.

**The Cornea**

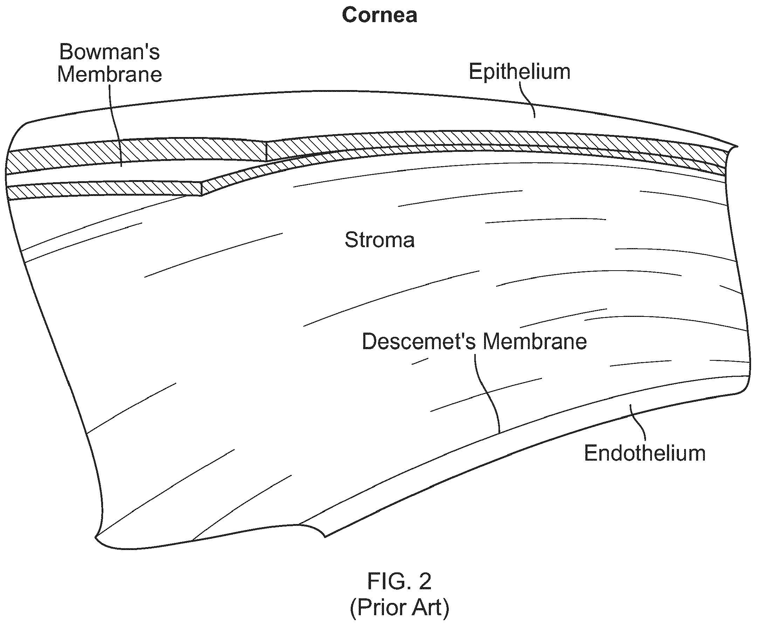

The cornea is a clear and transparent layer anterior on the eye. It is the eye's main refracting surface. **FIG. 2** is an illustration showing the cornea, which is avascular and exhibits the following five layers, from the anterior (nearer to the front) to posterior (nearer to the rear) direction: the epithelium, Bowman's layer, stroma, Descemet's membrane, and the endothelium.

The epithelium is a layer of cells that can be thought of as covering the surface of the cornea. Specifically, the cornea is covered externally by a stratified nonkeratinizing epithelium (5-6 layers of cells, about 50 microns in thickness) with three types of cells: superficial cells, wing cells and basal cells (deepest cell layer). Desmosomes form tight junctions in between the superficial cells. The basal cells are the only corneal epithelial cells capable of mitosis; the basement membrane of epithelial cells is 40-60 nm in thickness and is made up of type IV collagen and laminin secreted by basal cells. The epithelial layer is highly sensitive due to numerous nerve endings and has excellent regenerative power. There are differences between epithelium of central and peripheral cornea. In the central cornea, the epithelium has 5-7 layers, the basal cells are columnar; there are no melanocytes or Langerhans cells, and the epithelium is uniform to provide a smooth regular surface. In the peripheral cornea, the epithelium is 7-10 layered, the basal cells are cuboidal, there are melanocytes and Langerhans cells, and there are undulating extensions of the basal layer. (Sridhar, M.S., "Anatomy of cornea and ocular surface," Indian J. Ophthalmol. (2018) 66(2): 190-194).

Bowman membrane is structureless and acellular.

The stroma is the thickest layer of the cornea and gives the cornea much of its strength. Most refractive surgeries involve manipulating stroma cells. Specifically, the substantia propria (stroma) forms 90% of the cornea's thickness and is made up of keratocytes and extracellular matrix. Fibrils of the stroma crisscross at 90° angles; these fibrils are of types I, III, V, and VII collagen.

Descemet's membrane and the endothelium are considered the posterior portion of the cornea. Descemet membrane is structureless, homogeneous, and measures 3-12 microns; it is composed of the anterior banded zone and the posterior nonbanded zone; the Descemet membrane is rich in type IV collagen fibers.

The cornea is covered internally by the corneal endothelium, a single layer, 5 microns thick, of simple cuboidal and hexagonal cells with multiple orthogonal arrays of collagen in between. The endothelium is derived from the neural crest and functions to transport fluid from the anterior chamber to the stroma. Because the cornea is avascular, its nutrients are derived mainly from diffusion from the endothelium layer. (Duong, H-V. Q. "Eye Globe Anatomy," https://emedicine.medscape.com/article/1923010-0verview,updated Nov. 9, 2017). It is normally avascular due to the high concentration of soluble VEGFR-1, and is surrounded by a transitional margin, the corneal limbus, within which resides nascent endothelium and corneal epithelial stem cells. (Id.)

## Zones of the Cornea

The shape of the cornea is aspheric, meaning that it departs slightly from the spherical form. Typically, the central cornea is about 3D steeper than the periphery. (http://www.aao.org/bcsdsnippetdetail.aspx?id=65c7bff9-4fle-47'7-8585-40318390fc7c , visited 3/12/19)

Clinically, the cornea is divided into zones that surround fixation and blend into one another. The central zone of 1-2 mm closely fits a spherical surface. Adjacent to the central zone is the paracentral zone, a 3-4 mm doughnut with an outer diameter of 7-8 mm that represents an area of progressive flattening from the center. Together, the paracentral and central zones constitute the apical zone. The central and paracentral zones are primarily responsible for the refractive power of the cornea. Adjacent to the paracentral zone is the peripheral zone, with an outer diameter of approximately 11 mm. The peripheral zone is also known as the transitional zone, as it is the area of greatest flattening and asphericity of the normal cornea. Adjoining the peripheral zone is the limbus, with an outer diameter that averages 12 mm. (Id.)

The optical zone is the portion of the cornea that overlies the entrance pupil of the iris; it is physiologically limited to approximately 5.4 mm because of the Stiles-Crawford effect (the reduction of the brightness when a light beam's entry into the eye is shifted from the center to the edge of the pupil has from the outset been shown to be due to a change in luminous efficiency of radiation when it is incident obliquely on the retina. (See G. Westheimer, "Directional sensitivity of the retina: 75 years of Stiles-Crawford effect," Proc. R. Soc. B. (2008) 275 (1653): 2777-86).

The corneal apex is the point of maximum curvature. The corneal vertex is the point located at the intersection of a subject's line of fixation and the corneal surface..

The cornea must be clear, smooth and healthy for good vision. If it is scarred, swollen, or damaged, light is not focused properly into the eye.

## Corneal Wound Healing

The term "wound healing" refers to the process by which the body repairs trauma to any of its tissues, especially those caused by physical means and with interruption of continuity.

A wound-healing response often is described as having three distinct phases-injury, inflammation and repair. Injury often results in the disruption of normal tissue architecture, initiating a healing response. Generally speaking, the body responds to injury with an inflammatory response, which is crucial to maintaining the health and integrity of an organism. The closing phase of wound healing consists of an orchestrated cellular reorganization guided by a fibrin (a fibrous protein that is polymerized to form a "mesh" that forms a clot over a wound site)-rich scaffold formation, wound contraction, closure and re-epithelialization.

The response of the anterior segment of the eye to wound healing closely resembles the response of non-CNS tissues. (Friedlander, M. "Fibrosis and diseases of the eye," J. Clin. Invest. (2007) 117(3): 576-86).

The healing of corneal epithelial wounds involves a number of concerted events, including cell migration, proliferation, adhesion and differentiation, with cell layer stratification.

In brief, corneal epithelial healing largely depends on limbal epithelial stem cells (LESCs) stem cells, which, in many species, including humans, exclusively reside in the corneoscleral junction, and remodeling of the basement membrane. (Ljubimov , AV and Saghizadeh, M., "Progress in corneal wound healing," Prog. Retin. Eye Res. (2015) 49: 17-45). In response to injury, LESCs undergo few cycles of proliferation and give rise to many transit-amplifying cells (TACS), which appear to make up most of the basal epithelium in the limbus and peripheral cornea. (Id.) The LESCs are thought to migrate into the central cornea, proliferate rapidly afterwards, and eventually terminally differentiate into central corneal epithelial cells. (Id.) During stromal healing, keratocytes get transformed to motile and contractile myofibroblasts largely due to activation of the transforming growth factor $\beta$ system. (Id.) Endothelial cells heal mostly by migration and spreading, with cell proliferation playing a secondary role. (Id.)

**Epithelial wound healing**

The kinetics of epithelial wound healing includes two distinct phases: an initial latent phase and a closure phase. The initial latent phase includes cellular and subcellular reorganization to trigger migration of the epithelial cells at the wound edge. (Id., citing Kuwabara T, et al., Sliding of the epithelium in experimental corneal wounds. Invest. Ophthalmol. (1976) 15: 4-14; Crosson, CE et al., Epithelial wound closure in the rabbit cornea. A biphasic process. Invest. Ophthalmol. Vis. Sci. (1986) 27: 464-473). The closure phase includes several continuous processes starting with cell migration, which is independent of cell mitosis. (Id, citing Anderson, SC, et al., Rho and Rho-kinase (ROCK) signaling in adherens and gap junction assembly in corneal epithelium. Invest. Ophthalmol. Vis. Sci. (2002) 43: 978-986), followed by cell proliferation and differentiation, and eventually, by stratification to restore the original multicellular epithelial layer (Id., citing Crosson, CE et al., Epithelial wound closure in the rabbit cornea. A biphasic process. Invest. Ophthalmol. Vis. Sci. (1986) 27: 464-473)).

**Wound healing factors in epithelial wound healing**

When corneal epithelium is injured, nucleotides and neuronal factors are released to the extracellular milieu, generating a Ca(2+) wave from the origin of the wound to neighboring cells. (Id., citing Lee, A, et al., Hypoxia-induced changes in Ca2+ mobilization and protein phosphorylation implicated in impaired wound healing. Am. J. Physiol. Cell. Physiol. (2014) 306: C972-985). The release of ATP induced within one minute after injury results in mobilization of intracellular calcium upon activation of purinergic receptors P2Y or P2X (Id., citing Weinger, I et al Tri-nucleotide receptors play a critical role in epithelial cell wound repair. Purinerg. Signal. (2005) 1: 281-292; Hypoxia-induced changes in Ca2+ mobilization and protein phosphorylation implicated in impaired wound healing. Am. J. Physiol. Cell. Physiol. (2014) 306: C972-985). This activation appears to be one of the earliest events in the healing process. (Id., citing Lee, A. et al, Hypoxia-induced changes in Ca2+ mobilization and protein phosphorylation implicated in impaired wound healing. Am. J. Physiol. Cell. Physiol. (2014) 306: C972-985). Most recent data show that the effects of P2X7 on wound healing may be mediated by a rearrangement of the actin cytoskeleton enabling epithelial cells to better migrate (Id.).

Toll-like receptors (TLRs) also contribute to early corneal epithelial wound healing by enhancing cell migration and proliferation in vitro and in vivo (Id., citing Eslani, M et al, The role of toll-like receptor 4 in corneal epithelial wound healing. Invest. Ophthalmol. Vis. Sci. (2014) 55: 6108-6115). TLRs are a family of proteins that play a major role in the innate immune system and modulate inflammation via several pathways, such as nuclear factor κB (NF-κB), MAP kinases, and activator protein (AP)-1. (Id., citing Pearlman, E et al., Toll-like receptors at the ocular surface. Ocul. Surf. (2008) 6: 108-116; Kostarnoy, AV et al., Topical bacterial lipopolysaccharide application affects inflammatory response and promotes wound healing. J. Interferon Cytokine Res. (2013) 33: 514-522). The TLR signaling pathway is activated in response to its ligands, such as pathogen associated molecular patterns ("PAMPs", for viruses and bacteria) and damage-associated molecular patterns (DAMPs) as a result of tissue injury. This leads to production of proinflammatory cytokines, adhesion molecules and proteolytic enzymes during the inflammatory stage of wound healing. (Id., citing Pearlman, E et al., Toll-like receptors at the ocular surface. Ocul. Surf. (2008) 6: 108-116; Kostarnoy, AV et al., Topical bacterial lipopolysaccharide application affects inflammatory response and promotes wound healing. J. Interferon Cytokine Res. (2013) 33: 514-522) as well as to enhanced cell migration and proliferation.

In the initial or lag phase of wound healing, several parallel signaling pathways, which may cross-talk, are activated to reorganize cellular and subcellular structures initiating cell migration, the first step of the healing process. These initial factors include IL-1 and TNF-α (Id., citing Wilson SE, et al. Stromal-epithelial interactions in the cornea. Prog. Retin. Eye Res. (1999) 18: 293-309), EGF and PDGF (Id., citing Tuominen, IS et al, Human tear fluid PDGF-BB, TNF-α and TGF-β1 vs corneal haze and regeneration of corneal epithelium and subbasal nerve plexus after PRK. Exp. Eye Res. (2001) 72: 631-641), which trigger a series of responses leading to epithelial cell migration through ERK, MAP kinases, and/or NF-κB pathways. Additionally, a number of transcription factors, such c-fos, c-jun, jun-B, and fos-B, which become activated during the lag phase of wound healing before the cells start to migrate (Id., citing Oakdale, Y, Expression of fos family and jun family proto-oncogenes during corneal epithelial wound healing. Curr. Eye Res. (1996) 15: 824-832), can also lead to activation of other parallel pathways in underlying stroma, including IL-1 mediated keratocyte apoptosis via FAS/Fas ligand (Id., citing Wilson SE, et al., Stromal-epithelial interactions in the cornea. Prog. Retin. Eye Res. (1999) 18: 293-309), which leads to consecutive pathways of pro-inflammatory cascades in the first 24 hr following injury (Id., citing Wilson SE, et al., The corneal wound healing response: cytokine mediated interaction of the epithelium, stroma, and inflammatory cells. Prog. Retin. Eye Res. (2001) 20: 625-637). EGFR transactivation has been shown to enhance intracellular signaling in corneal epithelial wound healing in the presence of non-EGF ligands, such as IGF, insulin and HGF, by activating ERK and PI3K/Akt pathways (Id., citing Lyu J, Transactivation of EGFR mediates insulin-stimulated ERK1/2 activation and enhanced cell migration in human corneal epithelial cells. Mol. Vis. (2006) 12: 1403-1410; Spix JK, et al., Hepatocyte growth factor induces epithelial cell motility through transactivation of the epidermal growth factor receptor. Exp. Cell Res. (2007) 313: 3319-3325). Hepatocyte growth factor (HGF) and keratinocyte growth factor (KGF), as well as pigment epithelium-derived factor (PEDF) signaling during wound healing, converges on p38 and/or ERK1/2 pathways; the former mediates cell migration, whereas the latter induces proliferation (Id., citing Sharma GD, He J, Bazan HE. p38 and ERK1/2 coordinate cellular migration and proliferation in epithelial wound healing: evidence of cross-talk activation between MAP kinase cascades. J. Biol. Chem. (2003) 278: 21989-21997; Ho TC, et al., PEDF promotes self-renewal of limbal stem cell and accelerates corneal epithelial wound healing. Stem Cells. (2013) 31: 1775-1784).

Another initial wound healing factor is the release of matrix metalloproteinases (MMPs), which triggers a series of processes to disengage cell-cell and cell-matrix adhesion. This leads to initiation and facilitation of cell migration via cross-talk with integrins and the production of extracellular matrix (ECM) proteins, such as fibronectin, laminin and tenascin, in the wound area that act as a temporary scaffold for migratory cells (Id., citing Tuft SJ, et al., Photorefractive keratectomy: implications of corneal wound healing. Br. J. Ophthalmol. (1993) 77: 243-247). The release of cellular nucleotides (e.g., ATP) upon epithelial injury is also implicated as an initial factor causing rapid activation of purinergic signaling and increase of intracellular Ca2+ levels leading to epidermal growth factor receptor (EGFR) transactivation and cell migration, and, eventually, epithelial wound healing with corneal nerve involvement (Id., citing Weinger I, et al., Tri-nucleotide receptors play a critical role in epithelial cell wound repair. Purinerg. Signal. (2005) 1: 281-292; Boucher I. Injury and nucleotides induce phosphorylation of epidermal growth factor receptor: MMP and HB-EGF dependent pathway. Exp. Eye Res. (2007) 85: 130-141; Yin J, Xu K, Zhang J, Kumar A, Yu FS. Wound-induced ATP release and EGF receptor activation in epithelial cells. J. Cell Sci. (2007) 120: 815-825; Lee A, et al., Hypoxia-induced changes in Ca2+ mobilization and protein phosphorylation implicated in impaired wound healing. Am. J. Physiol. Cell. Physiol. (2014) 306: C972-985). EGFR and purinergic signaling are also involved in the phosphorylation of paxillin, a focal adhesion-associated phosphotyrosine-containing protein that contains a number of motifs that mediate protein-protein interactions (see Schaller, MD, "Paxillin: a focal adhesion associated adaptor protein," Oncogene (2001) 20: 6459-72) needed for cell migration (Id., citing Kimura K, et al., Role of JNK-dependent serine phosphorylation of paxillin in migration of corneal epithelial cells during wound closure. Invest. Ophthalmol. Vis. Sci. (2008) 49: 125-132; Mayo C, et al., Regulation by P2X7: epithelial migration and stromal organization in the cornea. Invest. Ophthalmol. Vis. Sci. (2008) 49: 4384-4391).

Cell migration during wound healing also may involve a cross-talk between growth factors and ECM. Insulin-like growth factor 1 (IGF1) was shown to induce cell migration directly through its receptor, as well as through stimulating the expression of corneal basement membrane component laminin-332, which facilitates epithelial cell migration in vitro (Id., citing Lee JG, Kay EP. FGF-2-induced wound healing in corneal endothelial cells requires Cdc42 activation and Rho inactivation through the phosphatidylinositol 3-kinase pathway. Invest. Ophthalmol. Vis. Sci. (2006) 47: 1376-1386). IGF1 receptor can also be engaged in cross-talk with β1 chain-containing integrins important for corneal epithelial cell migration (Id., citing Seomun Y, Joo CK. Lumican induces human corneal epithelial cell migration and integrin expression via ERK 1/2 signaling. Biochem. Biophys. Res. Commun. (2008) 372: 221-225) through their recruitment to lipid rafts (Id., citing Salani B, et al., IGF-I induced rapid recruitment of integrin β1 to lipid rafts is caveolin-1 dependent. Biochem. Biophys. Res. Commun. (2009) 380: 489-492). Overall, significant cross-talk in corneal wound healing has been revealed between several growth factors through transactivation of signaling pathways, and between growth factors and extracellular mediators of this process. This cross-talk underlines the complex nature of epithelial wound healing.

**ECM in epithelial wound healing**

Corneal epithelium makes its own ECM in the form of a specialized epithelial basement membrane that is positioned between basal epithelial cells and the stroma and apposed to the underlying collagenous Bowman's layer. It provides structural support and regulates, through various receptors, epithelial migration, proliferation, differentiation, adhesion and apoptosis (Id., citing Azar DT, et al., Altered epithelial-basement membrane interactions in diabetic corneas. Arch. Ophthalmol. (1992) 110: 537-40; Kurpakus MA, et al., The role of the basement membrane in differential expression of keratin proteins in epithelial cells. Dev. Biol. (1992) 150: 243-255; Zieske JD, et al., Basement membrane assembly and differentiation of cultured corneal cells: importance of culture environment and endothelial cell interaction. Exp. Cell Res. (1994) 214: 621-633; Ljubimov AV, et al., Extracellular matrix alterations in human corneas with bullous keratopathy. Invest. Ophthalmol. Vis. Sci. (1996) 37: 997-1007; Ljubimov AV, et al., Basement membrane abnormalities in human eyes with diabetic retinopathy. J Histochem Cytochem. (1996) 44: 1469-1479; Suzuki K, et al., Cell-matrix and cell-cell interactions during corneal epithelial wound healing. Prog. Retin. Eye Res. (2003) 22: 113-133). Corneal epithelial basement membrane is composed of specialized networks of type IV collagens, laminins, nidogens and perlecan, as are most basement membranes (Id., citing Nakayasu K, et al., Distribution of types I, II, III, IV and V collagen in normal and keratoconus corneas. Ophthalmic Res. (1986) 18: 1-10; Martin GR, Timpl R. Laminin and other basement membrane components. Annu. Rev. Cell Biol. (1987) 3: 57-85; Ljubimov AV, et al., Human corneal basement membrane heterogeneity: topographical differences in the expression of type IV collagen and laminin isoforms. Lab Invest. (1995) 72: 461-473; Ljubimov AV, et al., Extracellular matrix alterations in human corneas with bullous keratopathy. Invest. Ophthalmol. Vis. Sci. (1996) 37: 997-1007; Tuori A, et al., The immunohistochemical composition of the human corneal basement membrane. Cornea. (1996) 15: 286-294; Kabosova A, et al., Human diabetic corneas preserve wound healing, basement membrane, integrin and MMP-10 differences from normal corneas in organ culture. Exp. Eye Res. (2003) 77: 211-217; Schlötzer-Schrehardt U, et al., Characterization of extracellular matrix components in the limbal epithelial stem cell compartment. Exp. Eye Res. (2007) 85: 845-60) with additional components, such as TSP-1, matrilin-2, matrilin-4, types CV, XCII and XCIII collagen and fibronectin (FN) (Id., citing Kabosova A, et al., Compositional differences between infant and adult human corneal basement membranes. Invest. Ophthalmol. Vis. Sci. (2007) 48: 4989-4999; Schlötzer-Schrehardt U, et al., Characterization of extracellular matrix components in the limbal epithelial stem cell compartment. Exp. Eye Res. (2007) 85: 845-60; Dietrich-Ntoukas T, et al., Comparative analysis of the basement membrane composition of the human limbus epithelium and amniotic membrane epithelium. Cornea. (2012) 31: 564-569).

**Immune system involvement in epithelial wound healing**

Immune system cells, such as neutrophils, play a major role in corneal epithelial wound healing, which might be due to their ability to release growth factors that impact the epithelium (Li Z, et al., Lymphocyte function-associated antigen-1-dependent inhibition of corneal wound healing. Am. J. Pathol. (2006) 169: 1590-600; Li Z, et al., Platelet response to corneal abrasion is necessary for acute inflammation and efficient re-epithelialization. Invest. Ophthalmol. Vis. Sci. (2006) 47: 4794-4802).

The major function of corneal epithelium is to protect the eye interior by serving as a physical and chemical barrier against infection by tight junctions and sustaining the integrity and visual clarity of cornea. (Id). Wounded, damaged or infected epithelial cells secret the cytokine, IL-1$\alpha$, which is stored in epithelial cells and released when the cell membrane is damaged by external insults. (Id). Secreted IL-1$\alpha$ can cause increased immune infiltration of the cornea leading to neovascularization, which may result in visual loss. (Id). However, IL-1RN, an IL-1$\alpha$ antagonist, prevents leucocyte invasion of the cornea and suppresses neovascularization, which may help preserve vision (Id., citing Stapleton WM, et al., Topical interleukin-1 receptor antagonist inhibits inflammatory cell infiltration into the cornea. Exp. Eye Res. (2008) 86: 753-757). In animal models, corneal epithelial wounding prompts an acute inflammatory response in the limbal blood vessels leading to accumulation of leukocytes and neutrophils (Id., citing Li SD, Huang L. Non-viral is superior to viral gene delivery. J. Control Release. (2007) 123: 181-183; Yamagami S, et al., CCR5 chemokine receptor mediates recruitment of MHC class II-positive Langerhans cells in the mouse corneal epithelium. Invest. Ophthalmol. Vis. Sci. (2005) 46: 1201-1207), and migration of dendritic cells, macrophages and lymphocytes (Id., citing Jin Y, et al., The chemokine receptor CCR7 mediates corneal antigen-presenting cell trafficking. Mol. Vis. (2007) 13: 626-634; Li SD, Huang L. Non-viral is superior to viral gene delivery. J. Control Release. (2007) 123: 181-183; Gao N, et al., Dendritic cell-epithelium interplay is a determinant factor for corneal epithelial wound repair. Am. J. Pathol. (2011) 179: 2243-53) into the stroma and the wounded epithelium. Current evidence indicates that the innate inflammatory responses are necessary for corneal epithelial wound healing and nerve recovery (Id., citing Li Z, et al., Lymphocyte function-associated antigen-1-dependent inhibition of corneal wound healing. Am. J. Pathol. (2006) 169: 1590-600; Li SD, Huang L. Non-viral is superior to viral gene delivery. J. Control Release. (2007) 123: 181-183; 2011; Gao N, et al., Dendritic cell-epithelium interplay is a determinant factor for corneal epithelial wound repair. Am. J. Pathol. (2011) 179: 2243-53). Platelets also accumulate in the limbus and migrate to the stroma in response to wounded epithelium, which is necessary for efficient re-epithelialization through cell adhesion molecules such as P-selectin (Id., citing Li Z, et al., Platelet response to corneal abrasion is necessary for acute inflammation and efficient re-epithelialization. Invest. Ophthalmol. Vis. Sci. (2006) 47: 4794-4802; Lam FW, et al., Platelets enhance neutrophil transendothelial migration via P-selectin glycoprotein ligand-1. Am. J Physiol. Heart Circ. Physiol. (2011) 300: H468-H475).

**Epithelial Basement Membrane (BM) in Corneal Wound Healing**

Several studies have demonstrated the importance of epithelial BM in corneal wound healing. (Torricelli, AAM et al, The Corneal Epithelial Basement Membrane: Structure, Function and Disease, Invest. Ophthalmol. Vis. Sci. (2013) 54: 6390-6400, citing Fujikawa LS, et al., Basement membrane components in healing rabbit corneal epithelial wounds: immunofluorescence and ultrastructural studies. J Cell Biol. (1984) 98: 128-138; Sta Iglesia DD, Stepp MA. Disruption of the basement membrane after corneal debridement. Invest Ophthalmol Vis Sci. (2000) 41: 1045-1053; Chi C, Trinkaus-Randall V. New insights in wound response and repair of epithelium. J Cell Physiol. (2013) 228: 925-929; Pal-Ghosh S, et al., Removal of the basement membrane enhances corneal wound healing. Exp Eye Res. (2011) 93: 927-936). For example, Pal-Ghosh and coworkers demonstrated that removal of the epithelial BM enhances many wound healing processes in the cornea, including keratocyte apoptosis and nerve death. (Id., citing Pal-Ghosh S, et al, Removal of the basement membrane enhances corneal wound healing. Exp Eye Res. (2011) 93: 927-936). Corneal surgery, injury, or infection frequently triggers the appearance of stromal myofibroblasts associated with persistent corneal opacity (haze). (Torricelli AA, et al., Transmission electron microscopy analysis of epithelial basement membrane repair in rabbit corneas with haze. Invest Ophthalmol Vis Sci. (2013) 54: 4026-4033) The opacity develops as a result of diminished transparency of the cells themselves and the production of disordered extracellular matrix components by stromal cells. (Id., citing Jester JV, et al., Transforming growth factor (beta)-mediated corneal myofibroblast differentiation requires actin and fibronectin assembly. Invest Ophthalmol Vis Sci. (1999) 40: 1959-1967; Masur SK, et al., Myofibroblasts differentiate from fibroblasts when plated at low density. Proc Natl Acad Sci U S A. (1996) 93: 4219-4223; Wilson SE, et al., Stromal-epithelial interactions in the cornea. Prog Retin Eye Res. (1999) 18: 293-309). Singh et al. reported that the normally functioning epithelial BM critically modulates myofibroblast development through its barrier function preventing penetration of epithelial TGF-β1 and platelet-derived growth factor (PDGF) into the stroma at sufficient levels to drive myofibroblast development and maintain viability once mature myofibroblasts are generated. (Id., citing Singh V, et al., Stromal fibroblast-bone marrow-derived cell interactions: implications for myofibroblast development in the cornea. Exp Eye Res. (2012) 98: 1-8). This hypothesis holds that stromal surface irregularity after photorefractive keratectomy (PRK) or other cornea injury leads to structural and functional defects in the regenerated epithelial BM, which increases and prolongs penetration of epithelial TGF-β1 and PDGF into the anterior corneal stroma to promote myofibroblast development from either keratocyte-derived or bone marrow-derived precursor cells. (Id., citing Singh V, et al. Effect of TGFbeta and PDGF-B blockade on corneal myofibroblast development in mice. Exp Eye Res. (2011) 93: 810-817, and Singh, V, Wilson, SE, unpublished data, 2013).

The working hypothesis was that prominent mature myofibroblast generation and resulting disorganized extracellular matrix excretion in the anterior stroma of corneas with significant injury interfere with keratocyte contribution of critical components to the BM (collagen type VII, for example) that results in defective epithelial BM regeneration. (Id). Only when the epithelial BM is finally regenerated, which may take years in some corneas with haze, and epithelium-derived TGF-β1 levels fall, do myofibroblasts undergo apoptosis and keratocytes reabsorb disorganized extracellular matrix and thereby restore transparency. (Id., citing Singh V, et al., Stromal fibroblast-bone marrow-derived cell interactions: implications for myofibroblast development in the cornea. Exp Eye Res. (2012) 98: 1-8; Fini ME, Stramer BM. How the cornea heals: cornea-specific repair mechanisms affecting surgical outcomes. Cornea. (2005) 24: S2-S11; Stramer BM, et al., Molecular mechanisms controlling the fibrotic repair phenotype in cornea: implications for surgical outcomes. Invest Ophthalmol Vis Sci. (2003) 44: 4237-4246). Thus, the epithelial BM likely functions as a corneal regulatory structure that limits the fibrotic response in the stroma by modulating the availability of epithelium-derived TGF-β1, PDGF, and perhaps other growth factors and extracellular matrix components, to stromal cells, including myofibroblast precursors. (Id). It may also regulate levels of stromal cell-produced epithelial modulators of motility, proliferation, and differentiation like keratinocyte growth factor (KGF) that transition through the BM in the opposite direction. (Id., citing Wilson SE, et al., Hepatocyte growth factor, keratinocyte growth factor, their receptors, fibroblast growth factor receptor-2, and the cells of the cornea. Invest Ophthalmol Vis Sci. (1993) 34: 2544-2561; Wilson SE, et al. Effect of epidermal growth factor, hepatocyte growth factor, and keratinocyte growth factor, on proliferation, motility and differentiation of human corneal epithelial cells. Exp Eye Res. (1994) 59: 665-678). Thus, corneal epithelial BM may modulate epithelial-to-stroma and stroma-to-epithelial interactions by regulating cytokines and growth factor movement from one cell layer to the other. (Id).

Latvala et al. observed that the distribution of α6 and β4 integrins adjacent to the BM changes during epithelial wound healing after epithelial abrasion in the rabbit cornea. (Id., citing Latvala T, et al, Distribution of alpha 6 and beta 4 integrins following epithelial abrasion in the rabbit cornea. Acta Ophthalmol Scand. (1996) 74: 21-25). Stepp et al. have demonstrated that the re-epithelialization of small wounds is accompanied by increased α6β4 integrin. (Id., citing Stepp MA, et al., Changes in beta 4 integrin expression and localization in vivo in response to corneal epithelial injury. Invest Ophthalmol Vis Sci. (1996) 37: 1593-1601). Epithelial cell migration is also affected by the distribution of laminin and collagen IV during corneal wound healing and BM regeneration. (Id., citing Fujikawa LS, et al., Basement membrane components in healing rabbit corneal epithelial wounds: immunofluorescence and ultrastructural studies. J Cell Biol. (1984) 98: 128-138.) Thus, α3(IV) and α4(IV) collagen chains may be important for the healthy corneal epithelium. (Id). Upon injury, the BM is remodeled to include α1(IV) and α2(IV) collagen, recapitulating corneal epithelial expression during development. (Id).

**Corneal Stromal Wound Healing**

Stromal remodeling occurs upon direct damage to the stroma and its cells (as exemplified by photorefractive keratectomy (PRK) and LASIK (used for myopia correction)) and upon death of stromal cells (keratocytes) caused by damage to or removal of corneal epithelium by various physical or chemical factors (Id., citing Nakayasu K. Stromal changes following removal of epithelium in rat cornea. Jpn. J. Ophthalmol. (1988) 32: 113-125; Szerenyi KD, et al., Keratocyte loss and repopulation of anterior corneal stroma after de-epithelialization. Arch. Ophthalmol. (1994) 112: 973-976; Wilson SE, et al., Epithelial injury induces keratocyte apoptosis: hypothesized role for the interleukin-1 system in the modulation of corneal tissue organization and wound healing. Exp. Eye Res. (1996) 62:325-327; Wilson SE, et al, The corneal wound healing response: cytokine mediated interaction of the epithelium, stroma, and inflammatory cells.

Prog. Retin. Eye Res. (2001) 20: 625-637; Wilson SE, et al., Apoptosis in the initiation, modulation and termination of the corneal wound healing response. Exp. Eye Res. (2007) 85: 305-311). Such damage triggers a release of inflammatory cytokines from epithelial cells and/or tears (Id., citing Maycock NJ, Marshall J. Genomics of corneal wound healing: a review of the literature. Acta Ophthalmol. (2014) 92: e170-84), mainly IL-1 ($\alpha$ and $\beta$) that cause rapid apoptosis through Fas/Fas ligand system and later, necrosis of mainly anterior keratocytes. These cells die preferentially directly beneath the epithelial wounds, rather than also beyond their edges. The following stromal remodeling with replenishment of these cells from the areas adjacent to the depleted one (Id., citing Zieske JD, et al., Activation of epidermal growth factor receptor during corneal epithelial migration. Invest. Ophthalmol. Vis. Sci. (2000) 41: 1346-1355) also constitutes a wound healing process and may result in fibrotic changes, especially if the epithelial basement membrane was initially damaged (Id., citing Stramer BM, et al., Molecular mechanisms controlling the fibrotic repair phenotype in cornea: implications for surgical outcomes. Invest. Ophthalmol. Vis. Sci. (2003) 44:4237-4246; Fini ME, Stramer BM. How the cornea heals: cornea-specific repair mechanisms affecting surgical outcomes. Cornea. (2005) 24(Suppl 1): S2-S11; West-Mays JA, Dwivedi DJ. The keratocyte: corneal stromal cell with variable repair phenotypes. Int. J. Biochem. Cell Biol. (2006) 38: 1625-1631. This is a classic example of how stromal-epithelial interactions influence wound healing process by paracrine mediators. Id.

At the early stage of wound repair, quiescent keratocytes at the wound edges change their properties to become activated to fibroblasts. These cells enter the cell cycle and acquire migratory properties necessary to repopulate and close the wound (Id., citing West-Mays JA, Dwivedi DJ. The keratocyte: corneal stromal cell with variable repair phenotypes. Int. J. Biochem. Cell Biol. (2006) 38: 1625-1631). In culture, this transformation is mediated by some (fibroblast growth factor 2 (FGF-2) and PDGF-AB, TGF-$\beta$) growth factors, whereas others (IL-1, IGF-1) only confer mitogenic activity (Id., citing Jester JV, Ho-Chang J. Modulation of cultured corneal keratocyte phenotype by growth factors/cytokines control in vitro contractility and extracellular matrix contraction. Exp. Eye Res. (2003) 77: 581-592; Chen J, et al., Rho-mediated regulation of TGF-$\beta$1- and FGF-2-induced activation of corneal stromal keratocytes. Invest. Ophthalmol. Vis. Sci. (2009) 50: 3662-3670). These cells remodel their actin cytoskeleton to acquire stress fibers and change their morphology from stellate to an elongated one (Id., citing Jester JV, Ho-Chang J. Modulation of cultured corneal keratocyte phenotype by growth factors/cytokines control in vitro contractility and extracellular matrix contraction. Exp. Eye Res. (2003) 77: 581-592). Fibroblasts downregulate the expression of differentiated keratocyte proteins, such as corneal crystallins (transketolase and aldehyde dehydrogenase 1A1), and keratan sulfate proteoglycans, and start producing proteinases (mostly MMPs) needed to remodel the wound ECM (Id., citing Fini ME. Keratocyte and fibroblast phenotypes in the repairing cornea. Prog. Retin. Eye Res. (1999) 18: 529-551; Jester JV, et al., Corneal stromal wound healing in refractive surgery: the role of myofibroblasts. Prog. Retin. Eye Res. (1999) 18: 311-356; Carlson EC, et al., Altered KSPG expression by keratocytes following corneal injury. Mol. Vis. (2003) 9: 615-623; West-Mays JA, Dwivedi DJ. The keratocyte: corneal stromal cell with variable repair phenotypes. Int. J. Biochem. Cell Biol. (2006) 38: 1625-1631).

After they reach the wound bed, fibroblasts start expressing $\alpha$-smooth muscle actin (a-SMA) and desmin, upregulate the expression of vimentin (Id., citing Chaurasia SS, et al., "Dynamics of the expression of intermediate filaments vimentin and desmin during myofibroblast differentiation after corneal injury" Exp. Eye Res. (2009) 89: 590-59), and become highly motile and contractile myofibroblasts needed to remodel wound ECM and contract the wound. They also deposit provisional ECM rich in fibronectin and some other proteins including tenascin-C and type III collagen (Id., citing Tervo K, et al., Expression of tenascin and cellular fibronectin in the rabbit cornea after anterior keratectomy. Immunohistochemical study of wound healing dynamics. Invest. Ophthalmol. Vis. Sci. (1991) 32: 2912-2918; Fini ME. Keratocyte and fibroblast phenotypes in the repairing cornea. Prog. Retin. Eye Res. (1999) 18: 529-551). Myofibroblasts generate contractile forces to close the wound gap, and the expression of $\alpha$-SMA directly correlates with corneal wound contraction (Id., citing Jester JV, et al., Expression of alpha-smooth muscle (a-SM) actin during corneal stromal wound healing. Invest. Ophthalmol. Vis. Sci. (1995) 36: 809-819). When the wound does not really contract as in the case of PRK or phototherapeutic keratectomy (PTK), the appearance of myofibroblasts is delayed, and they start accumulating as late as four weeks after irregular PTK (Id., citing Barbosa FL, et al., Corneal myofibroblast generation from bone marrow-derived cells. Exp. Eye Res. (2010) 91:92-96).

It is generally accepted that myofibroblast transformation is triggered by transforming growth factor beta (TGF-$\beta$) in vivo, which has been confirmed by numerous studies in vitro (Id., citing Jester JV, et al., Corneal stromal wound healing in refractive surgery: the role of myofibroblasts. Prog. Retin. Eye Res. (1999) 18: 311-356). More recent work has also implicated a potent mitogen, PDGF (both AA and BB) in this process, with the combination of TGF-$\beta$ and PDGF being more potent than either factor alone (Id., citing Kaur H, et al., Corneal stroma PDGF blockade and myofibroblast development. Exp Eye Res. (2009) 88: 960-965; Singh V, et al., Transforming growth factor $\beta$ and platelet-derived growth factor modulation of myofibroblast development from corneal fibroblasts in vitro. Exp. Eye Res. (2014) 120: 152-160). Only TGF-$\beta$1 and TGF-$\beta$2 are active in this process, since TGF-$\beta$2 does not transform fibroblasts to myofibroblasts (Id., citing Karamichos D, et al., Reversal of fibrosis by TGF-$\beta$2 in a 3D in vitro model. Exp. Eye Res. (2014) 124: 31-36). Upon completion of wound healing, myofibroblasts apparently cease to express $\alpha$-SMA. Their fate in vivo is not completely clear, Although myofibroblast appearance is widely considered as a necessary part of stromal wound healing, the numbers of myofibroblasts in the corneal stroma after PRK differ widely among various mouse strains (Id., citing Singh V, et al., Mouse strain variation in SMA(+) myofibroblast development after corneal injury. Exp. Eye Res. (2013) 115: 27-30). Some data indicate that repopulation of keratocytes after epithelial debridement of mouse corneas occurs without the appearance of myofibroblasts (Id., citing Matsuba M, et al., Localization of thrombospondin-1 and myofibroblasts during corneal wound repair. Exp. Eye Res. (2011) 93: 534-540), possibly through stimulation of keratocyte migration by aquaporin-1 water channel (Id., citing Ruiz-Ederra J, Verkman AS. Aquaporin-1-facilitated keratocyte migration in cell culture and in vivo corneal wound healing models. Exp. Eye Res. (2009) 89: 159-165).

**Immune cells in stromal healing**

Corneal injury in animal models entails an inflammatory response by immune system cells, including monocytes/macrophages, T cells, polymorphonuclear (PMN) leukocytes and natural killer (NK) cells (Id., citing Gan L, et al., Effect of leukocytes on corneal cellular proliferation and wound healing. Invest. Ophthalmol. Vis. Sci. (1999) 40: 575-581; Wilson SE, et al., The corneal wound healing response: cytokine mediated interaction of the epithelium, stroma, and inflammatory cells. Prog. Retin. Eye Res. (2001) 20: 625-637; Wilson SE, et al., RANK, RANKL, OPG, and M-CSF expression in stromal cells during corneal wound healing. Invest. Ophthalmol. Vis. Sci. (2004) 45: 2201-2211; Liu Q, et al., NK Cells Modulate the Inflammatory Response to Corneal Epithelial Abrasion and Thereby Support Wound Healing. J. Pathol. (2012) 181: 452-462; Li S, et al., Macrophage depletion impairs corneal wound healing after autologous transplantation in mice. PLoS One. (2013) 8: e61799). These infiltrating cells are usually defined by staining for CD11b, although in some studies a better characterization of these cells is provided (Id., citing Wilson SE, et al., The corneal wound healing response: cytokine mediated interaction of the epithelium, stroma, and inflammatory cells. Prog. Retin. Eye Res. (2001) 20: 625-637; Liu Q, et al., NK Cells Modulate the Inflammatory Response to Corneal Epithelial Abrasion and Thereby Support Wound Healing. J. Pathol. (2012) 181: 452-462; Li S, et al., Macrophage depletion impairs corneal wound healing after autologous transplantation in mice. PLoS One. (2013) 8: e61799). Immune cells may come to the injured cornea from the limbal area or are mobilized from circulation (Id., citing Wilson SE, et al., The corneal wound healing response: cytokine mediated interaction of the epithelium, stroma, and inflammatory cells. Prog. Retin. Eye Res. (2001) 20: 625-637). A major attracting signal for such cells may be monocyte chemotactic protein-1 (MCP-1), a cytokine, which can be secreted by activated fibroblasts and triggered by IL-1 or TNF-$\alpha$ (Id., citing Wilson SE, et al., The corneal wound healing response: cytokine mediated interaction of the epithelium, stroma, and inflammatory cells. Prog. Retin. Eye Res. (2001) 20: 625-637). Another factor required for neutrophil influx following injury was identified as a stromal proteoglycan lumican (Id., citing Hayashi Y, et al., Lumican is required for neutrophil extravasation following corneal injury and wound healing. J. Cell Sci. (2010) 123: 2987-2995). It is still unclear what are the magnitude, infiltrating cell repertoire and origin, as well as kinetics of the immune response to corneal injury in humans.

Functions of immune cells infiltrating injured corneas may be diverse. They may scavenge remnants of apoptotic keratocytes and protect the cornea from possible infection (Id., citing Wilson SE, et al., The corneal wound healing response: cytokine mediated interaction of the epithelium, stroma, and inflammatory cells. Prog. Retin. Eye Res. (2001) 20: 625-637). Some of these cells may become myofibroblasts (Id., citing Barbosa FL, et al., Corneal myofibroblast generation from bone marrow-derived cells. Exp. Eye Res. (2010) 91: 92-96) and thus participate in wound contraction. Direct involvement of immune cells in the wound healing has been also suggested from recent studies. Blocking PMN entry into cornea by fucoidin (inhibitor of leucocyte adhesion to vascular endothelium) delayed wound healing after PRK in rabbits (Id., citing Gan L, et al., Effect of leukocytes on corneal cellular proliferation and wound healing. Invest. Ophthalmol. Vis. Sci. (1999) 40: 575-581). Functional blocking of NK cells after epithelial abrasion and keratocyte loss inhibited healing and nerve regeneration (Id., citing Liu Q, et al., NK Cells Modulate the Inflammatory Response to Corneal Epithelial Abrasion and Thereby Support Wound Healing. J. Pathol. (2012) 181: 452-462). Macrophage depletion impaired wound healing after autologous corneal transplantation, with a decrease in wound myofibroblasts (Id., citing Li S, et al., Macrophage depletion impairs corneal wound healing after autologous transplantation in mice. PLoS One. (2013) 8: e61799). These studies emphasize the importance of local and systemic immunity in corneal wound healing, both epithelial and stromal.

**Remodeling of stromal ECM during wound healing**

As described above, stromal wound healing is accompanied by several events that may be responsible for ECM changes in this location: death of keratocytes, secretion of proinflammatory and profibrotic cytokines including IL-1, TNF-$\alpha$, and MCP-1, transient appearance of cells that do not normally form the stroma (PMNs, macrophages, myofibroblasts), and production of ECM-degrading enzymes by activated cells. All these factors contribute to ECM remodeling including its degradation, expression of ectopic components (provisional matrix formation by new cell types), and reassembly of the new ECM to form a more or less normal structure (Id., citing Zieske JD, et al., Kinetics of keratocyte proliferation in response to epithelial debridement. Exp. Eye Res. (2001) 72: 33-39; Torricelli AA, Wilson SE. Cellular and extracellular matrix modulation of corneal stromal opacity. Exp. Eye Res. (2014) 129: 151-160). As a result, new ECM formed during wound healing often accumulates aberrant proteins, both in composition and structure. Over time, these proteins may form local scars persisting for a long time (Id., citing Ishizaki M, et al. Expression of collagen I, smooth muscle alpha-actin, and vimentin during the healing of alkali-burned and lacerated corneas. Invest. Ophthalmol. Vis. Sci. (1993) 34: 3320-3328; Ishizaki M, et al., Stromal fibroblasts are associated with collagen IV in scar tissues of alkali-burned and lacerated corneas. Curr. Eye Res. (1997) 16: 339-348; Maguen E, et al., Alterations of corneal extracellular matrix after multiple refractive procedures: a clinical and immunohistochemical study. Cornea. (1997) 16: 675-682; Ljubimov AV, et al., Extracellular matrix changes in human corneas after radial keratotomy. Exp. Eye Res. (1998) 67: 265-272; Maguen E, et al., Extracellular matrix and matrix metalloproteinase changes in human corneas after complicated laser-assisted in situ keratomileusis (LASIK) Cornea. (2002) 21: 95-100; Maguen E, et al., Immunohistochemical evaluation of two corneal buttons with post-LASIK keratectasia. Cornea. (2007) 26: 983-991; Kato T, et al., Expression of type XVIII collagen during healing of corneal incisions and keratectomy wounds. Invest. Ophthalmol. Vis. Sci. (2003) 44: 78-85; Kamma-Lorger CS, et al., Collagen ultrastructural changes during stromal wound healing in organ cultured bovine corneas. Exp. Eye Res. (2009) 88: 953-959; Torricelli AA, Wilson SE. Cellular and extracellular matrix modulation of corneal stromal opacity. Exp. Eye Res. (2014) 129: 151-160). Because of slow turnover of ECM proteins, the unusual scar components may still be present around the healed wounds for years, especially in human corneas (Id., citing Latvala T, et al., Expression of cellular fibronectin and tenascin in the rabbit cornea after excimer laser photorefractive keratectomy: a 12 month study. Br. J. Ophthalmol. (1995) 79: 65-69; Maguen E, et al., Extracellular matrix and matrix metalloproteinase changes in human corneas after complicated laser-assisted in situ keratomileusis (LASIK) Cornea. (2002) 21: 95-10; Maguen E, et al., Immunohistochemical evaluation of two corneal buttons with post-LASIK keratectasia. Cornea. (2007) 26: 983-991; Maguen E, et al., Alterations of extracellular matrix components and proteinases in human corneal buttons with INTACS for post-laser in situ keratomileusis keratectasia and keratoconus. Cornea. (2008) 27: 565-573). These components, which are normally scarce in or absent from adult corneal stroma, include type III, VIII, XIV, and XVIII collagen, limbal isoforms of type IV collagen, embryonic fibronectin isoforms, thrombospondin-1 (TSP-1), tenascin-C, fibrillin-1, and hevin (an ECM-associated secreted glycoprotein belonging to the secreted protein acidic and rich in cysteine (SPARC) family of matricellular proteins (Id., citing Saika S, et al., Epithelial basement membrane in alkali-burned corneas in rats. Immunohistochemical study. Cornea. (1993) 12: 383-390; Melles GR, et al., Immunohistochemical analysis of unsutured and sutured corneal wound healing. Curr. Eye Res. (1995) 14: 809-817; Nickeleit V, et al., Healing corneas express embryonic fibronectin isoforms in the epithelium, subepithelial stroma, and endothelium. Am. J. Pathol. (1996) 149: 549-558; Ishizaki M, et al., Stromal fibroblasts are associated with collagen IV in scar tissues of alkali-burned and lacerated corneas. Curr. Eye Res. (1997) 16: 339-348; Maguen E, et al., Alterations of corneal extracellular matrix after multiple refractive procedures: a clinical and immunohistochemical study. Cornea. (1997) 16: 675-682; Ljubimov AV, et al., Extracellular matrix changes in human corneas after radial keratotomy. Exp. Eye Res. (1998) 67: 265-272; Zieske JD, et al., Kinetics of keratocyte proliferation in response to epithelial debridement. Exp. Eye Res. (2001)72:33-39; Maguen E, et al., Extracellular matrix and matrix metalloproteinase changes in human corneas after complicated laser-assisted in situ keratomileusis (LASIK) Cornea. (2002) 21: 95-10; Maguen E, et al., Immunohistochemical evaluation of two corneal buttons with post-LASIK keratectasia. Cornea. (2007) 26: 983-991; Maguen E, et al., Alterations of extracellular matrix components and proteinases in human corneal buttons with INTACS for post-laser in situ keratomileusis keratectasia and keratoconus. Cornea. (2008) 27: 565-573; Kato T, et al., Expression of type XVIII collagen during healing of corneal incisions and keratectomy wounds. Invest. Ophthalmol. Vis. Sci. (2003) 44: 78-85; Javier JA, et al., Basement membrane and collagen deposition after laser subepithelial keratomileusis and photorefractive keratectomy in the leghorn chick eye. Arch. Ophthalmol. (2006) 124: 703-709; Matsuba M, et al., Localization of thrombospondin-1 and myofibroblasts during corneal wound repair. Exp. Eye Res. (2011) 93:534-540; Chaurasia SS, et al., Hevin plays a pivotal role in corneal wound healing. PLoS One. (2013) 8: e81544; Saika S, et al., Wakayama symposium: modulation of wound healing response in the corneal stroma by osteopontin and tenascin-C. Ocul. Surf. (2013) 11:12-15; Sullivan, MN, and Sage ,EH, Hevin/SCI, a matricellular glycoprotein and potential tumor suppressor of the SPARC/BM-40/Osteonectin family. Intl. J. Biochem. Cell Biol. (2004) 38 (6): 991-96).

**Signaling pathways associated with stromal wound healing**

Keratocyte activation to fibroblasts is mediated by FGF-2, TGF-β, and PDGF, and their proliferation by EGF, HGF, KGF, PDGF, IL-1 and IGF-I (Id., Citing Stern ME, et al., Effect of platelet-derived growth factor on rabbit corneal wound healing. Wound Repair Regen. (1995) 3: 59-65; Baldwin HC, Marshall J. Growth factors in corneal wound healing following refractive surgery: A review. Acta. Ophthalmol. Scand. (2002) 80: 238-247; Jester JV, Ho-Chang J. Modulation of cultured corneal keratocyte phenotype by growth factors/cytokines control in vitro contractility and extracellular matrix contraction. Exp. Eye Res. (2003) 77: 581-592; Carrington LM, Boulton M. Hepatocyte growth factor and keratinocyte growth factor regulation of epithelial and stromal corneal wound healing. J. Cataract Refract. Surg. (2005) 31: 412-423; Chen J, et al., Rho-mediated regulation of TGF-β1- and FGF-2-induced activation of corneal stromal keratocytes. Invest.

Ophthalmol. Vis. Sci. (2009) 50: 3662-3670). Although TGF-β is key to fibroblast to myofibroblast transformation, it actually inhibits keratocyte proliferation and migration (Id., citing Baldwin HC, Marshall J. Growth factors in corneal wound healing following refractive surgery: A review. Acta. Ophthalmol. Scand. (2002) 80: 238-247). Stromal cellular infiltration upon injury was found to be stimulated by such cytokines as MCP-1 and platelet-activating factor (PAF) (Id., citing Wilson SE, et al., The corneal wound healing response: cytokine mediated interaction of the epithelium, stroma, and inflammatory cells. Prog. Retin. Eye Res. (2001) 20: 625-637; Kakazu A, et al., Lipoxin A inhibits platelet-activating factor inflammatory response and stimulates corneal wound healing of injuries that compromise the stroma. Exp. Eye Res. (2012) 103:9-16). As noted above, TGF-β isoforms 1 and 2 (Id., citing Torricelli AA, Wilson SE. Cellular and extracellular matrix modulation of corneal stromal opacity. Exp. Eye Res. (2014) 129: 151-160), as well as bone morphogenetic protein 1 (BMP-1), which is capable of inducing formation of cartilage in vivo (Id., citing Malecaze F, et al., Upregulation of bone morphogenetic protein-1/mammalian tolloid and procollagen C-proteinase enhancer-1 in corneal scarring. Invest. Ophthalmol. Vis. Sci. (2014) 55: 6712-6721) may be responsible for myofibroblast emergence, wound contraction and fibrotic scar formation. TGF-β also promotes deposition of excessive ECM in the wound bed that may result in scar formation directly, as well as by stimulating production of other factors including connective tissue growth factor (CTGF) and IGF-I (Id., citing Izumi K, et al., Involvement of insulin-like growth factor-I and insulin-like growth factor binding protein-3 in corneal fibroblasts during corneal wound healing. Invest. Ophthalmol. Vis. Sci. (2006) 47:591-598; Shi L, et al., Activation of JNK signaling mediates connective tissue growth factor expression and scar formation in corneal wound healing. PLoS One. (2012) 7: e32128; Karamichos D, et al., Reversal of fibrosis by TGF-β3 in a 3D in vitro model. Exp. Eye Res. (2014) 124: 31-36; Torricelli AA, Wilson SE. Cellular and extracellular matrix modulation of corneal stromal opacity. Exp. Eye Res. (2014) 129: 151-160). Therefore, attenuation of TGF-β expression and signaling may provide means to counteract fibrotic changes. For instance, topical rosiglitazone, a ligand of peroxisome proliferator activated receptor γ (PPAR-γ) reduced α-SMA expression and scarring in cat corneas upon excimer laser ablation of anterior stroma without compromising wound healing. In corneal fibroblast cultures, it also counteracted TGF-β induced myofibroblast transformation (Id., citing Huxlin KR, et al., Topical rosiglitazone is an effective anti-scarring agent in the cornea. PLoS One. (2013) 8: e70785). Similar effects were seen with neutralizing antibody to TGF-β (Id., citing Møller-Pedersen T, et al., Neutralizing antibody to TGFβ modulates stromal fibrosis but not regression of photoablative effect following PRK. Curr. Eye Res. (1998) 17: 736-747). Inhibition of JNK signaling suppressed TGF-β induced CTGF expression and scarring in penetrating corneal wounds (Id., citing Shi L, et al., Activation of JNK signaling mediates connective tissue growth factor expression and scar formation in corneal wound healing. PLoS One. (2012) 7: e32128). Inhibitors of mechanistic target of rapamycin (mTOR) and p38 MAP kinase signaling were able to markedly reduce the expression of α-SMA and collagenase in corneal cells and injured corneas (Id., citing Jung JC, et al., Constitutive collagenase-1 synthesis through MAPK pathways is mediated, in part, by endogenous IL-1α during fibrotic repair in corneal stroma. J. Cell Biochem. (2007) 102: 453-462; Huh MI, et al., Distribution of TGF-β isoforms and signaling intermediates in corneal fibrotic wound repair. J. Cell Biochem. (2009) 108: 476-488; Milani BY, et al., Rapamycin inhibits the production of myofibroblasts and reduces corneal scarring after photorefractive keratectomy. Invest. Ophthalmol. Vis. Sci. (2013) 54: 7424-7430). In alkali-burned corneas, blocking of VEGF by neutralizing antibody bevacizumab also inhibited TGF-β expression and improved corneal transparency (Id., citing Lee SH, et al., Bevacizumab accelerates corneal wound healing by inhibiting TGF-β2 expression in alkali-burned mouse cornea. BMB Rep. (2009) 42: 800-805).

**Corneal endothelial wound healing**

Due to the relative inaccessibility of the corneal endothelial layer, there are fewer studies of endothelial healing. This process mostly occurs as a consequence of various burns (Id., citing Zhao B, et al., An investigation into corneal alkali burns using an organ culture model. Cornea. (2009) 28: 541-546.) and surgeries meant to replace dysfunctional endothelial cells (Descemet's stripping endothelial keratoplasty, DSEK) or endothelial cells with Descemet's membrane (Descemet's membrane endothelial keratoplasty, DMEK) (Id., citing Melles GR, et al., Descemet membrane endothelial keratoplasty (DMEK) Cornea. (2006) 25: 987-990; Price MO, Price FW. Descemet's stripping endothelial keratoplasty. Curr. Opin. Ophthalmol. (2007) 18: 290-294. 2007; Caldwell MC, et al., The histology of graft adhesion in Descemet stripping with endothelial keratoplasty. Am. J. Ophthalmol. (2009) 148: 277-281; Dirisamer M, et al., Patterns of corneal endothelialization and corneal clearance after Descemet membrane endothelial keratoplasty for Fuchs endothelial dystrophy. Am. J. Ophthalmol. (2011) 152: 543-555). The wound healing process of corneal endothelium has certain peculiarities. In many tissues, this process entails cell proliferation as a major mechanism of reducing and remodeling the wound bed. However, corneal endothelial cells, especially human, have very low proliferation rates (Id., citing Mimura T, et al., Corneal endothelial regeneration and tissue engineering. Prog. Retin. Eye Res. (2013) 35: 1-17). It is generally considered that corneal endothelium closes the wound gap mainly by migration and increased cell spreading. These two processes are pharmacologically separable and, depending on the wound nature, their relative contribution may vary (Id., citing Joyce NC, et al., In vitro pharmacologic separation of corneal endothelial migration and spreading responses. Invest. Ophthalmol. Vis. Sci. (1990) 31: 1816-1826.; Ichijima H, et al., Actin filament organization during endothelial wound healing in the rabbit cornea: comparison between transcorneal freeze and mechanical scrape injuries. Invest. Ophthalmol. Vis. Sci. (1993) 34: 2803-28012; Gordon SR. Cytological and immunocytochemical approaches to the study of corneal endothelial wound repair. Prog. Histochem. Cytochem. (1994) 28: 1-64; Mimura T, et al., Corneal endothelial regeneration and tissue engineering. Prog. Retin. Eye Res. (2013) 35: 1-17). Some data suggest that during healing, cell division remains very low (Id., citing Lee JG, Kay EP. FGF-2-induced wound healing in corneal endothelial cells requires Cdc42 activation and Rho inactivation through the phosphatidylinositol 3-kinase pathway. Invest. Ophthalmol. Vis. Sci. (2006) 47:1 376-1386), although this view is challenged by the fact that healing corneal endothelial cells mostly divide amitotically, with formation of binuclear cells (Id., citing Landshman N, et al., Cell division in the healing of the corneal endothelium of cats. Arch. Ophthalmol. (1989) 107: 1804-1808).

Endothelial wound healing is associated with a transient acquisition of fibroblastic morphology and actin stress fibers by migrating cells, which is consistent with endothelial-mesenchymal transformation (EnMT) (Id., citing Lee HT, et al., FGF-2 induced by interleukin-1 beta through the action of phosphatidylinositol 3-kinase mediates endothelial mesenchymal transformation in corneal endothelial cells. J. Biol. Chem. (2004) 279: 32325-32332; Miyamoto T, et al., Endothelial mesenchymal transition: a therapeutic target in retrocorneal membrane. Cornea. (2010) 29(Suppl 1): S52-56). In a model of freeze injury, EnMT to myofibroblasts occurs at the migrating front, where cells lose tight junction protein ZO-1 and start expressing $\alpha$-SMA (Id., citing Petroll WM, et al., ZO-1 reorganization and myofibroblast transformation of corneal endothelial cells after freeze injury in the cat. Exp. Eye Res. (1997) 64: 257-267). Inducers of EnMT and fibrotic changes in the endothelial layer include FGF-2, which may come from PMNs migrating to the cornea during epithelial and stromal wound healing (Id., citing Lee HT, et al., FGF-2 induced by interleukin-1 beta through the action of phosphatidylinositol 3-kinase mediates endothelial mesenchymal transformation in corneal endothelial cells. J. Biol. Chem. (2004) 279: 32325-32332) or IL-1$\beta$ (Id., citing Lee JG, et al., Endothelial mesenchymal transformation mediated by IL-1$\beta$-induced FGF-2 in corneal endothelial cells. Exp. Eye Res. (2012) 95: 35-39), and TGF-$\beta$ (Id. citing Sumioka T, et al., Inhibitory effect of blocking TGF-$\beta$/Smad signal on injury-induced fibrosis of corneal endothelium. Mol. Vis. (2008) 14: 2272-2281). Because EnMT may lead to fibrotic complications of healing, such as the formation of retrocorneal fibrous membrane, (Id., citing Ichijima H, et al., In vivo confocal microscopic studies of endothelial wound healing in rabbit cornea. Cornea. (1993) 12: 369-378.), some ways of attenuating EMT have been proposed. These include inhibiting the expression of connexin 43 (Id., citing Nakano Y, et al., Connexin 43 knockdown accelerates wound healing but inhibits mesenchymal transition after corneal endothelial injury in vivo. Invest. Ophthalmol. Vis. Sci. (2008) 49: 93-104) and TGF-$\beta$ type I receptor (Id., citing Okumura N, et al., Inhibition of TGF-$\beta$ signaling enables human corneal endothelial cell expansion in vitro for use in regenerative medicine. PLoS One. (2013) 8: e58000). The latter technique also facilitates endothelial cell propagation in culture.

Migration and spreading of corneal endothelial cells during wound healing is stimulated by a number of factors. The ECM proteins fibronectin and transpondin 1 (TSP-1) were shown to facilitate cell migration (Id., citing Munjal ID, et al., Thrombospondin: biosynthesis, distribution, and changes associated with wound repair in corneal endothelium. Eur. J. Cell Biol. (1990) 52:252-263; Gundorova RA, et al., Stimulation of penetrating corneal wound healing by exogenous fibronectin. Eur. J. Ophthalmol. (1994) 4: 202-210; Blanco-Mezquita JT, et al., Role of thrombospondin-1 in repair of penetrating corneal wounds. Invest. Ophthalmol. Vis. Sci. (2013) 54: 6262-6268.). Growth factors known to promote endothelial migration and wound healing include EGF, FGF-2, IL-1$\beta$, PDGF-BB, TGF-$\beta$2, and VEGF, whereas IGF-I and IGF-II are ineffective, and IL-4 reduces migration (Id., citing Joyce NC, et al., In vitro pharmacologic separation of corneal endothelial migration and spreading responses. Invest. Ophthalmol. Vis. Sci. (1990) 31: 1816-1826; Raphael B, et al., Enhanced healing of cat corneal endothelial wounds by epidermal growth factor. Invest. Ophthalmol. Vis. Sci. (1993) 34: 2305-2312; Soltau JB, McLaughlin BJ. Effects of growth factors on wound healing in serum-deprived kitten corneal endothelial cell cultures. Cornea. (1993) 12: 208-215; Hoppenreijs VP, et al., Basic fibroblast growth factor stimulates corneal endothelial cell growth and endothelial wound healing of human corneas. Invest. Ophthalmol. Vis. Sci. (1994) 35: 931-944; Hoppenreijs VP, et al., Effects of platelet-derived growth factor on endothelial wound healing of human corneas. Invest. Ophthalmol. Vis. Sci. (1994) 35: 150-161; Hoppenreijs VP, et al., Corneal endothelium and growth factors. Surv Ophthalmol. (1996) 41: 155-164; Bednarz J, et al., Influence of vascular endothelial growth factor on bovine corneal endothelial cells in a wound-healing model. Ger. J. Ophthalmol. (1996) 5: 127-31; Sabatier P, et al., Effects of human recombinant basic fibroblast growth factor on endothelial wound healing in organ culture of human cornea. J. Fr. Ophtalmol. (1996) 19: 200-207; Thalmann-Goetsch A, et al., Comparative study on the effects of different growth factors on migration of bovine corneal endothelial cells during wound healing. Acta. Ophthalmol. Scand. (1997) 75: 490-495; Rieck PW, et al., Intracellular signaling pathway of FGF-2-modulated corneal endothelial cell migration during wound healing in vitro. Exp. Eye Res. (2001) 73: 639-650.; Imanishi J, et al., Growth factors: importance in wound healing and maintenance of transparency of the cornea. Prog Retin Eye Res. (2000) 19: 113-129; Baldwin HC, Marshall J. Growth factors in corneal wound healing following refractive surgery: A review. Acta. Ophthalmol. Scand. (2002) 80: 238-247; Lee JG, Heur M. Interleukin-1$\beta$ enhances cell migration through AP-1 and NF-$\kappa$B pathway-dependent FGF2 expression in human corneal endothelial cells. Biol. Cell. (2013) 105: 175-189; Lee JG, Heur M. Interleukin-1$\beta$-induced Wnt5a enhances human corneal endothelial cell migration through regulation of Cdc42 and RhoA. Mol. Cell Biol. (2014) 34: 3535-3545).

The signaling pathways downstream of these factors that are important for wound healing are diverse. Prostaglandin E2 acting through cAMP pathway, ERK1/2 and p38 MAP kinase have been shown to participate in endothelial migration and wound healing (Id., citing Joyce NC, Meklir B. PGE2: a mediator of corneal endothelial wound repair in vitro. Am. J. Physiol. (1994) 266: C269-275; Sumioka T, et al., Inhibitory effect of blocking TGF-β/Smad signal on injury-induced fibrosis of corneal endothelium. Mol. Vis. (2008) 14: 2272-2281; Chen WL, et al., ERK1/2 activation regulates the wound healing process of rabbit corneal endothelial cells. Curr. Eye Res. (2009) 34: 103-111; Joko T, et al., Involvement of P38MAPK in human corneal endothelial cell migration induced by TGF-β2. Exp. Eye Res. (2013) 108: 23-32). FGF-2 stimulates migration through several pathways including p38, PI3K/Akt, and protein kinase C/phospholipase A2 (Id., citing Rieck PW, et al., Intracellular signaling pathway of FGF-2-modulated corneal endothelial cell migration during wound healing in vitro. Exp. Eye Res. (2001) 73: 639-650; L Lee HT, et al., FGF-2 induced by interleukin-1 beta (IL-1β) through the action of phosphatidylinositol 3-kinase mediates endothelial mesenchymal transformation in corneal endothelial cells. J. Biol. Chem. (2004) 279: 32325-32332; Joko T, et al., Involvement of P38MAPK in human corneal endothelial cell migration induced by TGF-β2. Exp. Eye Res. (2013) 108: 23-32). IL-1β stimulates migration through induction of FGF-2 (Id., citing Lee JG, et al., Endothelial mesenchymal transformation mediated by IL-1β-induced FGF-2 in corneal endothelial cells. Exp. Eye Res. (2012) 95: 35-39), as well as induction of Wnt5a that activate Cdc42 and inactivate RhoA (Id., citing Le JG, Kay EP. FGF-2-induced wound healing in corneal endothelial cells requires Cdc42 activation and Rho inactivation through the phosphatidylinositol 3-kinase pathway. Invest. Ophthalmol. Vis. Sci. (2006) 47:1376-1386; Lee JG, Heur M. Interleukin-1β enhances cell migration through activator protein 1 (AP-1) and NF-κB pathway-dependent FGF2 expression in human corneal endothelial cells. Biol. Cell. (2013) 105:175-189 Lee JG, Heur M. Interleukin-1β-induced Wnt5a enhances human corneal endothelial cell migration through regulation of Cdc42 and RhoA. Mol. Cell Biol. (2014) 34: 3535-3545).In the endothelial cells, interleukin-1β stimulates cell migration directly and indirectly.

**The Lens**

The eye lens is a flexible transparent biconvex structure embedded in the anterior segment of the eye and held in place by a ring of ciliary muscle. The main function of the lens alongside the cornea is to refract incoming light to be focused on the retina. The ciliary muscles help the lens change its shape, thereby changing its focal length. This process, which is generally referred to as accommodation, enables objects at various distances to be focused on the retina, producing sharp images.

**Optics of the Eye**

Focusing the eye contracts the ciliary muscle to reduce the tension or stress transferred to the lens by the suspensory ligaments. This results in increased convexity of the lens and, thus, increases the optical power of the eye. The term "accommodation" refers to the increase in thickness and convexity of the eye's lens in response to ciliary muscle contraction in order to focus the image of an external object on the retina. The term "amplitude of accommodation" refers to the difference in refractivity of the eye at rest and when fully accommodated.

The refractive power of the human eye is measured in diopters, which is a unit of measurement of the optical power of a lens and is equal to the reciprocal of the focal length measured in meters. In humans, the total refractive power (optical power) of the relaxed eye is approximately 60 diopters. The cornea accounts for approximately two-thirds of the refractive power (i.e., 40 diopters) and the lens accounts for the remaining one-third of the refractive power (i.e., 20 diopters). (Najjar, Dany MD. "Clinical Optics and Refraction," https://web.archive.org/web/20080323035251/http://www.eyeweb.org/optics.htm).

Emmetropia refers to an eye that has no visual defects. It is the state of vision where a faraway object at a distance of infinity is in sharp focus with the eye lens in a neutral or relaxed state. An emmetropic eye does not require vision correction.

**Abnormalities Of The Human Eye that can lead to vision impairment**

Abnormalities in the human eye can lead to vision impairment, such as myopia (near-sightedness), hyperopia (farsightedness), astigmatism, and presbyopia.

Myopia (or nearsightedness) occurs when the human eye is too long, relative to the focusing power of the cornea and the lens of the eye. This causes light rays to focus at a point in front of the retina, rather than directly on its surface.

Hyperopia (or farsightedness) occurs when light rays entering the eye focus behind the retina, rather than directly on it.

Astigmatism is a vision condition that causes blurred vision and occurs when the cornea is irregularly shaped. This prevents light rays from focusing properly on the retina.

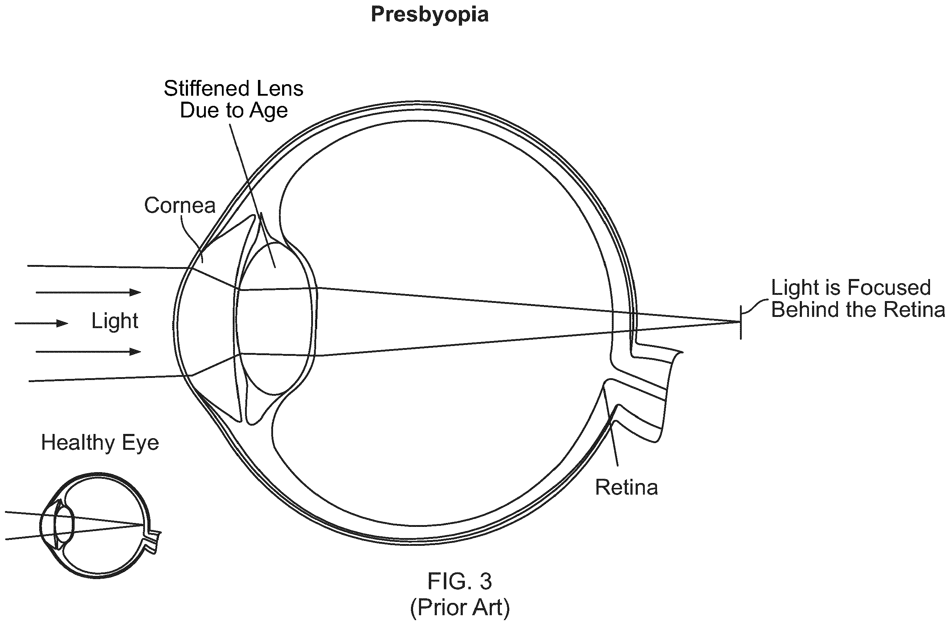

Presbyopia, as seen in **FIG. 3,** is generally characterized by a decrease in the eye's ability to increase its power to focus on nearby objects due to, for example, a loss of elasticity in the crystalline lens that occurs over time. Generally, young people under the age of 40 can easily focus both on distance and near objects. Near objects can be seen clearly due to contraction of the ciliary muscle resulting in increase in lens curvature. The focal length of the lens decreases, and the image of the object is focused on the retina. As we age past 40 years, the ciliary muscles progressively weaken and the lens starts to lose elasticity. Accommodation starts decreasing, making it difficult to see intermediate and near objects. This deficiency, which worsens as we age, is known as presbyopia. The most common solution to presbyopia is to wear reading glasses. Other solutions include multifocal or .monovision contact lenses, monovision presbyopia-correcting Intraocular Lens (IOLs), LASIK, multifocal LASIK, conductive keratoplasty (a surgical procedure that uses low level radiofrequency energy to reshape the cornea) and refractive lens exchange.

For example, ophthalmic devices and/or procedures (e.g., contact lenses, intraocular lenses, LASIK, inlays) can be used to address presbyopia using three common approaches. With a monovision prescription, the diopter power of one eye is adjusted to focus distant objects and the power of the second eye is adjusted to focus near objects. The appropriate eye is used to clearly view the object of interest. In the next two approaches, multifocal or bifocal optics are used to simultaneously, in one eye, provide powers to focus both distant and near objects. One common multifocal design includes a central zone of higher diopter power to focus near objects, surrounded by a peripheral zone of the desired lower power to focus distant objects. In a modified monovision prescription, the diopter power of one eye is adjusted to focus distance objects, and in the second eye a multifocal optical design is induced by the intracorneal inlay. The subject therefore has the necessary diopter power from both eyes to view distant objects, while the near power zone of the multifocal eye provides the necessary power for viewing near objects. In a bilateral multifocal prescription, the multifocal optical design is induced in both eyes. Both eyes therefore contribute to both distance and near vision.

## Corneal Ectatic Disorders

Ectatic corneal disease (ECD) or corneal ectasia is a group of progressive eye disorders characterized by progressive corneal steepening and thinning. Corneal ectasias are associated with decreased uncorrected visual acuity (UCVA), an increase in ocular aberrations, and often a loss of best-corrected distance visual acuity. Corneal ectasias can result in significant ocular morbidity and may require surgical intervention. [Guidelines, Corneal Ectasia Preferred Practice Pattern®, American Academy of Ophthalmology (2018) doi.org/10.1016/j.oophtha.2018.10.021].

Corneal ectasia encompasses both naturally occurring and surgically induced thinning and protrusion. Types of corneal ectasia include keratoconus, pellucid marginal degeneration, keratoglobus postkeratorefractive ectasia, and wound ectasia after penetrating keratoplasty (PK). The term "Forme fruste keratoconus (FFK)" is used to refer to a cornea that has no abnormal findings by both slit-lamp examinations and Placido-based corneal topography; it is considered an early form of keratoconus. [Ueki, R. et al. Differentiation of forme fruste keratoconus from normal cornea using parameters of corneal tomography, aberration, and biomechanics. Investigative Ophthalmology & Visual Sci. (2014) 55 (13): 3705].

Naturally occurring keratoconus can occur pre-puberty and progression past the age of 40 is not uncommon. It is typically bilateral, but can be asymmetrical. The overall prevalence of keratoconus has been reported to be between 50 and 230 per 100,000 in the general population, with both sexes equally affected. [Guidelines, Corneal Ectasia Preferred Practice Pattern®, American Academy of Ophthalmology (2018). doi.org/10.1016/j.oophtha.2018.10.021, citing Espandar, L., Meyer, J. Kertoconus: overview and update on treatment. Middle East Afr. J. Ophthalmol. (2010) 17(1): 15-20; Jhanji, V. et .al. Management of keratoconus: current scenario. Br. J. Ophthalmol. (2011) 95 (8): 1044-50; Pinero, DP et al. Characterization of corneal structure in keratoconus. J. Cataract Refract. Surg. (2012) 38 (12): 2167-83; Krachmer, JH et al. Surv. Ophthalmol. (19984) 28 (4): 293-322].

Keratoglobus may be seen in children and may be congenital. [Id.].

Pellucid marginal degeneration usually has its onset between the third and fifth decade of life. [Id., citing Jinabhai, A. et al. Pellucid corneal marginal degeneration: a review. Cont. Lens Anterior Eye (2011) 34 (2): 56-63; Feder, RS, Gan, TG. Noninflammatory ectatic disorders. In: Krachmer, JH, Mannis MJ, eds. *Cornea: Fundamentals, Diagnosis and Management.* Vol. 1. 33rd ed. London: Elsevier, Inc.: 2011: 879]..

Corneal ectasia can occur shortly after LASIK, small incision lenticule extraction (SMILE), and photorefractive keratectomy (PRK) in eyes with a pre-existing subclinical keratoconus or years later in eyes with no preoperative signs of keratoconus. [Id., citing Binder, PS. Analysis of ectasia after laser in situ keratomileusis: risk factors. J. Cataract Refrat. Surg. (2007) 33 (9): 1530-38; Moshirfar, M. et al. Ectasia following small-incision lenticule extraction (SMILE): a review of the literature. Clin. Ophthalmol. (2017) 11: 1683-88]. Findings in corneal ectasia that occur following keratorefractive surgery include the following: the residual stromal bed following surgery was thinner than expected, the flap was thicker than intended, or the patient had unrecognized preoperative signs of a pre-existing subclinical keratoconus. However, corneal ectasia can develop in the absence of these situations. [Id., citing Binder, PS. Analysis of ecasia after laser in situ keratomileusis: risk factors. J. Cataract Refract. Surg. (2007) 33 (9): 1530-8].

The etiology of corneal ectasia may include genetic factors, chromosomal and enzyme abnormalities, and mechanical factors (e.g., eye rubbing).

Genetic disorders associated with keratoconus include connective tissue diseases with abnormal collagen and hyperelasticity, such as Ehlers-Danlos syndrome, osteogenesis imperfect, congenital hip dysplasia, nail patella syndrome, pseudoxanthoma elasticum, hyper-immunoglobulin E syndrome associated with eczema and atopy, oculodentodigital dysplasia, Down syndrome and ichthyosis. Other genetic syndromes associated with keratoconus include those which are associated with eyerubbing and diminished mental capacity, such as in Apert syndrome, Crouzon syndrome, Down syndrome, hyperornithinemia, Angelman syndrome and Noonan syndrome. Keratoconus has been associated with disorders related to abnormal retinal function and oculodigital stimulation including albinism, Bardet-Biedl syndrome, Leber congenital amaurosis, tapetoretinal degeneration, retinitis pigmentosa, and Kurz syndrome. [Id., citing Sugar, J., Macsai, MS. What causes keratoconus? Cornea (2012) 31 96]: 716-19].

Keratoconus is also associated with atopic disease, including hay fever, asthma, eczema and vernal keratoconjunctivitis; in these patients, ocular inflammation should be controlled in order to decrease the propensity for eye rubbing. In addition, there are well-established associations with contact lens wear, particularly hard-contact lens wear.

Corneal hydrops, caused by the acute disruption of Descemet membrane in the setting of corneal ectasia, occurs in approximately 3% of patients with keratoconus. A history of eye rubbing and seasonal allergies is associated with hydrops development. [Id., citing Fan Gaskin, JC et al. The Aukland keratoconus study: identifying predictors of acute corneal hydrops in heratoconus. Clin. Exp. Optom. (2013) 96 (2): 208-13].

Recent advances in corneal imaging systems that allow for the detection of mild or subclinical stages of the disease have led to the realization that the true incidence of keratoconus is much higher than previously thought, making the true incidence of progression to keratoplasty much lower. [Id., citing Espandar, L., Meyer, J. Keratoconus: overview and update on treatment. Middle East Afr. J. Ophthalmol. (2010) 17 (1): 15-20; Jhanji, V. et al. Management of keratoconus: current scenario. Br. J. Ophthalmol. (2011) 95 (8): 1044-50; Weed, KH, et al. The Dundee University Scottish Keratoconus Study II: a prospective study of optical and surgical correction. Ophthalmic Physiol. Opt. (2007) 27 (6): 561-67; Gordon, MO, et al. Baseline factors predictive of incident penetrating keratoplasty in keratoconus. Am. J. Ophthalmology (2006) 142 (6): 923-30].

Thinning of the cornea in keratoconus occurs as a result of the degradation of corneal collagen. Altered enzyme activities and oxidative stress have been proposed as factors related to the pathogenesis of keratoconus and related corneal ectasias. In particular, increased matrix metalloproteinase levels, along with decreased tissue inhibitor of metalloproteinase, have been demonstrated in keratoconic corneas when compared with normal corneas. The findings indicate a probable role for these enzymes in matrix degradation found in keratoconus. [Id., citing Sugar, J., Macsai, MS. What causes keratoconus? Ornea 92012] 31 (6): 716-19; Atilano, SR et al. Accumulation of mitochondrial DNA damage in keratoconus corneas. Invest. Ophthalmol. Vis. Sci. (2005) 46 (4): 1256-63; Arnal, E. et al. Oxidative stress in keratoconus? Invest. Ophthalmol. Vis. Sci. (2011) 52 (12): 8592-97; Balasubramanian, SA et al. Are proteinases the reason for keratoconus? Curr. Eye Res. (2010) 35 (3): 185-91; Chwa, M. e al. Hypersensitive response to oxidative stress in keratoconus corneal fibroblasts. Invest. Ophthalmol. Vis. Sci. (2008) 49 (10): 4361-9; Kenney, MC et al. Increased levels of catalase and cathepsin V/L2 but deceased TIMP-1 in keratoconus corneas: evidence that oxidative stress plays a role in this disorder. Invest. Ophthalmol. Vis. Sci. (2005) 46 (30): 823-32; Smith, VA et al. Keratoconus: matrix metalloproteinase-2 activation and TIMP modulation. Biochim. Biophys. Acta (2006) 1762 (4): 431-9].

The tears of patients with keratoconus have been shown to contain increased levels of inflammatory mediators, such as interleukin-6, TNF-alpha, and MMP-9. [Id., citing Lema, I., Duran, JA. Inflammatory molecules in the tears of patients with keratoconus. Ophthalmology (2005) 112 (4): 654-9; Lema I., et al. Subclinical keratoconus and inflammatory molecules from tears. Br. J. Ophthalmol. (2009) 93 (6): 820-4]. These inflammatory mediators likely result in keratocyte apoptosis and the decreased keratocyte cell density associated with keratoconic corneas. It is therefore likely that this form of corneal thinning, classified as noninflammatory, may have an inflammatory component that is either directly or indirectly related to the pathogenesis and progression of the disease. [Id., citing Matthews, FJ et al. Changes in the balance of the tissue inhibitor of matrix metalloproteinases (TIMPS)-1 and -3 may promote keratocyte apoptosis in keratoconus. Exp. Eye Res. (2007) 84 (6): 1125-34].

Postkeratorefractive superficial ectasia has been postulated to occur as a result of insufficient corneal thickness, exacerbation of pre-existing, subclinical or clinical keratoconus by further weakening of the corneal structure, and/or the development of genetically predetermined ectasia occurring years after refractive surgery. [Id., citing Moshirar, M. et al. Ectasia following small-incision lenticule extraction (SMILE)L a review of the literature. Clin. Ophthalmol. (2017) 11: 1683-88' Randleman, JB, et al., Am. J.Ophthalol. (2008) 145 (5): 813-18; Binder, PS, Trattler WB. Evaluation of a risk factor scoring system for corneal ectasia after LASIK in eyes with normal topography. J. Refract. Surg. (2010) 26 (4): 241-50]. A genetic predisposition to keratoconus may exist in patients who undergo a second environmental insult, such as eye rubbing and/or the iatrogenic corneal thinning that occurs after laser vision correction. [Id., citing Espandar, L.,

Meyer, J. Keratoconus: overview and update on treatment. Middle East Afr. J. Ophthalmol. (2010) 17 (1): 15-20; Sugar, J. Macsai, MS. What causes keratoonus? Cornea (2012) 31 (6): 716-19].

Patients with corneal ectasia suffer from varying degrees of disability, including glare, halos, multiple images, ghosting, reduced visual acuity, and intolerance to eyeglasses and contact lenses. The loss of visual function may result in lost productivity, a reduced self-esteem, and difficulties when performing high-skill visual tasks.

The rationale for treatment depends on the severity of disease and the amount of vision loss. The greater the delay in diagnosis, the higher the risk of greater vision loss and of the patient requiring a corneal transplant. [Id., citing Reeves, SW et al. Risk factors for progression to penetrating keratoplasty in patients with keratoconus. Am. J. Ophthalmol. (2005) 140 (4): 607-11]. Once progression is observed, early detection and prompt treatment, e.g. with corneal cross-linking (CXL), can reduce or stop keratoconus progression and preserve good visual acuity with eyeglasses and/or contact lenses. [Id., citing Espandar, L., Meyer, J. Keratoconus: overview and update on treatment. Middle East Afr. J. Ophthalmol. (2010) 17 (1): 15-20; Jhanji, V. et al. Management of keratoconus: current scenario. Br. J. Ophthalmol. (2011) 95 (8): 1044-50; Mannis, MJ. Keratoconus: why and when do we turn to surgical therapy? Am J. Ophthalmol. (2006) 142 (6): 1044-5].

**Keratoconus**

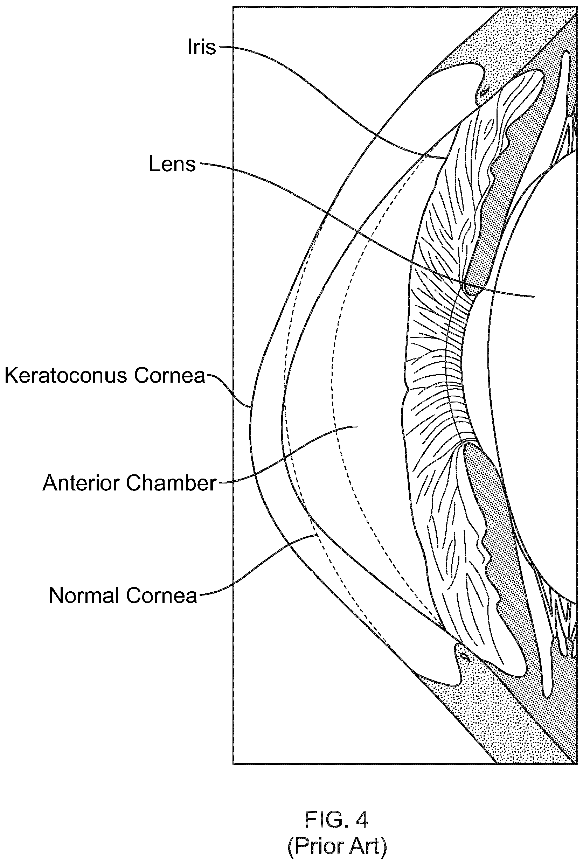

Structurally, the most common feature of keratoconus is a thinning of the inferior and nasal part of the corneal stroma. **FIG. 4** illustrates a normal cornea relative to a keratoconus cornea. Keratoconus often manifests as a conical protrusion with an epithelial iron line partially surrounding the base of the cone called Fleisher's ring. The degree of deformation, however, can vary greatly between patients. Both the anterior and posterior curvature of the cornea are affected, meaning that tracking corneal shape changes over time is the most reliable way of identifying keratoconus progression. [Volatier, TLA, et al. Keratoconus at a molecular level: A Review. The Anatomical Record (2020) 303: 1680-88, citing Schwiegerling, Cone dimensions in heratoconus using Zernike polynomials. J. Optom. Vis. Sci. (1997) 74: 963-9; Tomidokoro, A. et al., Changes in anterior and posterior corneal curvatures in keratoconus. Ophthalmology (2000) 107: 1328-32].

There are a number of characteristic clinical signs that can be used to set keratoconus apart from other ectatic conditions (e.g., pellucid marginal degeneration) and to determine the degree of severity. [Id., citing Sherwin, T., Brookes, NH. Morphological changes in keratoconus: pathology or pathogenesis (2004). Clin. Experiment. Ophthalmol. (2004) 32: 211-17]:

In moderate to advanced keratoconus, slit lamp examination can often capture clinical signs of keratoconus. These include:

(1) Fleischer's ring (a ferritin deposit in the epithelium that partially surrounds the base of the corneal cone). Ferritin is an intracellular iron storage protein. [Id., citing Loh, A et al. Iron homeostasis and eye disease. Biochim Biophys. Acta (2009) 1790: 637-49]. The source of the accumulated iron is likely to come from the tear film, where iron is bound to lactoferrin. The tear-pooling hypothesis suggests that the collection of tears along the lines of lid closure leads to increased iron deposition. It is also hypothesized that an altered iron metabolism may occur in keratoconic issue. Iron is required as a cofactor for the formation of amino acid hydrolysine, which is then used to regulate collagen fiber diameter. In keratoconic tissue, hydroxylysine content is reportedly lower [Id., citing Cannon, DJ, Foster CS. Collagen cross-linking in keratoconus. Invest. Ophthalmol. Vis. Sci. (1978) 17: 63-65], indicating that iron metabolism may be disrupted upstream.

(2) Vogt's striae (fine vertical lines located in the superficial stromal layers that occur due to deformation of the cornea).

(3) Thinning of the corneal stroma centrally and paracentrally. The thinning of the stroma is likely multifactorial.

(4) Thinned epithelia and proud nebulae, a small buildup of scar tissue at the very center of the cornea that often results in some loss of vision.

(5) Enlarged and more visible corneal nerves.

(6) Subepithelial fibrillary lines.

(7) Acute stromal swelling due to breaks in Descemet's membrane (termed Hydrops).

(8) Breaks in Bowman's layer centrally.

(9) Münson's sign (a wedge shaped indentation of the lower eyelid by the cornea when the patient is looking down. This is common in advanced cases of keratoconus and is due to the keratocone pushing into the lower eyelid.).

The manifestation and progression of keratoconus are highly variable and are most often asymmetric between the two eyes of the same patient.

**Pathophysiology**

The cause of keratoconus remains elusive. Without being limited by theory, a number of studies suggest that reactive oxygen species (ROS) within the cornea due to exposure to external oxidants-such as UV light or intracellular ROS produced as byproducts of cellular metabolism-may drive pathological thinning of the stroma [McKay, TB et al. Mechanisms of Collagen Crosslinking in Diabetes and Keratoconus. Cells (2019) 8: 1239, citing Kenney, MC et al. The elusive causes of keratoconus: A working hypothesis. CLAO J. (2000) 26: 10-13]. This hypothesis has been supported by metabolic studies identifying increased lactate production and lower reduced glutathione availability in KC-derived fibroblasts [Id., citing Karamichos, D. et al. Sci. Rep. (2014) 4: 4608] and tear samples [Id., citing Karamichos, D. et al. Exp. Eye Res. (2015) 132: 1-8] compared to healthy controls. These studies are consistent in showing increased susceptibility to damage caused by cytosolic ROS [Id., citing Chwa, M. et al. Hypersensitive response to oxidative stress in keratoconus corneal fibroblasts. Investig. Ophthalmol. Vis. Sci. (2008) 49:4361-4369] and hypoxia [Id., citing McKay, TB et al. Acute hypoxia influences collagen and matrix metalloproteinase expression by human keratoconus cells in vitro. PLoS ONE. 2017;12:e0176017] likely attributed to reduced expression of superoxide dismutase in KC-derived fibroblasts [Id., citing Atilano, SR. et al., Corneal Oxidative Damage in Keratoconus Cells due to Decreased Oxidant Elimination from Modified Expression Levels of SOD Enzymes, PRDX6, SCARA3, CPSF3, and FOXM1. J. Ophthalmic Vis. Res. 2019;14:62-70] with genetic studies likewise identifying splice variants of SOD1 associated with keratoconus in a human population [Id., citing Udar, N. et al. SOD1: A candidate gene for keratoconus. Investig. Ophthalmol. Vis. Sci. 2006;47:3345-3351; Udar, N. et al. SOD1 haplotypes in familial keratoconus. Cornea. 2009;28:902-907; Moschos, MM. et al. Polymorphism Analysis of VSX1 and SOD1 Genes in Greek Patients with Keratoconus. Ophthalmic Genet. 2015;36:213-217]. Furthermore, studies have suggested defects in mitochondrial function based on the reduced expression of key mitochondrial genes, including SOD2 and phorbol-12-myristate-13-acetate-induced protein 1 (PMAIP1) [Id., citing Sarker-Nag, A. et al. Mitochondrial Profile and Responses to TGF-beta Ligands in Keratoconus. Curr. Eye Res. 2016;41:900-907], consistent with altered metabolic flux favoring aerobic glycolysis in keratoconus over the tricarboxylic acid cycle and oxidative phosphorylation for energy production [Id., citing Karamichos, D. et al. In vitro model suggests oxidative stress involved in keratoconus disease. Sci. Rep. 2014;4:4608]. To combat the increased oxidative stress exhibited in keratoconus, studies utilizing the antioxidant quercetin have shown recovery of basal cellular metabolism in keratoconus-derived fibroblasts with reduced lactate production similar to control levels [Id., citing McKay, TB et al. Quercetin attenuates lactate production and extracellular matrix secretion in keratoconus. Sci. Rep. 2015;5:9003].

**Corneal changes in keratoconus**

In terms of corneal structure, keratoconus may present with up to a 60% decrease in central corneal thickness (<200 $\mu$m) for Stage 4 keratoconus, based on the Amsler-Krumeich grading system [Id., citing Mas Tur, V. et al. A review of keratoconus: Diagnosis, pathophysiology, and genetics. Surv. Ophthalmol. (2017) 62:770-783.]. Corneal thinning leads to detrimental effects on visual acuity and often corneal scarring due to severe steepening at the apex. Significant differences in the biomechanical parameters of early stages of keratoconus (i.e., forme fruste keratoconus) compared to controls may be difficult to identify in certain patients [Id., citing Luz, A. et al. Enhanced Combined Tomography and Biomechanics Data for Distinguishing Forme Fruste Keratoconus. J. Refract. Surg. (2016) 32:479-494], although a number of studies have identified significantly reduced stiffness compared to controls based on the corneal hysteresis and corneal resistance factor [Id., citing Ahmadi Housseini, SM et al. Efficacy of corneal tomography parameters and biomechanical characteristic in keratoconus detection. Cont Lens Anterior Eye. (2014) 37:26-30; Ayar, O. et al. In-vivo corneal biomechanical analysis of unilateral keratoconus. Int. J. Ophthalmol. 2015;8:1141-1145]. It is well-established that the advanced keratoconus cornea shows significant differences in biomechanical properties, including reduced stiffness nearly 50% lower than controls [Id., citing Mikula, ER et al. Measurement of an Elasticity Map in the Human Cornea Measurement of Elasticity Map in Human Cornea. Investig. Ophthalmol. Vis. Sci. 2016;57:3282-3286; Mikula, E. et al. Axial mechanical and structural characterization of keratoconus corneas. Exp. Eye Res. 2018;175:14-19; Andreassen, TT et al. Biomechanical properties of keratoconus and normal corneas. Exp. Eye Res. 1980;31:435-441].

The biomechanical changes observed in keratoconus are a result of differential collagen fibril microstructure and organization within the central cornea. Immunohistochemical analysis of keratoconus corneas has also shown alterations in basement membrane structure, as well as Bowman's and Descemet's layers [Id., citing Kenney, MC et al. Abnormalities of the extracellular matrix in keratoconus corneas. Cornea. 1997;16:345-351] that may arise with severe curvature of the cornea that develops during later stages of the disease. Differences in collagen organization imaged by acoustic radiation force elastic microscopy have shown reduced branching and interweaving of collagen fibers in the anterior and posterior keratoconus cornea compared to the normal basketweave pattern found in controls [Id., citing Mikula, E. et al. Axial mechanical and structural characterization of keratoconus corneas. Exp. Eye Res. 2018;175:14-19]. A number of studies have also identified differential angle directionality of collagen fibers in keratoconus corneas, particularly at the corneal apex, which is the region most susceptible to thinning [Id., citing Meek, KM, et al. Changes in collagen orientation and distribution in keratoconus corneas. Investig. Ophthalmol. Vis. Sci. 2005;46:1948-1956; Daxer, A. Fratzl, P. Collagen fibril orientation in the human corneal stroma and its implication in keratoconus. Investig. Ophthalmol. Vis. Sci. 1997;38:121-129; Mathew, JH et al. Lamellar changes in the keratoconic cornea. Acta Ophthalmol. 2015;93:767-773], further highlighting the defects in collagen organization that may contribute to the mechanical failings leading to ectasia.

**Post-LASIK keratectasia**

Post-LASIK keratectasia is a complication of corrective surgery procedures to the eye where procedures such as photorefractive keratectomy (PRK) and Laser-Assisted in-situ keratomileusis (LASIK) cause progressive thinning of the cornea. Because it becomes excessively thin and weak, and will not be able to hold the normal eye pressure, the cornea starts bulging out which, in turn, results in worsening vision. LASIK or PRK procedures also can remove too much tissue from the cornea, which causes a new form of astigmatism. Corneal ectasia signs and symptoms are progressive as the cornea adopts a bulging shape. The most commonly reported signs and symptoms are: inability to fully correct vision to 20/20 with contacts or glasses; shadows or ghosting figures in the visual field; refraction variations with constant prescription changes; and frequent squinting to improve visual acuity.

**Surgical correction of corneal damage**

Surgical procedures aimed at improving the focusing power of the eye are called refractive surgery.

Laser-assisted in situ keratomileusis ("LASIK") is a type of refractive laser eye surgery in which a laser is used to remodel a portion of the cornea after lifting a previous cut corneal flap. A special type of cutting laser is used for precise and controlled removal of corneal tissue to change the shape of the cornea, which changes its focusing power to improve vision.

Radial Keratotomy (RK) and Photorefractive Keratectomy (PRK) are other refractive surgeries used to reshape the cornea. In RK, a very sharp knife is used to cut slits in the cornea changing its shape. PRK was the first surgical procedure developed to reshape the cornea by sculpting using a laser. Later, LASIK was developed. Often the exact same laser is used for the two types of surgery. The major difference between the two surgeries is the way that the stroma (the middle layer of the cornea) is exposed before it is vaporized with the laser. In PRK, the epithelium (the top layer of the cornea) is scraped away to expose the stromal layer underneath. In LASIK, a flap is cut in the stromal layer and the flap is folded back.

Another type of refractive surgery is thermokeratoplasty in which heat is used to reshape the cornea. The source of the heat can be a laser, but it is a different kind of laser than that used for LASIK and PRK.

Small incision lenticule extraction (SMILE) is a type of laser refractive surgery in which a laser is used to treat myopia (nearsightedness) and astigmatism (irregularly shaped cornea). The laser is used to change the shape of the cornea, which improves the way light rays are focused on the retina.

Other refractive devices include corneal ring segments that are inserted into the stroma and special contact lenses that temporarily reshape the cornea (orthokeratology).

A diseased cornea may be replaced surgically with a clear, healthy cornea from a human donor (corneal transplantation) by a number of methods.

Phototherapeutic keratectomy (PTK) is a type of laser eye surgery that is used to treat corneal dystrophies (meaning abnormal buildup of foreign material in the cornea), corneal scars, and some corneal infections. The surgeon uses a laser to remove thin layers of diseased cornea tissue microscopically, allowing new tissue to grow on the smooth surface.

If the front and middle layers of the cornea are damaged, a deep anterior lamellar keratoplasty (DALK) or partial thickness corneal transplant is performed; only the front and middle layers of the cornea are removed, with the endothelial layer kept in place. Healing time after DALK is shorter than after a full corneal transplant. There is also less risk of having the new cornea rejected. DALK is commonly used to treat keratoconus or bulging of the cornea.

If both front and inner corneal layers are damaged, penetrating keratoplasty (PK) or full thickness corneal transplant is performed to remove and replace the damaged cornea. PK has a longer recovery period than other types of corneal transplants. Getting complete vision back after PK may take up to 1 year or longer. There is a slightly higher risk with PK than with other types of corneal transplants that the cornea will be rejected.

In some eye conditions, the endothelium, the innermost layer of the cornea, is damaged. Endothelial keratoplasty is a surgery to replace this layer of the cornea with healthy donor tissue. It is known as a partial transplant since only the endothelium is replaced. Examples of types of endothelial keratoplasty include DSEK (or DSAEK) - Descemet's Stripping (Automated) Endothelial Keratoplasty, and DMEK - Descemet's Membrane Endothelial Keratoplasty. Each procedure removes damaged cells from Descemet's membrane by removing the damaged corneal layer through a small incision, and putting the new tissue in place. Much of the cornea is left untouched.

**Keratoconus Interventions**

*Vision correction.* Although mild, non-progressing cases can be managed with eyeglasses or contact lenses, the significant asymmetry between eyes typically reduces the ability of spherocylindrical lenses to adequately correct vision. Since the effects of keratoconus in cornea shape distortion and stromal thinning are highly asymmetric, vision correction with eyeglasses and with spherical/toric soft contact lenses is suboptimal and only applicable to the early stages of keratoconus. As progressive keratoconus is characterized by increasing degrees of higher-order optical aberrations, the major shortcoming of traditional soft contact lens correction is their limited capacity to mask irregular astigmatism. [Downie, L., Lindsay, RG. Contact lens management of keratoconus. Clin. Exp. Optom. (2015) 98: 299-311, citing Jinabhai, A. et al. Visual performance and optical quality with soft lenses in heratoconus patients. Ophthalmic Physiol. Opt. (2012) 32: 100-16; Griffiths, M. et al. Masking of irregular corneal topography with contact lenses. CLAO J. (1998) 24: 76-81].

Rigid gas permeable contact lenses and scleral lenses are the mainstays of vision treatment for modest to advanced keratoconus. Their main advantage is the creation of the tear pool between the lens and the cornea, which naturally neutralizes the ocular aberrations associated with keratoconic ectasia, thus providing improved corrected vision. A disadvantage related to the use of rigid gas permeable contact lenses is that they may not be tolerated.

Three major subtypes of keratoconus are defined based on morphological criteria. [Downie, L., Lindsay, RG. Contact lens management of keratoconus. Clin. Exp. Optom. (2015) 98: 299-311, citing McMahon, T. et al. A new method for grading the severity of keratoconus: The keratoconus severity score (KSS). Cornea (2006) 25: 794-800; Perry, HD et al. Round and oval cones in keratoconus. Ophthalmology (1980) 87: 905-9]. The centered (nipple) cone subtype, which accounts for about half of cases, is characterized by a cone diameter of five millimeters or less that is round and positioned centrally or slightly inferior to the visual axis. The oval (sagging) cone subtype is characterized by cones that are larger in diameter (e.g., larger than five millimeters) and demonstrate either infero-nasal or infero-temporal displacement of the corneal apex. The least common subtype is the globus cone, in which the conical area involves at least 75% of the cornea. From a contact lens fitting perspective, since rigid contact lenses tend to align over the corneal apex, achieving adequate lens centration and pupil coverage for oval cones can pose a challenge. [Id, citing Perry, HD, et al. round and oval cones in kertoconus. Ophthalmology (1980) 87: 905-9; Romero-Jimenez, M. et al. Keratoconus: a review. Cont. Lens Anterior Eye (2010) 33: 157-66] Globus cone cases are arguably the most complex and typically warrant designs with larger diameters (e.g., corneo-scleral, mini-scleral, or scleral lenses) to achieve desired fitting.

*Non-surgical improvement of Corneal Stability.* The various alternatives to corneal transplantation for the management of corneal ectasia aim to support a weakened corneal structure by enhancing corneal rigidity.

*Collagen cross-linking.* There are three major reactions of collagen crosslinking associated with corneal biology: (1) lysyl oxidase-mediated crosslinking as a post-translational modification of secreted collagen that stabilizes collagen fibril structure; (2) advanced glycation end product (AGE)-mediated crosslinking that occurs as a result of reaction of glycated proteins, including collagen, to non-enzymatically react with surrounding proteins; and (3) photooxidative CXL mediated by riboflavin as a treatment option for KC and corneal ectasia. [Id.]

*Lysyl oxidase (LOX)-mediated crosslinking.* Lysyl oxidase is a copper amine oxidase primarily responsible for catalyzing the formation of crosslinks between collagen fibrils by generating reactive aldehydes on lysine residues found in the telopeptide domain of collagen that may cross-react with nearby groups [McKay, TB et al. Mechanisms of Collagen crosslinking in Diabetes and Keratoconus. Cells (2019) 8: 1239, citing Kagan H.M., Li W. Lysyl oxidase: Properties, specificity, and biological roles inside and outside of the cell. J. Cell. Biochem. (2003) 88:660-672]. This reaction proceeds with conversion of an $\varepsilon$-amine (e.g., lysine) group to an aldehyde (e.g., allysine) catalyzed by lysyl oxidase. This aldehyde group may then react with other surrounding $\varepsilon$-amine moieties or another proximal reactive aldehyde to generate a Schiff base or $\alpha,\beta$-unsaturated aldehyde, respectively. Spontaneous condensation of these species occurs to generate crosslinks between collagen molecules consisting of lysinorleucine, dihydroxylysinorleucine, hydroxylysinonorleucine, and histidinohydroxylysinonorleucine, which have all been detected within the cornea [Id., citing Takaoka. A. et al; An Evaluation of Lysyl Oxidase-Derived Cross-Linking in Keratoconus by Liquid Chromatography/Mass Spectrometry. Investig. Ophthalmol. Vis. Sci. (2016) 57:126-136; Yamauchi, M. et al. Cross-linking and the molecular packing of corneal collagen. Biochem. Biophys. Res. Commun. (1996) 219:311-315]. Lysyl oxidase is secreted as an inactive proenzyme that is cleaved by procollagen C-proteinase to an active form. A number of growth factors, including transforming growth factor-$\beta$1 (TGF-$\beta$1) and platelet-derived growth factor (PDGF), are known to increase the expression of lysyl oxidase, thus resulting in elevated collagen crosslinking during conditions associated with elevated levels, including wound healing [Id., citing 165] and cancer metastasis [Id., citing Erler, JT et al. Lysyl oxidase is essential for hypoxia-induced metastasis. Nature. (2006) 440:1222]. Stiffening of the ECM due to enhanced lysyl oxidase activity is also associated with cancer progression and fibrosis [Id., citing Levantal, KR et al. Matrix Crosslinking Forces Tumor Progression by Enhancing Integrin Signaling. Cell. (2009) 139:891-906; Akiri, G. et al. Lysyl oxidase-related protein-1 promotes tumor fibrosis and tumor progression in vivo. Cancer Res. (2003) 63:1657-1666]. Downregulation of lysyl oxidase is known to be promoted by basic fibroblast growth factor (bFGF) in osteoblasts. Conditions associated with hypertrophic scar have also been associated with increased lysyl oxidase activity, suggesting that strict regulation of this enzyme is important to minimize fibrosis as well as to maintain proper ECM structure [Id., citing Kagan H.M., Trackman P.C. Properties and function of lysyl oxidase. Am. J. Respir. Cell Mol. Biol. (1991) 5:206-210; Murray J.C. Keloids and hypertrophic scars. Clin. Dermatol. (1994) 12:27-37].

*Advanced glycation end product [AGE]-mediated crosslinking* Compared to enzymatic crosslinking by lysyl oxidase, non-enzymatic AGE-mediated crosslinking can occur between collagen helices between lysine and histidine or arginine, and thus contribute to stable crosslinks that are resistant to enzymatic degradation [Id., citing Robins S.P. Biochemistry and functional significance of collagen cross-linking. Biochem. Soc. Trans. (2007) 35:849-852].

*Photooxidative crosslinking mediated by riboflavin.* In contrast to LOX- and AGE-mediated crosslinking, the amine group present on lysine or arginine are thought to not be directly involved in crosslink formation in photooxidative CXL with riboflavin [Id., citing McCall, AS et al. Mechanisms of Corneal Tissue Cross-linking in Response to Treatment with Topical Riboflavin and Long-Wavelength Ultraviolet Radiation (UVA) Investig. Ophthalmol. Vis. Sci. (2010) 51:129-138]. The carbonyl groups found on histidine, hydroxyproline, tyrosine, or threonine appear to be the dominant functional group, as masking of the carboxyl moiety via chemical blockage using hydroxylamine or sodium azide inhibits crosslink formation [Id., citing McCall, AS et al. Mechanisms of Corneal Tissue Cross-linking in Response to Treatment with Topical Riboflavin and Long-Wavelength Ultraviolet Radiation (UVA) Investig. Ophthalmol. Vis. Sci. (2010) 51:129-138]. This mechanism proceeds via singlet oxygen formation, thus relying on the carbonyl group present on the side chain of select amino acids following exposure to UV light (360 nm) in the presence of riboflavin [Id., citing Raiskup F., Spoerl E. Corneal crosslinking with riboflavin and ultraviolet A. I. Principles. Ocul. Surf. (2013) 11:65-74]. Riboflavin functions as a photosensitizer in the presence of UV light, thereby absorbing light leading to conversion to an excited singlet riboflavin molecule that rapidly transforms to a triplet state [Id., citing Raiskup F., Spoerl E. Corneal crosslinking with riboflavin and ultraviolet A. I. Principles. Ocul. Surf. (2013) 11:65-74]. This reactive intermediate can then mediate hydride or electron transfer to collagen or other ECM proteins, which then converts to covalent bonds between substrates in a Type 1 process. Photooxidative CXL with riboflavin can also proceed via reaction of activated riboflavin with oxygen generating ROS, which can then mediate CXL between substrates in a Type II process. The primary amino acids thought to be involved in this reaction are hydroxyproline, hydroxylysine, tyrosine, or threonine, forming intramolecular and intermolecular bonds within the collagen backbone and with surrounding proteoglycans [Id., citing Raiskup F., Spoerl E. Corneal crosslinking with riboflavin and ultraviolet A. I. Principles. Ocul. Surf. (2013) 11:65-74]. Photooxidative CXL with riboflavin is also expected to proceed, via AGE-mediated mechanisms, primarily between highly glycosylated proteoglycans found in the stroma, e.g., keratocan and lumican, as well as collagen fibrils [Id., citing Zhang, Y. et al. Effects of ultraviolet-A and riboflavin on the interaction of collagen and proteoglycans during corneal cross-linking. J. Biol. Chem. (2011) 286:13011-13022]. The formation of dityrosine crosslinks has also been reported and may play a further role in tissue stiffening post-CXL [Id., citing Kato, Y. et al. Aggregation of collagen exposed to UVA in the presence of riboflavin: A plausible role of tyrosine modification. Photochem. Photobiol. 1994;59:343-349].

**CXL Biomechanical changes in ECM structure**

The structural modifications of the cornea due to CXL are thought to primarily occur within the collagenous-rich stroma. Ex vivo studies on the effects of CXL on human and porcine corneal tissue samples have demonstrated its association with increased collagen fiber diameter by about 12% in the anterior cornea exposed to the highest direct UV-A irradiation and about 5% in the posterior cornea [Id., citing Wollensak, G. et al. Collagen fiber diameter in the rabbit cornea after collagen crosslinking by riboflavin/UVA. Cornea. (2004) 23:503-507]. Some notable study findings include the study conducted by Wollensak et al., where the rigidity of the cornea was observed to have increased by 328.9% after collagen crosslinking [Id., citing Wollensak, G. et al. Stress-strain measurements of human and porcine corneas after riboflavin-ultraviolet-A-induced cross-linking. J. Cataract. Refract. Surg. (2003) 29:1780-1785] and increased Young's modulus up to 8 months post-CXL with no significant difference in corneal thickness [Id., citing Wollensak, G., Iomdina, E. Long-term biomechanical properties of rabbit cornea after photodynamic collagen crosslinking. Acta Ophthalmol. (2009) 87:48-51]. The effects of CXL on collagen structure have shown decreased collagen waviness or crimping in the anterior corneal stroma by 1-month post-riboflavin-mediated CXL in a rabbit model [Id., citing Bradford, SM et al. Collagen fiber crimping following in vivo UVA-induced corneal crosslinking. Exp. Eye Res. (2018) 177:173-180]. Riboflavin-mediated CXL of the cornea has also been associated with loss of corneal transparency by 1 month, although resolution of the haze is evident by 3 months post-CXL in rabbit models [Id., citing Collagen fiber crimping following in vivo UVA-induced corneal crosslinking. Exp. Eye Res. (2018) 177:173-180]. In a human clinical trial, the efficacy of CXL determined based on the maximum keratometry in the crosslinked group compared to sham controls showed an improvement in corneal steepening (Kmax) by 1.6 diopters with a parallel improvement in visual acuity by at least 1 line (corrected distance visual acuity) at 1-year post-CXL [Id., citing Hersh, PS et al. United States Multicenter Clinical Trial of Corneal Collagen Crosslinking for Keratoconus Treatment. Ophthalmology. (2017) 124:1259-1270]. Other studies have also concluded that CXL can help to reduce progression due to secondary corneal ectasia post-refractive surgery with similar halting of corneal steepening and stabilization of visual acuity [Id., citing Hersh, PS et al. Multicenter Clinical Trial of Corneal Collagen Crosslinking for Treatment of Corneal Ectasia after Refractive Surgery. Ophthalmology. (2017) 124:1475-1484].

**Cellular changes in the cornea post-photooxidative crosslinking**

In terms of photooxidative crosslinking, riboflavin/UV-A exposure has been reported to show cytotoxicity towards keratocytes in vitro at a much lower irradiance (0.5 mV/cm$^2$) compared to UV-A exposure alone [Id., citing Wollensak, G. et al. Keratocyte cytotoxicity of riboflavin/UVA-treatment in vitro. Eye. (2004) 18:718-722], suggesting that riboflavin-mediated ROS production may be key to this observed cytotoxicity. Studies have likewise shown a reduction in the keratocyte population found in the anterior cornea by 1 to 6 months post-CXL that appears to recover by 12 months to pre-operative levels, as assessed by in vivo confocal microscopy [Id., citing Mazzotta, C. et al. In Vivo Confocal Microscopy after Corneal Collagen Crosslinking. Ocul. Surf. (2015) 13:298-314; Jordan, C. et al. In vivo confocal microscopy analyses of corneal microstructural changes in a prospective study of collagen cross-linking in keratoconus. Ophthalmology. (2014) 121:469-474]. The CXL reaction also may lead to cellular damage through the production of ROS, which may be cytotoxic at high concentrations to other cell types within the cornea, including the endothelium [Id., citing Wollensak, G. et al. Corneal endothelial cytotoxicity of riboflavin/UVA treatment in vitro. Ophthalmic Res. (2003) 35:324-328].

In terms of downstream effectors modulated by CXL, in vitro studies evaluating the effects of CXL on primary corneal stromal fibroblasts isolated from KC patients have shown that CXL downregulates canonical TGF-β signaling based on lower pSMAD2/3 expression post-CXL in KC [Id., citing Sharif, R. et al. Human in vitro Model Reveals the Effects of Collagen Cross-linking on Keratoconus Pathogenesis. Sci. Rep. (2017) 7:12517]. A normalization of lysyl oxidase abundance [Id., citing Shhharif, R. et al. Human in vitro Model Reveals the Effects of Collagen Cross-linking on Keratoconus Pathogenesis. Sci. Rep. (2017) 7:12517], increased proteoglycan expression (e.g., lumican, mimecan, and decorin) [Id., citing Sharif, R. et al. Collagen cross-linking impact on keratoconus extracellular matrix. PLoS ONE. (2018) 13: e0200704], and a reduction in lactate levels in KC-derived corneal fibroblasts following CXL have been reported [Id., citing Sharif, R. et al. Collagen cross-linking impact on keratoconus extracellular matrix. PLoS ONE. (2018) 13:e0200704 ] suggesting that riboflavin/UV-A CXL may modulate ECM deposition and collagen crosslinking at the cellular level in addition to the direct structural modifications to collagen.

*Surgical interventions.* If the cornea is not capable of compensating for keratoconus by toughening the remaining stromal ECM, then a surgical cross-linking treatment can aid in slowing or more often halting the disease process by hardening the collagen matrix [Volatier, TLA, et al. Keratoconus at a molecular level: A Review. The Anatomical Record (2020) 303: 1680-88., citing Snibson,GR. Collagen cross-linking: a new treatment paradigm in corneal disease - a review. Clin. Experiment Ophthalmol. (2010) 38: 141-53]. The cross-linking treatment exposes photosensitizing riboflavin drops in the stroma to UV radiation, forming molecular links between the collagen fibrils existing matrix in the cornea [Id., citing Wollensak, G. et al. Riboflavin/ultraviolet-A-induced collagen crosslinking for the treatment of kertoconus. Am. J. Ophthalmol. (2003) 135: 620-7]. The effects of UV irradiation are multiple: there is a marked increase in crosslinking proteoglycans like lumican, mimecan, and decorin as well as a decrease in MMPs [Id., citing Sharif, R. et al. Collagen cross-linking impact on keratoconus extracellular matrix. PLoS One (2018) 13: e0200704]. Pre-treating patient corneas with UV has been shown to regress lymphatic and blood vessels, reducing subsequent graft rejection [Id., citing Hou, Y. et al. UV light crosslinking regresses mature corneal blood and lymphatic vessels and promotes subsequent high-risk corneal transplant survival. Am J. Transplant. (2018) 18: 2873-84].

In cases of advanced keratoconus where contact lenses no longer fit or where scar tissue occupies a significant portion of the cornea affecting the visual axis, a corneal transplant also known as keratoplasty (i.e., penetrating or deep anterior lamellar) would be recommended [Id., citing Jhanji, V. et al. Management of keratoconus: current scenario. Br. J. Ophthalmol. (2011) 95: 1044-50]. Penetrating keratoplasty saw 70% of patients develop keratometric astigmatism in the 10 years that followed suture removal with most cases occurring after 7 years [Id., citing De Toledo, JA et al. Long-term progression of astigmatism after penetrating keratoplasty for keratoconus: evidence of late recurrence. Cornea (2003) 22: 317-23]. The recurrence of keratoconus following deep anterior lamellar keratoplasty has not yet been fully investigated, although a case has been reported where signs of keratoconus reemerged 49 months after surgery [Id., citing Feizi, S. et al. Recurrent keratoconus in a corneal graft after deep anterior lamellar keratoplasty. (2012) J. Ophthalmic Vis. Res. (2012) 7: 328-31].

**Post-LASIK ectasia Interventions**

Treatments for post-LASIK ectasia are the same as for keratoconus. Treating with contact lenses is most common; penetrating keratoplasty (PK) is the treatment of last resort. There are surgical alternatives to conventional penetrating keratoplasty, including anterior lamellar keratoplasty, which enables targeted replacement or augmentation of corneal stroma, without replacement of endothelium.[Tan, DTH, Por, Y-M. Current treatment options for corneal ectasia. Curr. Opin. Ophthalmol. (2007) 18 (4): 284-89]

Corneal cross-linking (CXL) is designed to stabilize and strengthen the cornea through a minimally invasive approach consisting of UV light, eye drops, and brushing off the surface of the cornea.

Intrastromal corneal ring segments (INTACS) are implants placed outside of the cornea to flatten the corneal curvature [Riau, AK et al. Femtosecond laser-assisted stromal keratophakia for keratoconus: a systemic review and meta-analysis. Int. Ophthalmol. (2021) doi.org/10.1007/s10792-021091745-w, citing Pinero, DP, Alio, JL. Intracorneal ring segments in ectatic corneal disease- a review. Clin. Experiment. Ophthalmol. (2010) 38: 154-67]. The implants are placed at two-thirds depth around the cornea, after making a small incision in the stroma. After INTACS, patients with keratoconus will be able to tolerate contact lenses once again, and it is an alternative to corneal transplantation. Typically acrylic-based INTACS are not an ideal treatment option due to their poor biointegration within the cornea, which can lead to clinical complications, such as corneal melting, device protrusion, and inflammation [Riau, AK et al. Femtosecond laser-assisted stromal keratophakia for keratoconus: a systemic review and meta-analysis. Int. Ophthalmol. (2021) doi.org/10.1007/s10792-021091745-w, citing Riau, AK, et al. Surface modification of corneal prosthesis with nano-hydroxyapatite to enhance in vivo biointegration. Acta Biomater. (2020) 107: 299-312; Coskunseven, E. et al. Complications of intrastromal corneal ring segment implantation using a femtosecond laser for channel creation: a survey of 850 eyes with keratoconus. Acta Ophthalmol. (2011) 89: 54-57; K anellopoulos, AJ et al. Modified intracorneal ring segment implantations (INTACS) for the management of moderate to advanced keratoconus: efficacy and complications. Cornea (2006) 25: 29-33].

In moderate and severe cases, corneal transplants are a suitable option. It consists of replacing the cornea, but preserving Descemet membrane. The surgical technique (DALK corneal transplant, also known as deep anterior lamellar keratoplasty) is appropriate in cases of corneal scarring, advanced keratoconus, corneal ectasia after LASIK procedures, corneal degeneration and dystrophy. DALK features a lower rejection rate compared to other procedures, with a rapid stabilization of the patient and healing of the wound. It also features quicker return to daily activities, including physical activity. Topical steroids can be prescribed after DALK, but only for a very short time to allow it to heal faster and reduce the risk of cataracts and glaucoma.

Stromal keratophakia or tissue additive keratoplasty has undergone a revival due to advancements in femtosecond laser technologies, particularly in the laser's capability to optically sculpt frozen donor corneal tissue to a desired geometry before the shaped corneal tissue is inserted into a pocket or flap of the cornea of a patient and its capability to create a precise intrastromal pocket [Riau, AK et al. Femptosecond laser-assisted stromal keratophakia for keratoconus: A systemic review and meta-analysis. Int. Ophthalmol. (2020). Doi.org/10.1007/s10792-021-0175-w]. . Various clinical trials have been conducted to show the clinical application of the modern iteration of stromal keratophakia in managing presbyopia [Id., citing Jacob, S. et al. Preliminary evidence of successful near vision enhancement with a new technique: Presbyopic allogeneic Refractive Lenticule (PEARL) corneal inlay using a SMILE lenticule. J. Refract. Surg. (2017) 33: 224-29] and hyperopia [Id., citing Ganesh, S. et al. Cryopreservation of extracted corneal lenticules after small incision lenticule extraction for potential use in human subjects. Cornea (2014) 33: 1355-62; Pradhan, KR et al. Femtosecond laser-assisted keyhole endokeratophakia: correction of hyperopia by implantation of an allogeneic lenticule obtained by SMILE from a myopic donor. J. Refract. Surg. (2013) 29: 777-82].

**Corneal Inlay Structure and Function**

A corneal inlay is an implant that is surgically inserted within the cornea beneath a portion of corneal tissue to correct a decrease in near vision in people with presbyopia. These tiny devices increase the depth of focus or the refractive power of the central or paracentral cornea. Corneal inlays do not restore the ability to accommodate. Corneal inlays can be positioned by, for example, cutting a flap in the cornea and positioning the inlay beneath the flap. The corneal flap is created by making an incision in the corneal tissue and separating the corneal tissue from the underlying stroma, with one segment remaining attached, which acts like a hinge. The corneal inlay can also be positioned within a pocket (meaning a sac-like cavity) formed in the cornea. Corneal inlays can alter the refractive power of the cornea by changing the shape of the anterior surface of the cornea, by creating an optical interface between the cornea and an implant by having an index of refraction; different from that of the cornea (i.e., has intrinsic power), or both. The cornea is the strongest refracting optical element in the eye, and altering the shape of the anterior surface of the cornea can therefore be a particularly useful method for correcting vision impairments caused by refractive errors.

**Corneal Inlay Procedures**

Regardless of the vision correction procedure and/or devices implanted, it is important to understand the cornea's natural response to the procedure to understand how the cornea will attempt to reduce or minimize the impact of the vision correction procedure.

In a simple biomechanical model proposed by Watsky et al., Investigative Ophthalmology and Visual Science, vol. 26, pp. 240-243 (1985) ("Watsky model"), the anterior corneal surface radius of curvature is assumed to be equal to the thickness of the lamellar corneal material (i.e., flap) between the anterior corneal surface and the anterior surface of a corneal inlay plus the radius of curvature of the anterior surface of the inlay. Reviews of clinical outcomes for implanted inlays or methods for design generally discuss relatively thick inlays (e.g., greater than 200 microns thick) for which the Watsky simple biomechanical response model has some validity, because the physical size of the inlay dominates the biomechanical response of the cornea and dictates the primary anterior surface change.

When an inlay is relatively small and thin, however, the material properties of the cornea contribute significantly to the resulting change in the anterior corneal surface. Petroll et al. reported that implantation of inlays induced a thinning of the central corneal epithelium overlying the inlay. (Petroll, et al., "Confocal assessment of the corneal response to intracorneal lens insertion and laser in situ keratomileusis with flap creation using IntraLase," J. Cataract Refract. Surg., vol. 32, pp 1119-1128 (July 2006)).

Huang et al. reported central epithelial thickening after myopic ablation procedures and peripheral epithelial thickening and central epithelial thinning after hyperopic ablation procedures. (Huang, et al., "Mathematical Model of Corneal Surface Smoothing After Laser Refractive Surgery," America Journal of Ophthalmology, March 2003, pp 267-278). The theory in Huang does not address correcting for presbyopia, nor does it accurately predict changes to the anterior surface, which create a center near portion of the cornea for near vision while allowing distance vision in an area of the cornea peripheral to the center near portion. Additionally, Huang reports on removing cornea tissue by ablation as opposed to adding material to the cornea, such as an intracorneal inlay.

An understanding of the cornea's response to the correction of presbyopia, using, for example, a corneal inlay, allows the response to be compensated for when performing the procedure on the cornea.

**Corneal Onlay Procedures**

A synthetic corneal onlay is an alternative approach to refractive correction that offers an adjustable and reversible procedure with minimal surgical intervention of the central optical zone. [See Evans, MDM et al., Progress in the Development of a Synthetic Corneal Onlay," Investigative Ophthalmology & Visual Sci. (2002) 43 (10): 3196-3201]. In general, a corneal onlay as currently conceptualized requires debridement or removal of the corneal epithelium and placement of a synthetic lenticule on the exposed stromal surface, leaving Bowman's membrane intact.

Attempts to create lenticules for onlay application have largely involved collagen-based materials; however, their success has been limited by remodeling of the lenticule and abnormalities of the corneal epithelium covering the lenticule. [Id., citing Lass, JH et al. Epikeratoplasty: the surgical correction of aphakia, myopia and keratoconus. Ophthalmology (1987) 94: 912-25; Rao, GN et al. Specular microscopy of corneal epithelium after epikeratophakia. Am. J. Ophthalmol. (1987) 103: 392-96; Rodrigues, M. et al. Clinical and histopathologic changes in the host cornea after epikeratophakia for keratoconus. Am. J. Ophthalmol. (1992) 114: 161-170; Thompson, KP et al. Synthetic epikeratoplasty in rhesus monkeys with human type IV collagen. Cornea (1993) 12: 35-45; Kornmehl, EW et al. In vivo evaluation of collagen corneal allograft derived from rabbit dermis. J. Refract. Sur. (1995) 11: 502-6; McCarey, BE. Collagen onlay epikeratoplasty after one year in the monkey model (ARVO Abstract). Invet. Ophthalmol. Vis. Sci. (1997) 40 (4): S511, Abstract No. 2362]. The success of a synthetic material requires that the onlay material be transparent, nontoxic, biocompatible, biostable, and nutrient permeable and have surface characteristics that permit the migration and persistent adhesion of corneal epithelial tissue. [Id., citing Kaufman, H.E. et al., eds. The Cornea. New York: Churchill Livingstone; (1988) 697-712; Leibowitz, HM et al. Progress in the development of a synthetic cornea. Prog. Retinal Eye Res. (1994) 13: 605-21].

There are few published reports of synthetic materials for corneal onlay applications. Lidofilcon A (Allergan, Irvine CA) a high water content contact lens material, failed to epithelialize when tested in vivo. [Id., citing McDonald, MB. The future direction of refractive surgery. J. Refract. Surg. (1988) 4: 158-68]. A plasma-modified poly(vinyl alcohol) material supported epithelial growth when tested in vitro [Id., citing Latkany, R. et al. Plasma surface modification of artificial corneas for optimal epithelialization. J. Biomed. Mater. Res. (1997) 36: 29-37] but only partially epithelialized when implanted in vivo [Id., citing Trinkaus-Randall, V. et al. Implantation of a synthetic cornea: design, development and biological response. Artif. Organs (1997) 21: 1185-91]. A porous perfluoropolyether (PFPE)-based polymer for use as a corneal onlay also has been described. [Id., citing J. Appl. Polym. Sci. (2001) 80: 1756-63].

## Corneal Haze

The idea of using corneal reshaping inlays for presbyopia correction dates back to about 50 years. Early developments used purely synthetic polymer implants which, because of their nature, were associated with stromal necrosis. When it became clear that this was as a result of impermeability of the implants, resulting in the prevention of flow of water and nutrients through the cornea, scientists starting using hydro gels for fabricating corneal inlays. These hydrogels, which were made from synthetic polymers, had high water content (e.g., greater than about 92% by weight) and were permeable to nutrients. In animal studies, these materials showed compatibility with the corneas; however over time, the materials became hazy rendering the implant impracticable. Commercially available products have also shown this phenomenon, whereby the inlay is compatible within the first few months then becomes hazy as time goes on.

Existing corneal implants can therefore lead to the development of a cloudy or opaque appearance of the cornea, which can cause blurry vision or glare by clouding the cornea or by changing the focusing power of the eye. The impact of this corneal haze on a patient's vision is dependent on the severity of the haze and its location in the cornea. Although steroid eye drops are commonly used to treat corneal haze, in cases where the steroid eye drops are ineffective, the corneal implant is commonly removed.

The present disclosure provides methods for treating an ectatic corneal disease by implanting a biocompatible synthetic corneal lenticule effective to support a corneal structure weakened by ectatic corneal disease. According to some embodiments, the corneal lenticule is a corneal inlay device, comprising a biocompatible hydrogel molding with a water content ranging from 78% to 92%, inclusive, which can decrease/eliminate the risk of a patient developing corneal haze, even after long-term implantation without the use of antibiotic and/or steroid regimen.

The present disclosure further provides methods for changing anterior curvature of the cornea in a subject with an ectatic corneal disease by implanting a synthetic corneal lenticule designed to correct visual acuity by increasing depth of focus or the refractive power of the central or paracentral cornea. According to some embodiments, the corneal lenticule is a corneal inlay device, comprising a biocompatible hydrogel molding with a water content ranging from 78% to 92%, inclusive, which can decrease/eliminate the risk of a patient developing corneal haze. According to some embodiments, the corneal lenticule is a synthetic corneal onlay device that offers an adjustable and reversible implantation procedure even in the instance of significant asymmetry with minimal surgical intervention of the central optical zone.

## SUMMARY OF THE INVENTION

In one aspect, the present disclosure provides a method of treating ectatic corneal disease, comprising: implanting in or on a cornea of a mammalian subject a corneal device of water content of between 78%-92%% (w/w), the corneal device comprising a thickness, a diameter, a base surface and a top surface, wherein the corneal device is effective to support a corneal structure weakened by the ectatic corneal disease, and to improve vision of an eye of the mammalian subject.

In some embodiments, the corneal device is a corneal inlay device implanted in the cornea. In some embodiments, the corneal device is implanted on an anterior surface of the cornea. In some embodiments, the base surface defines a convex configuration. In some embodiments, the top surface defines a concave configuration. In some embodiments, the top surface of the corneal device extends along an entire central area of the corneal device.

In some embodiments, the corneal device is a corneal inlay device implanted in the cornea, the corneal device comprises an opening extending through the corneal device to define a ring or donut shape. In some embodiments, the corneal device is a corneal inlay device implanted in the cornea, the corneal device comprises a convex section in the top surface. In some embodiments, the corneal device is a corneal inlay device implanted in the cornea, the implanting of the corneal device is by cutting a flap in the cornea and positioning the corneal device beneath the flap. In some embodiments, the corneal device is a corneal inlay device implanted in the cornea, the implanting of the corneal device is by positioning the corneal device within a pocket formed in the cornea. In some embodiments, the implanting of the corneal device is in the cornea at a depth of about 100 microns to about 250 microns, inclusive. In some embodiments, the corneal device is a corneal inlay device implanted in the cornea, the implanting of the corneal device is in the cornea at a depth of about 130 microns to about 160 microns, inclusive. In some embodiments, the corneal device is a corneal inlay device implanted in the cornea, the thickness of the corneal device ranges from at least 11.5 microns to about 290 microns, inclusive. In some embodiments, the corneal device is a corneal inlay device implanted in the cornea, a diameter of the corneal device is from about 5.5 mm to about 7.5 mm, inclusive. In some embodiments, the corneal device is a corneal inlay device implanted in the cornea, the corneal device is molded from a hydrogel. In some embodiments, the corneal device is a corneal inlay device implanted in the cornea, the corneal device comprises water, a natural polymer, and two synthetic polymers, the two synthetic polymers forming an interpenetrating polymer network, wherein the synthetic polymers and the natural polymer are at least partially interlaced on a molecular scale but not covalently bonded to each other and cannot be separated. In some embodiments, the corneal device is a corneal inlay device implanted in the cornea, the natural polymer is a collagen. In some embodiments, the corneal device is a corneal inlay device implanted in the cornea, the collagen is a porcine collagen. In some embodiments, the corneal device is a corneal inlay device implanted in the cornea, a hydrogel for fabricating the corneal device comprise at least 1%, at least 2%, at least 3%, at least 4%, or at least 5% by weight of the natural polymer. In some embodiments, the corneal device is a corneal inlay device implanted in the cornea, one of the two synthetic polymers is poly(2-methacryloyloxyethyl phosphorylcholine) (MPC).

In some embodiments, the corneal device is a corneal inlay device implanted in the cornea, one of the two synthetic polymers is Poly(ethylene glycol) diacrylate (PEGDA). In some embodiments, the corneal device is a corneal inlay device implanted in the cornea, the water content of the corneal device ranges from about 78% to about 90% inclusive. In some embodiments, the corneal device is a corneal inlay device implanted in the cornea, the water content of the corneal device ranges from about 78% to about 88%, inclusive. In some embodiments, the corneal device is a corneal inlay device implanted in the cornea, the water content of the corneal device ranges from about 78% to about 84%, inclusive. In some embodiments, the corneal device is a corneal inlay device implanted in the cornea, the corneal device is optically transparent, biocompatible, permeable and refractive. In some embodiments, the corneal device is a corneal inlay device implanted in the cornea, the ectatic corneal disease is naturally occurring or derived from refractive surgery. In some embodiments, the corneal device is a corneal inlay device implanted in the cornea, the naturally occurring ectatic corneal disease is one or more of keratoconus, forme fruste keratoconus; keratoglobus, or pellucid marginal degeneration. In some embodiments, the corneal device is a corneal inlay device implanted in the cornea, the ectatic corneal disease derived from refractive surgery is derived from LASIK, small incision lenticule extraction (SMILE), or photorefractive keratectomy (PRK).

In another aspect, the present disclosure provides a method of treating ectatic corneal disease, comprising: implanting in or on a cornea of a mammalian subject a corneal device of water content of between 78%-92%% (w/w), the corneal device comprising a thickness, a diameter, a base surface and a top surface, wherein the corneal device is effective to change an anterior curvature of the cornea to improve vision of an eye of the mammalian subject. In some embodiments, the ectatic corneal disease is naturally occurring or derived from refractive surgery. In some embodiments, the naturally occurring ectatic corneal disease is one or more of keratoconus, forme fruste keratoconus; keratoglobus, or pellucid marginal degeneration. In some embodiments, the ectatic corneal disease derived from refractive surgery is derived from LASIK, small incision lenticule extraction (SMILE), or photorefractive keratectomy (PRK).

In another aspect, the present disclosure provides a use of a corneal device with low water content to correct vision of a mammalian subject with corneal structure weakened by ectatic corneal disease, the corneal device comprising a thickness, a diameter, a base surface, and a top surface, wherein the corneal device when placed in or on the cornea is effective to support the corneal structure weakened by the ectatic corneal disease or change an anterior curvature of the cornea to improve vision of the eye of the mammalian subject.

In another aspect, the present disclosure provides a corneal device for treating ectatic corneal disease, comprising: a body defining a thickness, a diameter, a base surface, and a top surface, wherein the corneal device is fabricated from a hydrogel composition comprising an interpenetrating polymer network of two polymers at least partially interlaced on a molecular scale but not covalently bonded to each other that cannot be separated, comprising a water content ranging from 78%-92%, inclusive. In some embodiments, the ectatic corneal disease is naturally occurring or derived from refractive surgery. In some embodiments, the naturally occurring ectatic corneal disease is one or more of keratoconus, forme fruste keratoconus; keratoglobus, or pellucid marginal degeneration. In some embodiments, the ectatic corneal disease derived from refractive surgery is derived from LASIK, small incision lenticule extraction (SMILE), or photorefractive keratectomy (PRK).

These and other advantages of the invention will be apparent to those of ordinary skill in the art by reference to the following detailed description and the accompanying drawings.

In the various views of the drawings, like reference characters designate like or similar parts.

## BRIEF DESCRIPTION OF THE DRAWINGS

FIG. 1 shows an illustrative view of the human eye (from Allaboutvision.com/resources/anatomy.htm, Accessed March 2019);

FIG. 2 shows an illustrative view of the five layers of the cornea;

FIG. 3 shows an illustrative view of the effects of presbyopia on the human eye;

FIG. 4 shows an illustrative view of the effects of keratoconus on the human eye;

**FIG. 5** shows an illustrative embodiment of the corneal inlay device of the present disclosure;

**FIG. 6** is a diagram showing the corneal inlay of the present disclosure implanted in a cornea;

**FIG. 7** shows an example of how a corneal inlay can provide near vision to a subject's eye while retaining some distance vision according to an embodiment of the present disclosure;

**FIG. 8** is a graph showing a change in anterior corneal surface height and the corresponding induced added power.

**FIG. 9** is a diagram showing a preoperative optical coherence tomography ("OCT") and a postoperative OCT including an example location for the corneal inlay of the present disclosure;

**FIG. 10** shows an illustrative embodiment of the corneal onlay device of the present disclosure;

**FIG. 11** is a diagram showing the corneal onlay of the present disclosure implanted under an epithelium layer of a cornea;

**FIG. 12** is a perspective view of an exemplary mold assembly for fabricating an exemplary hydrogel inlay in accordance with the present disclosure;

**FIG. 13** is a side view of the exemplary mold assembly of **FIG. 12;**

**FIG. 14** is a cross-sectional view of the exemplary mold assembly of **FIG. 12;**

**FIG. 15** is a detailed view of the exemplary mold assembly of **FIG. 14** showing a cavity formed between a first and second mold section;

**FIG. 16** is a detailed view of the exemplary mold assembly of **FIG. 15;**

**FIG. 17** is a perspective view of a first mold section of the exemplary mold assembly of **FIG. 12;**

**FIG. 18** is a top view of a first mold section of **FIG. 17;**

**FIG. 19** is a side view of a first mold section of **FIG. 17;**

**FIG. 20** is a cross-sectional view of a first mold section of **FIG. 19;**

**FIG. 21** is a detailed view of a first mold section of **FIG. 20** showing a cavity formed in a top surface of the first mold section;

**FIG. 22** is a perspective view of a second mold section of an exemplary mold assembly of **FIG. 12;**

**FIG. 23** is a top view of a second mold section of **FIG. 22;**

**FIG. 24** is a side view of a second mold section of **FIG. 22;**

**FIG. 25** is a cross-sectional view of a second mold section of **FIG. 24;**

**FIG. 26** is a detailed view of a second mold section of **FIG. 25;**

**FIG. 27** is a top view of an exemplary hydrogel inlay in accordance with the present disclosure.

**FIG. 28** is a side view of the exemplary hydrogel inlay of **FIG. 27;**

**FIG. 29** is a cross-sectional view of the exemplary hydrogel inlay of **FIG. 27;**

**FIG. 30** is a detailed cross-sectional view of the exemplary hydrogel inlay of **FIG. 29;**

FIG. 31 is a side view of an exemplary hydrogel meniscus inlay in accordance with the present disclosure;

FIG. 32 is a cross-sectional view of the exemplary hydrogel inlay of **FIG. 31**

FIG. 33 is a detailed cross-sectional view of the exemplary hydrogel inlay of **FIG. 32;**

FIG. 34 is an image of cell coverage on a biocompatible material after seven days;

FIG. 35 is an image of cell coverage on a non-biocompatible material after seven days;

FIGS. 36A, 36B, 36C, 36D, and 36E illustrate guideline haze grading schemes for corneal haze scoring;

FIG. 37 is a bar graph showing thickness for different samples tested in the cell attachment assay at day 4;

FIG. 38 is a bar graph showing thickness for different samples tested in the cell attachment assay at day 7;

FIG. 39 is a bar graph showing thickness over time for different samples tested in the cell attachment assay;

FIGS. 40A, 40B, 40C, 40D, 40E, 40F, and 40G are microscopy images for different samples tested in the cell attachment assay at day 4, with **FIG. 40A** showing a control, **FIG. 40B** showing Nippi 10%, **FIG. 40C** showing Nippi 12%, **FIG. 40D** showing Nippi 15%, **FIG. 40E** showing Nippon 10%, **FIG. 40F** showing Ferentis 1823B, and **FIG. 40G** showing Ferentis 1837A;

FIGS. 41A, 41B, 41C, 41D, 41E, 41F, and 41G are microscopy images for different samples tested in the cell attachment assay at day 7, with **FIG. 41A** showing a control, **FIG. 41B** showing Nippi 10%, **FIG. 41C** showing Nippi 12%, **FIG. 41D** showing Nippi 15%, **FIG. 41E** showing Nippon 10%, **FIG. 41F** showing Ferentis 1823B, and **FIG. 41G** showing Ferentis 1837A;

FIG. 42 is a diagram illustrating placement of materials then seeded with cells during a cell attachment assay;

FIG. 43 is a bar graph showing thickness over time for different samples tested in the cell attachment assay;

FIGS. 44A, 44B, 44C, 44D, 44E, 44F, 44G, 44H, and 44I are microscopy images for different samples tested in the cell attachment assay at day 4, with **FIG. 44A** showing a control, **FIG. 44B** showing Ferentis 1842A, **FIG. 44C** showing Nippi 12%D12%, **FIG. 44D** showing Nippi 10%D10%, **FIG. 44E** showing Nippi 12%D10%, **FIG. 44F** showing Nippon 10%, **FIG. 44G** showing SA-13-31B, **FIG. 44H** showing SA-13-92A edge, and **FIG. 44I** showing SA-13-92A on sample;

FIGS. 45A, 45B, 45C, 45D, 45E, 45F, 45G, 45H, and 45I are microscopy images for different samples tested in the cell attachment assay at day 7, with **FIG. 45A** showing a control, **FIG. 45B** showing Ferentis 1842A, **FIG. 45C** showing Nippi 12%D12%, **FIG. 45D** showing Nippi 10%D10%, **FIG. 45E** showing Nippi 12%D10%, **FIG. 45F** showing Nippon 10%, **FIG. 45G** showing SA-13-31B, **FIG. 45H** showing SA-13-92A edge, and **FIG. 45I** showing SA-13-92A on sample;

FIG. 46 is a schematic diagram illustrating an MTT (3-(4,5-dimethylthiazol-2-yl)-2,5-diphenyltetrazolium bromide) assay;

FIGS. 47A, 47B, 47C, 47D, 47E, and 47F are microscopy images for control samples tested in the cell attachment assay, with each of **FIGS. 47A-47F** showing control samples and, in particular, **FIGS. 47A-47D** showing control sample images for 4/6 samples, 80-100% confluent, and **FIGS. 47E-47F** showing control sample images for 2/6 samples mostly confluent, and a few patches in center;

FIGS. 48A, 48B, 48C, 48D, 48E, 48F, 48G, 48H, 48I, and 48J are microscopy images for 1745A samples tested in the cell attachment assay, with **FIGS. 48A-48C** showing 1745A sample images for 3/10 confluent at edges and nearly confluent in center, **FIGS. 48D-48E** showing 1745A sample images for 2/10 60-70% confluent in center, confluent at edges, and **FIGS. 48F-48J** showing 1745A sample images for 5/10 samples 30-40% confluent in center, patchy, some holes;

**FIG. 49** is an image of an MTT plate illustrating the setup for samples tested in the cell attachment assay;

**FIG. 50** is a bar graph showing cell numbers for MTT results in the cell attachment assay for a sample and control;

**FIG. 51** is a diagram of seeding materials during a cell attachment assay;

**FIGS. 52A and 52B** are light microscopy images of PC-MPC cells attached to a sample;

**FIG. 53** is a confocal microscopy image of a cross-section of PC-MPC material following incubation with epithelial cells; there are few multi-layer spots on top of the material;

**FIGS. 54A and 54B** are confocal microscopy images of a cross-section of controls showing some multilayered structures as a baseline for comparison;

**FIGS. 55A and 55B** are confocal microscopy images of a cross-section of 3D PEG gelatin material with epithelial cells on a plate and on surface demonstrating the ability of cells to form cell layers; both have multi-layered spots;

**FIG. 56** is a perspective view of an exemplary mold assembly for fabricating an exemplary hydrogel inlay having a disc-shaped configuration in accordance with the present disclosure;

**FIG. 57** is a side view of the exemplary mold assembly of **FIG. 56;**

**FIG. 58** is a cross-sectional view of the exemplary mold assembly of **FIG. 56;**

**FIG. 59** is a perspective view of a first mold section of the exemplary mold assembly of **FIG. 56;**

**FIG. 60** is a top view of a first mold section of **FIG. 59;**

**FIG. 61** is a side view of a first mold section of **FIG. 59;**

**FIG. 62** is a cross-sectional view of a first mold section of **FIG. 59;**

**FIG. 63** is a detailed view of a first mold section of **FIG. 62** showing a cavity formed in a top surface of the first mold section;

**FIG. 64** is a perspective view of a second mold section of an exemplary mold assembly of **FIG. 56;**

**FIG. 65** is a top view of a second mold section of **FIG. 64;**

**FIG. 66** is a side view of a second mold section of **FIG. 64;**

**FIG. 67** is a cross-sectional view of a second mold section of **FIG. 64;**

**FIG. 68** is a detailed view of a second mold section of **FIG. 67;**

**FIG. 69A, 69B and 69C** are diagrammatic top and cross-sectional views of inlay and onlay configurations for treatment of keratoconus;

**FIG. 70** is a top view of an exemplary hydrogel corneal implant defining a concave shape for treatment of keratoconus;

**FIG. 71** is a side view of the exemplary hydrogel corneal implant of **FIG. 70;**

**FIG. 72** is a cross-sectional view of the exemplary hydrogel corneal implant of **FIG. 70;**

**FIG. 73** is a top view of an exemplary hydrogel corneal implant defining a donut or ring shape for treatment of keratoconus;

**FIG. 74** is a side view of the exemplary hydrogel corneal implant of **FIG. 73;**

**FIG. 75** is a cross-sectional view of the exemplary hydrogel corneal implant of **FIG. 73;**

**FIG. 76** is a top view of an exemplary hydrogel corneal implant defining a convex shape for treatment of keratoconus;

**FIG. 77** is a side view of the exemplary hydrogel corneal implant of **FIG. 76;** and

**FIG. 78** is a cross-sectional view of the exemplary hydrogel corneal implant of FIG. 76;

## DETAILED DESCRIPTION OF THE INVENTION

### Glossary

The term "about" or "approximately" as used herein when referring to a measurable value such as an amount, a temporal duration, and the like, is meant to encompass variations of $\pm 20\%$, $\pm 10\%$, $\pm 5\%$, $\pm 1\%$, $\pm 0.9\%$, $\pm 0.8\%$, $\pm 0.7\%$, $\pm 0.6\%$, $\pm 0.5\%$, $\pm 0.4\%$, $\pm 0.3\%$, $\pm 0.2\%$ or $\pm 0.1\%$ from the specified value, as such variations are appropriate to perform the disclosed methods.

The term "adaptive immune response" refers to an immune response mediated by uniquely specific recognition or a non-self entity by lymphocytes whose activation leads to elimination of the entity and the production of specific memory lymphocytes. Because these memory lymphocytes forestall disease in subsequent attacks by the same pathogen, the host immune system is said to have "adapted" to copy with the entity.

The term "adhere" and its other grammatical forms as used herein means to stick fast to a surface or substance.

### Anatomical terms

When referring to animals that typically have one end with a head and mouth, with the opposite end often having the anus and tail, the head end is referred to as the cranial end, while the tail end is referred to as the caudal end. Within the head itself, rostral refers to the direction toward the end of the nose, and caudal is used to refer to the tail direction. The surface or side of an animal's body that is normally oriented upwards, away from the pull of gravity, is the dorsal side; the opposite side, typically the one closest to the ground when walking on all legs, swimming or flying, is the ventral side. On the limbs or other appendages, a point closer to the main body is "proximal"; a point farther away is "distal". Three basic reference planes are used in zoological anatomy. A "sagittal" plane divides the body into left and right portions. The "midsagittal" plane is in the midline, i.e. it would pass through midline structures such as the spine, and all other sagittal planes are parallel to it. A "coronal" plane divides the body into dorsal and ventral portions. A "transverse" plane divides the body into cranial and caudal portions.

When referring to humans, the body and its parts are always described using the assumption that the body is standing upright. Portions of the body which are closer to the head end are "superior" (corresponding to cranial in animals), while those farther away are "inferior" (corresponding to caudal in animals). Objects near the front of the body are referred to as "anterior" (corresponding to ventral in animals); those near the rear of the body are referred to as "posterior" (corresponding to dorsal in animals). A transverse, axial, or horizontal plane is an X-Y plane, parallel to the ground, which separates the superior/head from the inferior/feet. A coronal or frontal plane is an Y-Z plane, perpendicular to the ground, which separates the anterior from the posterior. A sagittal plane is an X-Z plane, perpendicular to the ground and to the coronal plane, which separates left from right. The midsagittal plane is the specific sagittal plane that is exactly in the middle of the body.

Structures near the midline are called medial and those near the sides of animals are called lateral. Therefore, medial structures are closer to the midsagittal plane, lateral structures are further from the midsagittal plane. Structures in the midline of the body are median. For example, the tip of a human subject's nose is in the median line.

The term "ipsilateral" as used herein means on the same side, the term "contralateral" as used herein means on the other side, and the term "bilateral" as used herein means on both sides. Structures that are close to the center of the body are proximal or central, while ones more distant are distal or peripheral. For example, the hands are at the distal end of the arms, while the shoulders are at the proximal ends.

The term "biocompatible" as used herein, means causing no clinically relevant tissue irritation, injury, toxic reaction, or immunologic reaction to human tissue based on a clinical risk/benefit assessment.

The term "collagen" as used herein refers to a natural, chemically synthesized, or synthetic protein rich in glycine and proline that *in vivo* is a major component of the extracellular matrix and connective tissues.

The term "corneal apex" as used herein refers to the point of maximum curvature.

The term "corneal inlay" as used herein refers to an implantable device that is embedded within the corneal stroma.

The term "corneal lenticule" as used herein refer to a disc-shaped piece of corneal tissue or a piece of synthetic material manufactured to produce a given curvature and thickness that is implanted into or on top of the cornea to change its anterior curvature.

The term "corneal onlay" as used herein refers to an implantable device placed onto the corneal surface.

The term "corneal vertex" as used herein refers to the point located at the intersection of an individual's line of fixation and the corneal surface.

The term "curvature" as used herein refers to a degree of curving of a continuously bending line, without angles.

The term "cytokine" as used herein refers to small soluble protein substances secreted by cells which have a variety of effects on other cells. Cytokines mediate many important physiological functions including growth, development, wound healing, and the immune response. They act by binding to their cell-specific receptors located in the cell membrane, which allows a distinct signal transduction cascade to start in the cell, which eventually will lead to biochemical and phenotypic changes in target cells. Generally, cytokines act locally. They include type I cytokines, which encompass many of the interleukins, as well as several hematopoietic growth factors; type II cytokines, including the interferons and interleukin-10; tumor necrosis factor ("TNF")-related molecules, including TNF$\alpha$ and lymphotoxin; immunoglobulin super-family members, including interleukin 1 ("IL-1"); and the chemokines, a family of molecules that play a critical role in a wide variety of immune and inflammatory functions. The same cytokine can have different effects on a cell depending on the state of the cell. Cytokines often regulate the expression of, and trigger cascades of, other cytokines.

The term "demolding" as used herein refers to a process of removing a mold from a model or a casting from a mold. The process can be, for example, by mechanical means, by hand, by the use of compressed air, etc.

The term "diopter" as used herein refers to a unit of measurement of the refractive power of a lens equal to the reciprocal of the focal length in meters.

The term "elasticity" as used herein refers to a measure of the deformation of an object when a force is applied. Objects that are very elastic like rubber have high elasticity and stretch easily.

The term "extracellular matrix" (or ECM) as used herein refers to a complex network of polysaccharides and proteins secreted by cells that serves as a structural element in tissues and also influences their development and physiology. It is composed of an interlocking mesh of fibrous proteins and glycosaminoglycans (GAGs). Examples of fibrous proteins found in the extracellular matrix include collagen, elastin, fribronectin, and laminin. Examples of GAGs found in the extracellular matrix include proteoglycans (e.g., heparin sulfate), chondroitin sulfate, keratin sulfate, and non-proteoglycan polysaccharide (e.g., hyaluronic acid). The term "proteoglycan" refers to a group of glycoproteins that contain a core protein to which is attached one or more glycosaminoglycans. The extracellular matrix serves many functions, including, but not limited to, providing support and anchorage for cells, segregating one tissue from another tissue, and regulating intracellular communication.

The term "fibroblast" as used herein refers to a common cell type in connective tissue that secretes an extracellular matrix rich in collagen and other extracellular matrix macromolecules and that migrates and proliferates readily in wounded tissue and in tissue culture.

The term "fixation" or "visual fixation" as used herein refers to an optic skill that allows one to sustain gaze at a stationary object.

The term "focal length" of a lens as used herein refers to the distance at which a lens focuses parallel rays of light. Given its diopter, the focal length of a lens can be calculated from the equation: focal length in mm = 1000/diopter.

The term "gently" and its various grammatical forms as used herein refers to mildly, not harsh or rough.

The term "growth" as used herein refers to a process of becoming larger, longer or more numerous, or an increase in size, number, or volume of cells in a cell population.

The term "growth factor" as used herein refers to an extracellular polypeptide signal molecule that can stimulate a cell to grow or proliferate. Nonlimiting examples include epidermal growth factor (EGF) and platelet-derived growth factor (PDGF). Most growth factors also have other actions.

The term "hydrogel" as used herein refers to a substance resulting in a solid, semisolid, pseudoplastic, or plastic structure containing a necessary aqueous component to produce a gelatinous or jelly-like mass.

The term "hydrophilic" as used herein refers to a material or substance having an affinity for polar substances, such as water.

The terms "immune response" and "immune mediated" are used interchangeably herein to refer to any functional expression of a subject's immune system, against either foreign or self-antigens, whether the consequences of these reactions are beneficial or harmful to the subject.

The term "immune system" as used herein refers to a complex arrangement of cells and molecules that maintain immune homeostasis to preserve the integrity of the organism by elimination of all elements judged to be dangerous. Responses in the immune system may generally be divided into two arms, referred to as "innate immunity" and "adaptive immunity." The two arms of immunity do not operate independently of each other, but rather work together to elicit effective immune responses.

The term "implant" as used herein refers to material inserted or grafted into a tissue.

The term "index of refraction" as used herein refers to a measure of the extent to which a substance/medium slows down light waves passing through it. Its value determines the extent to which light is refracted (bent) when entering or leaving the substance/medium. It is the ratio of the velocity of light in a vacuum to its speed in a substance or medium.

The terms "innate immunity" or innate immune response" are used interchangeably to refer to a nonspecific fast response to pathogens that is predominantly responsible for an initial inflammatory response via a number of soluble factors, including the complement system and the chemokine/cytokine system; and a number of specialized cell types, including mast cells, macrophages, dendritic cells (DCs), and natural killer cells (NKs).

The term "integrins" as used herein refers to the principal receptors used by animal cells to bind to the extracellular matrix. They are heterodimers and function as transmembrane linkers between the extracellular matrix and the actin cytoskeleton. A cell can regulate the adhesive activity of its integrins from within.

The term "interlaced" and its other grammatical forms as used herein refers to a state of being united by intercrossing; of being passed over and under each other; of being weaved together; intertwined; or being connected intricately.

The term "isolated" as used herein refers to material, such as, but not limited to, a nucleic acid, peptide, polypeptide, or protein, which is: (1) substantially or essentially free from components that normally accompany or interact with it as found in its naturally occurring environment. The terms "substantially free" or "essentially free" are used herein to refer to considerably or significantly free of, or more than about 95% free of, more than about 96% free of, more than about 97% free of, more than about 98% free of, or more than about 99% free of. The isolated material optionally comprises material not found with the material in its natural environment; or (2) the material has been synthetically (non-naturally) altered by deliberate human intervention.

The term "matrix" as used herein refers to a three dimensional network of fibers that contains voids (or "pores") where the woven fibers intersect. The structural parameters of the pores, including the pore size, porosity, pore interconnectivity/ tortuosity and surface area, can affect how substances (e.g., fluid, solutes) move in and out of the matrix.

The term "MPC" is an abbreviation for methacryloyloxyethyl phosphorylcholine.

The term "miosis" as used herein means excessive constriction (shrinking) of the pupil. In miosis, the diameter of the pupil is less than 2 millimeters (mm),

The term "myeloid" as used herein means of or pertaining to bone marrow. Granulocytes and monocytes, collectively called myeloid cells, are differentiated descendants from common progenitors derived from hematopoietic stem cells in the bone marrow. Commitment to either lineage of myeloid cells is controlled by distinct transcription factors followed by terminal differentiation in response to specific colony-stimulating factors and release into the circulation. Upon pathogen invasion, myeloid cells are rapidly recruited into local tissues via various chemokine receptors, where they are activated for phagocytosis as well as secretion of inflammatory cytokines, thereby playing major roles in innate immunity. [Kawamoto, H., Minato, N. Intl J. Biochem. Cell Biol. (2004) 36 (8): 1374-9].

The term "myofibroblast" as used herein refers to a differentiated cell type essential for wound healing that participates in tissue remodeling following an insult. Myofibroblasts are typically activated fibroblasts, although they can also be derived from other cell types, including epithelial cells, endothelial cells, and mononuclear cells.

The term "PEGDA" is an abbreviation for poly(ethylene glycol)diacrylate.

The term "permeable" as used herein means permitting the passage of substances, such as oxygen, glucose, water and ions, as through a membrane or other structure.

The term "porosity" as used herein refers to the ratio between the pore volume and the total volume of a material.

The term "peptide" as used herein refers to a molecule of two or more amino acids chemically linked together. A peptide may refer to a polypeptide, protein or peptidomimetic.

The term "peptidomimetic" refers to a small protein-like chain designed to mimic or imitate a peptide. A peptidomimetic may comprise non-peptidic structural elements capable of mimicking (meaning imitating) or antagonizing (meaning neutralizing or counteracting) the biological action(s) of a natural parent peptide.

The terms "polypeptide" and "protein" are used herein in their broadest sense to refer to a sequence of subunit amino acids, amino acid analogs, or peptidomimetics. The subunits are linked by peptide bonds, except where noted. The polypeptides described herein may be chemically synthesized or recombinantly expressed. Polypeptides of the described invention can be chemically synthesized. Synthetic polypeptides, prepared using the well-known techniques of solid phase, liquid phase, or peptide condensation techniques, or any combination thereof, can include natural and unnatural amino acids. Amino acids used for peptide synthesis may be standard Boc (N-$\alpha$-amino protected N-$\alpha$-t-butyloxycarbonyl) amino acid resin with the standard deprotecting, neutralization, coupling and wash protocols of the original solid phase procedure of Merrifield (1963, J. Am. Chem. Soc 85:2149-2154), or the base-labile N-$\alpha$-amino protected 9-fluorenylmethoxycarbonyl (Fmoc) amino acids first described by Carpino and Han (1972, J. Org. Chem. 37:3403-3409). Both Fmoc and Boc N-$\alpha$-amino protected amino acids can be obtained from Sigma, Cambridge Research Biochemical, or other chemical companies familiar to those skilled in the art. In addition, the polypeptides can be synthesized with other N-$\alpha$-protecting groups that are familiar to those skilled in this art. Solid phase peptide synthesis may be accomplished by techniques familiar to those in the art and provided, for example, in Stewart and Young, 1984, Solid Phase Synthesis, Second Edition, Pierce Chemical Co., Rockford, Ill.; Fields and Noble, 1990, Int. J. Pept. Protein Res.

35:161-214, or using automated synthesizers. The polypeptides of the invention may comprise D-amino acids (which are resistant to L-amino acid-specific proteases in vivo), a combination of D- and L-amino acids, and various "designer" amino acids (e.g., β-methyl amino acids, C-α-methyl amino acids, and N-α-methyl amino acids, etc.) to convey special properties. Synthetic amino acids include ornithine for lysine, and norleucine for leucine or isoleucine. In addition, the polypeptides can have peptidomimetic bonds, such as ester bonds, to prepare peptides with novel properties. For example, a peptide may be generated that incorporates a reduced peptide bond, i.e., $R^1\text{-}CH_2\text{-}NH\text{-}R^2$, where $R_1$ and $R_2$ are amino acid residues or sequences. A reduced peptide bond may be introduced as a dipeptide subunit. Such a polypeptide would be resistant to protease activity, and would possess an extended half-live in vivo. Accordingly, these terms also apply to amino acid polymers in which one or more amino acid residue is an artificial chemical analogue of a corresponding naturally occurring amino acid, as well as to naturally occurring amino acid polymers. When incorporated into a protein, that protein is specifically reactive to antibodies elicited to the same protein but consisting entirely of naturally occurring amino acids. The terms "polypeptide", "peptide" and "protein" also are inclusive of modifications including, but not limited to, glycosylation, lipid attachment, sulfation, gamma-carboxylation of glutamic acid residues, hydroxylation and ADP-ribosylation. It will be appreciated, as is well known and as noted above, that polypeptides may not be entirely linear. For instance, polypeptides may be branched as a result of ubiquitination, and they may be circular, with or without branching, generally as a result of posttranslational events, including natural processing event and events brought about by human manipulation which do not occur naturally. Circular, branched and branched circular polypeptides may be synthesized by non-translation natural process and by entirely synthetic methods, as well.

The term "polymer" as used herein refers to any of various chemical compounds made of smaller, identical molecules (called monomers) linked together. Polymers generally have high molecular weights. The incorporation of two different monomers, A and B, into a polymer chain in a statistical fashion leads to copolymers. In the limit, single monomers may alternate regularly in the chain and these are known as alternating copolymers. The monomers can be combined in a more regular fashion, either by linking extended linear sequences of one to linear sequences of the other by end-to-end addition to give block copolymers, or by attaching chains of B at points on the backbone chain of A, forming a branched structure known as a graft copolymer.

The term "proliferate" and its various grammatical forms as used herein means to increase rapidly in numbers; to multiply.

The term "protein" is used herein to refer to a large complex molecule or polypeptide composed of amino acids. The sequence of the amino acids in the protein is determined by the sequence of the bases in the nucleic acid sequence that encodes it.

The term "range" and its various grammatical forms as used herein refers to varying between the stated limits and includes the stated limits and all points or values in between.

The term "recombinant DNA" refers to a DNA molecule formed by laboratory methods whereby DNA segments from different sources are joined to produce a new genetic combination.

The term "recombinant protein" as used herein refers to a protein encoded by recombinant DNA that has been cloned in a system that supports expression of the gene and translation of messenger RNA within a living cell. To make a human recombinant protein, for example, a gene of interest is isolated, cloned into an expression vector, and expressed in an expression system. Exemplary expression systems include prokaryotic organisms, as bacteria, and eukaryotic organisms, such as yeast, insect cells, plants, and mammalian cells in culture.

The term "refraction" as used herein refers to the deflection of a ray of light when it passes from one medium into another of different optical density. A denser medium provides more matter from which the light can scatter, so light will travel more slowly in a dense medium. A slower speed means a higher index of refraction. Light travels much faster in rarer medium (meaning a less dense medium, for example: air). In passing from a denser into a rarer medium the light is deflected away from a line perpendicular to the surface of the refracting medium. In passing from a rarer to a denser medium, it is bent towards this perpendicular line. The term "refraction" also refers to the act of determining the nature and degree of the refractive errors in the eye and correction of the same.

"Refractive power" of a lens as used herein refers to the reciprocal of its focal length in meters, or $D=1/f$, where D is the power in diopters and f is the focal length in meters.

The term "RGD motif' as used herein refers to arginylglycylaspartic acid, the binding motif of fibronectin to cell adhesion molecules, which can serve as a cell adhesion site of extracellular matrix, cell surface proteins, and integrins.

The term "shape" as used herein refers to the quality of a distinct object or body in having an external surface or outline of specific form or figure.

The term "sign" as used herein refers to a healthcare provider's evidence of disease.

The term "slit lamp" as used herein refers to a lamp for projecting a narrow beam of intense light that is used in conjunction with a biomicroscope for examining the anterior parts (as the conjunctiva or cornea) of an eye.

The terms "subject" or "individual" or "patient" are used interchangeably to refer to a member of an animal species of mammalian origin, including but not limited to, mouse, rat, cat, goat, sheep, horse, hamster, ferret, pig, dog, guinea pig, rabbit and a primate, such as, for example, a monkey, ape, or human.

The term "symptom" as used herein refers to a patient's subjective evidence of disease.

The term "thickness" as used herein refers to a measure between opposite surfaces, from top to bottom, or in a direction perpendicular to that of the length and breadth.

The term "tolerance limits" as used herein refers to the end points of a tolerance interval.

The term "transactivation" as used herein refers to stimulating transcription of a gene in a host cell by binding to DNA. Genes can be transactivated naturally (e.g., by a virus or a cellular protein) or artificially.

The term "viscosity", as used herein refers to the property of a fluid that resists the force tending to cause the fluid to flow. Viscosity is a measure of the fluid's resistance to flow. The resistance is caused by intermolecular friction exerted when layers of fluids attempt to slide by one another. Viscosity can be of two types: dynamic (or absolute) viscosity and kinematic viscosity. Absolute viscosity or the coefficient of absolute viscosity is a measure of the internal resistance. Dynamic (or absolute) viscosity is the tangential force per unit area required to move one horizontal plane with respect to the other at unit velocity when maintained a unit distance apart by the fluid. Dynamic viscosity is usually denoted in poise (P) or centipoise (cP), wherein 1 poise = 1 $g/cm^2$, and 1 cP = 0.01 P. Kinematic viscosity is the ratio of absolute or dynamic viscosity to density. Kinematic viscosity is usually denoted in Stokes (St) or Centistokes (cSt), wherein 1 St = $10-4$ $m^2/s$, and 1 cSt = 0.01 St.

The term "wt %" or "weight percent" or "percent by weight" or "wt/wt%" or w/w of a component, unless specifically stated to the contrary, refers to the ratio of the weight of the component to the total weight of the composition in which the component is included, expressed as a percentage.

The term "Young's modulus" as used herein refers to a measure of elasticity, equal to the ratio of the stress acting on a substance to the strain produced. The term "stress" as used herein refers to a measure of the force put on an object over an area. The term "strain" as used herein refers to the change in length divided by the original length of the object. Change in length is proportional to the force put on it and depends on the substance from which the object is made. Change in length is proportional to the original length and inversely proportional to the cross-sectional area. Fracture is caused by a strain placed on an object such that it deforms (a change of shape) beyond its elastic limit and breaks.

### Embodiments - Corneal Inlay Device

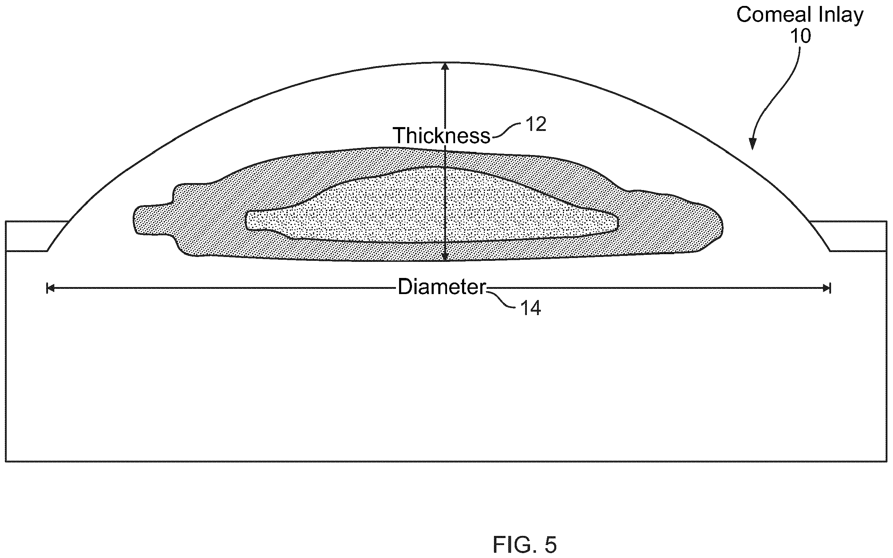

The present disclosure relates to a corneal inlay device, insertion means, and construction means, as discussed in detail below in connection with **FIGS. 5-9.**

**FIG. 5** is a diagram showing an example of a corneal inlay 10 of the present disclosure. The corneal inlay 10 includes a thickness 12 and a diameter 14. The corneal inlay 10 can have a dome or meniscus shape, including a flat or flat-like base and a dome or more or less spherical, convex shaped top. The corneal inlay 10 is biocompatible with the eye. The corneal inlay 10 can define a diameter 14 dimensioned smaller than the diameter of the pupil and is capable of correcting presbyopia while reducing or eliminating the risk of a patient developing corneal haze. To provide near vision, the corneal inlay 10 can be implanted centrally in the cornea to induce an "effect" zone on the anterior corneal surface that is smaller than the optical zone of the cornea, wherein the "effect" zone is the area of the anterior corneal surface affected by the corneal inlay 10. The implanted corneal inlay 10 increases the curvature of the anterior corneal surface within the "effect" zone, thereby increasing the diopter power of the cornea within the "effect" zone. Because the corneal inlay 10 is smaller than the diameter of the pupil, light rays from distant objects bypass the inlay and refract through the region of the cornea peripheral to the "effect" zone to create an image of distant objects on the retina.

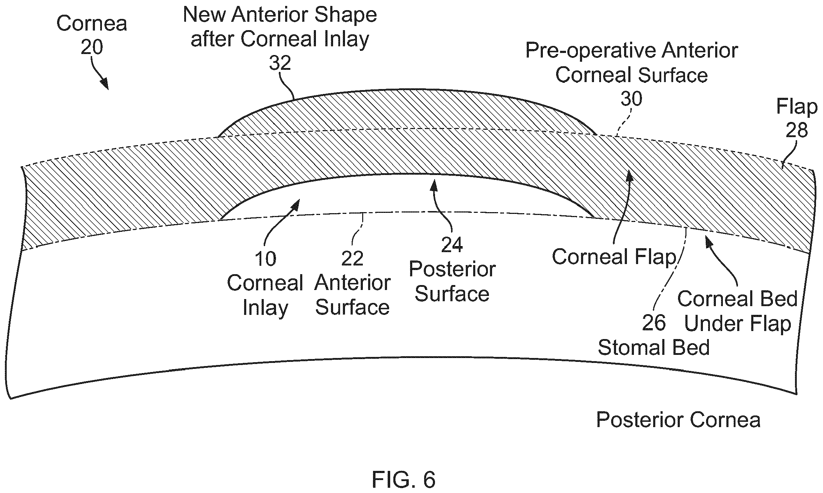

**FIG. 6** is a diagram showing the corneal inlay 10 implanted in a cornea 20. The corneal inlay 10 can have a substantially dome shape with an anterior surface 22 and a posterior surface 24. The corneal inlay 10 can be implanted in the cornea at a depth of about 50% or less of the cornea (approximately 250 μm or less), and is placed on the stromal bed 26 of the cornea 20 created by a microkeratome or any other suitable surgical instrument. For example, the corneal inlay 10 can be implanted in the cornea 20 by cutting a flap 28 into the cornea 20, lifting the flap 28 to expose an interior of the cornea 20, placing the corneal inlay 10 on the exposed area of the interior, and repositioning the flap 28 over the corneal inlay 10. The flap 28 can be cut using a laser (e.g., a femtosecond laser, a mechanical keratome, etc.) or manually by an ophthalmic surgeon. When the flap 28 is cut into the cornea 20, a small section of corneal tissue is left intact to create a hinge for the flap 28 so that the flap 28 can be repositioned accurately over the corneal inlay 10. After the flap 28 is repositioned over the corneal inlay 10, the cornea 20 heals around the flap 28 and seals the flap 28 back to the uncut peripheral portion of the anterior corneal surface. Alternatively, a pocket or well having side walls or barrier structures may be cut into the cornea 20, and the corneal inlay 10 inserted between the side walls or barrier structures through a small opening or "port" in the cornea 20.

In some embodiments, the corneal inlay 10 changes the refractive power of the cornea by altering the shape of the anterior corneal surface. In **FIG. 6,** the pre-operative anterior corneal surface is represented by dashed line 30 and the post-operative anterior corneal surface induced by the underlying corneal inlay 10 is represented by solid line 32.

In some embodiments in which a corneal inlay is positioned beneath a flap, the inlay 10 is implanted between about 100 microns (micrometers) and about 200 microns deep in the cornea. In some embodiments the inlay is positioned at a depth of between about 130 microns to about 160 microns. In some embodiments, the inlay 10 is positioned at depth of 100 microns. In some embodiments, the inlay 10 is positioned at depth of 101 microns. In some embodiments, the inlay 10 is positioned at depth of 102 microns. In some embodiments, the inlay 10 is positioned at depth of 103 microns. In some embodiments, the inlay 10 is positioned at depth of 104 microns. In some embodiments, the inlay 10 is positioned at depth of 105 microns. In some embodiments, the inlay 10 is positioned at depth of 106 microns. In some embodiments, the inlay 10 is positioned at depth of 107 microns. In some embodiments, the inlay 10 is positioned at depth of 108 microns. In some embodiments, the inlay 10 is positioned at depth of 109 microns. In some embodiments, the inlay 10 is positioned at depth of 110 microns. In some embodiments, the inlay 10 is positioned at depth of 111 microns. In some embodiments, the inlay 10 is positioned at depth of 112 microns. In some embodiments, the inlay 10 is positioned at depth of 113 microns. In some embodiments, the inlay 10 is positioned at depth of 114 microns. In some embodiments, the inlay 10 is positioned at depth of 115 microns. In some embodiments, the inlay 10 is positioned at depth of 116 microns. In some embodiments, the inlay 10 is positioned at depth of 117 microns. In some embodiments, the inlay 10 is positioned at depth of 118 microns. In some embodiments, the inlay 10 is positioned at depth of 119 microns. In some embodiments, the inlay 10 is positioned at depth of 120 microns. In some embodiments, the inlay 10 is positioned at depth of 121 microns. In some embodiments, the inlay 10 is positioned at depth of 122 microns. In some embodiments, the inlay 10 is positioned at depth of 123 microns. In some embodiments, the inlay 10 is positioned at depth of 124 microns. In some embodiments, the inlay 10 is positioned at depth of 125 microns. In some embodiments, the inlay 10 is positioned at depth of 126 microns. In some embodiments, the inlay 10 is positioned at depth of 127 microns. In some embodiments, the inlay 10 is positioned at depth of 128 microns. In some embodiments, the inlay 10 is positioned at depth of 129 microns. In some embodiments, the inlay 10 is positioned at depth of 130 microns. In some embodiments, the inlay 10 is positioned at depth of 131 microns. In some embodiments, the inlay 10 is positioned at depth of 132 microns. In some embodiments, the inlay 10 is positioned at depth of 133 microns. In some embodiments, the inlay 10 is positioned at depth of 134 microns. In some embodiments, the inlay 10 is positioned at depth of 135 microns. In some embodiments, the inlay 10 is positioned at depth of 136 microns. In some embodiments, the inlay 10 is positioned at depth of 137 microns. In some embodiments, the inlay 10 is positioned at depth of 138 microns. In some embodiments, the inlay 10 is positioned at depth of 139 microns. In some embodiments, the inlay 10 is positioned at depth of 140 microns. In some embodiments, the inlay 10 is positioned at depth of 141 microns. In some embodiments, the inlay 10 is positioned at depth of 142 microns. In some embodiments, the inlay 10 is positioned at depth of 143 microns. In some embodiments, the inlay 10 is positioned at depth of 144 microns. In some embodiments, the inlay 10 is positioned at depth of 145 microns. In some embodiments, the inlay 10 is positioned at depth of 146 microns. In some embodiments, the inlay 10 is positioned at depth of 147 microns. In some embodiments, the inlay 10 is positioned at depth of 148 microns. In some embodiments, the inlay 10 is positioned at depth of 149 microns. In some embodiments, the inlay 10 is positioned at depth of 150 microns. In some embodiments, the inlay 10 is positioned at depth of 151 microns. In some embodiments, the inlay 10 is positioned at depth of 152 microns. In some embodiments, the inlay 10 is positioned at depth of 153 microns. In some embodiments, the inlay 10 is positioned at depth of 154 microns. In some embodiments, the inlay 10 is positioned at depth of 155 microns. In some embodiments, the inlay 10 is positioned at depth of 156 microns. In some embodiments, the inlay 10 is positioned at depth of 157 microns. In some embodiments, the inlay 10 is positioned at depth of 158 microns. In some embodiments, the inlay 10 is positioned at depth of 159 microns. In some embodiments, the inlay 10 is positioned at depth of 160 microns. In some embodiments, the inlay 10 is positioned at depth of 161 microns. In some embodiments, the inlay 10 is positioned at depth of 162 microns. In some embodiments, the inlay 10 is positioned at depth of 163 microns. In some embodiments, the inlay 10 is positioned at depth of 164 microns. In some embodiments, the inlay 10 is positioned at depth of 165 microns. In some embodiments, the inlay 10 is positioned at depth of 166 microns. In some embodiments, the inlay 10 is positioned at depth of 167 microns. In some embodiments, the inlay 10 is positioned at depth of 168 microns. In some embodiments, the inlay 10 is positioned at depth of 169 microns. In some embodiments, the inlay 10 is positioned at depth of 170 microns. In some embodiments, the inlay 10 is positioned at depth of 171 microns. In some embodiments, the inlay 10 is positioned at depth of 172 microns. In some embodiments, the inlay 10 is positioned at depth of 173 microns. In some embodiments, the inlay 10 is positioned at depth of 174 microns. In some embodiments, the inlay 10 is positioned at depth of 175 microns. In some embodiments, the inlay 10 is positioned at depth of 176 microns. In some embodiments, the inlay 10 is positioned at depth of 177 microns. In some embodiments, the inlay 10 is positioned at depth of 178 microns. In some embodiments, the inlay 10 is positioned at depth of 179 microns. In some embodiments, the inlay 10 is positioned at depth of 180 microns. In some embodiments, the inlay 10 is positioned at depth of 181 microns. In some embodiments, the inlay 10 is positioned at depth of 182 microns. In some embodiments, the inlay 10 is positioned at depth of 183 microns. In some embodiments, the inlay 10 is positioned at depth of 184 microns. In some embodiments, the inlay 10 is positioned at depth of 185 microns. In some embodiments, the inlay 10 is positioned at depth of 186 microns. In some embodiments, the inlay 10 is positioned at depth of 187 microns. In some embodiments, the inlay 10 is positioned at depth of 188 microns. In some embodiments, the inlay 10 is positioned at depth of 189 microns. In some embodiments, the inlay 10 is positioned at depth of 190 microns. In some embodiments, the inlay 10 is positioned at depth of 191 microns. In some embodiments, the inlay 10 is positioned at depth of 192 microns. In some embodiments, the inlay 10 is positioned at depth of 193 microns. In some embodiments, the inlay 10 is positioned at depth of 194 microns. In some embodiments, the inlay 10 is positioned at depth of 195 microns. In some embodiments, the inlay 10 is positioned at depth of 196 microns. In some embodiments, the inlay 10 is positioned at depth of 197 microns. In some embodiments, the inlay 10 is positioned at depth of 198 microns. In some embodiments, the inlay 10 is positioned at depth of 199 microns. In some embodiments, the inlay 10 is positioned at depth of 200 microns. In some embodiments, the depth in the cornea for a pocket may be greater than for a flap. According to some exemplary embodiments, because depth in the cornea for the pocket is greater than for the flap, a thicker inlay may be needed in order to impart a refractive correction.

The elastic (Young's) modulus of the corneal inlay 10 can, by way of example, be 0.18 megapascals ("MPa") with a tolerance of $\pm 0.06$ MPa. However, in some embodiments, the elastic modulus of the corneal inlay 10 can exceed the tolerance. In some embodiments, the elastic modulus of the corneal inlay 10 can be at least 0.05 MPa. In some embodiments, the elastic modulus of the corneal inlay 10 can be at least 0.06 MPa. In some embodiments, the elastic modulus of the corneal inlay 10 can be at least 0.07 MPa. In some embodiments, the elastic modulus of the corneal inlay 10 can be at least 0.08 MPa. In some embodiments, the elastic modulus of the corneal inlay 10 can be at least 0.09 MPa. In some embodiments, the elastic modulus of the corneal inlay 10 can be at least 0.10 MPa. In some embodiments, the elastic modulus of the corneal inlay 10 can be at least 0.11 MPa. In some embodiments, the elastic modulus of the corneal inlay 10 can be at least 0.12 MPa. In some embodiments, the elastic modulus of the corneal inlay 10 can be at least 0.13 MPa. In some embodiments, the elastic modulus of the corneal inlay 10 can be at least 0.14 MPa. In some embodiments, the elastic modulus of the corneal inlay 10 can be at least 0.15 MPa. In some embodiments, the elastic modulus of the corneal inlay 10 can be at least 0.16 MPa. In some embodiments, the elastic modulus of the corneal inlay 10 can be at least 0.17 MPa. In some embodiments, the elastic modulus of the corneal inlay 10 can be at least 0.18 MPa. In some embodiments, the elastic modulus of the corneal inlay 10 can be at least 0.19 MPa. In some embodiments, the elastic modulus of the corneal inlay 10 can be at least 0.20 MPa. In some embodiments, the elastic modulus of the corneal inlay 10 can be at least 0.21 MPa. In some embodiments, the elastic modulus of the corneal inlay 10 can be at least 0.22 MPa. In some embodiments, the elastic modulus of the corneal inlay 10 can be at least 0.23 MPa. In some embodiments, the elastic modulus of the corneal inlay 10 can be at least 0.24 MPa. In some embodiments, the elastic modulus of the corneal inlay 10 can be at least 0.25 MPa. In some embodiments, the elastic modulus of the corneal inlay 10 can be at least 0.26 MPa. In some embodiments, the elastic modulus of the corneal inlay 10 can be at least 0.27 MPa. In some embodiments, the elastic modulus of the corneal inlay 10 can be at least 0.28 MPa. In some embodiments, the elastic modulus of the corneal inlay 10 can be at least 0.29 MPa. In some embodiments, the elastic modulus of the corneal inlay 10 can be at least 0.30 MPa.

"Elongation at break", also called "fracture strain" or "tensile elongation at break" is the percentage increase in length that a material will achieve before breaking. It is a measurement that shows how much a material can be stretched - as a percentage of its original dimensions - before it breaks. In other words, it's a percentage that shows a material's ductility. A material with high ductility means it's more likely to deform (but not break). Low ductility indicates that it's brittle and will fracture easily under a tensile load.

In some embodiments, the elongation at break of the corneal inlay 10 may be 58.30% with a tolerance of $\pm 4.49\%$. However, in some embodiments, the elongation at break of the corneal inlay 10 may exceed the tolerance. In some embodiments, the elongation at break of the corneal inlay 10 may be at least 48%. In some embodiments, the elongation at break of the corneal inlay 10 may be at least 49%. In some embodiments, the elongation at break of the corneal inlay 10 may be at least 50%. In some embodiments, the elongation at break of the corneal inlay 10 may be at least 21%. In some embodiments, the elongation at break of the corneal inlay 10 may be at least 52%. In some embodiments, the elongation at break of the corneal inlay 10 may be at least 53%. In some embodiments, the elongation at break of the corneal inlay 10 may be at least 54%. In some embodiments, the elongation at break of the corneal inlay 10 may be at least 55%. In some embodiments, the elongation at break of the corneal inlay 10 may be at least 56%. In some embodiments, the elongation at break of the corneal inlay 10 may be at least 57%. In some embodiments, the elongation at break of the corneal inlay 10 may be at least 58%. In some embodiments, the elongation at break of the corneal inlay 10 may be at least 59%. In some embodiments, the elongation at break of the corneal inlay 10 may be at least 60%. In some embodiments, the elongation at break of the corneal inlay 10 may be at least 61%. In some embodiments, the elongation at break of the corneal inlay 10 may be at least 62%. In some embodiments, the elongation at break of the corneal inlay 10 may be at least 63%. In some embodiments, the elongation at break of the corneal inlay 10 may be at least 64%. In some embodiments, the elongation at break of the corneal inlay 10 may be at least 65%. In some embodiments, the elongation at break of the corneal inlay 10 may be at least 66%. In some embodiments, the elongation at break of the corneal inlay 10 may be at least 67%. In some embodiments, the elongation at break of the corneal inlay 10 may be at least 68%. In some embodiments, the elongation at break of the corneal inlay 10 may be at least 69%. In some embodiments, the elongation at break of the corneal inlay 10 may be at least 70%.

In some embodiments, the tensile strength (meaning the resistance of a material to breaking under tension) of the corneal inlay 10 may be 0.07 MPa with a tolerance of ±0.02 MPa. In some embodiments, the tensile strength of the corneal inlay may exceed the tolerance. In some embodiments, the tensile strength of the corneal inlay 10 may be at least 0.01 MPa. In some embodiments, the tensile strength of the corneal inlay 10 may be at least 0.02 MPa. In some embodiments, the tensile strength of the corneal inlay 10 may be at least 0.03 MPa. In some embodiments, the tensile strength of the corneal inlay 10 may be at least 0.04 MPa. In some embodiments, the tensile strength of the corneal inlay 10 may be at least 0.05 MPa. In some embodiments, the tensile strength of the corneal inlay 10 may be at least 0.06 MPa. In some embodiments, the tensile strength of the corneal inlay 10 may be at least 0.07 MPa. In some embodiments, the tensile strength of the corneal inlay 10 may be at least 0.08 MPa. In some embodiments, the tensile strength of the corneal inlay 10 may be at least 0.09 MPa. In some embodiments, the tensile strength of the corneal inlay 10 may be at least 0.10 MPa. In some embodiments, the tensile strength of the corneal inlay 10 may be at least 0.11 MPa. In some embodiments, the tensile strength of the corneal inlay 10 may be at least 0.12 MPa. In some embodiments, the tensile strength of the corneal inlay 10 may be at least 0.13 MPa. In some embodiments, the tensile strength of the corneal inlay 10 may be at least 0.14 MPa. In some embodiments, the tensile strength of the corneal inlay 10 may be at least 0.15 MPa.

In some embodiments, the backscatter (meaning deflection of radiation or particles through an angle of 180°) of the corneal inlay 10 may be 0.90% with a tolerance of ±0.17%. However, in some embodiments, the backscatter of the corneal inlay 10 may exceed the tolerance. In some embodiments, the backscatter of the corneal inlay 10 may be at least 0.65%. In some embodiments, the backscatter of the corneal inlay 10 may be at least 0.66%. In some embodiments, the backscatter of the corneal inlay 10 may be at least 0.67%. In some embodiments, the backscatter of the corneal inlay 10 may be at least 0.68%. In some embodiments, the backscatter of the corneal inlay 10 may be at least 0.69%. In some embodiments, the backscatter of the corneal inlay 10 may be at least 0.70%. In some embodiments, the backscatter of the corneal inlay 10 may be at least 0.71%. In some embodiments, the backscatter of the corneal inlay 10 may be at least 0.72%. In some embodiments, the backscatter of the corneal inlay 10 may be at least 0.73%. In some embodiments, the backscatter of the corneal inlay 10 may be at least 0.74%. In some embodiments, the backscatter of the corneal inlay 10 may be at least 0.75%. In some embodiments, the backscatter of the corneal inlay 10 may be at least 0.76%. In some embodiments, the backscatter of the corneal inlay 10 may be at least 0.77%. In some embodiments, the backscatter of the corneal inlay 10 may be at least 0.78%. In some embodiments, the backscatter of the corneal inlay 10 may be at least 0.79%. In some embodiments, the backscatter of the corneal inlay 10 may be at least 0.80%. In some embodiments, the backscatter of the corneal inlay 10 may be at least 0.81%. In some embodiments, the backscatter of the corneal inlay 10 may be at least 0.82%. In some embodiments, the backscatter of the corneal inlay 10 may be at least 0.83%. In some embodiments, the backscatter of the corneal inlay 10 may be at least 0.84%. In some embodiments, the backscatter of the corneal inlay 10 may be at least 0.85%. In some embodiments, the backscatter of the corneal inlay 10 may be at least 0.86%. In some embodiments, the backscatter of the corneal inlay 10 may be at least 0.87%. In some embodiments, the backscatter of the corneal inlay 10 may be at least 0.88%. In some embodiments, the backscatter of the corneal inlay 10 may be at least 0.89%. In some embodiments, the backscatter of the corneal inlay 10 may be at least 0.90%. In some embodiments, the backscatter of the corneal inlay 10 may be at least 0.91%. In some embodiments, the backscatter of the corneal inlay 10 may be at least 0.92%. In some embodiments, the backscatter of the corneal inlay 10 may be at least 0.93%. In some embodiments, the backscatter of the corneal inlay 10 may be at least 0.94%. In some embodiments, the backscatter of the corneal inlay 10 may be at least 0.95%. In some embodiments, the backscatter of the corneal inlay 10 may be at least 0.96%. In some embodiments, the backscatter of the corneal inlay 10 may be at least 0.97%. In some embodiments, the backscatter of the corneal inlay 10 may be at least 0.98%. In some embodiments, the backscatter of the corneal inlay 10 may be at least 0.99%. In some embodiments, the backscatter of the corneal inlay 10 may be at least 1.00%. In some embodiments, the backscatter of the corneal inlay 10 may be at least 1.01%. In some embodiments, the backscatter of the corneal inlay 10 may be at least 1.02%. In some embodiments, the backscatter of the corneal inlay 10 may be at least 1.03%. In some embodiments, the backscatter of the corneal inlay 10 may be at least 1.04%. In some embodiments, the backscatter of the corneal inlay 10 may be at least 1.05%. In some embodiments, the backscatter of the corneal inlay 10 may be at least 1.06%. In some embodiments, the backscatter of the corneal inlay 10 may be at least 1.07%. In some embodiments, the backscatter of the corneal inlay 10 may be at least 1.08%. In some embodiments, the backscatter of the corneal inlay 10 may be at least 1.09%. In some embodiments, the backscatter of the corneal inlay 10 may be at least 1.10%. In some embodiments, the backscatter of the corneal inlay 10 may be at least 1.11%. In some embodiments, the backscatter of the corneal inlay 10 may be at least 1.12%. In some embodiments, the backscatter of the corneal inlay 10 may be at least 1.13%. In some embodiments, the backscatter of the corneal inlay 10 may be at least 1.14%. In some embodiments, the backscatter of the corneal inlay 10 may be at least 1.15%.

In some embodiments, the light transmission (meaning the moving of electromagnetic waves through) of the corneal inlay 10 can be 92.4% with a tolerance of ±0.95%. In some embodiments, the elastic modulus of the corneal inlay 10 can exceed the tolerance. In some embodiments, the light transmission of the corneal inlay 10 can be at least 85.0%. In some embodiments, the light transmission of the corneal inlay 10 can be at least 86.0%. In some embodiments, the light transmission of the corneal inlay 10 can be at least 87.0%. In some embodiments, the light transmission of the corneal inlay 10 can be at least 88.0%. In some embodiments, the light transmission of the corneal inlay 10 can be at least 89.0%. In some embodiments, the light transmission of the corneal inlay 10 can be at least 90.0%. In some embodiments, the light transmission of the corneal inlay 10 can be at least 91.0%. In some embodiments, the light transmission of the corneal inlay 10 can be at least 92.0%. In some embodiments, the light transmission of the corneal inlay 10 can be at least 93.0%. In some embodiments, the light transmission of the corneal inlay 10 can be at least 94.0%. In some embodiments, the light transmission of the corneal inlay 10 can be at least 95.0%. In some embodiments, the light transmission of the corneal inlay 10 can be at least 96.0%. In some embodiments, the light transmission of the corneal inlay 10 can be at least 97.0%. In some embodiments, the light transmission of the corneal inlay 10 can be at least 98.0%. In some embodiments, the light transmission of the corneal inlay 10 can be at least 99.0%. In some embodiments, the light transmission of the corneal inlay 10 can be 100.0%.

In some embodiments, the morphology (meaning form) of the corneal inlay 10 can be that of a fibrillary network with nano-pores. In some embodiments, the nano-pores of the corneal inlay 10 can have a diameter of at least 0.1 $\mu$m. In some embodiments, the nano-pores of the corneal inlay 10 can have a diameter of at least 0.2 $\mu$m. In some embodiments, the nano-pores of the corneal inlay 10 can have a diameter of at least 0.3 $\mu$m. In some embodiments, the nano-pores of the corneal inlay 10 can have a diameter of at least 0.4 $\mu$m. In some embodiments, the nano-pores of the corneal inlay 10 can have a diameter of at least 0.5 $\mu$m. In some embodiments, the nano-pores of the corneal inlay 10 can have a diameter of at least 0.6 $\mu$m. In some embodiments, the nano-pores of the corneal inlay 10 can have a diameter of at least 0.7 $\mu$m. In some embodiments, the nano-pores of the corneal inlay 10 can have a diameter of at least 0.8 $\mu$m. In some embodiments, the nano-pores of the corneal inlay 10 can have a diameter of at least 0.9 $\mu$m. In some embodiments, the nano-pores of the corneal inlay 10 can have a diameter of at least 1.0 $\mu$m. In some embodiments, the nano-pores can have a diameter of approximately 0.4 $\mu$m. In some embodiments, the storage temperature for the corneal inlay 10 may range from about 2° - 6° Celsius, i.e., about 2°C, 2.5°C, 3°C, 3.5°C, 4°C, 4.5°C, 5°C, 5.5°C, 6°C.

**Presbyopic Inlays**

In some embodiments, the diameter of the corneal inlay 10 is small in comparison with the diameter of the pupil for correcting presbyopia. In some embodiments, a corneal inlay 10 (e.g., 1 mm to 3 mm in diameter, 1.8 mm in diameter, or the like) is implanted substantially centrally in the cornea to induce an "effect" zone on the anterior corneal surface that is smaller than the optical zone of the cornea for providing near vision. Here, the "effect" zone is the area of the anterior corneal surface affected by the corneal inlay 10. The implanted corneal inlay 10 increases the curvature of the anterior corneal surface within the "effect" zone, thereby increasing the diopter power of the cornea within the "effect" zone. Distance vision is provided by the region of the cornea peripheral to the "effect" zone.

Presbyopia is characterized by a decrease in the ability of the eye to increase its power to focus on nearby objects due to a loss of elasticity in the crystalline lens with age. Typically, a person suffering from presbyopia requires reading glasses to provide near vision.

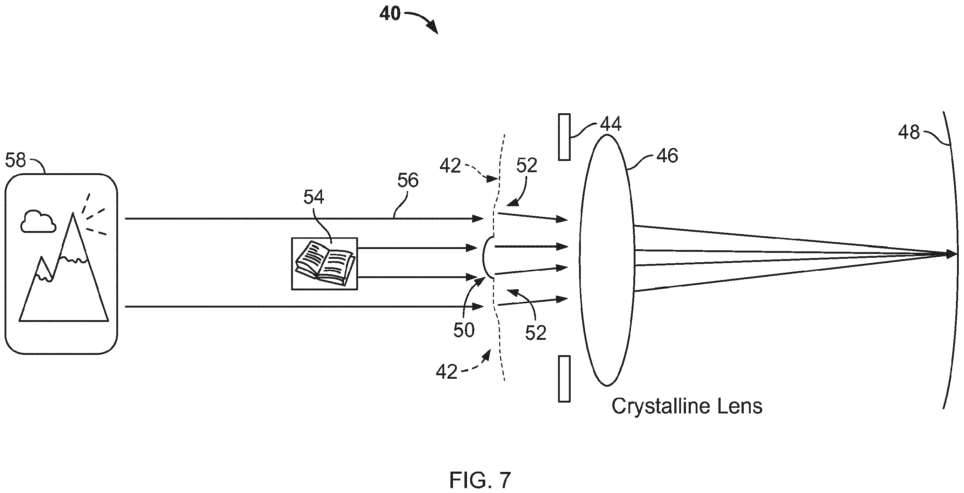

**FIG. 7** shows an example of how a corneal inlay 10 can provide near vision to a subject's eye while retaining some distance vision according to an embodiment of the invention. The eye 40 comprises a cornea 42, a pupil 44, a crystalline lens 46 and a retina 48. In this example, the corneal inlay 10 (not shown) is implanted substantially centrally in the cornea 42 to create a small diameter "effect" zone 50. The corneal inlay 10 has a smaller diameter than the pupil 44 so that the resulting "effect" zone 50 has a smaller diameter than the optical zone of the cornea 42. The "effect" zone 50 provides near vision by increasing the curvature of the anterior corneal surface, and therefore the diopter power within the "effect" zone 50. The region 52 of the cornea peripheral to the "effect" zone provides distance vision.

To increase the diopter power within the "effect" zone 50, the corneal inlay 10 has a curvature higher than the curvature of the pre-implant anterior corneal surface in order to increase the curvature of the anterior corneal surface within the "effect" zone 50. The corneal inlay 10 can further increase the diopter power within the "effect" zone 52 by having an index of refraction that is higher than the index of refraction of the cornea ($n_{corea}$=1.376). Thus, the increase in the diopter power within the "effect" zone 50 can be due to the change in the anterior corneal surface induced by the corneal inlay 10 or a combination of the change in the anterior cornea surface and the index of refraction of the corneal inlay 10. For early presbyopia (e.g., about 45 to 55 years of age), at least 1 diopter is typically required for near vision. For complete presbyopia (e.g., about 60 years of age or older), between 2 and 3 diopters of additional power are required.

An advantage of corneal inlay 10 is that when concentrating on nearby objects 54, the pupil naturally becomes smaller (e.g., near point miosis) making the corneal inlay effect even more effective. Further increases in the corneal inlay effect can be achieved by increasing the illumination of a nearby object (e.g., turning up a reading light).

Because the inlay is smaller than the diameter of the pupil 44, light rays 56 from distant objects 58 bypass the inlay and refract using the region of the cornea peripheral to the "effect" zone to create an image of the distant objects on the retina 48, as shown in **FIG. 7.** This is particularly true with larger pupils. At night, when distance vision is most important, the pupil naturally becomes larger, thereby reducing the inlay effect and maximizing distance vision.

A subject's natural distance vision is in focus only if the subject is emmetropic (i.e., does not require glasses for distance vision). Many subjects are ammetropic, requiring either myopic or hyperopic refractive correction. Especially for myopes, distance vision correction can be provided by myopic Laser in Situ Keratomileusis ("LASIK"), Laser Epithelial Keratomileusis ("LASEK"), Photorefractive Keratectomy ("PRK") or other similar corneal refractive procedures. After the distance corrective procedure is completed, the corneal inlay 10 can be implanted in the cornea to provide near vision. Since LASIK requires the creation of a flap, the corneal inlay 10 may be inserted concurrently with the LASIK procedure. The corneal inlay 10 can also be inserted into the cornea after the LASIK procedure since the flap can be re-opened. Therefore, the corneal inlay 10 can be used in conjunction with other refractive procedures, such as LASIK for correcting myopia or hyperopia.

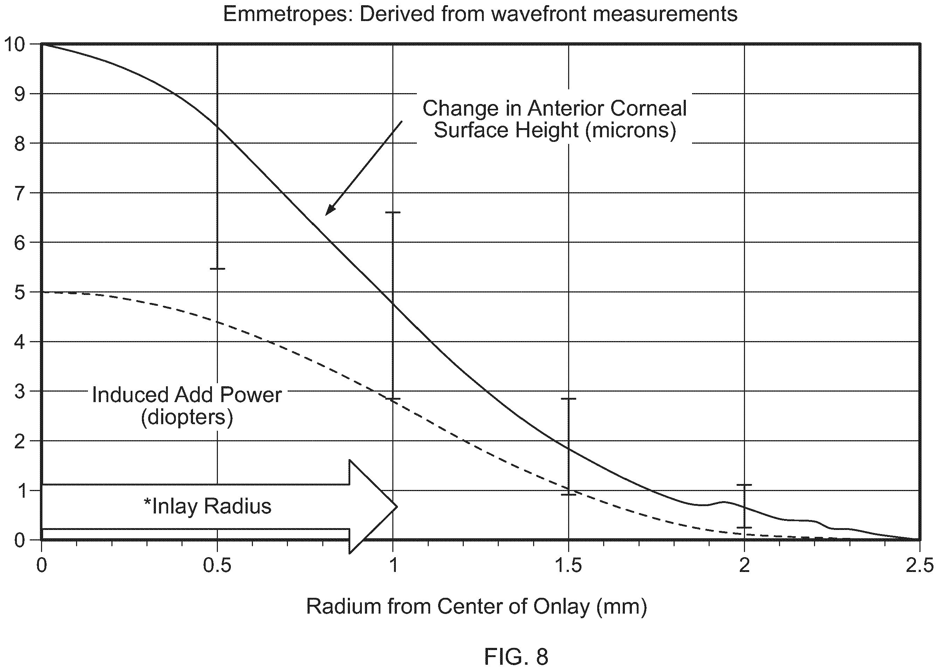

**FIG. 8** is a plot of anterior corneal surface height (in microns) (y axis) vs. radius from center of inlay (mm) (x-axis). The graph shows the change in anterior corneal surface height (in microns) and the corresponding induced added power (e.g., diopters).

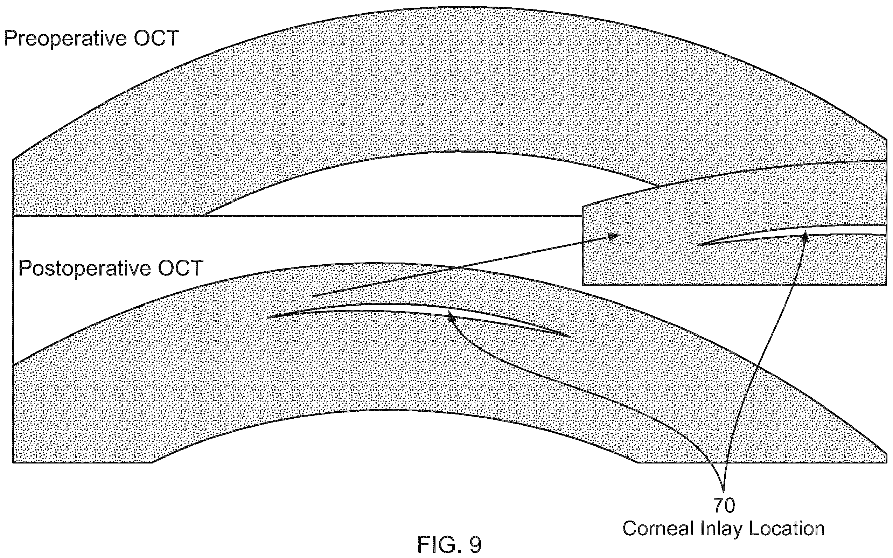

**FIG. 9** is a diagram showing a preoperative optical coherence tomography ("OCT") and a postoperative OCT. In the postoperative OCT, an example location 70 for the corneal inlay 10 is shown.

It should be understood that the illustrations of **FIGS. 7-9** similarly apply to and have substantially the same effect as the presbyoptic corneal onlay device discussed herein.

**Material Chemistry of the Inlay**

The section below discusses the material chemistry of the corneal inlay device. However, it should be understood that the corneal onlay device discussed herein can have the same or substantially the same material chemistry as the corneal inlay device.

In some embodiments, the percentage by weight of the collagen within the hydrogel composition can be about, e.g., 1%-5%, inclusive 1-4% inclusive, 1-3% inclusive, 1-2%, inclusive 2-5% inclusive, 3-5%, inclusive 4-5% inclusive, 2-4% inclusive, 3-4% inclusive, 1%, 1.1%, 1.2%, 1.3%, 1.4%, 1.5%, 1.6%, 1.7%, 1.8%, 1.9, 2%, 2.1%, 2.2%, 2.3%, 2.4% 2.5%, 2.6%, 2.7%, 2.8%, 2.9%, 3%, 3.1%, 3.2%, 3.3%, 3.4%, 3.5%, 3.6%, 3.7%, 3.8%, 3.9% , 4%, 4.1%, 4.2%, 4.3%, 4.4%, 4.5%, 4.6%, 4.7%, 4.8%, 4.9%, 5%, or the like. In some embodiments, the percentage by weight of the synthetic polymer within the hydrogel composition can be about, e.g., 1.5-7.2%, inclusive, 1.5-7% inclusive, 1.5-6% inclusive, 1.5-5% inclusive, 1.5-4% inclusive, 1.5-3% inclusive, 1.5-2% inclusive, 2-7.2% inclusive, 3-7.2% inclusive, 4-7.2% inclusive, 5-7.2% inclusive, 6-7.2% inclusive, 7-7.2% inclusive, 1.5-7%, inclusive 2-7% inclusive, 3-7% inclusive, 4-7% inclusive, 5-7% inclusive, 6-7% inclusive, 1.5%, 1.6%, 1.7%, 1.8%, 1.9%, 2%, 2.1%, 2.2%, 2.3%, 2.4% 2.5%, 2.6%, 2.7%, 2.8%, 2.9%, 3%, 3.1%, 3.2%, 3.3%, 3.4%, 3.5%, 3.6%, 3.7%, 3.8%, 3.9% 4%, 4.1%, 4.2%, 4.3%, 4.4%, 4.5%, 4.6%, 4.7%, 4.8%, 4.9%, 5%, 5.1%, 5.2%, 5.3%, 5.4%, 5.5%, 5.6%, 5.7%, 5.8%, 5.9%, 6%, 6.1%, 6.2%, 6.3%, 6.4%, 6.5%, 6.6%, 6.7%, 6.8%, 6.9%, 7%, 7.1%, 7.2%, or the like. In some embodiments, the collagen can be a porcine atelocollagen, type 1, obtained from Nippi Collagen of North America Inc. However, it should be understood that any similar collagen can be used.

In some embodiments, the inlay material comprises a biopolymer. In some embodiments, the natural biopolymer is a collagen. In some embodiments, the natural biopolymer is different from the collagen. In some embodiments, the percentage by weight of the natural polymer within the hydrogel composition can be substantially equal to the percentage by weight of the collagen. In some embodiments, the natural polymer can be a collagen. In some embodiments, the biopolymer is a synthetic self-assembling biopolymer. In some embodiments, the biopolymer is a naturally-occurring biopolymer. Exemplary naturally-occurring biopolymers include, but are not limited to, protein polymers, collagen, polysaccharides, and photopolymerizable compounds. Exemplary protein polymers synthesized from self-assembling protein polymers include, for example, silk fibroin, elastin, collagen, and combinations thereof. In some embodiments, the synthetic self-assembling biopolymer is a synthetic collagen. In some embodiments, the synthetic self-assembling biopolymer is a recombinant human collagen. In some embodiments, the collagen is a collagen mimetic peptide. As used herein, the term "mimetic" refers to chemicals containing chemical moieties that mimic the function of a peptide. For example, if a peptide contains two charged chemical moieties having functional activity, a mimetic places two charged chemical moieties in a spatial orientation and constrained structure so that the charged chemical function is maintained in three-dimensional space.

In some embodiments, the inlay materials comprise a synthetic polymeric material. In some embodiments the synthetic material is an optically transparent material. In some embodiments the synthetic materials is a biocompatible material. In some embodiments the synthetic material is a hydrophilic material. In some embodiments the synthetic materials is a material permeable to low molecular weight nutrients so as to maintain corneal health. In some embodiments the synthetic materials is a refractive material. In some embodiments the synthetic material is optically transparent, biocompatible, hydrophilic, permeable and refractive.

Exemplary biocompatible biodegradable polymers include, without limitation, a poly(lactide); a poly(glycolide); a poly(lactide-co-glycolide); a poly(lactic acid); a poly(glycolic acid); a poly(lactic acid-co-glycolic acid); a poly(caprolactone); a poly(orthoester); a polyanhydride; a poly(phosphazene); a polyhydroxyalkanoate; a poly(hydroxybutyrate); a polycarbonate; a tyrosine polycarbonate; a polyamide; a polyesteramide; a polyester; a poly(dioxanone); a poly(alkylene alkylate); a polyether (such as polyethylene glycol, PEG, and polyethylene oxide, PEO); polyvinyl pyrrolidone or PVP; a polyurethane; a polyetherester; a polyacetal; a polycyanoacrylate; a poly(oxyethylene)/poly(oxypropylene) copolymer; a polyacetal, a polyketal; a polyphosphate; a (phosphorous-containing) polymer; a polyphosphoester; a polyhydroxyvalerate; a polyalkylene oxalate; a polyalkylene succinate; or a poly(maleic acid). The water-soluble, biocompatible polymer poly(2-methacryloyloxyethyl phosphorylcholine) (PMPC) is a zwitterionic polymer that is able to form a more compact conformation in aqueous solution than poly(ethylene glycol) (PEG).

Exemplary non-degradable biocompatible polymers include, without limitation, polysiloxane, polyvinyl alcohol, polyimide a polyacrylate; a polymer of ethylene-vinyl acetate, EVA; cellulose acetate; an acyl-substituted cellulose acetate; a non-degradable polyurethane; a polystyrene; a polyvinyl chloride; a polyvinyl fluoride; a poly(vinyl imidazole); a silicone-based polymer (for example, Silastic® and the like), a chlorosulphonate polyolefin; a polyethylene oxide; polysiloxane, polyvinyl alcohol, and polyimide, or a blend or copolymer thereof.

Exemplary copolymers may include, hydroxyethyl methacrylate and methyl methacrylate, and hydroxyethyl methacrylate copolymerized with polyvinyl pyrrolidone (PVP, to increase water retention) or ethylene glycol dimethacrylic acid (EGDM). Nexofilcon A (Bausch & Lomb) is a hydrophilic copolymer of 2-hydroxyethyl methacrylate and N-vinyl pyrrolidone.

Exemplary block polymers comprising blocks of hydrophilic biocompatible polymers or biopolymers or biodegradable polymers may include polyethers, including polyethylene glycol, PEG; polyethylene oxide, PEO; polypropylene oxide, PPO, perfluoropolyethers (PFPEs) and block copolymers comprised of combinations thereof.

In some embodiments, the hydrophilic polymer comprises a hydrogel polymer. Hydrogels are water-swollen, cross-linked polymeric structures produced by the polymerization reaction of one or more monomers or by association of bonds, such as hydrogen bonds and strong van der Waals interactions between chains that exist in a state between rigid solids and liquid. Aqueous gels are formed when high molecular weight polymers or high polymer concentration are incorporated in the formulations. Hydrogels generally comprise a variety of polymers. Exemplary polymers include acrylic acid, acrylamide and 2-hydroxyethylmethacrylate (HEMA). For example, Cross-linked poly (acrylic acid) of high molecular weight is commercially available as Carbopol® (B.F./ Goodrich Chemical Co., Cleveland, OH). Polyethylene glycol diacrylate (PEGDA 400) is a long-chain, hydrophilic, crosslinking monomer. Methacryloyloxyethyl phosphorylcholine (MPC), containing a phosphorylcholine group in the side chain, is a monomer to mimic the phospholipid polar groups contained with cell membranes. Polyoxamers, commercially available as Pluronic® (BASF-Wyandotte, USA), are thermal setting polymers formed by a central hydrophobic part (polyoxypropylene) surrounded by a hydrophilic part (ethylene oxide). (4-(4,6-dimethoxy-1,3,5-triazin-2-yyl)-4methylmorpholinium chloride (DMTMM) or N-3-dimethylaminopropyl)-N'-ethylcarbodiimide hydrochloride and N-hydroxysuccinimide (EDC/NHS) may be useful to synthesize hyaluronan derivatives. See, D'Este, M. et al, Carbohydrate Polymers (2014) 108: 239-246). Cellulosic derivatives most commonly used in ophthalmology include: methylcellulose; hydroxyethylcellulose (HEC), hydroxypropylcellulose (HPC), hydroxypropylmethylcellulose (HPMC) and sodium carboxymethylcellulose (CMC Na). Photocrosslinked poly(ethylene glycol) diacrylate (PEGDA) hydrogels displaying collagen mimetic peptides (CMPs) that can be further conjugated to bioactive molecules via CMP-CMP triple helix association are described in Stahl, PJ et al. Soft Matter (2012) 8: 10409-10418.

In some embodiments, a first polymer and a second polymer comprise one or more different non-repeating units, such as, for example, an end group, or a non-repeating unit in the backbone of the polymer. In some embodiments, the first polymer and the second polymer comprise one or more different end groups. For example, the first polymer can have a more polar end group than one or more end group(s) of the second polymer. According to some such embodiments, the first polymer will be more hydrophilic, relative to a second polymer (with the less polar end group) alone. According to some such embodiments, the first polymer comprises one or more carboxylic acid end groups, and the second polymer comprises one or more ester end groups.

In some embodiments, the inlay material comprises a polymer matrix.

In some embodiments, the inlay materials may comprise an ultraviolet blocker that is added to the hydrogel composition before fabrication of the inlay material. In some embodiments, the inlay materials may comprise a dye (e.g., for easy handling of implant) added to the hydrogel composition before fabrication of the inlay material. According to some embodiments, the dye can be a UV dye added to the hydrogel composition to allow for visibility of the device during illumination with UV light.

The corneal inlay 10 can have properties similar to those of the cornea in nature, and may be made of a hydrogel or other clear biocompatible material. To increase the optical power of the inlay, the inlay may be made of a material with a higher index of refraction than the cornea, e.g., > 1.376.

In some embodiments, the water content can range from 78%-92% (w/w), inclusive. In some embodiments, the water content can range from 78%-88% inclusive. In some embodiments, the water content is at least 78%. In some embodiments, the water content is at least 79%. In some embodiments, the water content is at least 80%. In some embodiments, the water content is at least 81%. In some embodiments, the water content is at least 82%. In some embodiments, the water content is at least 83%. In some embodiments, the water content is at least 84%. In some embodiments, the water content is at least 85%. In some embodiments, the water content is at least 86%. In some embodiments, the water content is at least 87%. In some embodiments, the water content is at least 88%. In some embodiments, the water content is at least 89%. In some embodiments, the water content is at least 90%. In some embodiments, the water content is at least 91%. In some embodiments, the water content is at least 92%. In some embodiments, the water content is less than 92%. In some embodiments, the water content is less than 90%. In some embodiments, the water content is less than 88%. In some embodiments, the water content ranges from at least 78%-91%., inclusive In some embodiments, the water content ranges from at least 78%-90% inclusive. In some embodiments, the water content ranges from at least 78%-89% inclusive. In some embodiments, the water content ranges from at least 78%-88% inclusive. In some embodiments, the water content ranges from at least 78%-87% inclusive. In some embodiments, the water content ranges from at least 78%-86% inclusive. In some embodiments, the water content ranges from at least 78%-85% inclusive. In some embodiments, the water content ranges from at least 78%-85% inclusive. In some embodiments, the water content ranges from at least 78%-84% inclusive. In some embodiments, the water content ranges from at least 78%-83%. inclusive In some embodiments, the water content ranges from at least 78%-82% inclusive. In some embodiments, the water content ranges from at least 78%-81% inclusive. In some embodiments, the water content ranges from at least 78%-80% inclusive. In some embodiments, the water content ranges from at least 78%-79% inclusive. In some embodiments, the water content ranges from at least 79%-92% inclusive. In some embodiments, the water content ranges from at least 80%-92% inclusive. In some embodiments, the water content ranges from at least 81%-92% inclusive. In some embodiments, the water content ranges from at least 82%-92% inclusive. In some embodiments, the water content ranges from at least 83%-92% inclusive. In some embodiments, the water content ranges from at least 84%-92% inclusive. In some embodiments, the water content ranges from at least 85%-92% inclusive. In some embodiments, the water content ranges from at least 86%-92% inclusive. In some embodiments, the water content ranges from at least 87%-92% inclusive. In some embodiments, the water content ranges from at least 88%-92% inclusive. In some embodiments, the water content ranges from at least 89%-92% inclusive. In some embodiments, the water content ranges from at least 90%-92% inclusive. In some embodiments, the water content ranges from at least 91%-92% inclusive. In some embodiments, the water content ranges from at least 80%-88% inclusive. In some embodiments, the water content ranges from at least 82%-84% inclusive.

The low water content of the hydrogel composition used to fabricate the exemplary inlay can allow for ease of handling of the inlay. For example, too high of water content (e.g., above 92% w/w) can create more flexibility in the inlay, resulting in potentially greater difficulty for handling and damage to the inlay. The water content range between 78%-92%, inclusive, can provide a pliable yet sufficiently strong/stiff material that can be easily handled during manufacturing and surgery. The low water content range of the hydrogel composition also substantially matches the 78%-80% water content of the cornea, allowing for improved biocompatibility. For example, inlays with water content above the 78%-92% range, inclusive, i.e., at least 78%, at least 79%, at least 80%, at least 81%, at least 82%, at least 83%, at least 84%, at least 85%, at least 86%, at least 87%, at least 88%, at least 89%, at least 90%, at least 91%, or 92%, can have reduced biocompatibility with the cornea due to the lack of a match between the water content of the inlay and the cornea. The substantial match in water content with the cornea results in the same or substantially same refractive index of the inlay and cornea. The semi-synthetic composition of the inlay (e.g., synthetic and collagen) further assists with biocompatibility.

**Interpenetrating Polymer Networks**

In some embodiments, the inlay and/or onlay comprises an interpenetrating polymer network (IPN). An IPN is a polymer comprising two or more networks that are at least partially interlaced on a molecule scale but not covalently bonded to each other and cannot be separated unless chemical bonds are broken (Intl Union of Pure& Applied Chemistry Compendium of Chemical Terminology (IUPAC Gold Book, v. 2.3.3 (2014-02-24), page 750). Mixtures of two or more polymers cannot be termed IPNs and are instead multicomponent polymer material.

The main advantage of IPNs is their mechanical strength and stability. Also IPNs provide an opportunity to have two or more polymers with distinguishing properties. By modifying the interaction of the IPNs, a synergy can be achieved, which results in enhanced performance that surpasses that of either of the original polymers. Purkait, MK, et al. Interface Science and Technology (2018) vol. 25, chapter 3, section 3.2.3: 67-113).

In some embodiments, the hydrogel polymer comprises an interpenetrating network (IPN) which includes two or more polymeric units in the network in which the polymers are interlaced with each other (Maity, S. et al. Green approaches in medicinal chemistry for sustainable drug design. Advances in Green Chemistry (2020) 617-49, citing Dragan, E.S. "Design and applications of interpenetrating polymer network hydrogels. A review. Chem. Eng. J. (2014) 243: 572-90).

For the preparation of an IPN hydrogel, the polymers should meet the following criteria. First, there should be one polymer which can be synthesized and/or cross-linked with the other. Second, the polymers should have similar reaction rates. Lastly, there should not be any phase separation between/among the polymers (Id., citing (Bajpai, AK et al. Responsive polymers in controlled drug delivery. Prog. Polym. Sci. (2008) 33: 1088-18). Advantages of IPN hydrogels include their viscoelastic properties and easy swelling behavior without dissolving in any solvent (Id.). IPNs can be prepared by chemistry and (ii) by structure [Id., citing Myung, D. et al. Polym. Adv. Technol. (2008) 647-57; Naseri, N. et al. Biomacromolecules (2016) 17: 3714-23.

Depending on the chemistry of preparation, IPN hydrogels can be divided into simultaneous IPNs or sequential IPNs. In simultaneous IPNs, both the networks are prepared simultaneously from the precursors by independent, noninterfering routs that will not interfere with one another. In sequential IPNs, a network is made of a single network hydrogel by swelling into a solution comprising the mixture of monomer, initiator and activator, with or without a cross-linker. (Id.).

Depending on the structure, IPN hydrogels can be categorized into the following types:

(a) full IPNS which are composed of two networks that are ideally juxtaposed, with many entanglements and interactions between the networks;

(b) homo-IPNs, where the two polymers used in the networks are the same;

(c) Semi- or pseudo-IPNs, which is a way of blending of two polymers, where one is cross-linked in the presence of the other to produce a mixture of fine morphology; additional noncovalent interaction between the two polymers can influence the surface mo9rphology and the thermal properties of the semi-IPN gel;

(d) latex IPNs, which result from emulsion polymerization. The morphology of the latex IPN depends on the polymerization techniques of the IPN components;

(e) thermoplastic IPNs, which can be moldable, extruded and recycled. At least one component generally is a block copolymer. (Id.).

**Embodiments - Corneal Onlay Device**

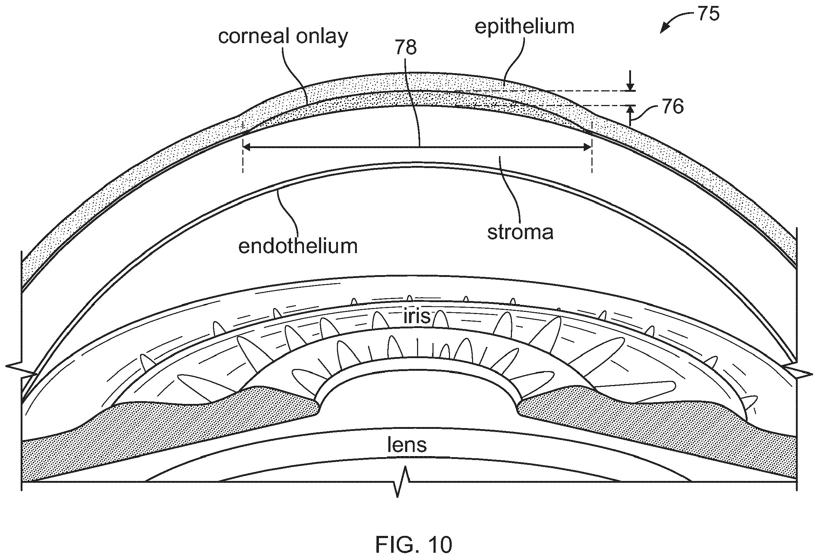

The present disclosure relates to a corneal onlay device, insertion means, and construction means, as discussed in detail below in connection with **FIGS. 10-11.**

**FIG. 10** is a diagram showing an example of a corneal onlay 75 of the present disclosure. The corneal onlay 75 includes a thickness 76 and a diameter 78. The corneal onlay 75 can have a dome or meniscus shape, including a flat or flat-like base and a dome or more or less spherical, convex shaped top. According to some embodiments, the onlay 75 can include a base curve and a front curve, or be dome-shaped. The thickness 76 can range from about 15 microns to about 30 microns. Because the corneal onlay 75 is biocompatible with the eye, it is capable of correcting presbyopia while reducing or eliminating the risk of a patient developing corneal haze. The corneal onlay 75 can define a diameter 78 dimensioned smaller than the diameter of the pupil To provide near vision, the corneal onlay 75 can be implanted centrally in the cornea under the epithelium and over the Bowman's membrane to induce an "effect" zone on the anterior epithelium surface that is smaller than the optical zone of the cornea, wherein the "effect" zone is the area of the anterior corneal surface affected by the corneal onlay 75. In some embodiments, the corneal onlay 75 can be a corneal implant device implanted on an anterior surface of the cornea. The implanted corneal onlay 75 increases the curvature of the anterior corneal surface within the "effect" zone, thereby increasing the diopter power of the cornea within the "effect" zone by +1.5 to +3 diopters, inclusive. Because the corneal onlay 75 is smaller than the diameter of the pupil, light rays from distant objects bypass the onlay and refract through the region of the cornea peripheral to the "effect" zone to create an image of distant objects on the retina.

To provide near vision, the corneal onlay of the present disclosure may be implanted centrally on Bowman's membrane after removal of at least a portion of the epithelium, oriented substantially centrally to the cornea to induce an "effect" zone on the anterior surface of the regrown epithelium. A regrowth period of 24-48 hours may allow the epithelium to regrow over the onlay. The implanted corneal onlay may increase the curvature of the anterior surface of the regrown epithelium within the "effect zone". Because the corneal onlay is smaller than the diameter of the pupil, light rays from distant objects bypass the onlay and refract through the region of the cornea peripheral to the "effect" zone to create an image of distant objects on the retina.

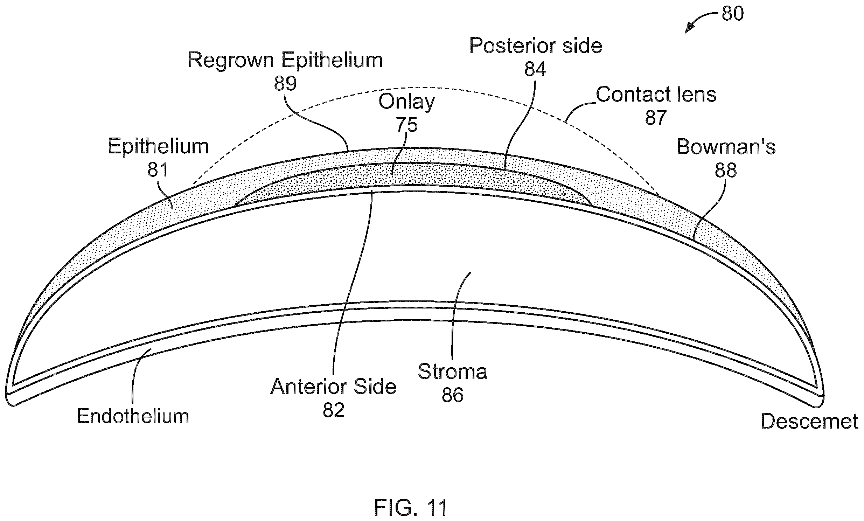

**FIG.** 11 is a schematic diagram showing the corneal onlay 75 implanted on Bowman's membrane 88. The corneal onlay 75 may have a substantially dome shape with an anterior surface 82 and a posterior surface 84. According to some embodiments, the surgical procedure may initially involve removal of at least a portion (a central portion) of the epithelium 81 dimensioned to allow for positioning of the corneal onlay 75 onto the Bowman's membrane 88. The onlay 75 can be oriented substantially centrally relative to the cornea 80. A biocompatible adhesive may be used to assist in adhering the onlay 75 to the Bowman's membrane 88. The stroma 86 remains unharmed during this procedure. Optionally, a contact lens 87 may be positioned on the epithelium 81 to cover the onlay 75 and the opening in the epithelium 81. After the epithelium regrows into the surgical area 89 to cover the onlay 75, the contact lens 87 may be removed.

According to some embodiments, the surgical procedure may initially involve cutting of a flap in the epithelium 81 to expose the Bowman's membrane 88. According to some embodiments, a portion of the anterior side of the epithelium 81 may be removed to accommodate positioning of the onlay 75 onto the Bowman's membrane 88. According to some embodiments, a biocompatible adhesive may be used to assist in adhering the onlay 75 to the Bowman's membrane 88. According to some embodiments, the flap of the epithelium 81 may be positioned over the onlay 75. Optionally, a contact lens 87 may be positioned on the epithelium 81 to cover the onlay 75. After the epithelium heals, the contact lens 87 may be removed. In some embodiments, the corneal onlay 75 may change the refractive power of the cornea by altering the shape of the anterior corneal surface.

The elastic (Young's) modulus of the corneal onlay 75 may, by way of example, be 0.18 megapascals ("MPa") with a tolerance of ±0.06 MPa. However, in some embodiments, the elastic modulus of the corneal onlay 75 may exceed the tolerance. In some embodiments, the elastic modulus of the corneal onlay 75 may be at least 0.05 MPa. In some embodiments, the elastic modulus of the corneal onlay 75 may be at least 0.06 MPa. In some embodiments, the elastic modulus of the corneal onlay 75 may be at least 0.07 MPa. In some embodiments, the elastic modulus of the corneal onlay 75 may be at least 0.08 MPa. In some embodiments, the elastic modulus of the corneal onlay 75 may be at least 0.09 MPa. In some embodiments, the elastic modulus of the corneal onlay 75 may be at least 0.10 MPa. In some embodiments, the elastic modulus of the corneal onlay 75 may be at least 0.11 MPa. In some embodiments, the elastic modulus of the corneal onlay 75 may be at least 0.12 MPa. In some embodiments, the elastic modulus of the corneal onlay 75 may be at least 0.13 MPa. In some embodiments, the elastic modulus of the corneal onlay 75 may be at least 0.14 MPa. In some embodiments, the elastic modulus of the corneal onlay 75 may be at least 0.15 MPa. In some embodiments, the elastic modulus of the corneal onlay 75 may be at least 0.16 MPa. In some embodiments, the elastic modulus of the corneal onlay 75 may be at least 0.17 MPa. In some embodiments, the elastic modulus of the corneal onlay 75 may be at least 0.18 MPa. In some embodiments, the elastic modulus of the corneal onlay 75 may be at least 0.19 MPa. In some embodiments, the elastic modulus of the corneal onlay 75 may be at least 0.20 MPa. In some embodiments, the elastic modulus of the corneal onlay 75 may be at least 0.21 MPa. In some embodiments, the elastic modulus of the corneal onlay 75 may be at least 0.22 MPa. In some embodiments, the elastic modulus of the corneal onlay 75 may be at least 0.23 MPa. In some embodiments, the elastic modulus of the corneal onlay 75 may be at least 0.24 MPa. In some embodiments, the elastic modulus of the corneal onlay 75 may be at least 0.25 MPa. In some embodiments, the elastic modulus of the corneal onlay 75 may be at least 0.26 MPa. In some embodiments, the elastic modulus of the corneal onlay 75 may be at least 0.27 MPa. In some embodiments, the elastic modulus of the corneal onlay 75 may be at least 0.28 MPa. In some embodiments, the elastic modulus of the corneal onlay 75 may be at least 0.29 MPa. In some embodiments, the elastic modulus of the corneal onlay 75 may be at least 0.30 MPa.

In some embodiments, the elongation at break of the corneal onlay 75 may be 58.30% with a tolerance of ±4.49%. However, in some embodiments, the elongation at break of the corneal onlay 75 may exceed the tolerance. In some embodiments, the elongation at break of the corneal onlay 75 may be at least 48%. In some embodiments, the elongation at break of the corneal onlay 75 may be at least 49%. In some embodiments, the elongation at break of the corneal onlay 75 may be at least 50%. In some embodiments, the elongation at break of the corneal onlay 75 may be at least 21%. In some embodiments, the elongation at break of the corneal onlay 75 may be at least 52%. In some embodiments, the elongation at break of the corneal onlay 75 may be at least 53%. In some embodiments, the elongation at break of the corneal onlay 75 may be at least 54%. In some embodiments, the elongation at break of the corneal onlay 75 may be at least 55%. In some embodiments, the elongation at break of the corneal onlay 75 may be at least 56%. In some embodiments, the elongation at break of the corneal onlay 75 may be at least 57%. In some embodiments, the elongation at break of the corneal onlay 75 may be at least 58%. In some embodiments, the elongation at break of the corneal onlay 75 may be at least 59%. In some embodiments, the elongation at break of the corneal onlay 75 may be at least 60%. In some embodiments, the elongation at break of the corneal onlay 75 may be at least 61%. In some embodiments, the elongation at break of the corneal onlay 75 may be at least 62%. In some embodiments, the elongation at break of the corneal onlay 75 may be at least 63%. In some embodiments, the elongation at break of the corneal onlay 75 may be at least 64%. In some embodiments, the elongation at break of the corneal onlay 75 may be at least 65%. In some embodiments, the elongation at break of the corneal onlay 75 may be at least 66%. In some embodiments, the elongation at break of the corneal onlay 75 may be at least 67%. In some embodiments, the elongation at break of the corneal onlay 75 may be at least 68%. In some embodiments, the elongation at break of the corneal onlay 75 may be at least 69%. In some embodiments, the elongation at break of the corneal onlay 75 may be at least 70%.

In some embodiments, the tensile strength (meaning the resistance of a material to breaking under tension) of the corneal onlay 75 may be 0.07 MPa with a tolerance of ±0.02 MPa. In some embodiments, the tensile strength of the corneal onlay 75 may exceed the tolerance. In some embodiments, the tensile strength of the corneal onlay 75 may be at least 0.01 MPa. In some embodiments, the tensile strength of the corneal onlay 75 may be at least 0.02 MPa. In some embodiments, the tensile strength of the corneal onlay 75 may be at least 0.03 MPa. In some embodiments, the tensile strength of the corneal onlay 75 may be at least 0.04 MPa. In some embodiments, the tensile strength of the corneal onlay 75 may be at least 0.05 MPa. In some embodiments, the tensile strength of the corneal onlay 75 may be at least 0.06 MPa. In some embodiments, the tensile strength of the corneal onlay 75 may be at least 0.07 MPa. In some embodiments, the tensile strength of the corneal onlay 75 may be at least 0.08 MPa. In some embodiments, the tensile strength of the corneal onlay 75 may be at least 0.09 MPa. In some embodiments, the tensile strength of the corneal onlay 75 may be at least 0.10 MPa. In some embodiments, the tensile strength of the corneal onlay 75 may be at least 0.11 MPa. In some embodiments, the tensile strength of the corneal onlay 75 may be at least 0.12 MPa. In some embodiments, the tensile strength of the corneal onlay 75 may be at least 0.13 MPa. In some embodiments, the tensile strength of the corneal onlay 10 may be at least 0.14 MPa. In some embodiments, the tensile strength of the corneal onlay 75 may be at least 0.15 MPa.

In some embodiments, the backscatter (meaning deflection of radiation or particles through an angle of 180°) of the corneal onlay 75 may be 0.90% with a tolerance of ±0.17%. However, in some embodiments, the backscatter of the corneal onlay 75 may exceed the tolerance. In some embodiments, the backscatter of the corneal onlay 75 may be at least 0.65%. In some embodiments, the backscatter of the corneal onlay 75 may be at least 0.66%. In some embodiments, the backscatter of the corneal onlay 75 may be at least 0.67%. In some embodiments, the backscatter of the corneal onlay 75 may be at least 0.68%. In some embodiments, the backscatter of the corneal onlay 75 may be at least 0.69%. In some embodiments, the backscatter of the corneal onlay 75 may be at least 0.70%. In some embodiments, the backscatter of the corneal onlay 75 may be at least 0.71%. In some embodiments, the backscatter of the corneal onlay 75 may be at least 0.72%. In some embodiments, the backscatter of the corneal onlay 75 may be at least 0.73%. In some embodiments, the backscatter of the corneal onlay 75 may be at least 0.74%. In some embodiments, the backscatter of the corneal onlay 75 may be at least 0.75%. In some embodiments, the backscatter of the corneal onlay 75 may be at least 0.76%. In some embodiments, the backscatter of the corneal onlay 75 may be at least 0.77%. In some embodiments, the backscatter of the corneal onlay 75 may be at least 0.78%. In some embodiments, the backscatter of the corneal onlay 75 may be at least 0.79%. In some embodiments, the backscatter of the corneal onlay 75 may be at least 0.80%. In some embodiments, the backscatter of the corneal onlay 75 may be at least 0.81%. In some embodiments, the backscatter of the corneal onlay 75 may be at least 0.82%. In some embodiments, the backscatter of the corneal onlay 75 may be at least 0.83%. In some embodiments, the backscatter of the corneal onlay 75 may be at least 0.84%. In some embodiments, the backscatter of the corneal onlay 75 may be at least 0.85%. In some embodiments, the backscatter of the corneal onlay 75 may be at least 0.86%. In some embodiments, the backscatter of the corneal onlay 75 may be at least 0.87%. In some embodiments, the backscatter of the corneal onlay 75 may be at least 0.88%. In some embodiments, the backscatter of the corneal onlay 75 may be at least 0.89%. In some embodiments, the backscatter of the corneal onlay 75 may be at least 0.90%. In some embodiments, the backscatter of the corneal onlay 75 may be at least 0.91%. In some embodiments, the backscatter of the corneal onlay 75 may be at least 0.92%. In some embodiments, the backscatter of the corneal onlay 75 may be at least 0.93%. In some embodiments, the backscatter of the corneal onlay 75 may be at least 0.94%. In some embodiments, the backscatter of the corneal onlay 75 may be at least 0.95%. In some embodiments, the backscatter of the corneal onlay 75 may be at least 0.96%. In some embodiments, the backscatter of the corneal onlay 75 may be at least 0.97%. In some embodiments, the backscatter of the corneal onlay 75 may be at least 0.98%. In some embodiments, the backscatter of the corneal onlay 75 may be at least 0.99%. In some embodiments, the backscatter of the corneal onlay 75 may be at least 1.00%. In some embodiments, the backscatter of the corneal onlay 75 may be at least 1.01%. In some embodiments, the backscatter of the corneal onlay 75 may be at least 1.02%. In some embodiments, the backscatter of the corneal onlay 75 may be at least 1.03%. In some embodiments, the backscatter of the corneal onlay 75 may be at least 1.04%. In some embodiments, the backscatter of the corneal onlay 75 may be at least 1.05%. In some embodiments, the backscatter of the corneal onlay 75 may be at least 1.06%. In some embodiments, the backscatter of the corneal onlay 75 may be at least 1.07%. In some embodiments, the backscatter of the corneal onlay 75 may be at least 1.08%. In some embodiments, the backscatter of the corneal onlay 75 may be at least 1.09%. In some embodiments, the backscatter of the corneal onlay 75 may be at least 1.10%. In some embodiments, the backscatter of the corneal onlay 75 may be at least 1.11%. In some embodiments, the backscatter of the corneal onlay 75 may be at least 1.12%. In some embodiments, the backscatter of the corneal onlay 75 may be at least 1.13%. In some embodiments, the backscatter of the corneal onlay 75 may be at least 1.14%. In some embodiments, the backscatter of the corneal onlay 75 may be at least 1.15%.

In some embodiments, the light transmission (meaning the moving of electromagnetic waves through) of the corneal onlay 75 may be 92.4% with a tolerance of ±0.95%. In some embodiments, the elastic modulus of the corneal onlay 75 may exceed the tolerance. In some embodiments, the light transmission of the corneal onlay 75 may be at least 85.0%. In some embodiments, the light transmission of the corneal onlay 75 may be at least 86.0%. In some embodiments, the light transmission of the corneal onlay 75 may be at least 87.0%. In some embodiments, the light transmission of the corneal onlay 75 may be at least 88.0%. In some embodiments, the light transmission of the corneal onlay 75 may be at least 89.0%. In some embodiments, the light transmission of the corneal onlay 75 may be at least 90.0%. In some embodiments, the light transmission of the corneal onlay 75 may be at least 91.0%. In some embodiments, the light transmission of the corneal onlay 75 may be at least 92.0%. In some embodiments, the light transmission of the corneal onlay 75 may be at least 93.0%. In some embodiments, the light transmission of the corneal onlay 75 may be at least 94.0%. In some embodiments, the light transmission of the corneal onlay 75 may be at least 95.0%. In some embodiments, the light transmission of the corneal onlay 75 may be at least 96.0%. In some embodiments, the light transmission of the corneal onlay 75 may be at least 97.0%. In some embodiments, the light transmission of the corneal onlay 75 may be at least 98.0%. In some embodiments, the light transmission of the corneal onlay 75 may be at least 99.0%. In some embodiments, the light transmission of the corneal onlay 75 may be 100.0%.

An adhesive material for adhering the onlay to Bowman's membrane would need to be tested to confirm its refractive index, and safety when used in the eye. Many commercial adhesives are available and described by [Nam, S. and Mooney, D. Chem. Rev. (2020) doi.org/10.1021/acs.chemistry/0c00798]. For example, naturally derived proteins can be used to fabricate tissue adhesives. For example, collagen itself may have adhesive properties. Similarly, gelatin, an irreversibly hydrolyzed form of collagen, is widely used in adhesives. [Id., citing Gomez-Guillen, MC et al. Food Hydrocolloids (2011) 25: 1813-27; Yue, K. et al. Biomaterial (2015) 73: 254-71]. Albumin combined with functionalized PEG forms a hydrogel adhesive. [Id., citing Fuller, C. J. Cardiothorac. Surg. (2013) 8. DOI: 10.1186/1749-8090-8-90]. Fibrin-based adhesives (e.g., Tisseal, Baxter) are known; fibrin glues, which are biologically compatible and biodegradable, usually do not lead to inflammatory responses, foreign-body reactions, tissue necrosis or fibrosis, and have been widely used in a variety of surgical applications. [Id., citing Duarte, AP et al. Prog. Polym. Sci. (2012) 37: 1031-50,Seyednejad, H. et al. Br. J. Surg. (2008) 95: 1197-1225]. PEG is FDA approved in a number of applications and is broadly considered to be nontoxic, biocompatible and nonimmunogenic. [Id., citing Mehdizadeh, M. and Yang, J. Macromol. Biosci. (2013) 13: 271-88]. For example DuraSeal Xact Adhesion Barrier and Sealant System (DSX) utilizes an NHS ester to achieve a hydrogel with adhesive cross-linking and bonding to tissues with reduced swelling [Id., citing Preul, MC et al. J. Neurosurg. Spin (2010) 12: 381-90; Fransen, P. Spine J. (2010) 10: 751-61; Kim, K. D et al. Global Spine J. (2019) 9: 272-8]. A recent study explored a tissue adhesive with reversible adhesion in which adhesive patches of poly(vinyl alcohol) and poly (acrylic acid) (PAA) were grafted with NHS esters; The NHS ester groups were linked to the PAA chains through disulfide bonds that act as cleavable linkages. [Id., citing Chen, CX. et al. (2020) Proc. Natl Acad. Sci. USA 117: 15497-15503}. Another product, Cell-Tak is a nonimmunogenic extracellular matrix protein preparation isolated from the marine mussel, Mytilus edulis (Corning] In some embodiments, photochemical tissue bonding (PTB) using either Riboflavin or Rose Bengal and UV light may be implemented to secure or glue the onlay in the desired position. [Redmond, Robert W. et al. (2019) Photochemistry and Photobiology, 95: 1097-1115]. In some embodiments, cyanoacrylate glue (typically used for corneal lacerations/perforations and gluing small corneal patches on corneal perforations, corneal melts, and corneal wound leaks) may be used to secure or glue the onlay in the desired position. [Vote, BJ et al., (2000) Clin Exp Ophthalmol., Dec;28(6):437-42].

In some embodiments, the morphology (meaning form) of the corneal onlay 75 may be that of a fibrillary network with nano-pores. In some embodiments, the nano-pores of the corneal onlay 75 may have a diameter of at least 0.1 $\mu$m. In some embodiments, the nano-pores of the corneal onlay 75 may have a diameter of at least 0.2 $\mu$m. In some embodiments, the nano-pores of the corneal onlay 75 may have a diameter of at least 0.3 $\mu$m. In some embodiments, the nano-pores of the corneal onlay 75 may have a diameter of at least 0.4 $\mu$m. In some embodiments, the nano-pores of the corneal onlay 75 may have a diameter of at least 0.5 $\mu$m. In some embodiments, the nano-pores of the corneal onlay 75 may have a diameter of at least 0.6 $\mu$m. In some embodiments, the nano-pores of the corneal onlay 75 may have a diameter of at least 0.7 $\mu$m. In some embodiments, the nano-pores of the corneal onlay 75 may have a diameter of at least 0.8 $\mu$m. In some embodiments, the nano-pores of the corneal onlay 75 may have a diameter of at least 0.9 $\mu$m. In some embodiments, the nano-pores of the corneal onlay 75 may have a diameter of at least 1.0 $\mu$m. In some embodiments, the nano-pores may have a diameter of approximately 0.4 $\mu$m. In some embodiments, the storage temperature for the corneal onlay 75 may range from about 2° - 8° Celsius, i.e., about 2°C, 2.5°C, 3°C, 3.5°C, 4°C, 4.5°C, 5°C, 5.5°C, 6°C, 6.5°C, 7°C, 7.5°C, 8°C.

**Fabrication**

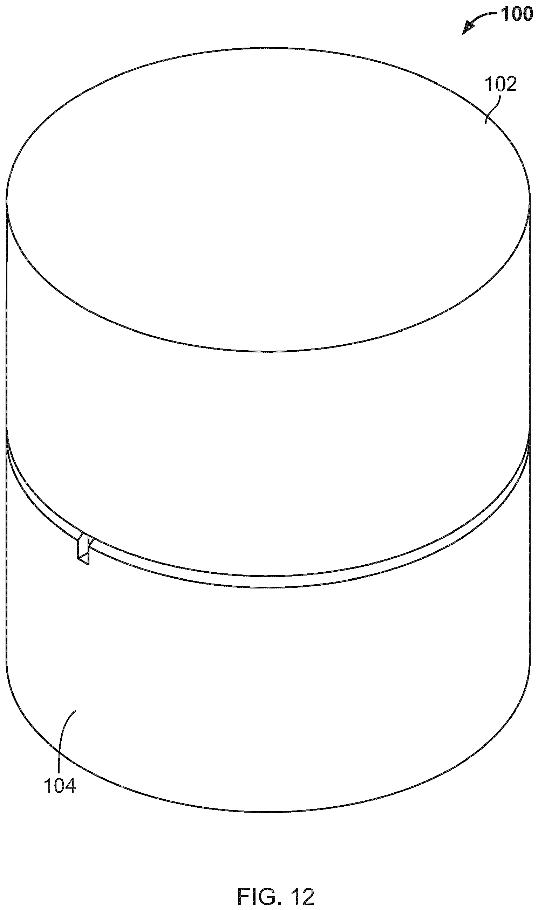

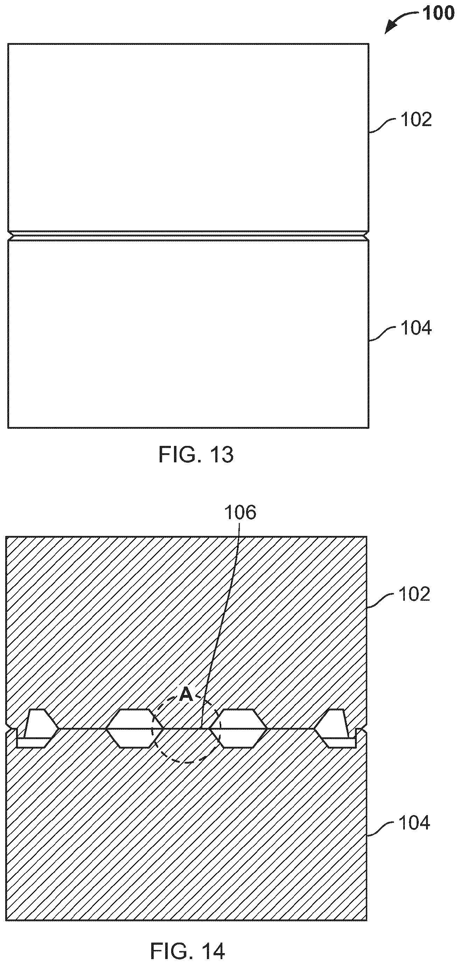

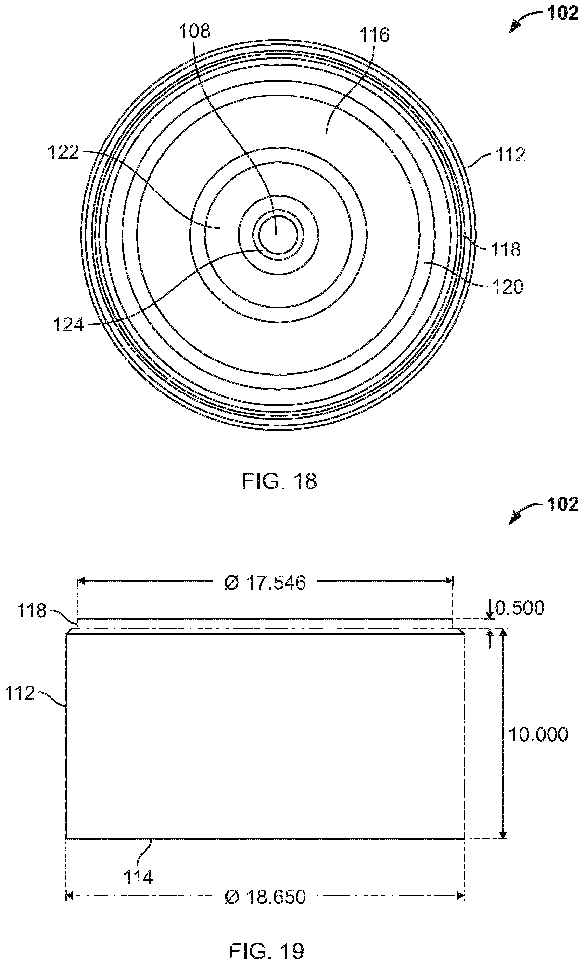

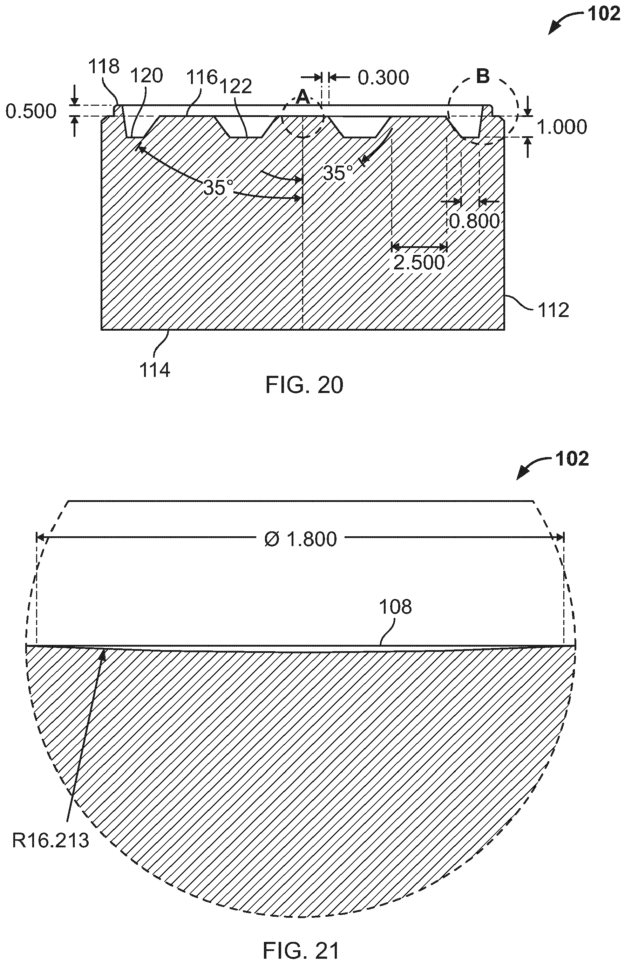

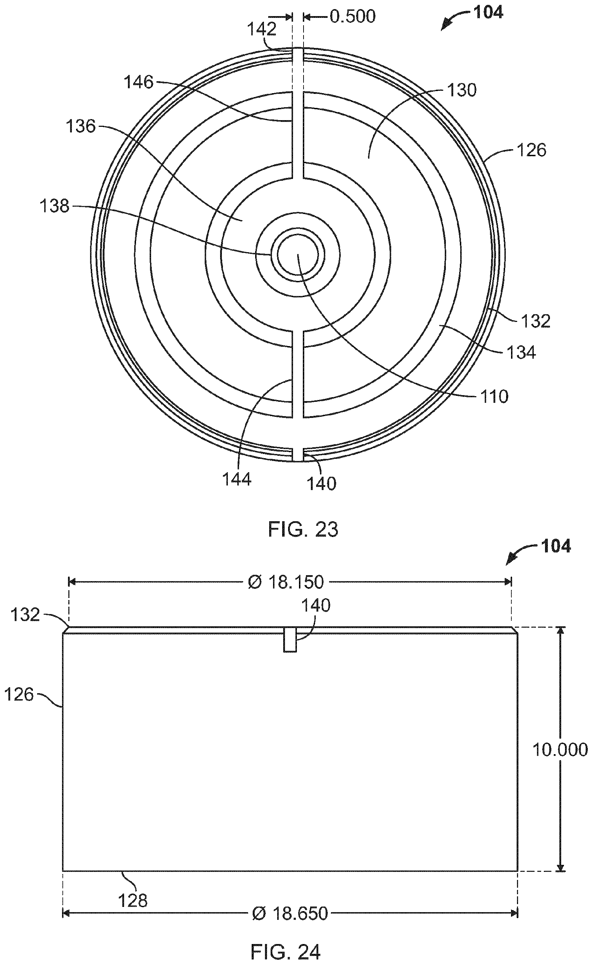

Molding can be used to fabricate the hydrogel inlays discussed herein. Due to the softness and presence of water in the pre-mix, molding is used to fabricate the inlay (instead of lathing or machining the inlay into a final shape). In particular, the high and low water content compositions were used to mold hydrogel inlays for testing. To fabricate the inlay, uncrosslinked hydrogel composition was cast in a cavity mold assembly made from, e.g., Poly(methyl methacrylate) (PMMA), or the like. **FIGS. 12-26** show perspective, cross-sectional and detailed views of components of an exemplary mold assembly 100 for fabricating or forming the hydrogel inlays discussed herein. Although the mold assembly 100 is used to fabricate or form the inlays, it should be understood that a substantially similar mold assembly 100 can be used to form the onlay, except as discussed herein.

With respect to **FIGS. 2-16,** the mold assembly 100 generally includes a first mold section 102 (e.g., a male mold section) and a second mold section 104 (e.g., a female mold section). The mold assembly 100 can be fabricated for single-use purposes. The material of fabrication of the mold assembly 100 can be transparent to UV light, allowing for cross-linking of the hydrogel composition within the mold assembly 100. The mold sections 102, 104 are configured to fit complementary to each other to form a cavity 106 therebetween. The cavity 106 is in the form of the inlay to be fabricated. As discussed below, each of the mold sections 102, 104 includes one or more radial complementary channels that can assist with escape of air during molding.

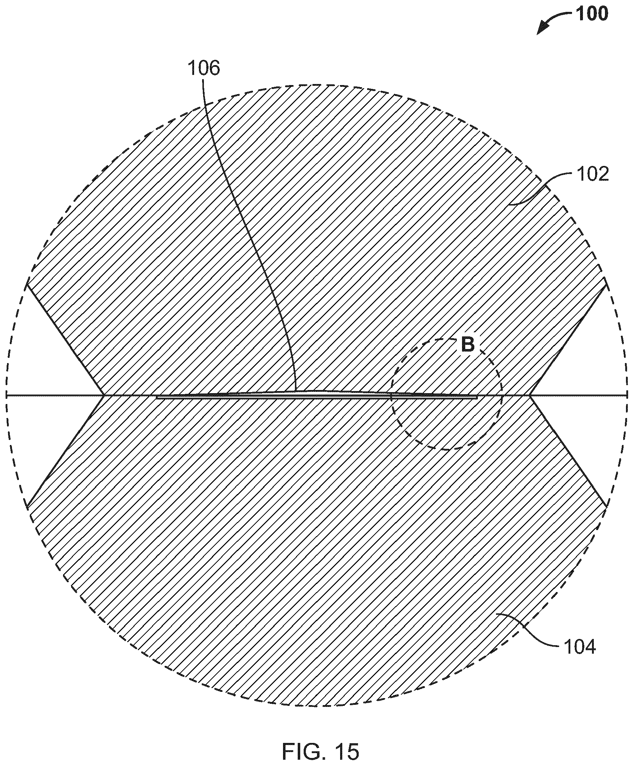

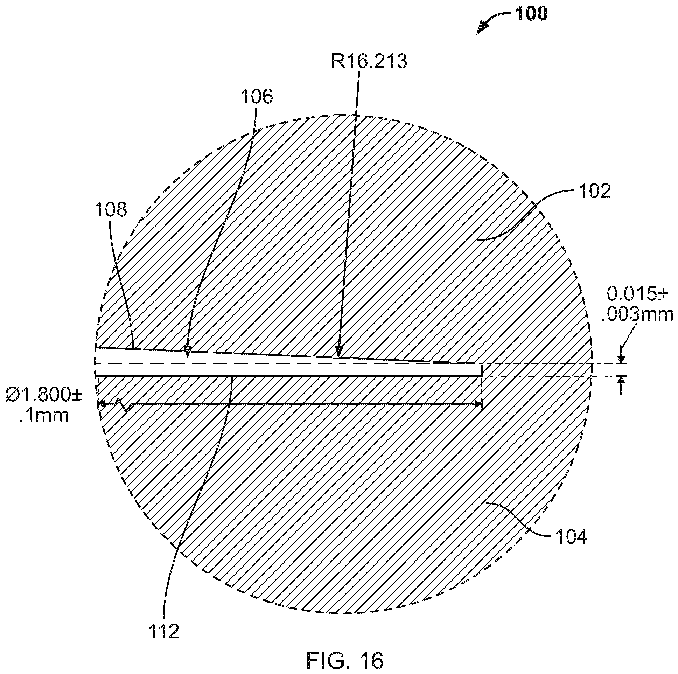

As illustrated in **FIGS. 15 and 16,** the first mold section 102 includes a hemispherical cavity 108 formed in the mating surface, while the second mold section 104 includes a flat cavity 110 formed in the mating surface. When mated against each other, the cavities 108, 110 align to define the overall shape or form of the hydrogel inlay. Both cavities 108, 110 define a substantially circular shape. In some embodiments, the diameter of the hemispherical cavity 108 can be about, e.g., 1.8-2.2 mm, inclusive, 1.8-2.1 mm inclusive, 1.8-2.0 mm inclusive, 1.8-1.9 mm inclusive, 1.9-2.2 mm inclusive, 2.0-2.2 mm inclusive, 2.1-2.2 mm inclusive, 1.8 mm, 1.9 mm, 2.0 mm, 2.1 mm, 2.2 mm, or the like. In some embodiments, the radius of the hemispherical cavity 108 can be about, e.g., 16.213 mm. In some embodiments, the diameter of the flat cavity 110 can be about, e.g., 1.8-2.2 mm, inclusive, 1.8-2.1 mm inclusive, 1.8-2.0 mm inclusive, 1.8-1.9 mm inclusive, 1.9-2.2 mm inclusive, 2.0-2.2 mm inclusive, 2.1-2.2 mm inclusive, 1.8 mm, 1.9 mm, 2.0 mm, 2.1 mm, 2.2 mm, or the like. In some embodiments, the edge thickness of the cavity (i.e., cavities 108, 110 mated together) can be about, e.g., 0.015-0.025 mm, 0.015-0.02 mm, 0.02-0.025 mm, 0.015 mm, 0.02 mm, 0.025 mm, or the like. In some embodiments, the center thickness of the entire cavity can be about, e.g., 40-60 nm, inclusive, 40-55 nm, inclusive, 40-50 nm, inclusive, 40-45 nm, inclusive, 45-60 nm, inclusive, 50-60 nm, inclusive, 55-60 nm, inclusive, 45-55 nm, inclusive, 40 nm, 45 nm, 50 nm, 55 nm, 60 nm, or the like.

With respect to formation of an onlay, the first mold section 102 can include a hemispherical cavity 108 formed in the mating surface, while the second mold section 104 can include a substantially flat cavity 110 formed in the mating surface. When mated against each other, the cavities 108, 110 align to define the overall shape or form of the hydrogel onlay (e.g., a dome-shaped onlay). Accordingly to some embodiments, the cavity 110 can define an upwardly curved or hemispherical form such that when mated against each other, the cavities 108, 110 align to define an onlay having curved front (posterior) and base (posterior) surfaces). Both cavities 108, 110 define a substantially circular shape. In some embodiments, the diameter of the hemispherical cavity 108 can be about, e.g., 1.8-2.2 mm, inclusive, 1.8-2.1 mm inclusive, 1.8-2.0 mm inclusive, 1.8-1.9 mm inclusive, 1.9-2.2 mm inclusive, 2.0-2.2 mm inclusive, 2.1-2.2 mm inclusive, 1.8 mm, 1.9 mm, 2.0 mm, 2.1 mm, 2.2 mm, or the like. In some embodiments, the radius of the hemispherical cavity 108 can be about, e.g., 16.213 mm. In some embodiments, the diameter of the flat cavity 110 can be about, e.g., 1.8-2.2 mm, inclusive, 1.8-2.1 mm inclusive, 1.8-2.0 mm inclusive, 1.8-1.9 mm inclusive, 1.9-2.2 mm inclusive, 2.0-2.2 mm inclusive, 2.1-2.2 mm inclusive, 1.8 mm, 1.9 mm, 2.0 mm, 2.1 mm, 2.2 mm, or the like. In some embodiments, the edge thickness of the cavity (i.e., cavities 108, 110 mated together) can be about, e.g., 0.015-0.025 mm, 0.015-0.02 mm, 0.02-0.025 mm, 0.015 mm, 0.02 mm, 0.025 mm, or the like. In some embodiments, the center thickness of the entire cavity can be about, e.g., 15-30 microns, 15-25 microns, inclusive, 15-20 microns, inclusive, 20-30 microns, inclusive, 25-30 microns, inclusive, 15 microns, 20 microns, 25 microns, 30 microns, or the like.

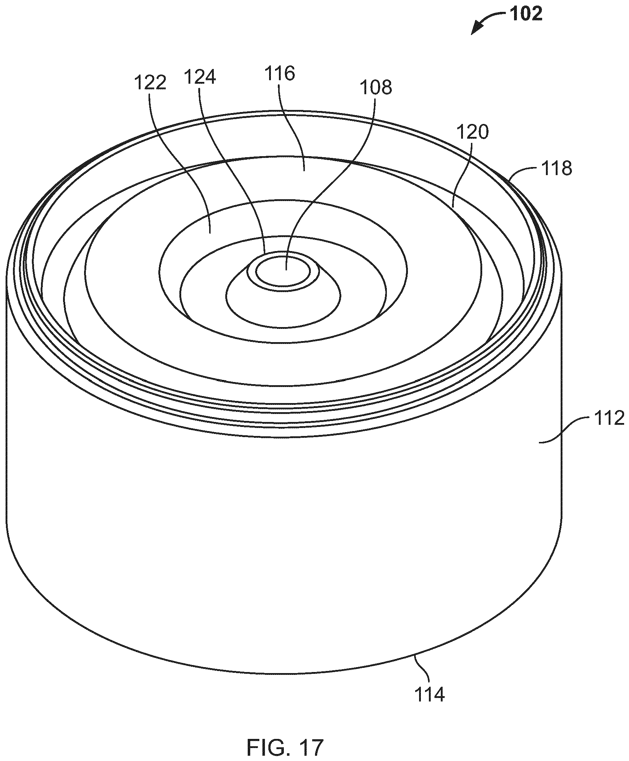

**FIGS. 17-21** are perspective, top, side, cross-sectional and detailed views of the first mold section 102. The mold section 102 includes a body 112 defining a substantially cylindrical configuration. The body 112 includes a bottom surface 114 and an opposing top surface defining the substantially planar mating surface 116. The mold section 102 includes a radial flange 118 extending near the outer perimeter of the body 112 from the top surface, and positioned slightly inwardly offset from the outer perimeter or edge of the body 112. The mold section 102 includes a first radial channel 120 inwardly formed in the top surface adjacent to the radial flange 118. The mold section 102 includes a second radial channel 122 inwardly formed in the top surface. The second radial channel 122 defines a smaller diameter than the first radial channel 120, and is therefore disposed closer to the central longitudinal axis of the mold section 102. As illustrated in **FIG. 17,** the inner walls of the channels 120, 122 taper inwardly. The inwardly extending hemispherical cavity 108 is formed in the top surface of the body 112 and is substantially aligned with a central longitudinal axis of the mold section 102. A perimeter section 124 surrounds the cavity 108 and forms part of the mating surface 116.

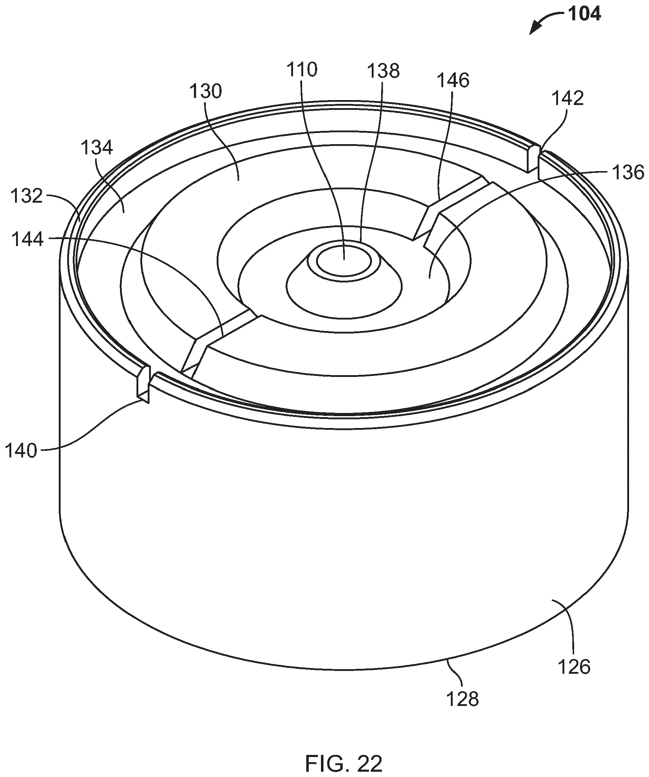

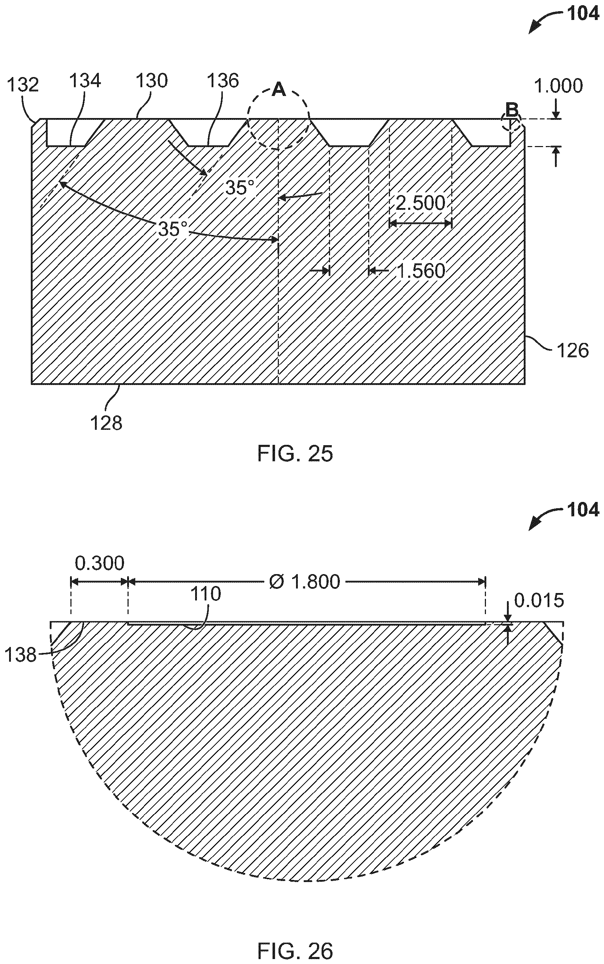

**FIGS. 22-26** are perspective, top, side, cross-sectional and detailed views of the second mold section 104. The mold section 104 includes a body 126 defining a substantially cylindrical configuration complementary to the body 112 of the mold section 102. The body 126 includes a bottom surface 128 and an opposing top surface defining the substantially planar mating surface 130. The mold section 104 includes a radial edge 132 extending at or near the perimeter of the body 126 from the top surface. The mold section 104 includes a first radial channel 134 inwardly formed in the top surface adjacent to the radial edge 132. The mold section 104 includes a second radial channel 136 inwardly formed in the top surface. The second radial channel 136 defines a smaller diameter than the first radial channel 134, and is therefore disposed closer to the central longitudinal axis of the mold section 104. The diameters and edges of the radial channels 134, 136 are dimensioned substantially complementary to the diameters and edges of the respective radial channels 120, 122 of the mold section 102. As illustrated in **FIG. 25,** the inner walls of the channels 134, 136 taper inwardly.

The flat cavity 110 is formed in the top surface of the body 126 and is substantially aligned with a central longitudinal axis of the mold section 104. A perimeter section 138 surrounds the cavity 110 and forms part of the mating surface 130. The mold section 104 includes a transverse channel, cutout or slit extending through the radial flange 132 and the raised section of the body 126 between the channels 134, 136. In particular, the transverse channel includes channels 140, 142 that extend through the radial flange 132 on opposing sides of the body 126, and channels 144, 146 that extend through the raised section of the body 126 between the channels 134, 136. The bottom of the channels 140-146 is substantially aligned with the bottom surface of the channels 134, 136. The channels 140-146 provide a fluidic connection between the channels 134, 136 (and also channels 120, 122 of the mold section 102 when both mold sections 102, 104 are mated to each other).

During fabrication, the uncrosslinked hydrogel composition can be placed within the cavity 110 of the mold section 104, and the mold section 102 can be aligned with and positioned over the mold section 104 such that the mating surfaces 116, 130 are positioned against each other. As illustrated in **FIGS. 12-26,** the radial flange 118 of the mold section 102 is configured and dimensioned to fit within the radial edge 132 of the mold section 104, and against the outer wall of the channel 134 of the mold section 104, to ensure alignment and mating of the mold sections 102, 104 relative to each other. In the mated position, the cavities 108, 110 are enclosed by the mating surfaces to ensure proper distribution of the hydrogel during formation of the inlay. In the mated position, the channels 120, 122, 134, 136 are aligned to allow for escape of air and/or gas from the mold assembly 100. In some embodiments, a clamp can be used to secure the mold sections 102, 104 to each other prior to crosslinking, as long as the clamp does not obstruct UV light from reaching the hydrogel in the mold cavity. Once UV crosslinking is complete, the inlay is alloyed to further crosslink at ambient conditions before the inlay is demolded, further processed and characterized.

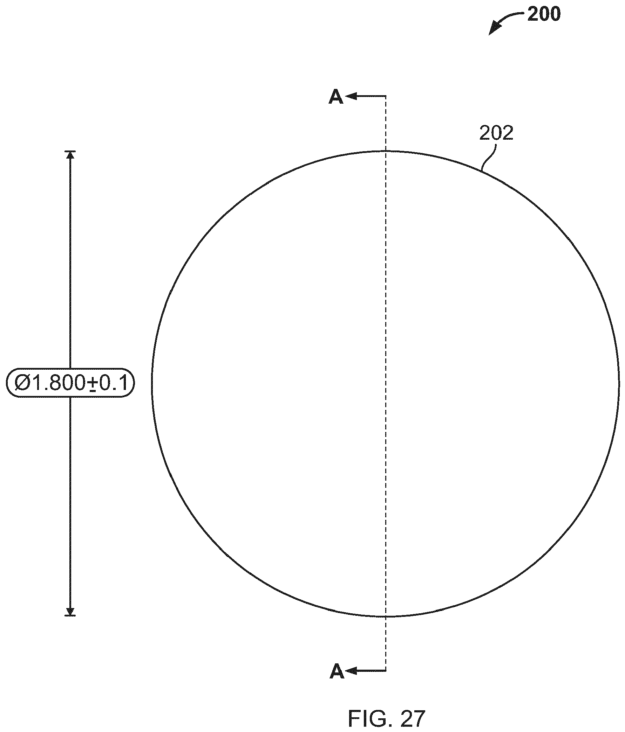

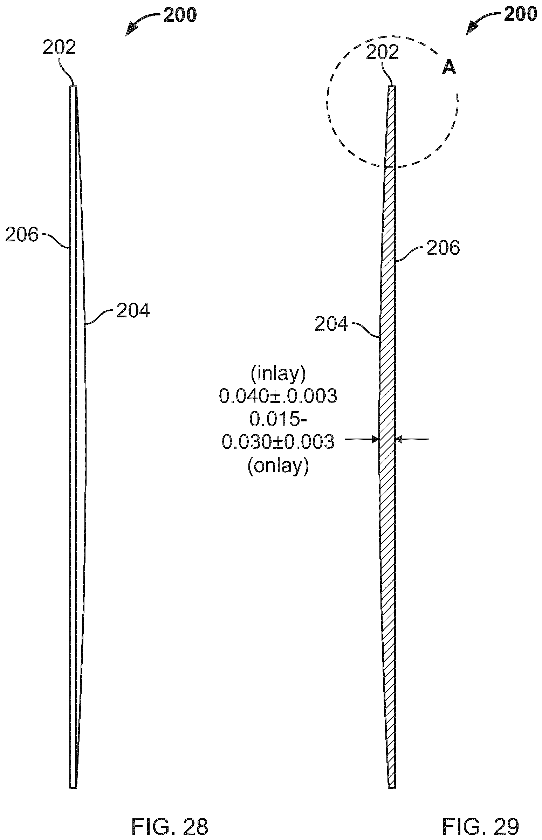

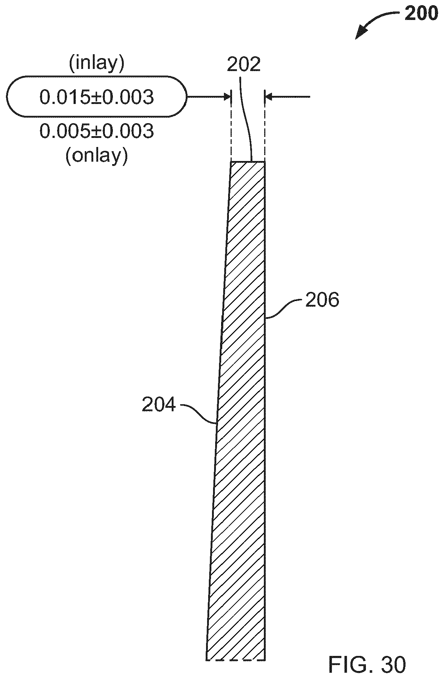

**FIGS. 27-30** are front, side and cross-sectional views of an exemplary hydrogel inlay 200 fabricated using the mold assembly 100. The inlay 200 can define a substantially circular shape of a diameter of about, e.g., 1.8-2.2 mm inclusive, 1.8-2.1 mm inclusive, 1.8-2.0 mm inclusive, 1.8-1.9 mm inclusive, 1.9-2.2 mm inclusive, 2.0-2.2 mm inclusive, 2.1-2.2 mm inclusive, 1.8 mm, 1.9 mm, 2.0 mm, 2.1 mm, 2.2 mm, or the like. The inlay 200 can define an edge thickness of about, e.g., 0.015-0.025 mm inclusive, 0.015-0.02 mm inclusive, 0.02-0.025 mm inclusive, 0.015 mm, 0.02 mm, 0.025 mm, or the like. The inlay 200 can define a center thickness of about, e.g., 0.04-0.06 mm inclusive, 0.05-0.06 mm inclusive, 0.04-0.05 mm inclusive, 0.04 mm, 0.05 mm, 0.06 mm, or the like. The inlay 200 includes a substantially flat perimeter edge 202, a convex top surface 204, and a substantially flat bottom surface 206. As illustrated in **FIG. 29,** the body of the inlay 200 is solidly formed from the hydrogel (e.g., no openings or hollow cavities), with the inlay 200 tapering from the thickest area at the central longitudinal axis to the thinnest area at the perimeter edge 202.

According to some embodiments, the onlay can define a center thickness of about, e.g., 0.015 mm to 0.03 mm, 0.02 mm to 0.03 mm, 0.025 mm to 0.03 mm, 0.015 mm to 0.025 mm, 0.015 mm to 0.02 mm, 0.02 mm to 0.025 mm, 0.015 mm, 0.02 mm, 0.025 mm, 0.03 mm, or the like.

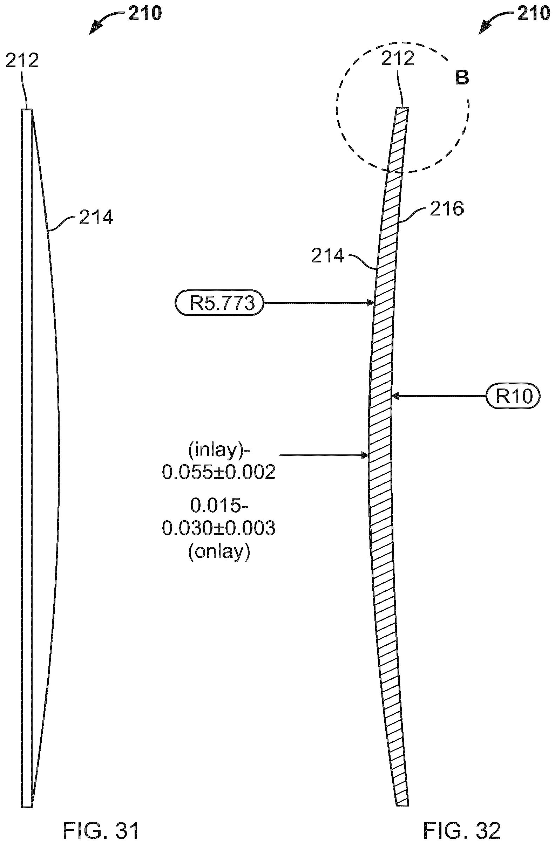

In some embodiments, the cavities 108, 110 of the mold assembly 100 can be adjusted to form a hydrogel meniscus inlay 210 shown in **FIGS. 31-33.** The inlay 210 can include a substantially circular shape having a diameter of about, e.g., 1.8-2.2 mm inclusive, 1.8-2.1 mm, 1.8-2.0 mm inclusive, 1.8-1.9 mm inclusive, 1.9-2.2 mm inclusive, 2.0-2.2 mm inclusive, 2.1-2.2 mm inclusive, 1.8 mm, 1.9 mm, 2.0 mm, 2.1 mm, 2.2 mm, or the like. The inlay 210 can have an edge thickness of about, e.g., 0.018-0.025 mm inclusive, 0.018-0.024 mm inclusive, 0.018-0.023 mm inclusive, 0.018-0.022 mm inclusive, 0.018-0.021 mm inclusive, 0.018-0.02 mm inclusive, 0.018-0.019 mm inclusive, 0.019-0.025 mm inclusive, 0.02-0.025 mm inclusive, 0.021-0.025 mm inclusive, 0.022-0.025 mm inclusive, 0.023-0.025 mm inclusive, 0.024-0.025 mm inclusive, 0.018 mm, 0.019 mm, 0.02 mm, 0.021 mm, 0.022 mm, 0.023 mm, 0.024 mm, 0.025 mm, or the like. The inlay 210 can have a center thickness of about, e.g., 0.03-0.055 mm inclusive, 0.03-0.05 mm inclusive, 0.03-0.045 mm inclusive, 0.03-0.04 mm inclusive, 0.03-0.035 mm inclusive, 0.035-0.055 mm inclusive, 0.04-0.055 mm inclusive, 0.045-0.055 mm inclusive, 0.05-0.055 mm inclusive, 0.03 mm, 0.035 mm, 0.04 mm, 0.045 mm, 0.05 mm, 0.055 mm, or the like. The inlay 210 can have an outer radius of about 5.773 mm, and an inner radius of about 10 mm. The inlay 210 includes a substantially flat perimeter edge 212, a convex top surface 214, and a concave bottom surface 216. As illustrated in **FIG. 32,** the body of the inlay 210 is solidly formed from the hydrogel, with the inlay 210 tapering from the thickest area at the central longitudinal axis to the thinnest area at the perimeter edge 212.

With respect to a hydrogel meniscus onlay 210, the onlay 210 can include a substantially circular shape having a diameter of about $1.9 \pm 0.3$ mm (e.g., 1.6-2.2 mm inclusive, 1.6-2.1 mm inclusive, 1.6-2.0 mm inclusive, 1.6-1.9 mm inclusive, 1.6-1.8 mm inclusive, 1.6-1.7 mm inclusive, 1.7-2.2 mm inclusive, 1.8-2.2 mm inclusive, 1.9-2.2 mm inclusive, 2.0-2.2 mm inclusive, 2.1-2.2 mm inclusive, 1.6 mm, 1.7 mm, 1.8 mm, 1.9 mm, 2.0 mm, 2.1 mm, 2.22 mm, or the like), an edge thickness of about 0.005-0.012 mm inclusive (e.g., 0.005-0.011 mm inclusive, 0.005-0.010 mm inclusive, 0.005-0.009 mm inclusive, 0.005-0.008 mm inclusive, 0.005-0.007 mm inclusive, 0.005-0.006 mm inclusive, 0.006-0.012 mm inclusive, 0.007-0.012 mm inclusive, 0.008-0.012 mm inclusive, 0.009-0.012 mm inclusive, 0.010-0.012 mm inclusive, 0.011-0.012 mm inclusive, 0.005 mm, 0.006 mm, 0.007 mm, 0.008 mm, 0.009 mm, 0.010 mm, 0.011 mm, 0.012 mm, or the like), a center thickness of about 0.01-0.03 mm inclusive (e.g., 0.01-0.02 mm, 0.02-0.03 mm, 0.01 mm, 0.02 mm, 0.03 mm, or the like), an outer radius of about 5.773 mm, and an inner radius of about 10 mm. The onlay 210 includes a substantially flat perimeter edge 212, a convex top surface 214, and a concave bottom surface 216. As illustrated in **FIG. 32,** the body of the onlay 210 is solidly formed from the hydrogel, with the onlay 210 tapering from the thickest area at the central longitudinal axis to the thinnest area at the perimeter edge 212.

In some embodiments, the inlay can define a substantially disc-shaped configuration. In some embodiments, the disc-shaped inlay can define a diameter of about, e.g., 1.8-2.2 mm inclusive, 1.8-2.1 mm inclusive, 1.8-2.0 mm inclusive, 1.8-1.9 mm inclusive, 1.9-2.2 mm inclusive, 2.0-2.2 mm inclusive, 2.1-2.2 inclusive, 1.8 mm, 1.9 mm, 2.0 mm, 2.1 mm, 2.2 mm, or the like. In some embodiments, the disc-shaped inlay can define a thickness of about, e.g., 20-50 $\mu$m inclusive, 20-45 $\mu$m inclusive, 20-40 $\mu$m inclusive, 20-35 $\mu$m inclusive, 20-30 $\mu$m inclusive, 20-25 $\mu$m inclusive, 25-50 $\mu$m inclusive, 30-50 $\mu$m inclusive, 35-50 $\mu$m inclusive, 40-50 $\mu$m inclusive, 45-50 $\mu$m inclusive, 20 $\mu$m, 25 $\mu$m, 30 $\mu$m, 35 $\mu$m, 40 $\mu$m, 45 $\mu$m, 50 $\mu$m, or the like. As an example, to obtain an approximately 2 mm diameter (about 40 $\mu$m thick) disc-shaped inlay, an approximately 10 mm diameter cavity mold can be used to fabricate the 10 mm disc. Once the 10 mm disc is fully hydrated, a 2 mm biopsy punch can be used to punch out the 2 mm x 40 $\mu$m disc-shaped inlay from the 10 mm disc. A similar process can be used to punch out a 15-20 mm diameter disc-shaped inlay. In some embodiments, instead of using biopsy punches, a laser beam can be used to create the disc and/or disc-shaped inlays. In some embodiments, a disc 4 mm in diameter can be implanted into the cornea, and a femtosecond laser can be used to cut out an approximately 2 mm disc-shaped inlay. The peripheral material is subsequently removed from the cornea, leaving the approximately 2 mm disc-shaped inlay implanted in the cornea.

In some embodiments, the onlay can define a substantially disc-shaped configuration. In some embodiments, the disc-shaped inlay can define a diameter of about, e.g., 1.8-2.2 mm inclusive, 1.8-2.1 mm inclusive, 1.8-2.0 mm inclusive, 1.8-1.9 mm inclusive, 1.9-2.2 mm inclusive, 2.0-2.2 mm inclusive, 2.1-2.2 inclusive, 1.8 mm, 1.9 mm, 2.0 mm, 2.1 mm, 2.2 mm, or the like. In some embodiments, the disc-shaped inlay can define a thickness of about, e.g., 10-30 $\mu$m inclusive, 10-25 $\mu$m inclusive, 10-20 $\mu$m inclusive, 10-15 $\mu$m inclusive, 15-30 $\mu$m inclusive, 20-30 $\mu$m inclusive, 25-30 $\mu$m inclusive, or the like. As an example, to obtain an approximately 2 mm diameter (about 30 $\mu$m thick) disc-shaped onlay, an approximately 10 mm diameter cavity mold can be used to fabricate the 10 mm disc. In some embodiments, 15-20 mm inclusive diameter molds can be used to fabricate the disc. Once the 10 mm disc is fully hydrated, a 2 mm biopsy punch can be used to punch out the 2 mm x 30 $\mu$m disc-shaped onlay from the 10 mm disc. In some embodiments, instead of using biopsy punches, a laser beam can be used to create the disc and/or disc-shaped inlays. In some embodiments, a disc 4 mm in diameter can be implanted into the cornea, and a femtosecond laser can be used to cut out an approximately 2 mm disc-shaped inlay. The peripheral material is subsequently removed from the cornea, leaving the approximately 2 mm disc-shaped inlay implanted in the cornea.

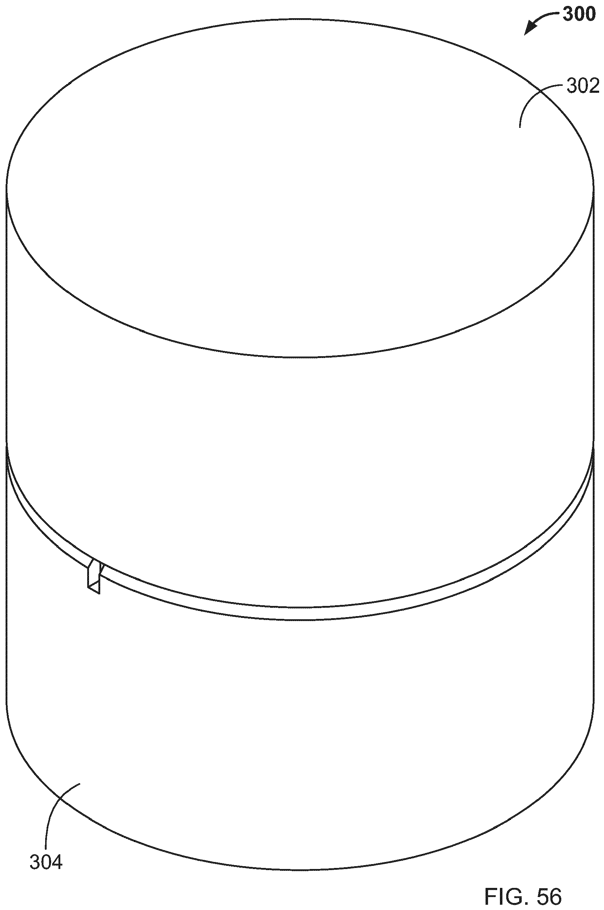

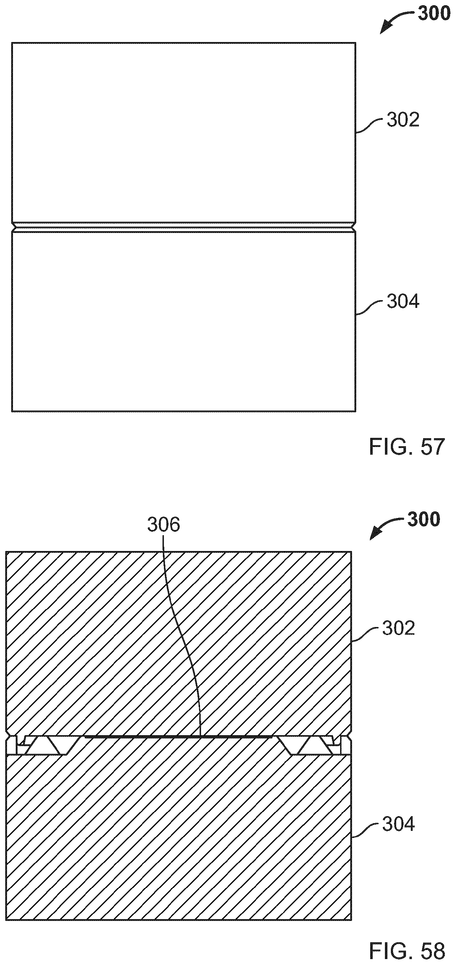

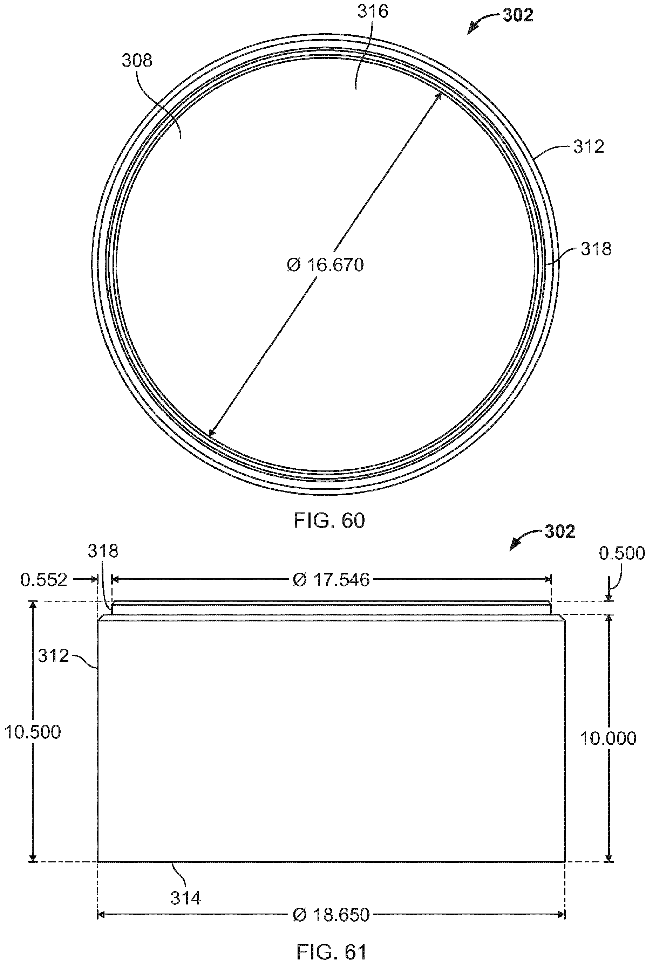

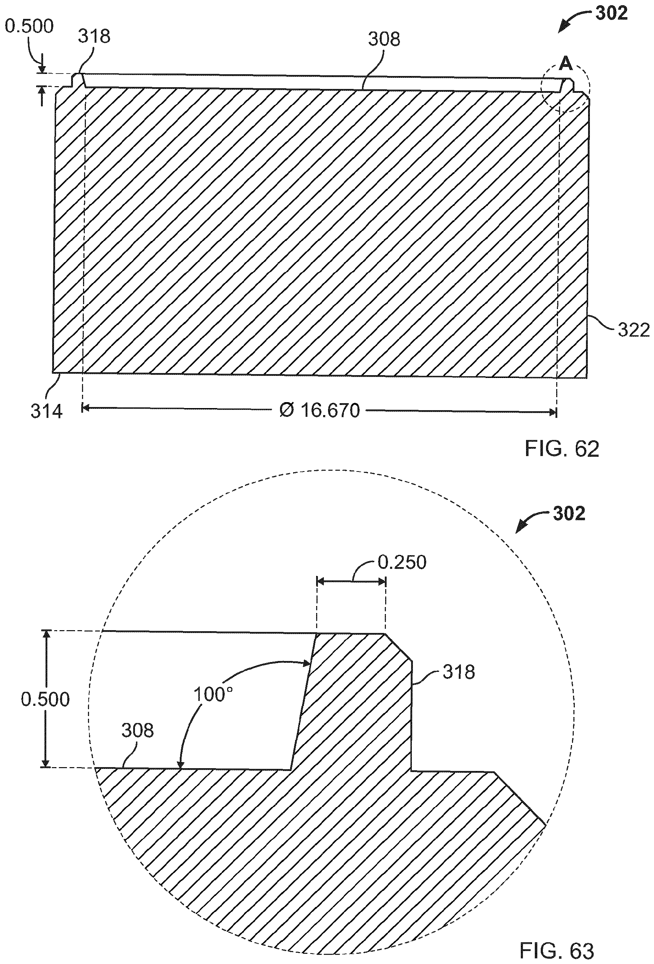

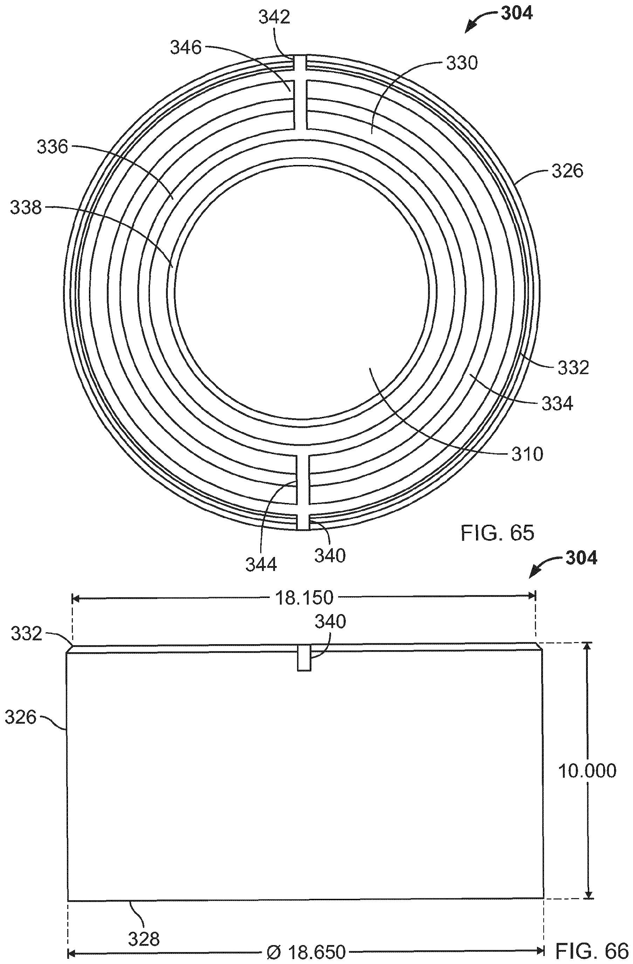

**FIGS. 56-68** show perspective, cross-sectional and detailed views of components of an exemplary mold assembly 300 for fabricating or forming the hydrogel inlays having a disc-shaped configuration discussed herein. The mold assembly 300 can be substantially similar in structure and/or function to the mold assembly 100, except for the distinctions noted herein.

With respect to **FIGS. 56-58,** the mold assembly 300 generally includes a first mold section 302 (e.g., a male mold section) and a second mold section 304 (e.g., a female mold section). The mold assembly 300 can be fabricated for single-use purposes. The material of fabrication of the mold assembly 300 can be transparent to UV light, allowing for cross-linking of the hydrogel composition within the mold assembly 300. The mold sections 302, 304 are configured to fit complementary to each other to form a cavity 306 therebetween. The cavity 306 is in the form of the disc-shaped inlay to be fabricated. As discussed below, each of the mold sections 302, 304 includes one or more radial complementary channels that can assist with escape of air during molding.

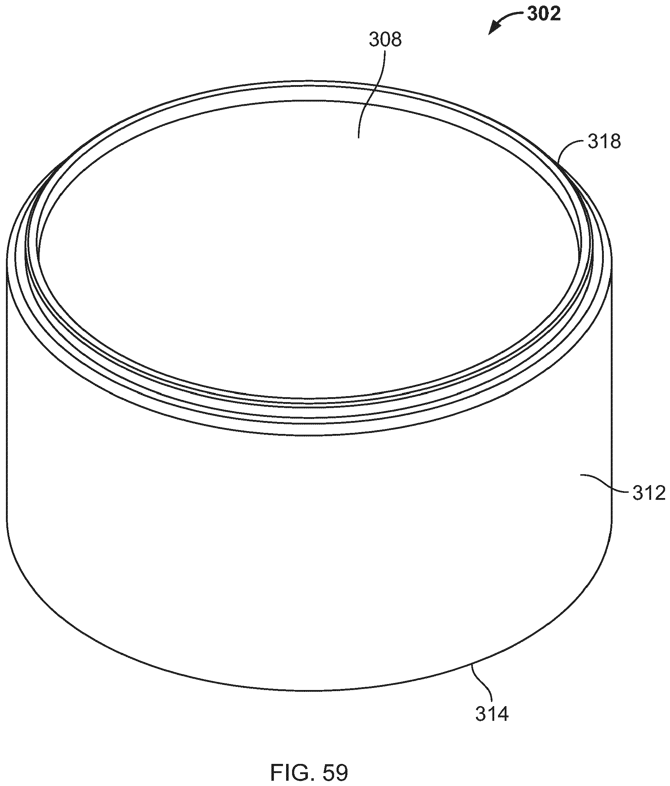

As illustrated in **FIGS. 59-68,** the first mold section 302 includes a flat cavity 308 formed in the mating surface, and the second mold section 304 includes a flat cavity 310 formed in the mating surface. When mated against each other, the cavities 308, 310 align to define the overall shape or form of the hydrogel disc that can be used to punch out a disc-shaped inlay. Both cavities 308, 310 define a substantially circular shape. In some embodiments, the diameter of the cavity 308 can be about, e.g., 14-24 mm inclusive, 14-23 mm inclusive, 14-22 mm inclusive, 14-21 mm inclusive, 14-20 mm inclusive, 14-19 mm inclusive, 14-18 mm inclusive, 14-17 mm inclusive, 14-16 mm inclusive, 14-15 mm inclusive, 15-24 mm inclusive, 16-24 mm inclusive, 17-24 mm inclusive, 18-24 mm inclusive, 19-24 mm inclusive, 20-24 mm inclusive, 21-24 mm inclusive, 22-24 mm inclusive, 23-24 mm inclusive, 14 mm, 15 mm, 16 mm, 17 mm, 18 mm, 19 mm, 20 mm, 21 mm, 22 mm, 23 mm, 24 mm, 16.67 mm, or the like. In some embodiments, the depth of the cavity 308 can be about, e.g., 0.4-0.6 mm inclusive, 0.4-0.55 mm inclusive, 0.4-0.5 mm inclusive, 0.4-0.45 mm inclusive, 0.45-0.6 mm inclusive, 0.5-0.6 mm inclusive, 0.55-0.6 mm inclusive, 0.4 mm, 0.45 mm, 0.5 mm, 0.55 mm, 0.6 mm, or the like. In some embodiments, the diameter of the cavity 310 can be about, e.g., 8-12 mm inclusive, 8-11.5 mm inclusive, 8-11 mm inclusive, 8-10.5 mm inclusive, 8-10 mm inclusive, 8-9.5 mm inclusive, 8-9 mm inclusive, 8-8.5 mm inclusive, 8.5-12 mm inclusive, 9-12 mm inclusive, 9.5-12 mm inclusive, 10-12 mm inclusive, 10.5-12 mm inclusive, 11-12 mm inclusive, 11.5-12 mm inclusive, 8 mm, 8.5 mm, 9 mm, 9.5 mm, 10 mm, 10.5 mm, 11 mm, 11.5 mm, 12 mm, or the like. In some embodiments, the depth of the cavity 310 can be about, e.g., 0.3-0.5 mm inclusive, 0.3-0.45 mm inclusive, 0.3-0.4 mm inclusive, 0.3-0.35 mm inclusive, 0.35-0.5 mm inclusive, 0.4-0.5 mm inclusive, 0.45-0.5 mm inclusive, 0.3 mm, 0.35 mm, 0.4 mm, 0.45 mm, 0.5 mm, or the like.

**FIGS. 59-63** are perspective, top, side, cross-sectional and detailed views of the first mold section 302. The mold section 302 includes a body 312 defining a substantially cylindrical configuration. The body 312 includes a bottom surface 314 and an opposing top surface defining the substantially planar mating surface 316. The mold section 302 includes a radial flange 318 extending near the outer perimeter of the body 312 from the top surface, and positioned slightly inwardly offset from the outer perimeter or edge of the body 312.

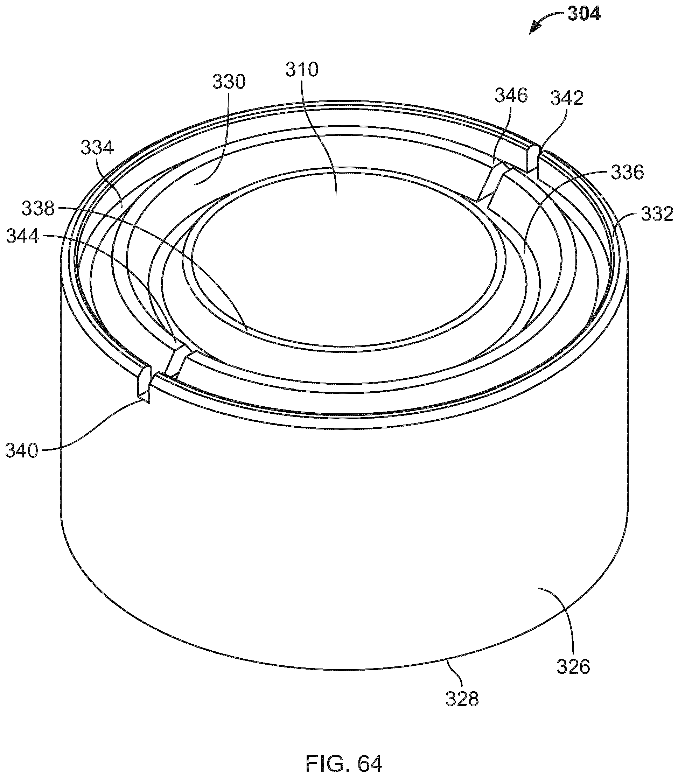

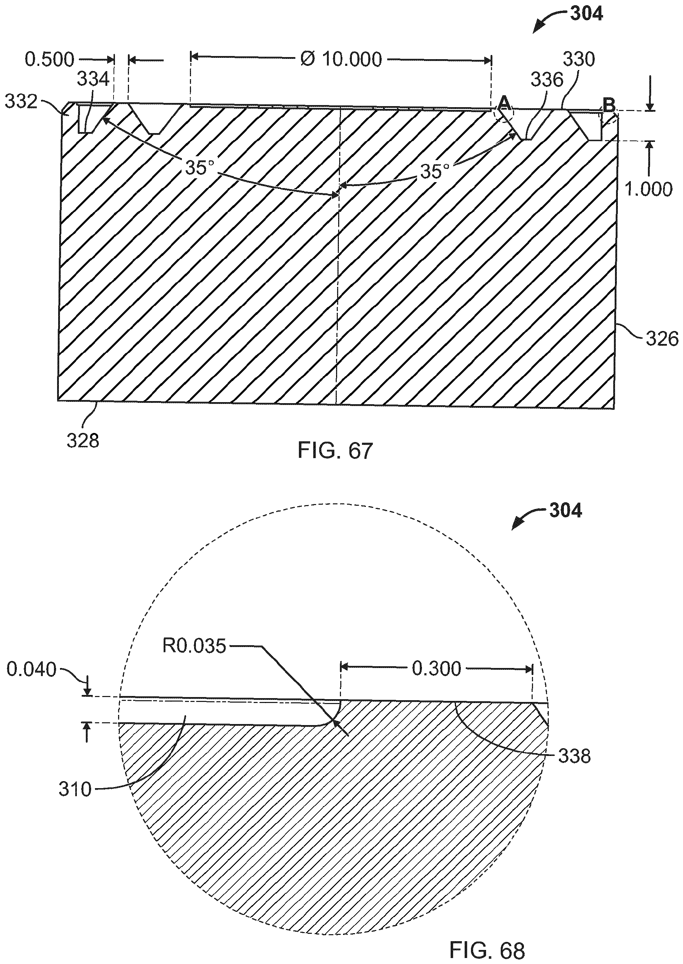

**FIGS. 64-68** are perspective, top, side, cross-sectional and detailed views of the second mold section 304. The mold section 304 includes a body 326 defining a substantially cylindrical configuration complementary to the body 312 of the mold section 302. The body 326 includes a bottom surface 328 and an opposing top surface defining the substantially planar mating surface 330. The mold section 304 includes a radial edge 332 extending at or near the perimeter of the body 226 from the top surface. The mold section 204 includes a first radial channel 234 inwardly formed in the top surface adjacent to the radial edge 332. The mold section 304 includes a second radial channel 336 inwardly formed in the top surface. The second radial channel 336 defines a smaller diameter than the first radial channel 334, and is therefore disposed closer to the central longitudinal axis of the mold section 304. The diameters and edges of the radial channels 334, 336 are dimensioned substantially complementary to the diameters and edges of the mold section 302. As illustrated in **FIG. 67,** the inner walls of the channels 334, 336 taper inwardly.

The flat cavity 310 is formed in the top surface of the body 326 and is substantially aligned with a central longitudinal axis of the mold section 304. A perimeter section 338 surrounds the cavity 310 and forms part of the mating surface 330. The mold section 304 includes a transverse channel, cutout or slit extending through the radial flange 332 and the raised section of the body 326 between the channels 334, 336. In particular, the transverse channel includes channels 340, 342 that extend through the radial flange 332 on opposing sides of the body 326, and channels 344, 346 that extend through the raised section of the body 326 between the channels 334, 336. The bottom of the channels 340-346 is substantially aligned with the bottom surface of the channels 134, 136. The channels 140-146 provide a fluidic connection between the channels 334, 336.

During fabrication, the uncrosslinked hydrogel composition can be placed within the cavity 310 of the mold section 304, and the mold section 302 can be aligned with and positioned over the mold section 304 such that the mating surfaces 316, 330 are positioned against each other. The radial flange 318 of the mold section 302 is configured and dimensioned to fit within the radial edge 332 of the mold section 304, and against the outer wall of the channel 334 of the mold section 304, to ensure alignment and mating of the mold sections 302, 304 relative to each other. In the mated position, the cavities 308, 310 are enclosed by the mating surfaces to ensure proper distribution of the hydrogel during formation of the disc for the inlay. In the mated position, the channels 134, 136 allow for escape of air and/or gas from the mold assembly 300. In some embodiments, a clamp can be used to secure the mold sections 302, 304 to each other prior to crosslinking, as long as the clamp does not obstruct UV light from reaching the hydrogel in the mold cavity. Once UV crosslinking is complete, the disc is alloyed to further crosslink at ambient conditions before the disc is demolded, further processed and characterized. As noted above, the disc-shaped inlay can be punched out from the hydrogel disc based on the desired diameter of the disc-shaped inlay.

Based on testing performed on exemplary inlay 200, the refractive index for the inlay 200 (and the onlay) can range from about, e.g., 1.33-1.36 inclusive, 1.33-1.35 inclusive, 1.33-1.34 inclusive, 1.34-1.36 inclusive, 1.35-1.36 inclusive, 1.34-1.35 inclusive, 1.33, 1.34, 1.35, 1.36, or the like, when measured at about $23 \pm 1°C$. Such exemplary inlay 200 has a percent transmittance ranging from about, e.g., 94-98% inclusive, 94-97% inclusive, 94-96% inclusive, 94-95% inclusive, 95-98% inclusive, 96-98% inclusive, 97-98% inclusive, 95-97% inclusive, 95-96% inclusive, 94%, 95%, 96%, 97%, 98%, or the like, at about 300 nm.

According to some embodiments, the hydrogel can be injected into the mold and corneal inlays or onlays can then be polymerized by a method appropriate for the particular polymer(s) employed, e.g., chemically, by successive cross-linking of precursors using UV light, cross-linking agents, thermally, or by photopolymerization. After polymerization, the inlay can be removed from the mold (demolded), washed and stored in buffer with a preservative until use. According to one embodiment, the cross-linking agent is Poly(ethylene glycol) diacrylate (PEGDA). According to one embodiment, the cross-linking agent is any multi arm PEG acrylate or methacrylate (e.g., 3 or 4 or 8 arm PEG acrylate or methacrylate).

According to some embodiments, the hydrogel composition can be formed by initially hydrating the natural polymer, e.g., a collagen, in an acidic medium. The dry collagen is mixed with a buffer at a pH of about 3.0, and sits at about $5 \pm 3°C$ for about 10 days (at a minimum 8 days). The cross-linker(s), monomer(s) and/or polymer(s), and UV initiator are added to the hydrated collagen. In some embodiments, the cross-linker for the collagen ranges from about, e.g., 0.25-0.75% inclusive, 0.25-0.65% inclusive, 0.25-0.55% inclusive, 0.25-0.45% inclusive, 0.25-0.35% inclusive, 0.35-0.75% inclusive, 0.45-0.75% inclusive, 0.55-0.75% inclusive, 0.65-0.75% inclusive, 0.25%, 0.35%, 0.45%, 0.55%, 0.65%, 0.75%, or the like, by weight of the hydrogel composition. In some embodiments, the monomer ranges from about, e.g., 1.2-4.8% inclusive, 1.2-4.0% inclusive, 1.2-3.5% inclusive, 1.2-3.0% inclusive, 1.2-2.5% inclusive, 1.2-2.0% inclusive, 1.2-1.5% inclusive, 1.5-4.8% inclusive, 2.0-4.8% inclusive, 2.5-4.8% inclusive, 3.0-4.8% inclusive, 3.5-4.8% inclusive, 4.0-4.8% inclusive, 4.5-4.8% inclusive, 1.2%, 1.5%, 2.0%, 2.5%, 3.0%, 3.5%, 4.0%, 4.5%, 4.8%, or the like, by weight of the hydrogel composition. In some embodiments, the cross-linker for the synthetic polymer (after monomer polymerization)ranges from about, 0.2-0.6% inclusive, 0.2-0.5% inclusive, 0.2-0.4% inclusive, 0.2-0.3% inclusive, 0.3-0.6% inclusive, 0.4-0.6% inclusive, 0.5-0.6% inclusive, 0.2%, 0.3%, 0.4%, 0.5%, 0.6%, or the like. The hydrogel mixture is degassed and then added to the mold prior to cross-linking. UV light is used to crosslink the hydrogel composition for about 15-30 minutes. The inlay is then allowed to cross-link in ambient conditions for about 12-20 hours, resulting in complete polymerization of the composition. According to some embodiments, the UV wavelength intensity ranges from about, e.g., 360-405 nm inclusive, 360-400 nm inclusive, 360-390 nm inclusive, 360-380 nm inclusive, 360-370 nm inclusive, 370-405 nm inclusive, 380-405 nm inclusive, 390-405 nm inclusive, 400-405 nm inclusive, 360 nm, 370 nm, 380 nm, 390 nm, 400 nm, 405 nm, or the like. The crosslinked hydrogel can remain in a chamber overnight. Once removed, the inlay is hydrated in 1x phosphate buffer then washed several times in phosphate buffer to extract unpolymerized material and residual crosslinker and/or UV initiator.

According to some embodiments, a single cavity mold (e.g., male and female molds) can be used to fabricate the corneal implant. According to some embodiments, 2-cavity molding (e.g., multi cavity with two or more male and/or two or more female molds) can be used to fabricate the inlay. Such process can render a shape similar to the meniscus inlay/onlay 210 lens or dome shape discussed above (e.g., flat on one side, curved on the other side).

According to some embodiments, cannula molding can be used to fabricate the inlay. One side of a lenticule can be molded against a surface, the other side can be molded in free air. The curved side is formed due to surface tension only, and is not in contact with a plastic surface as in 2-cavity molding. The result is a dome-shaped lenticule.

According to some embodiments, surface energy molding can be used to fabricate the corneal implant. Based on the surface energy of a solid surface or substrate, the pre-mix can be dropped onto the surface and forms a shape. The shape is a dome geometry meaning flat on one side and curved on the other. The surface energy of the substrate and the volume of pre-mix determine the final geometry of the molded article.

According to some embodiments, the corneal implant may be cast as a flat, thin, round disc. According to some embodiments, the fabricated corneal implant may be cast as a hemispherical dome. According to some embodiments, the fabricated corneal implant may be cast as a spherical lens. According to some embodiments, the fabricated corneal implant may be cast as a thin sheet. The mold can be adjusted as needed depending on the configuration of the desired corneal implant.

The corneal inlay and onlay improve near vision and decrease dependence on near-vision correction modalities on presbyopic patients. The inlay can resemble a small contact lens implanted into the cornea, and the onlay can resemble a small contact lens implanted onto the cornea. In the clinical application, one corneal implant (e.g., lens) can be implanted into (or onto for the onlay) the dominant eye resulting in only one corneal inlay per patient. The mode of action is based on displacement of a small section of the corneal stroma. This, in turn, gives rise to a micron size bump anterior to the cornea. This causes a small refractive change giving rise to about 1-3, inclusive, or 1.5-3 diopters, inclusive, of correction, hence, enabling the patient to view items at near distance. Visual acuity at far distance is still maintained.

According to some embodiments, the inlay can have a center thickness of about 40-50 microns, inclusive. The reported surface area and weight of the inlay can be about 0.063 cm$^2$ and about 0.0001 gram (100 $\mu$g). According to some embodiments, the onlay can have a center thickness of about 15-30 microns, inclusive; for example, about 15, 16, 17, 18, 19, 20, 21, 22, 23, 24, 25, 26, 27, 29, or 30 microns. The inlay and/or onlay is considered to be an optically clear hydrogel with about a 78-92% inclusive water content produced from porcine collagen. According to some embodiments, an 80% water content, collagen-based hydrogel can be used as a corneal or stromal implant. Fabrication of the inlay and/or onlay can involve accurate control and consideration of several parameters, e.g., overall geometry, center thickness, edge thickness, surface quality, smoothness/roughness, front radius of curvature, back radius of curvature.

The exemplary inlay provides several advantages that alone or in combination provide improved results post-implantation. As noted herein, the inlay includes a water content ranging from about 78%-92%, inclusive, or from about 78%-80%, inclusive, with collagen hydrogel materials having defined properties. The collagen inlay (cytophilic property) and assay yields a biocompatible device in the cornea. Such true biocompatibility results in haze-free vision, even after extended periods of time (e.g., beyond 2 years). In particular, the exemplary inlay has produced positive results showing excellent biocompatibility with the cornea without use of any drugs post-op. No haze has been seen in animal subjects approaching 3 years post-implantation. The inlay can be fabricated using cannula molding or surface energy molding, and defines a dome-shaped geometry. The corneal implant can be implanted into a deep pocket (e.g., about 180-200 microns) as well as a flap (e.g., about 150 microns similar to a LASIK procedure). The highly biocompatible material of the inlay allows for such implantation successfully.

Traditional corneal implants fail to be truly biocompatible. The exemplary inlay solves such issues by providing a truly biocompatible device. The inlay has the potential for excellent efficacy together with excellent safety. Future delivery systems or insertion devices can allow quick ease of insertion (as compared to traditional designs). Excellent biocompatibility of the implant can lead to flexibility with surgeons and patients involving specific procedures to be employed (e.g., both flap and pocket procedures can be employed).

As discussed herein, the inlay can be implanted using either a flap or pocket technique formed by a femtosecond laser. The inlay can be positioned onto the stromal bed and centered against the pupil or visual axis. The flap is closed (no closure needed for pocket), and smoothed out. The inlay creates a central bump over the cornea, e.g., a 15-20 micron bump. Such bump can provide for correction in vision of about +1.5 to +3 diopters.

The exemplary onlay provides several advantages that alone or in combination provide improved results post-implantation. Lower water content is a stiffer material, which means it may be easier to manufacture and handle. Our experience is that at a water content ranging from about 78%-92%, inclusive, or from about 78%-80%, inclusive, the index of refraction is similar to the index of refraction of both water and the cornea, therefore the onlay does not cause any refraction change. The vision correction of the cornea is primarily due to the shape of the onlay.

According to some embodiments, the onlay can be adhered to the Bowman's membrane with, e.g., a biological coating (e.g., fibronectin, collagen, RGDS (Kobayashi, H. and Ikacia, Y., Current Eye Res. (2009) 10 (10): 899-908); or a biocompatible adhesive [see Rostron, CK et al. Arch. Ophthalmol. (1988) 106: 1103-6; Nam, S. and Mooney, D. Chem. Rev. (2021) doi.org/10.1021/acs.chemrev.0c00798].An adhesive material for adhering the onlay to Bowman's membrane would need to be tested to confirm its refractive index, and safety when used in the eye. Many commercial adhesives are available and described by [Nam, S. and Mooney, D. Chem. Rev. (2020) doi.org/10.1021/acs.chemistry/0c00798]. For example, naturally derived proteins can be used to fabricate tissue adhesives. For example, collagen itself may have adhesive properties. Similarly, gelatin, an irreversibly hydrolyzed form of collagen, is widely used in adhesives. [Id., citing Gomez-Guillen, MC et al. Food Hydrocolloids (2011) 25: 1813-27; Yue, K. et al. Biomaterial (2015) 73: 254-71]. Albumin combined with functionalized PEG forms a hydrogel adhesive. [Id., citing Fuller, C. J. Cardiothorac. Surg. (2013) 8. DOI: 10.1186/1749-8090-8-90]. Fibrin-based adhesives (e.g., Tisseal, Baxter) are known; fibrin glues, which are biologically compatible and biodegradable, usually do not lead to inflammatory responses, foreign-body reactions, tissue necrosis or fibrosis, and have been widely used in a variety of surgical applications. [Id., citing Duarte, AP et al. Prog. Polym. Sci. (2012) 37: 1031-50,Seyednejad, H. et al. Br. J. Surg. (2008) 95: 1197-1225]. PEG is FDA approved in a number of applications and is broadly considered to be nontoxic, biocompatible and nonimmunogenic. [Id., citing Mehdizadeh, M. and Yang, J. Macromol. Biosci. (2013) 13: 271-88]. For example DuraSeal Xact Adhesion Barrier and Sealant System (DSX) utilizes an NHS ester to achieve a hydrogel with adhesive cross-linking and bonding to tissues with reduced swelling [Id., citing Preul, MC et al. J. Neurosurg. Spin (2010) 12: 381-90; Fransen, P. Spine J. (2010) 10: 751-61; Kim, K. D et al. Global Spine J. (2019) 9: 272-8]. A recent study explored a tissue adhesive with reversible adhesion in which adhesive patches of poly(vinyl alcohol) and poly (acrylic acid) (PAA) were grafted with NHS esters; The NHS ester groups were linked to the PAA chains through disulfide bonds that act as cleavable linkages. [Id., citing Chen, CX. et al. (2020) Proc. Natl Acad. Sci. USA 117: 15497-15503}. Another product, Cell-Tak is a nonimmunogenic extracellular matrix protein preparation isolated from the marine mussel, Mytilus edulis (Corning]. In some embodiments, photochemical tissue bonding (PTB) using either Riboflavin or Rose Bengal and UV light may be implemented to secure or glue the onlay in the desired position. [Redmond, Robert W. et al. (2019) Photochemistry and Photobiology, 95: 1097-1115]. In some embodiments, cyanoacrylate glue (typically used for corneal lacerations/perforations and gluing small corneal patches on corneal perforations, corneal melts, and corneal wound leaks) may be used to secure or glue the onlay in the desired position. [Vote, BJ et al., (2000) Clin Exp Ophthalmol., Dec;28(6):437-42].

At least a portion of the epithelial layer may be removed from the cornea and the remaining surface cleaned before implantation of the onlay onto the Bowman's membrane. For example, a portion of the epithelial layer [e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or 100%] may be removed to reduce the thickness from the anterior side of the epithelial layer, thereby accommodating the onlay. As another example, a central section of the epithelial layer may be removed entirely to accommodate implantation of the onlay, with the epithelial layer growing over the onlay in about 24-48 hours. Once implanted with biocompatible adhesive, the onlay creates a bump in the cornea that improves presbyopia. According to some embodiments, as the hydrogel composition dries against the surface of the Bowman's membrane, the onlay sticks to the Bowman's membrane without the use of a biocompatible adhesive. According to some embodiments, a contact lens can be positioned over the onlay to assist with the healing process.

Unlike traditional corneal implants, the described onlay provides no inflammation post implantation, no necrosis, and no haze, even after implanting materials in rabbits for more than 2 years. It therefore is a truly biocompatible device.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges which may independently be included in the smaller ranges is also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, exemplary methods and materials have been described. All publications mentioned herein are incorporated herein by reference to disclose and described the methods and/or materials in connection with which the publications are cited.

It must be noted that as used herein and in the appended claims, the singular forms "a", "and", and "the" include plural references unless the context clearly dictates otherwise.

The publications discussed herein are provided solely for their disclosure prior to the filing date of the present application and each is incorporated by reference in its entirety. Nothing herein is to be construed as an admission that the present invention is not entitled to antedate such publication by virtue of prior invention. Further, the dates of publication provided may be different from the actual publication dates which may need to be independently confirmed.

**EXAMPLES**

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric.

**Example 1. Exemplary inlays**

*Exemplary inlay 1.* A collagen-synthetic polymer hydrogel inlay was formed from a collagen-2-Methacryloyloxyethyl phosphorylcholine (MPC)-Poly(ethylene glycol) diacrylate (PEGDA) composition. The hydrogel composition included an IPN made of a natural polymer (e.g., collagen), and two synthetic polymers (i.e., MPC and PEGDA). The water content of the composition ranged from about 94% to about 98% (referred to herein as a "high water content composition"). In some instances the hydrogel composition had a water content of between about 90% to about 96%.

The IPN for the high water content composition had a collagen/PEGDA weight ratio of about 4:1, and a PEGDA/MPC weight ratio varying from about 1:3 to about 1:1 (i.e.., 1:3, 1:2, or 1:1). The length of PEGDA was small enough to serve as both a crosslinking agent and a macro-monomer i.e., between about 200 to about 700 Da. A crosslinking agent was used to crosslink collagen, while an ultraviolet (UV) initiator was used for simultaneously initiating the polymerization and crosslinking of MPC with PEGDA as a crosslinker. Instead of using a redox initiator (as generally used with traditional compositions), the hydrogel composition was cross-linked with a UV initiator which can extend the mold fabrication time up to about 5 minutes. In contrast, with traditional redox initiators, cross-linking must occur in less than 30 seconds, minimizing the time allotted to ensuring the hydrogel is properly positioned in the mold.

*Exemplary inlay 2.* A second inlay was formed from a collagen-MPC-PEGDA composition. The hydrogel composition included an IPN made of a natural polymer (e.g., collagen), and two synthetic polymers (i.e., MPC and PEGDA). The composition had a water content ranging from about 78% to about 92% (referred to herein as a "low water content composition"). The IPN for the low water content composition had a collagen/PEGDA weight ratio varying from about 1:3 to about 1:10, and a PEGDA/MPC weight ratio varying from about, e.g., 1:0.5-0.5:1, 1:0.6-0.5:1, 1:0.7-0.5:1, 1:0.8-0.5:1, 1:0.9-0.5:1, 1:1-0.5:1, 1:0.5-0.6-:, 1:0.5-0.7:1, 1:0.5-0.8:1, 1:0.5-0.9:1, 1:0.5-1:1, or the like. The length of PEGDA was greater than about 700 Da. A crosslinking agent was used to crosslink collagen, while an initiator (e.g., UV) was used for simultaneously initiating polymerization and crosslinking of MPC and PEDGA.

**Example 2. Method of Making IPN Hydrogels for Corneal Inlay**

The collagen and the non-collagen components of the hydrogel mixture were simultaneously crosslinked in the mold cavity. While collagen was crosslinked only via DMT-MM (4-(4,6-Dimethoxy-1,3,5-triazin-2-yl)-4-methylmorpholinium chloride), two different crosslinking chemistries can be used to polymerize/crosslink the non-collagen moieties of the hydrogel.

One crosslinking chemistry can be, e.g., DMT-MM-APS/TMEDA. DMT-MM was used to slowly crosslink collagen, while the redox pair, Ammonium Persulfate (APS)/TMEDA was used to polymerize and crosslink MPC, with PEGDA as the crosslinker. The entire process was performed at room temperature.

Another crosslinking chemistry can be, e.g., DMT-MM-LAP. DMT-MM was used to slowly crosslink collagen, while a UV initiator Lithium phenyl-2,4,6-trimethylbenzoylphosphinate (LAP) was used to polymerize and crosslink MPC, with PEGDA as the crosslinker. In some embodiments, the UV initiator can be 2,2-Dimethoxy-2-phenylacetophenone (DMPA) or Irgacure. UV polymerization/crosslinking was performed in a UV chamber. After the completion of UV polymerization/crosslinking, the sample was removed from the UV chamber and the crosslinking of collagen was continued for about 12 more hours.

## Example 3. Method of Making DMT-MM-APS/TMEDA Hydrogel Inlays With Water Content From About 94% to About 98%

50 mg of collagen powder was weighed into a 2 ml micro-centrifuge tube labeled 1. 450 mg of 2-(N-Morpholino)ethanesulfonic acid (MES) buffer pH 2.90 was then added and the tube placed in 5 °C for 7 - 10 days to hydrate the collagen. Once collagen was completely hydrated, 200 mg of MES buffer pH 2.90, 125 mg of 10% w/w MPC in MES buffer pH 2.90 and 4.71 PEGDA, molecular weight (Mw) 575 were added sequentially to the micro centrifuge tube 1. The tube was vortexed after each addition to properly homogenize the mixture. The tube was then centrifuge and placed in 5 °C. The following crosslinking/initiating reagents were then prepared; 4% w/w solution of APS in MES buffer pH 2.90, 4% w/w solution of TMEDA solution in MES buffer pH 2.90, and a 12% solution of DMT-MM in MES buffer pH 2.90. The tube labeled 1 was removed from 5 °C and 12.19 mg TMEDA solution was added and mixture was homogenized by vortexing. 52 mg of DMT-MM solution and 15.63 mg of APS solution were added to the tube and vortexed to properly mix. The mixture was then centrifuged at 15 °C to remove air bubbles before casting in PMMA cavity molds and allowed to polymerize/crosslink for approximately 12 hours at room temperature, in a humidity chamber. After crosslinking the inlay was demolded and washed several times in 1x phosphate buffer saline (PBS) buffer to remove residual reagents.

## Example 4. Method of Making DMT-MM-LAP Hydrogel Inlays With Water Content Ranging From About 94% to About 98% for Comparison Testing

60 mg of collagen powder was weighed into a 2 ml micro-centrifuge tube labeled 1. 440 mg of MES buffer pH 2.90 was then added and the tube placed in 5 °C for 7 - 10 days to hydrate the collagen. Once collagen was completely hydrated, 410 mg of MES buffer pH 2.90, 150 mg of 10% w/w MPC in MES buffer pH 2.90 and 5.0 PEGDA, Mw 575 were added sequentially to the micro centrifuge tube 1. The tube was vortexed after each addition to properly homogenize the mixture. The tube was then centrifuge and placed in 5 °C. The following crosslinking/initiating reagents were then prepared. 0.25% w/w solution of LAP in MES buffer pH 2.90, and a 12% solution of DMT-MM also in MES buffer pH 2.90. The tube labeled 1 was removed from 5 °C and 62.26 mg of DMT-MM solution and 120.0 mg of LAP solution were added to the tube and vortexed to properly mix. The mixture was then centrifuged at 15 °C to remove air bubbles before casting in PMMA cavity molds. The molds were placed in a UV chamber for 30 minutes and then in a humidified chamber for approximately 12 hours at room temperature. After polymerization/crosslinking the inlay was demolded and washed several times in 1x PBS buffer to remove residual reagents.

## Example 5. Method of Making Hydrogel Inlays With Water Content Ranging From About 78% to About 92%

43.33 mg of 12% w/w collagen hydrated in MES buffer pH = 2.90 was weighed into a 2 ml micro-centrifuge tube labeled 1. 340 mg of MES buffer pH = 2.90, 25 mg MPC, and 50 mg PEGDA (Mw = 700). The tube was then vortexed to homogenized and the pH checked and adjusted to between 2.8 and 3.8 with 1 N hydrochloric acid (HCl) and 1 N sodium hydroxide (NaOH) solutions. The mixture was then placed in 5 °C. The following crosslinking/initiating reagents were then prepared. 0.15% w/w solution of LAP in MES buffer pH 2.90, and a 12% solution of DMT-MM also in MES buffer pH 2.90. The tube labeled 1 was removed from 5 °C and 5.41 mg of DMT-MM solution and 50.0 mg of LAP solution were added to the tube and vortexed to properly mix. The mixture was then centrifuged at 15 °C to remove air bubbles before casting in PMMA cavity molds. The molds were placed in a UV chamber for 30 minutes and then in a humidified chamber for approximately 12 hours at room temperature. After polymerization/crosslinking the inlay was demolded and washed several times in 1x PBS buffer to remove residual reagents.

## TEST METHODS

### Mechanical properties

Mechanical properties of the corneal inlays were determined by means of profilometry-based indentation e.g., burst strength measurements following ASTM Standard F2392-04. For the indentation measurements, samples of about 2-6 mm in diameter were tested in 1x PBS in glass vials under spherical indentation in order to obtain the profilometry of the sample. Young's Modulus was subsequently obtained from the resulting profile of the sample.

### Water Content

To determine water content of a sample of hydrogel, excess water from a fully hydrated 10 mm diameter disc with a thickness of ~300 $\mu$m is thoroughly blotted with the aid of a KimWipe. The weight of the material, $W_1$ is taken by placing material on an analytical balance. The inlay is then placed in an oven set at 100 °C for minimum of two and a half hours to completely dry the sample. The weight of the dried sample is measured and recorded as $W_2$. The water content $\%WC$ recorded as a percentage is calculated as:

$$\%WC = \left(\frac{W_1 - W_2}{W_1}\right) x \ 100$$

**Refractive Index**

A refractometer is used to determine the refractive index. The refractometer is first calibrated with HPLC water and a calibration oil before measurements. To measure the refractive index of the inlay, excess water on the inlay (10 mm diameter by 100 $\mu$m thickness) is blotted with the aid of KimWipes before it is placed on the measuring prism. Once the prism is closed, an adjustment knob is used to adjust and align a shadow-line to intersects a crosshairs. Once this is completed, the refractive index of the material as well as the temperature are read and recorded.

**Percent Transmission**

Percent transmission of a fully hydrated inlay sample is measured using a spectrophotometer, calibrated with HPLC water. The sample (10 mm diameter by 100 $\mu$m thickness) is placed inside the cuvette, along with HPLC water and adjusted so that it is touching the bottom of the cuvette and pressed up against the front side. The cuvette is then inserted into the cell holder compartment for a UV scan. The percent transmission at 300 nm is recorded as the % transmittance of the materials.

**Example 6. Evaluating Biocompatibility of Inlay Material In Vitro**

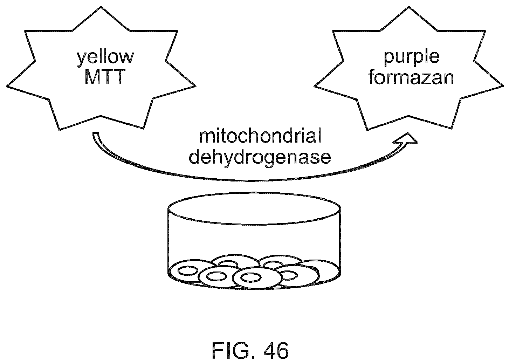

(1) MTT assay to quantify cell viability on different material samples. **(FIG. 46)**

Rationale: MTT is used to measure cellular metabolic activity as an indicator of cell viability, proliferation and cytotoxicity. The darker the solution, the greater the number of viable metabolically active cells.

Protocol: 12 mm discs are added to cover most of the well area of 24 well plates. The following numbers of rabbit corneal fibroblasts were seeded in duplicate per well: 200K, 100K, 50K, 25K, 12.5K, 6.25K. Cells were cultured in standard culture medium. 10 wells correspond to test samples; 6 wells correspond to controls (no discs). An MTT calibration plate is seeded on day 3 with the same cell numbers. The calibration plate is evaluated by microscopy before the assay and confluency estimated. On day 4, water soluble yellow MTT (4, 5-dimethylthiazol-2-yl)-2,5-diphenyltetrazolium bromide) is added to the cultures. MTT is reduced to purple insoluble formazan by mitochondrial dehydrogenase in the mitochondria of viable cells. The formazan is solubilized with detergent and measured spectrophotometrically. Viable cells with active metabolism convert MTT into a purple colored formazan product with an absorbance maximum near 570 nm.

(2) **In vitro cytophilicity assay**

Rationale: An in vitro cytophilicity assay using rabbit corneal fibroblasts measures migration of rabbit keratocytes onto a test material and their attachment. The test therefore addresses whether the material of the exemplary inlays is toxic to cells and whether cells can attach and grow on the material. If cell coverage on the material is judged acceptable (e.g., confluent, no dead cells, others), then the material is a candidate for a more detailed animal implant study. All materials passing this test have shown excellent biocompatibility in subsequent in vivo animal studies in rabbit eyes.

Protocol: Passaged NZW rabbit corneal fibroblasts were seeded in media containing test article. Cell growth was monitored for up to seven days to see if cells attach and grow on the test article, if cell morphology is altered in the presence of the test article, and to measure the thickness of test article. Fully biocompatible test articles showed 100% cell confluence between four and seven days. When implanted in animals, these materials remained clear and transparent even after two years. Test articles that were not biocompatible showed less than 30% cell confluence after seven days. In some cases, no cell growth was observed. When implanted in animals, these materials became hazy between three to six months.

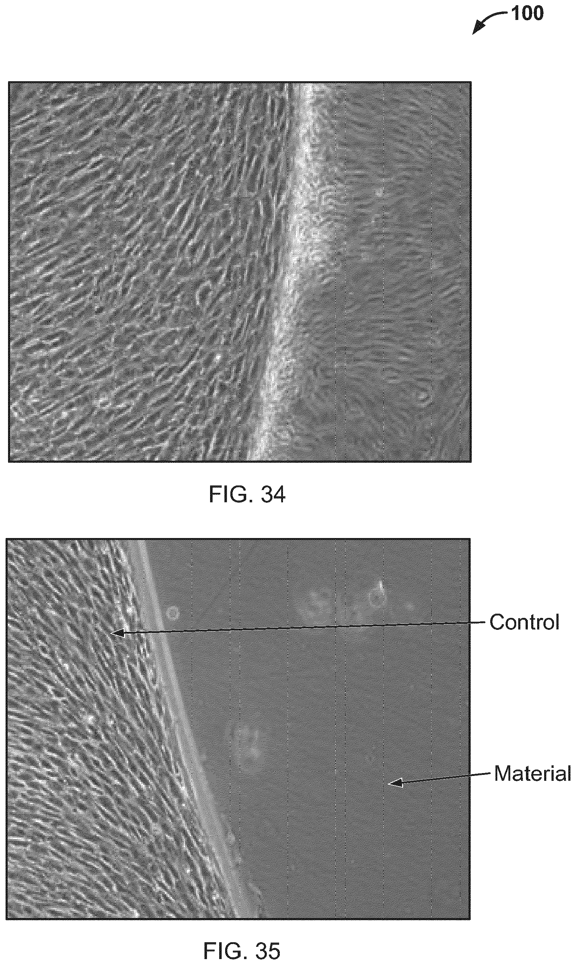

**FIG. 34** shows an image of cell coverage on a biocompatible material, and **FIG. 35** shows an image of cell coverage on a non-biocompatible material. The whitish or light-colored line in the images is the edge of material on the wall plate which serves as the control. By day seven, cells were confluent on both material and control as seen in **FIG. 34.** While cells were confluent on the control in **FIG. 35,** no cells were found on the material.

### (3) First Cell Attachment Assay

Sample materials: OM-PC-MPC 1% to 3% collagen.

While these materials passed an in vitro cell growth assay, the materials were found to degrade easily and showed a small degree of haze in in vivo studies. Further tests are to be performed to optimize the material.

### Questions Asked

Can cells grow in the presence of the material/are the materials toxic to cells?

Do cells attach and grow on the materials?

Is cell morphology altered in the presence of material?

What is the thickness of the material samples?

### Experimental Protocol

Microscopy Evaluation on Days 4, and 7; thickness - evaluated by microscopy; done in 6 well plates;

Materials: Ferentis secondary and non-secondary; and OM PC-MPC. The OM-PC-MPC materials included a 1% collagen sample and a 2.5% collagen sample, each having water content of about 79-82% inclusive.

Cells: Rabbit corneal fibroblasts, passage 4.

Table 1 is a list of samples tested in the cell attachment assay, including a Group ID, a description, and the number of samples/size. The samples included (1) Nippon 07.14.20, 07.24.20 DMTMM 10%; (2) Nippi 07.24.20, 08.11.20 DMTMM 10%; (3) Nippi 07.24.20, 08.17.20 DMTMM 12%; (4) Nippi 07.24.20, 08.19.20 DMTMM 15%; and (5) Ferentis 1823B, Ferentis 1837A.

Table 1. Samples for cell attachment assay

| Group-ID | Description | Number of Samples/ Size |
|---|---|---|
| Nippon 07.14.20 07.24.20 DMTMM 10% | New OM PC-MPC stored in CHCl3/PBS, rinsed with fresh PBS - placed in 6-well plate | 3/8 mm |
| Nippi 07.24.20 08.11.20 DMTMM 10% | New OM PC-MPC stored in CHCl3/PBS, rinsed with fresh PBS - placed in 6-well plate | 3/8 mm |
| Nippi 08.24.20 08.17.20 DMTMM 12% | New OM PC-MPC stored in CHCl3/PBS, rinsed with fresh PBS - placed in 6-well plate | 3/8 mm |
| Nippi 07.24.20 08.19.20 DMTMM 15% | New OM PC-MPC stored in CHCl3/PBS, rinsed with fresh PBS - placed in 6-well plate | 3/8 mm |
| Ferentis 1823B | No secondary crosslinking Thickness = 70 $\mu$m | 3/8 mm |
| Ferentis 1837A | Secondary crosslinking Thickness = 60 $\mu$m | 3/8 mm |

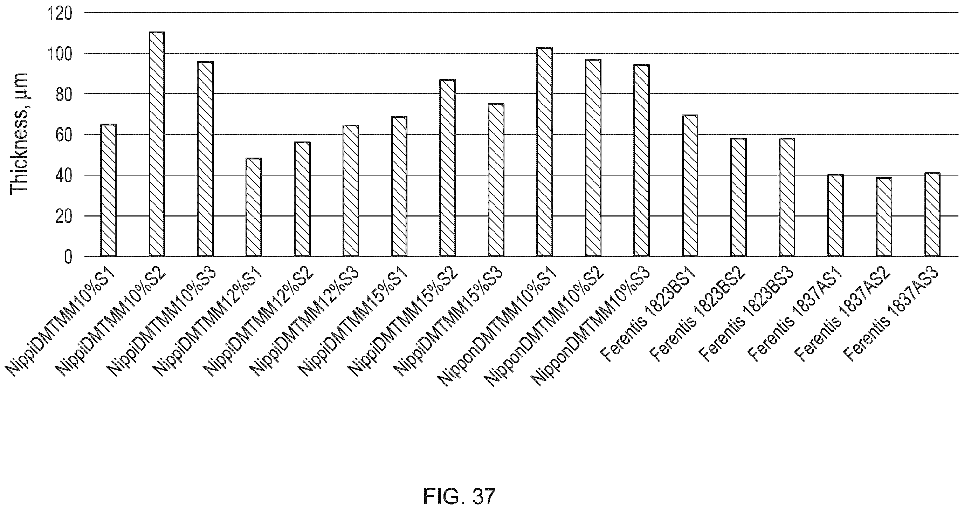

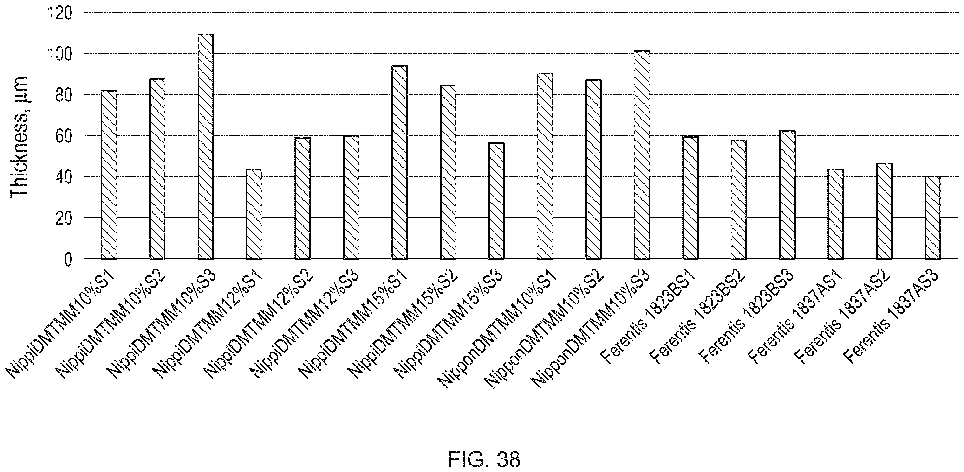

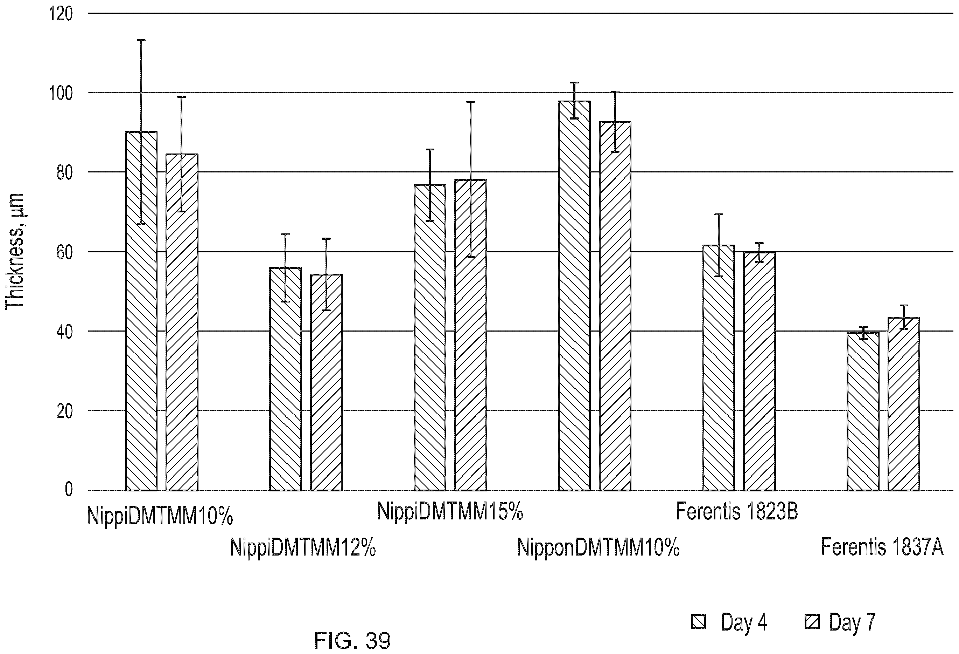

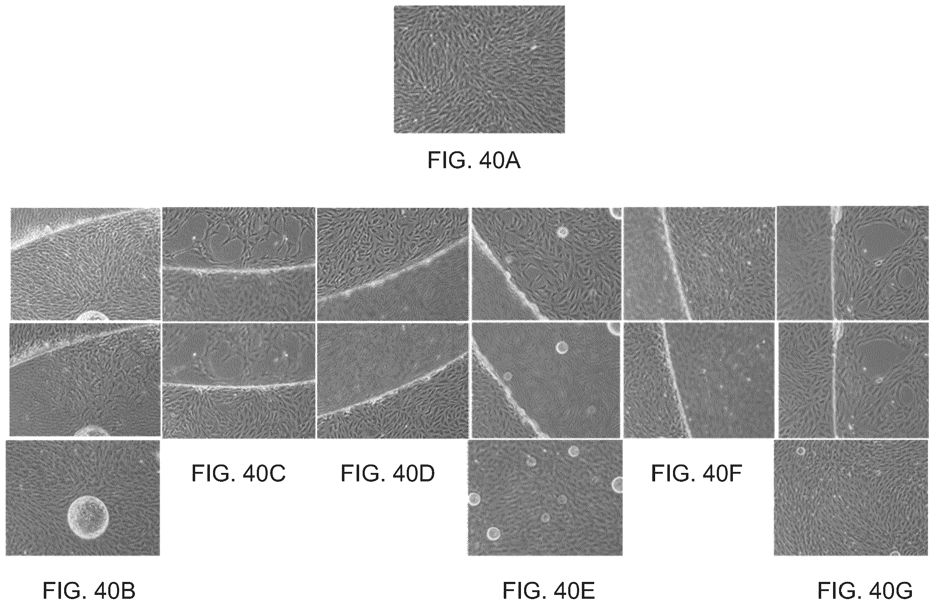

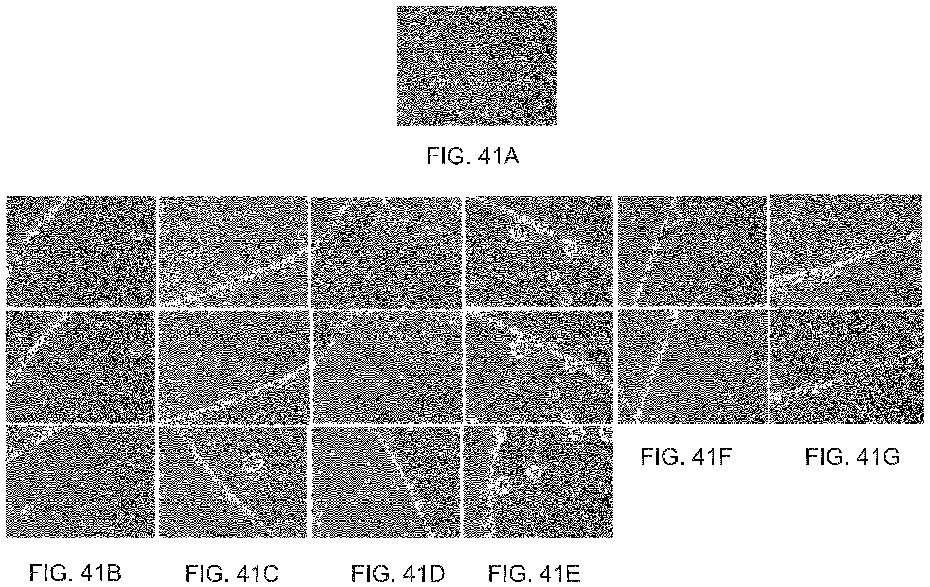

**FIGS. 37** and **38** show the measured thickness of the samples based on cell growth at days 4 and 7, respectively, and **FIG. 39** shows the measured thickness over time for each of the samples. Tables 2 and 3 show confluency and additional details regarding cell growth for each of the samples based on microscopy imaging at days 4 and 7, respectively. **FIGS. 40A-40G** and **41A-41G** are microscopy images for days 4 and 7, with **FIGS. 40A** and **41A** showing an image of the control, **FIGS. 40B** and **41B** showing images for Nippi 10%, **FIGS. 40C** and **41C** showing images for Nippi 12%, **FIGS. 40D** and **41D** showing images for Nippi 15%, **FIGS. 40E** and **41E** showing images or Nippon 10%, **FIGS. 40F** and **41F** showing images for Ferentis 1823B, and **FIGS. 40G** and **41G** showing images for Ferentis 1837A. The first and second row images of **FIGS. 40B-40G** and **41B-41G** are general microscopy images of the samples, and the third row of images of **FIGS. 40B, 40E,40G** and **41B-41E** show bubbles formed.

Table 2. Microscopy results for different samples tested in cell attachment assay at day 4.

| Sample | Description |
|---|---|
| Controls | Confluent/overconfluent |

(continued)

| Sample | Description |
|---|---|
| Nippi 10% | Confluent on and off material |
| Ferentis 1823B | Overconfluent on and off material |
| Nippi 12%, Nippi 15%, Nippon 10%, Ferentis 1837A | Mostly confluent on and off, a few less confluent spots |
| Nippi 10%, Nippon 10%, Ferentis 1837A | Bubble structure sin material - more in Nippon 10% than others. Bubbles do not move, focal plane is between the cell on the material and the cells on the plate |
| Nippi 10%, Ferentis 1837A | Cells growing under |

Table 3. Microscopy results for different samples tested in cell attachment assay at day 7.

| Sample | Description |
|---|---|
| Controls | Overconfluent, some cells floating |
| Nippon 10%, Nippi 10% | Confluent on and off material |
| Ferentis 1837A, Ferentis 1823B | Overconfluent on and off material |
| Nippi 12% | A few spots still not totally confluent on material |
| Nippi 10%, Nippon 10% (a few), Nippi 12% (a few), Nippi 15% (a few) | Bubble structures in material |
| Nippon 10%, Nippi 12%, Ferentis 1837A | Cells growing under |

The cell attachment assay provided the following results. With respect to thickness:

Nippi 10% and Nippon 10% materials are the thickest (85-98 $\mu$m).

Nippi 15% are 76-78 $\mu$m.

Nippi 12% and Ferentis 1823B are 55-60 $\mu$m.

Ferentis 1837A materials are around 40 $\mu$m.

Thickness remained steady over culture time.

The cells attached and grew well on all materials, becoming confluent on all samples by day 7 (except one spot on one Nippi 12% sample). Bubbles were observed in the material itself in the following samples:

Nippon 10%, Nippi 10% (a few), and Ferentis 1837A (a few) on day 4.

Nippon 10%, Nippi 10% (a few), Nippi 12% (a few), and Nippi 15% (a few) on Day 7

(4) **Second Cell Attachment Assay**

**Questions Asked**

Can cells grow in the presence of the material/are the materials toxic to cells?
Do cells attach and grow on the materials?
Is cell morphology altered in the presence of material?
What is the thickness of the material samples?

## Experimental Protocol

Materials were sterilized either in 0.65% chloroform in 1x PBS or in an antibiotic cocktail in 1x PBS.

Materials were soaked for 20-30 minutes in cell media prior to cell seeding.

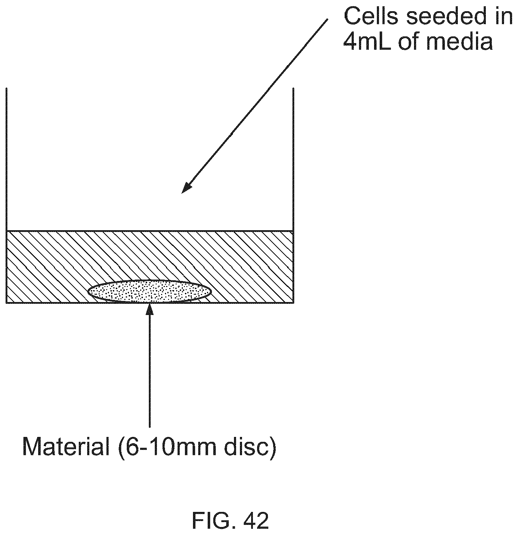

Passaged NZW rabbit corneal keratocytes were seeded at 5000 cells/cm$^2$ as shown in **FIG. 42** in 6 well plates and incubated. As shown in **FIG. 42,** cells were seeded in 4 mL of media, and the material was a 6-10 mm disc.

As a control, cells were added to the well plate in the absence of material for each experiment.

Cells were grown for 7 days.

Cells were imaged on Days 4 and 7 using a Nikon Ti100 infrared camera for (1) thickness, and (2) cell attachment and confluency on materials.

Potential modifications to the protocol included Picro Sirius Red staining for collagen content, and evaluation for potential degradation/loss over time in culture. Another potential modification to the protocol includes using Western Blot analysis to evaluate cell activation.

Table 4 provides a summary of the samples for a cell attachment assay. The samples included (1) Nippi 08.25.20 12%, DMTMM 10% -APS 09.16.20; (2) Nippi 08.25.20 12%, DMTMM 12% -APS 09.16.20; (3) Nippi 09.03.20 10%, DMTMM 10%-Lithium 09.17.20; (4) Nippon 07.30.20 10%, DMTMM 10%-Lithium 09.15.20; (5) Ferentis 1842A; (6) SA-13-31B, Non collagen; and (7) SA-13-92A, Collagen 1%.

Table 4. Samples for cell attachment assay

| Group-ID | Description | Number of Samples/ Size |
|---|---|---|
| Nippi 08.25.20 12% DMTMM 10% - APS 09.16.20 | New OM PC-MPC stored in CHCl3/PBS, rinsed with fresh PBS - placed in 6-well plate | 3/8mm |
| Nippi 08.25.20 12% DMTMM 12% - APS 09.16.20 | New OM PC-MPC stored in CHCl3/PBS, rinsed with fresh PBS - placed in 6-well plate | 3/8mm |
| Nippi 09.03.20 10% DMTMM 10%-Lithium 09.17.20 | New OM PC-MPC stored in CHCl3/PBS, rinsed with fresh PBS - placed in 6-well plate | 3/8mm |
| Nippon 07.30.20 10% DMTMM 10%-Lithium 09.15.20 | New OM PC-MPC stored in CHCl3/PBS, rinsed with fresh PBS - placed in 6-well plate | 3/8mm |
| Ferentis 1842A | 300 $\mu$m | 3/8mm |
| SA-13-31B Non-collagen | >200 $\mu$m | 3/8mm |
| SA-13-92A Collagen 1% | >700 $\mu$m | 3/8mm |

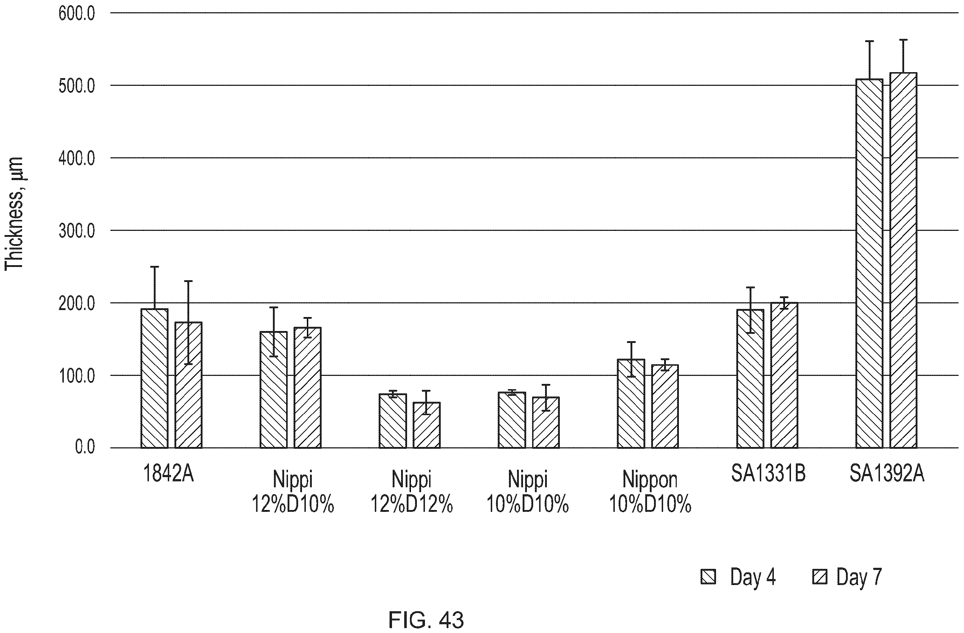

**FIG. 43** is a bar graph showing thickness over time for different samples tested in the cell attachment assay at days 4 and 7 is provided.

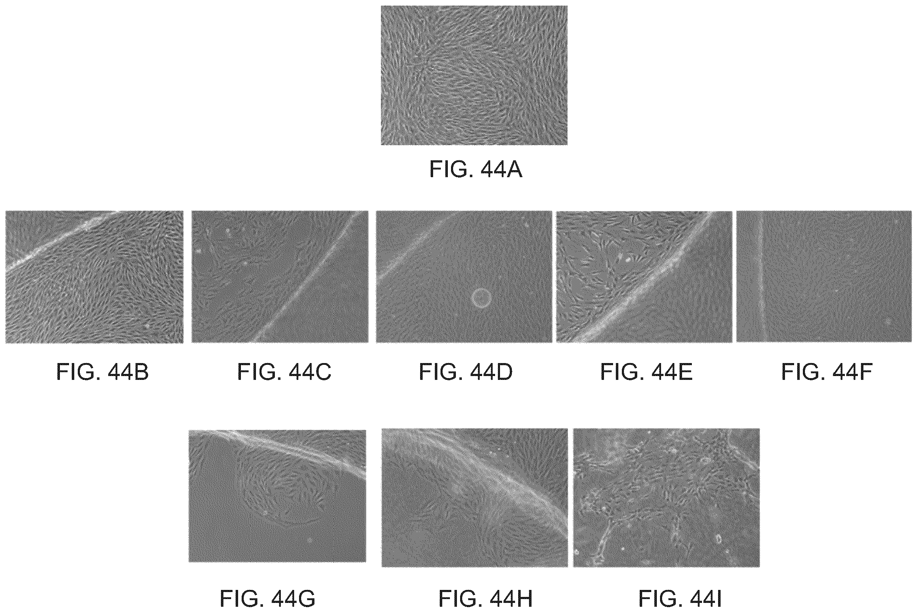

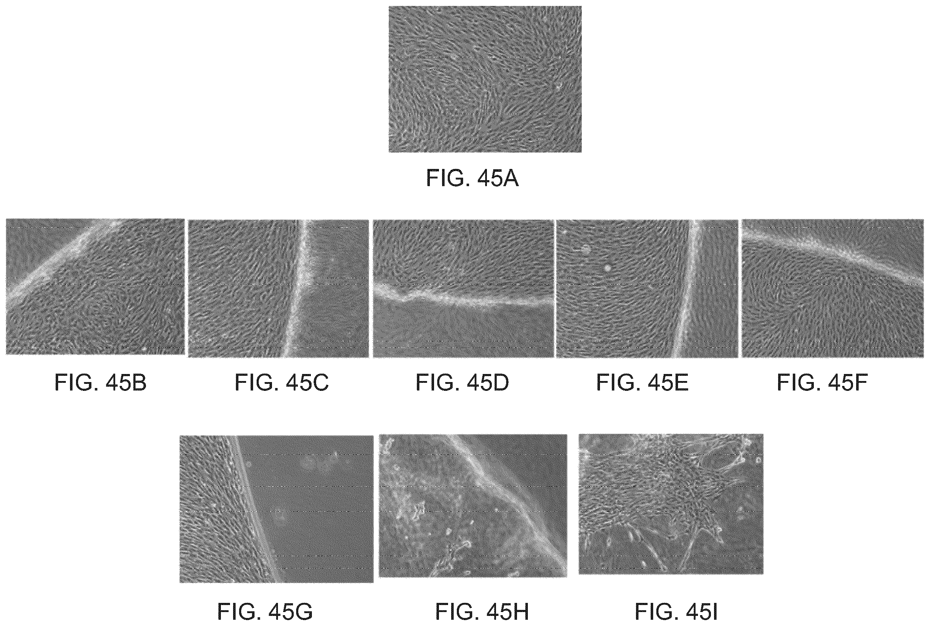

Tables 5 and 6 microscopy results for different samples tested in the cell attachment assay at days 4 and 7, respectively, are provided. The tables include descriptions related to confluency of the cells. **FIGS. 44A-44I** and **FIG. 45A-45I** show microscopy images for different samples tested in the cell attachment assay at days 4 and 7, respectively. **FIG. 44A** and **FIG. 45A** show images for the control, **FIG. 44B** and **FIG. 45B** show images for Ferentis 1842A, **FIG. 44C** and **FIG. 45C** show images for Nippi 12% D12%, **FIG. 44D** and **FIG. 45D** show images for Nippi 10%D10%, **FIG. 44E** and **FIG. 45E** show images for Nippi 12%D10%; **FIG. 44F** and **FIG.45F** show images for Nippon 10%, **FIG. 44G** and **FIG. 45G** show images for SA-13-31B, **FIG. 44H** and **FIG. 45H** show images for SA-13-92A edge, and **FIG. 44I** and **FIG. 45I** show images for SA-13-92A on sample.

Table 5. Microscopy results for different samples tested in cell attachment assay at day 4.

| Sample | Description |
|---|---|
| Controls | Confluent |
| Ferentis 1842A | Confluent on and off material, some cells under, one sample floating |
| Nippi 10%D10% | Confluent on and off material |
| Nippon 10%D10% | Confluent on and off material, bubbles in material |

(continued)

| Sample | Description |
|---|---|
| Nippi 12%D10%, Nippi 12%D12% | Mostly confluent on and off, some less confluent spots, some bubbles in material |
| SA1392A | A few cells on material, some dead cells, confluent on plate |
| S1331B | No cells on material, confluent on plate |

Table 6. Microscopy results for different samples tested in cell attachment assay at day 7.

| Sample | Description |
|---|---|
| Controls | Overconfluent |
| Ferentis 1842A | Confluent on and off material, some cells under, one sample floating |
| Nippi10%D10% | Confluent on and off material |
| Nippon 10%D10% | Confluent on and off material, bubbles in material; material appears grainy |
| Nippi 12%D10%, Nippi 12%D12% | Confluent on and off, bubbles in material |
| SA1392A | A few cell patches on material, some dead cells, confluent on plate, cells under |
| S1331B | No cells on material, confluent on plate, dead cells |

## Summary of Assay

Thickness findings were as follows:

Nippi 10%D10% and Nippi 12%12% are thinnest (65-80 $\mu$m).

Nippon 10%D10% are 115-120 $\mu$m in thickness.

Nippi 12%D10% about are 160 $\mu$m in thickness.

Ferentis 1842A and SA-13-31B materials are around 170-200 $\mu$m in thickness.

SA-13-92A materials are around 500 $\mu$m in thickness.

Thickness remains steady over culture time.

The cells attached and grew well on all materials, becoming confluent on all samples by day 7.
Control non-collagen samples did not support cell growth (but are not toxic to the cells on the plate).
Collagen coated control samples had a few patches of cells attached.
Bubbles were observed in the material itself in: Nippi 12%D10%, Nippi 12%D12%, and Nippon 10%D10%

## Microscopy

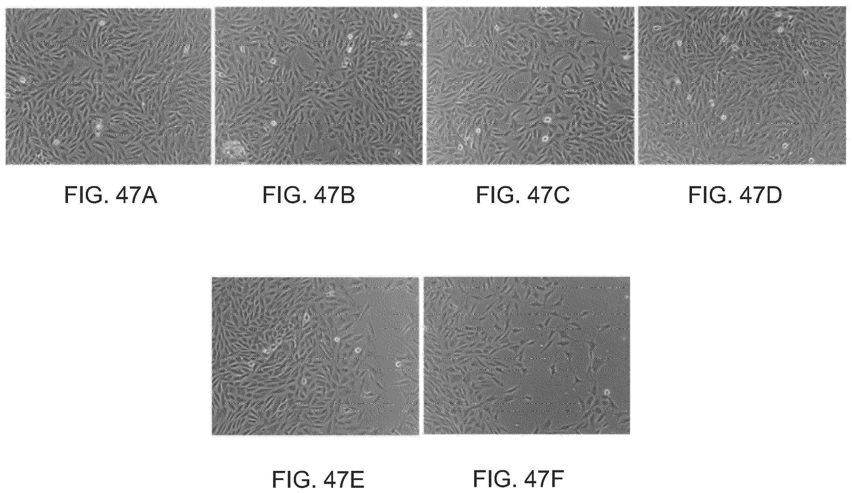

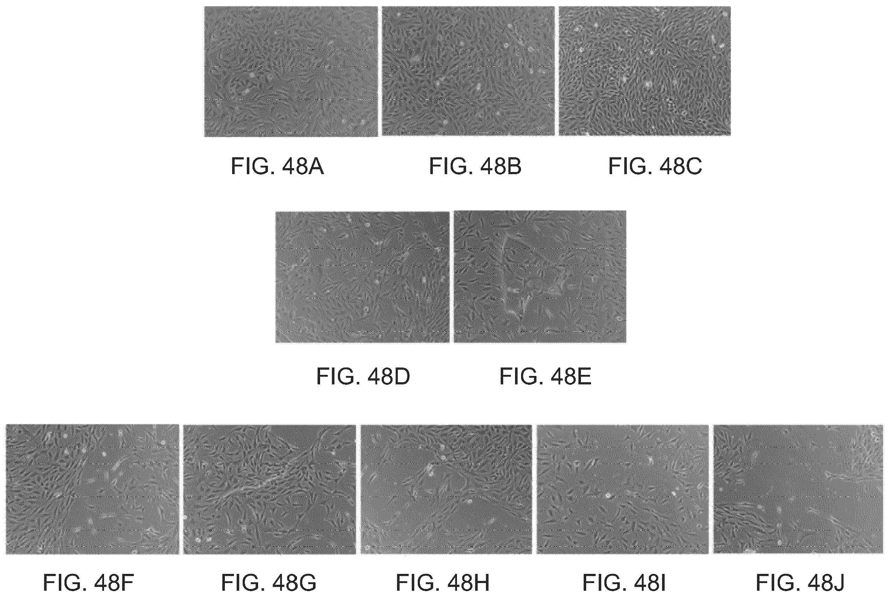

**FIGS. 47A-47F** show microscopy images for control samples tested in the cell attachment assay. **FIGS. 48A-48J** show microscopy images for 1745A samples tested in the cell attachment assay. At day 3 microscopy imaging, it was hard to image edges due to 24 well plate. Imaged the center of each well to get an idea of cell growth on the materials compared to controls. Controls included: (1) 4/6 samples 70-100% confluent, and (2) 2/6 samples mostly confluent, a few patches in center. 1746A samples included: (1) 4/10 confluent at edges and nearly confluent in center, (2) 1/10 about 60% confluent in center, confluent at edges, and (3) 5/10 samples 30-40% confluent in center, patchy, some holes. Controls are more confluent overall than samples, but not a stark difference and might be hard to pick up on MTT assay. **FIGS. 7A-47D** show control sample images for 4/6 samples, 80-100% confluent. **FIGS. 47E-47F** show control sample images for 2/6 samples mostly confluent, a few patches in center. **FIGS. 48A-48C** show 1745A sample images for 3/10 confluent at edges and nearly confluent in center. **FIGS. 48D-48E** show 1745A sample images for 2/10 60-70% confluent in center, confluent at edges. **FIGS. 48F-48J** show 1745A sample images for 5/10 samples 30-40% confluent in center, patchy, some holes.

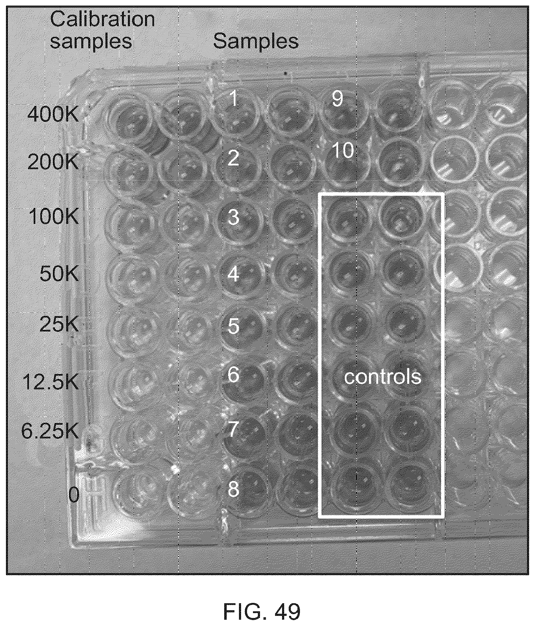

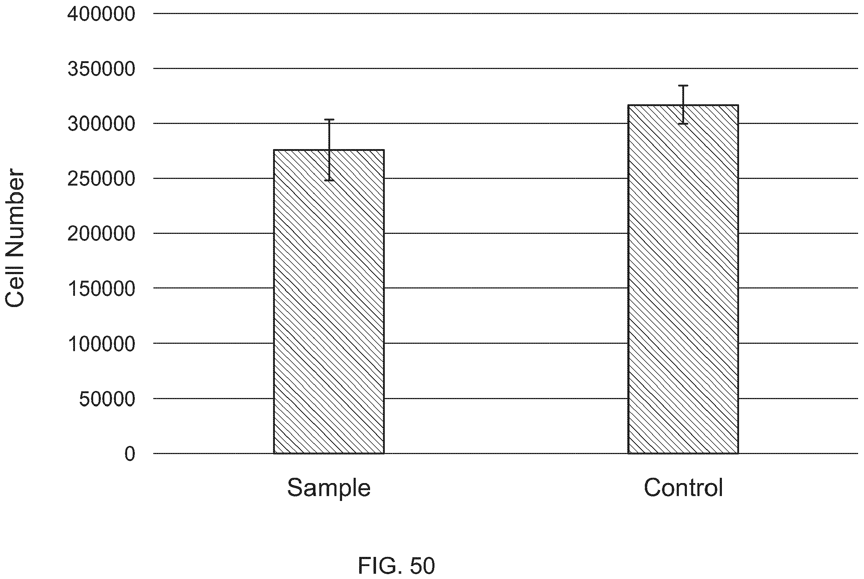

**FIG. 49** is an image of an MTT plate illustrating the setup for samples tested in the cell attachment assay. **FIG. 50** is a bar graph showing cell numbers for MTT results in the cell attachment assay for a sample and control.

### Onlay/Epithelial Cell Studies

Questions Asked

Can corneal epithelial cells migrate over material X?

Do epithelial cells attach to the material?

Do epithelial cells form multiple layers on the material?

### Protocol

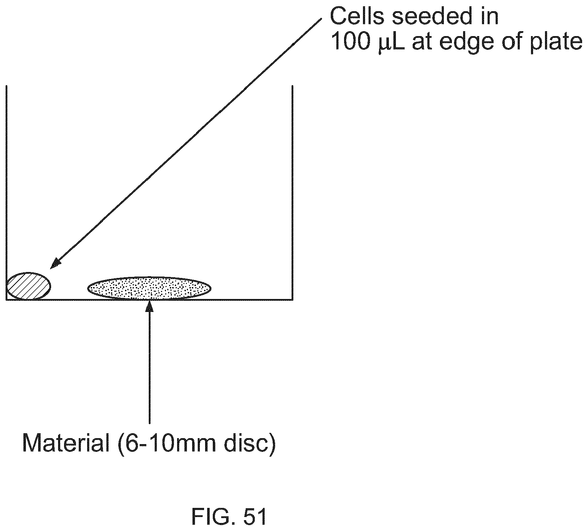

With respect to **FIG. 51,** a diagram of seeding materials during a cell attachment assay is provided. Cells were seeded in 100 μL at edge of plate. The material was a 6-10 mm disc.

Samples were cultured with passaged corneal epithelial cells.

Cells were not seeded on samples to evaluate cell migration onto material. Cells were seeded at edge in small volume of media. 4 mL added gently after 30 minutes.

Possible control material: denuded cornea.

Sample evaluation: (1) Migration onto material/attachment to material (light microscopy); and (2) Ability to form cell layers (confocal microscopy).

Possible addition: (1) Stimulate differentiation; and (2) Confocal microscopy for focal adhesion proteins, epithelial cell differentiation markers.

### Microscopy

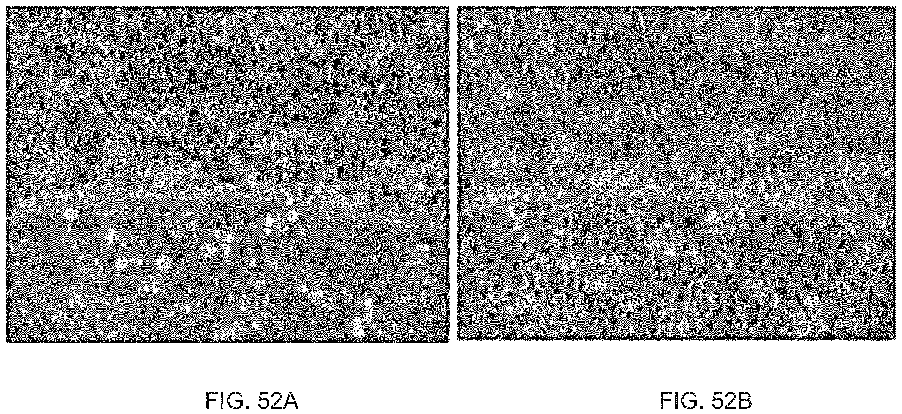

With respect to **FIGS. 52A-52B,** light microscopy images of PC-MPC cells attached to a sample are provided. The cells are passaged corneal epithelial cells that have migrated over or attached to the material, PC-MPC (porcine collagen-methacryloyloxyethyl phosphorylCholine).

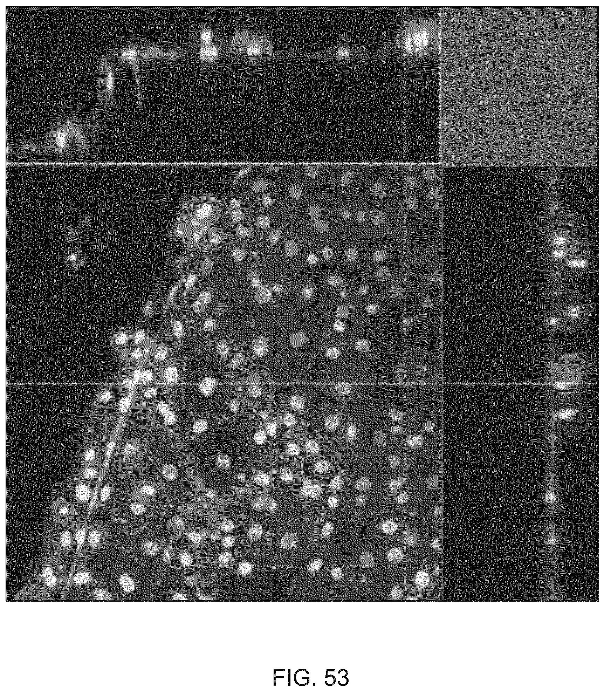

With respect to **FIG. 53,** a confocal microscopy image of a cross-section of PC-MPC material following incubation with epithelial cells. The nuclei of the cells fluoresce in green and hence can be viewed under a confocal fluorescence microscope. There are few multi-layer spots on top of the material.

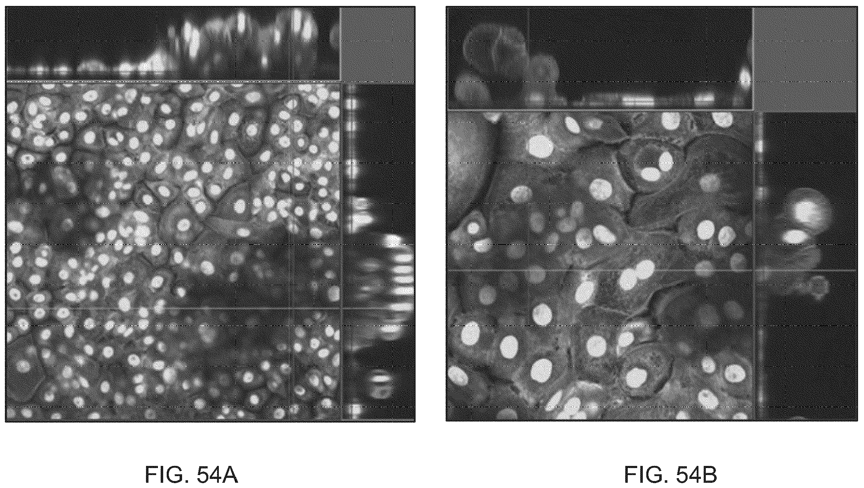

With respect to **FIGS. 54A-54B,** confocal microscopy images of a cross-section of controls showing some multilayered structures as a baseline for comparison are provided. The control was the bare tissue culture plate. Epithelial cells were able to grow over the plate surface. Denuded cornea (e.g., cornea with the epithelial layer scrapped off) could be used as another control, and it is expected that epithelial cells will grow over the denuded cornea.

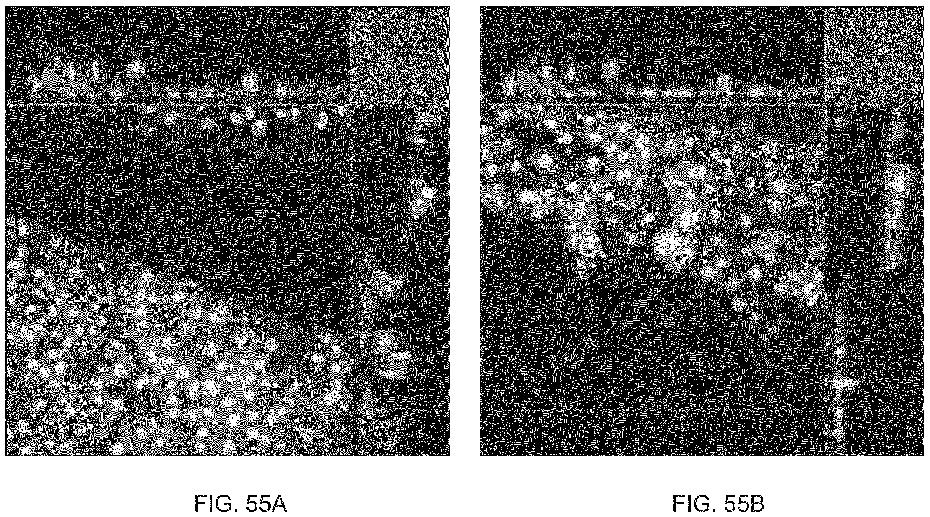

With respect to **FIGS. 55A-55B,** confocal microscopy images of a cross-section of 3D PEG gelatin material with epithelial cells on a plate and on surface demonstrating the ability of cells to form cell layers. Both have multi-layered spots;

### Example 7. Evaluating Haze After Administration of the Sponsor's Corneal Implants in New Zealand White Rabbits (Non-GLP) (Sponsor: RVO 2.0)

Purpose/Objective(s): The purpose of this animal study is to evaluate haze after implantation of the Sponsor's corneal implants comprising the described hydrogel composition in the eyes of New Zealand White rabbits (non-GLP) compared to control test articles shown in Table 7.

Regulatory Status: This study is not intended to be conducted in accordance with U.S. FDA regulations 21 CFR Part 58 Good Laboratory Practice for Nonclinical Laboratory Studies (and all amendments, effective June 20, 1979). However, it is conducted in compliance with the Standard Operating Procedures established at ASC.

Background: This study is designed to evaluate haze after administration of the Sponsor's corneal implants in rabbits. This information can only be obtained from living systems treated with the Sponsor's test articles. A non-animal model would not provide the data on the test articles that are being evaluated; therefore, a whole body test system is required. The rabbit is a standard species used in ocular studies based upon historical data and FDA requirements. Rabbits are recognized as a preferred and optimal model for assessing ocular study endpoints. Rabbits have proven to be useful in ophthalmic research considering their ocular anatomy and physiology is similar to humans and their eyes have similar metabolic pathways.

A. **Proposed study duration:** 5-6 months.

B. **Experimental design**

*1. Test System*

| | |
|---|---|
| Species: | Oryctolagus cuniculus |
| Strain: | New Zealand White rabbits (naive) |
| Sex: | Male or female (all same sex) |
| Age: | Commensurate with weight |
| Weight: | Approximately 3.5 to 4.5 kilograms at study start |
| Number: | 10 (+ up to 2 extras) |
| Vendor: | approved vendor |
| Method ofIdentification: | Ear tag and cage label per ASC SOPs |
| Caging: | As per ASC SOPs |
| MinimumAcclimation: | 5 days |

2. Specialized animal husbandry and/or restraint

Fasting - None

Restraint - Animals will be manually restrained per ASC SOPs to facilitate ophthalmic examinations and imaging.

Housing - Animals will be singly housed prior to and during the study in order to decrease the likelihood of ocular injuries from cage mates.

C. **Test Articles are shown in Table 7.**

Table 7. Test articles*

| Test article | Name | Physicochemical Characteristics and Composition Description | Manufacturer | Storage conditions |
|---|---|---|---|---|
| 1 | **Raindrop Inlay** | Raindrop near vision inlay; 78% water content; Diameter: -2.0 mm; Thickness: -34 microns; | ReVision Optics | Room temperature |
| 2 | **Ferentis PC-MPC Disc** | Porcine collagen/2- methacryloyloxethyl phosphorylcholine (PC-MPC) polymer Diameter 2.0 mm 92-97% (w/w) water content | Ferentis | Refrigerate at 2-8 C |
| 3 | **OM PC- MPC- APS Disc** | Porcine collagen/2- methacryloyloxethyl phosphorylcholine (PC-MPC polymer Diameter: 1.8-2.0 mm 90-94% (w/w) water content | Optics Medical | Refrigerate at 2-8 C |

(continued)

| Test article | Name | Physicochemical Characteristics and Composition Description | Manufacturer | Storage conditions |
|---|---|---|---|---|
| 4 | **OM PC- MPC- APS Molded Implant** | Porcine collagen/2- methacryloyloxethyl phosphorylcholine (PC-MPC polymer Diameter: 1.8-2.0 mm 90-94% (w/w) water content | Optics Medical | Refrigerate at 2-8 C |
| 5 | **OM PC- MPC- LAP Disc** | Porcine collagen/2- methacryloyloxethyl phosphorylcholine (PC-MPC polymer Diameter: 1.8-2.0 mm 92-96% (w/w) water content | Optics Medical | Refrigerate at 2-8 C |
| 6 | **OM-PC- MPC- LAP molded implant** | Porcine collagen/2- methacryloyloxethyl phosphorylcholine (PC-MPC polymer Diameter: 1.8-2.0 mm 92-96% (w/w) water content | Optics Medical | Refrigerate at 2-8 C |
| 7 | **OM-PC- MPC-1% collagen- Low H$_2$O content** | Porcine collagen/2- methacryloyloxethyl phosphorylcholine/polyethylene glycol (PC-MPC-PEG) polymer Diameter: 1.8-2.0 mm 79-82% (w/w) water content | Optics Medical | Refrigerate at 2-8 C |
| 8 | **OM PC- MPC- 2.5% Collagen- Low H2O content** | Porcine collagen/2- methacryloyloxethyl phosphorylcholine/polyethylene glycol (PC-MPC-PEG) polymer Diameter: 1.8-2.0 mm 79-82% (w/w) water content | Optics Medical | Refrigerate at 2-8 C |
| 9 | **MDB PEG Synthetic Disc** | PEG synthetic disc Diameter: 1.8-2.0 mm Water content range unavailable | Medical Device Biopolymers | Refrigerate at 2-8 C |
| *ID (Lot #) and Date of Expiry (or retest date) will be recorded in the raw data. | | | | |

10. Test Article Preparation, Accounting, and Disposition

Test articles will be supplied by the Sponsor sterile for implantation and ready to administer. Test material accountability will be maintained according to ASC SOPs. After test article administration has completed, any leftover test articles will be directly transferred to the Sponsor by hand or shipped to the Sponsor on cold packs.

11. **Pre-Treatment Procedures and Details of Test Article Administration**

Pre-Treatment Examinations

Prior to placement on study, each animal will undergo an ophthalmic examination (slit-lamp biomicroscopy and indirect ophthalmoscopy) to be performed by the Study Director or Director of Ophthalmology. Ocular findings will be scored according to a modified McDonald-Shadduck Scoring System per SOP ASC-OC-001. The acceptance criteria for placement on study will be scores of "0" for all variables.

Anesthesia

Animals will be anesthetized with an intramuscular (IM) injection of ketamine hydrochloride (up to approximately 50 mg/kg) and xylazine (up to approximately 10 mg/kg) or dexmedetomidine (approximately 0.25 mg/kg). Glycopyrrolate (approximately 0.01 mg/kg, IM) may be administered concurrently. Atipamezole hydrochloride (up to 1 mg/kg, IM) may be used as a reversal agent.

Alternative anesthesia regimens may be used as advised by the veterinary staff in consultation with the Study Director and the Sponsor.

After the area has been surgically prepped and prior to the surgical procedure, one to two drops of topical proparacaine hydrochloride anesthetic (0.5%) will be applied to the animals' eyes. Additional topical ocular anesthesia dosing may be utilized during the procedures if needed.

Animals may be placed on a thermal heating pad to help maintain body temperature, if necessary. If anesthesia persists longer than 15 minutes, vital signs will be monitored every 15 minutes throughout anesthesia and recovery.

Nictitating Membrane Removal

Each rabbit will have the nictitating membrane removed from both eyes prior to test article administration. Since humans do not have nictitating membranes, removal of these membranes provides a model that more closely mimics human eyes. Nictitating membranes will be removed at least 14 days prior to test article administration.

Animals will be anesthetized as described herein. A 5% Betadine solution will be used to clean the eye and surrounding area. Betadine will be applied for ~5 minutes, after which the eye will be rinsed with balanced salt solution (BSS) and the surrounding area wiped with gauze.

One to two drops of topical proparacaine hydrochloride anesthetic (0.5%) will be applied to the nictitating membrane of each eye prior to the surgical procedure.

The nictitating membrane will be grasped with a pair of forceps and gently clamped at its base with a pair of hemostats. The nictitating membrane will be excised along the clamp line with scissors. Using a cauterizing unit, the excised area will be sealed to stop any further bleeding. BSS will be applied to the cauterized area. The area may be blotted and medicated with topical gentamicin (0.3%) and/or antibiotic ophthalmic ointment.

The contralateral eye will have its nictitating membrane removed by the same procedure.

Animals will be monitored during anesthesia recovery per ASC SOPs. Antibiotic ointment will be applied topically once immediately following nictitating membrane removal and daily for up to 3 days post-operatively. One injection of buprenorphine (0.02-0.05 mg/kg, IM/SC) will be given perioperatively. Additional buprenorphine injections (0.02-0.05 mg/kg, IM/SC, twice daily or sustained-release buprenorphine 0.1 mg/kg, SC, every 72 hours) may be administered in the days after surgery as deemed necessary by the veterinary staff.

The day after surgery, animals will be examined to ensure there were no post-surgical complications. If post-surgical complications occur, the Study Director and/or veterinary staff will be consulted as to the appropriate course of action to maintain the animal's health and well-being.

Group Assignment

One animal will be assigned per group.

Test Article Administration

Test articles will be implanted in the corneas of both eyes of all study animals on Day 1 according to the study design in Table 2 (below).

All surgical procedures will be performed by the Sponsor's designated surgeon. ASC will provide technician support, anesthesia monitoring, and basic surgical equipment (including a surgical table, surgical microscope, and slit-lamp) and supplies (including sutures, gowns, etc.). Specialized surgical tools and supplies, including the laser and/or microkeratome, will be provided by the Sponsor.

Animals will be anesthetized as outlined herein. A 5% Betadine solution will be used to clean the eye and surrounding area. Betadine will be applied for ~5 minutes, after which the eye will be rinsed with BSS and the surrounding area wiped with gauze.

A flap or pocket will be cut into each cornea using a laser or a microkeratome. The surgery type utilized for each eye will be noted in the study data.

The appropriate test article for each eye will be inserted into the flap or pocket. Test articles will be stained with 10 or 25% fluorescein solution (provided by the Sponsor) to facilitate visualization during the implantation procedure.

Further details of surgical procedures may be noted in the raw data.

Animals will be monitored during anesthesia recovery per ASC SOPs. One injection of buprenorphine (0.02-0.05 mg/kg IM/SC) will be given for post-surgical analgesia. Additional buprenorphine injections (0.02-0.05 mg/kg, IM/SC, twice daily or sustained-release buprenorphine 0.1 mg/kg, SC, every 72 hours) may be administered in the days after surgery as deemed necessary by the veterinary staff.

Antibiotic ophthalmic ointment (e.g. triple antibiotic ointment) or antibiotic eye drops (e.g. 0.3% tobramycin) and anti-inflammatory topical steroids (e.g. 0.1% prednisolone acetate) will be administered on Days 2-4

Analgesic, anti-inflammatory, and/or antibiotic regimens may be extended or otherwise modified as necessary at the discretion of the veterinary staff in consultation with the Study Director and the Sponsor. All post-surgical treatments will be recorded in the raw data.

12. **Safety Precautions**

Standard laboratory safety procedures will be employed for handling the test articles. Specifically, gloves and lab coat along with appropriate vivarium attire will be worn while preparing and administering the test articles. Eye protection will be worn as appropriate during operation of the surgical laser.

Table 8. Study Design

| Group/ Animal | Eye | Test Article | Surgery Technique# | Slit-lamp Examination | Corneal OCT* | Termination Time | Tissues Collected |
| --- | --- | --- | --- | --- | --- | --- | --- |

(continued)

| Group/ Animal | Eye | Test Article | Surgery Technique# | Slit-lamp Examination | Corneal OCT* | Termination Time | Tissues Collected |
|---|---|---|---|---|---|---|---|
| 1 | OS | Raindrop Inlay | Flap or Pocket | | | | |
| 1 | OD | Raindrop Inlay | Flap or Pocket | | | | |
| 2 | OS | Ferentis PC-MPC | Flap or Pocket | | | | |
| 2 | OD | Ferentis PC-MPC | Flap or Pocket | | | | |
| 3 | OS | OMPC-MPC-APS | Flap or Pocket | | | | |
| 3 | OD | OMPC-MPC-APS | Flap or Pocket | | | | |
| 4 | OS | OMPC-MPC-APS | Flap or Pocket | | | | |
| 4 | OD | OMPC-MPC-APS | Flap or Pocket | | | | |
| 5 | OS | OMPC-MPC-LAD | Flap or Pocket | | | | |
| 5 | OD | OMPC-MPC-LAD | Flap or Pocket | | | | |
| 6 | OS | OMPC-MPC-LAD | Flap or Pocket | Baseline" and Days 8(±1), 30 (±3), 60(±5), & 90 (±7) | Baseline and Days 8 (±1) & 90 (±7) | Day 90(±7) | Eyes (Whole Globes) OU |
| 6 | OD | OMPC-MPC-LAD | Flap or Pocket | | | | |
| 7 | OS | OMPC-MPC-1% Collagen - Low H2O | Flap or Pocket | | | | |
| 7 | OD | OMPC-MPC-1% Collagen - Low H2O | Flap or Pocket | | | | |
| 8 | OS | OMPC-MPC-2.5% Collagen - Low H2O | Flap or Pocket | | | | |
| 8 | OD | OMPC-MPC-2.5% Collagen - Low H2O | Flap or Pocket | | | | |
| 9 | OS | OMPC-MPC-1% Collagen - Low H2O | Flap or Pocket | | | | |
| 9 | OD | OMPC-MPC-2.5% Collagen - Low H2O | Flap or Pocket | | | | |
| 10 | OS | MDB PEG Synthetic Disc | Flap or Pocket | | | | |
| 10 | OD | MDB PEG Synthetic Disc | Flap or Pocket | | | | |

OD: right eye; OS: left eye; OU: both eyes; OCT: optical coherence tomography.

"Baseline examinations will include indirect ophthalmoscopy and assess full set of ocular observation variables; remaining examinations will assess variables related to corneal haze/opacity only.

#Surgical technique and type (laser flap or pocket or microkeratome flap) will be chosen on the day of surgery at the discretion of the Sponsor and recorded in the raw data for each eye.

*Additional time points may be added at the discretion of the Sponsor with the approval of the Study Director and Attending Veterinarian.

tMay optionally be extended for some or all animals at the Sponsor's discretion with the agreement of the Study Director and Attending Veterinarian. Alternatively, animals may be euthanized earlier in case of corneal damage developing.

## DETAILS OF IN-LIFE OBSERVATIONS AND MEASUREMENTS

### Body Weights

Animals will be weighed prior to test article administration, monthly thereafter, and prior to termination.

**Cageside Observations**

Cageside observations will be performed once daily starting on Day 1 and continuing throughout the study. A survival check will be performed for each animal, and the animal will be evaluated for moribundity or other obvious illness or injury. Any findings of death, moribundity, or obvious illness or injury will be reported on the same day to the Study Director, the Sponsor, and the Attending Veterinarian.

**Detailed Clinical Observations**

Detailed clinical observations will be performed at baseline (prior to test article administration) and once weekly thereafter throughout the duration of the study.

**Slit-Lamp Examinations**

Slit-lamp examinations will be performed on both eyes of all animals at baseline (prior to test article administration) and on Days 8($\pm$1), 30($\pm$3), 60($\pm$5), and 90($\pm$7). Additional time points may be added at the Sponsor's discretion with the agreement of the Study Director and Attending Veterinarian.

Ocular findings will be scored according to a modified McDonald-Shadduck Scoring System per SOP ASC-OC-001. Baseline examinations will include both slit-lamp biomicroscopy and indirect ophthalmoscopy and will assess all ocular observation variables. All other examinations will include slit-lamp biomicroscopy only and will assess only the ocular observation variables related to corneal haze/opacity, namely "Cornea" (severity of corneal haze/opacity) and "Surface Area of Cornea Involvement" (area of corneal haze/opacity).

In addition, examinations will include scoring of corneal haze following a study-specific scoring system.

Examinations will be performed either by the Study Director, the Director of Ophthalmology, and/or the Sponsor Representative.

**Corneal Optical Coherence Tomography (OCT)**

Optical coherence tomography (OCT) of the cornea will be performed at baseline (prior to test article administration) and on Days 8($\pm$1) and 90($\pm$7). Additional time points may be added at the Sponsor's discretion with the agreement of the Study Director and Attending Veterinarian.

Heidelberg Spectralis® Eye Explorer image processing software (HEYEX Software Version 1.9.10.0, Heidelberg Engineering GmbH, Heidelberg, Germany) will be used to measure the thickness of the cornea near the center of the cornea and the depth of the test article implant in the cornea at each time point.

**Terminal Procedures**

**1. Early Death/Unscheduled Sacrifice**

If an animal dies on study, the time of death will be estimated as closely as possible and recorded. The animal may be necropsied; if so, necropsy will be performed as soon as possible. If the necropsy cannot be performed immediately, the animal will be refrigerated (not frozen) to minimize tissue autolysis. Animals that are prematurely terminated may be necropsied after discussion with the Sponsor, and major organ findings noted.

If an animal is moribund as defined by SOPs ASC-AC-007 and ASC-QP-015, it will be euthanized as described below, which is in accordance with ASC's policies on humane care of animals. If an animal possesses any of the following signs it will be considered as indicative of moribund condition: impaired ambulation which prevents the animal from reaching food or water, excessive weight loss and emaciation (>20%), lack of physical or mental alertness, difficult labored breathing, or inability to remain upright. Animals with other less severe clinical signs will be treated (antibiotics or analgesics, fluids, etc.) or euthanized after discussion with Attending Veterinarian and Study Director. Any alternate endpoints (e.g. death, allowing ill animals to remain untreated and alive [i.e. moribund endpoints], etc.) must be justified in study documentation.

If possible, blood or other specimens will be collected and analyzed as appropriate (e.g., for clinical pathology parameters) to help reveal the cause of malaise/morbidity. All unscheduled-sacrifice animals may be necropsied; if so, necropsy will be performed immediately, or, if this cannot be performed, the animal will be refrigerated to minimize autolysis and necropsied no later than 12 hours after death. All tissues listed in this protocol will be preserved.

Specific ocular endpoints necessitating treatment and/or euthanasia would be:

• Ocular infection

- Ocular hemorrhage, hyperemia

- Visual impairment that is manifesting in behavior abnormalities, pain, and/or distress to the animal

- Loss of globe integrity

In the event that an animal dies or is euthanized during the study, terminal procedures will be conducted as per SOP ASC-AC-007.

**2. Euthanasia**

Animals will be euthanized on Day 90 ($\pm$7). Alternatively, the study may be extended for some or all study animals at the Sponsor's discretion with the agreement of the Study Director and Attending Veterinarian, or animals may be euthanized earlier in case of corneal damage developing; in this case, the day(s) and time(s) when animals are euthanized will be recorded in the study data.

Animals will be euthanized by an intravenous injection of a commercial barbiturate based euthanasia solution (approximately 150 mg/kg, to effect). The euthanasia procedure will be performed in compliance with the American Veterinary Medical Association (AVMA) Guidelines for Euthanasia of Animals: 2020 Edition.

**3. Tissue Collection**

Immediately following euthanasia, both eyes will be collected from all animals following on of the below methods. The Sponsor will be contacted before euthanasia to determine which method should be used for each eye of each animal.

Method 1

A 2% paraformaldehyde (PFA) solution in phosphate-buffered saline (PBS), pH 7.4, will be made fresh on the day of tissue collection.

Immediately following euthanasia, the anterior chamber of the eye will be perfused with 2% PFA in PBS for 4 minutes to fix the cornea. Perfusion will be performed using handheld syringes using a push-pull technique (two needles, one for pushing in PFA, one for pulling out aqueous humor). A slow push/pull will be used to maintain the internal pressure of the eye and avoid damage to the cornea. After perfusion, the eye will be harvested. The cornea plus 1-2 mm limbal tissue will be excised using scalpel puncture and curved corneal scissors. The remaining eye will be discarded.

The excised cornea will be placed into a chilled container with 2% PFA. The container will be sealed to prevent leakage or evaporation and immediately placed on wet ice until being stored refrigerated at 2-8°C.

Samples collected via this method will be shipped on cold packs via overnight shipment within 2 days of collection (to ensure receipt of samples within 3 days of collection) to the Sponsor's designated laboratory.

Method 2

Immediately following euthanasia, the eye will be harvested. Excess tissues will be trimmed off, and the whole globe will be immediately placed in Davidson's solution and stored at room temperature. To ensure consistent fixation, a gauze pad will be used to keep the eye submerged if necessary.

After 48 hours in Davidson's solution for 48 hours, the eye will be transferred into 70% ethanol.

Samples collected via this method will be shipped at ambient temperatures to the Sponsor's designated laboratory.

**F. Calculations and Statistical Analysis**

Data will be presented in tabular format and no calculations or statistical analysis will be performed on the data collected during the in-life portion of the study.

**G. Test System (Animals and Animal Care)**

**1. Species/Strain, Number and Sex**

Ten (10) male or female (single sex) New Zealand White (NZW) rabbits (plus up to 2 extras), weighing between approximately 3.5 to 4.5 kilograms at study start, will be used for this study.

## 2. Source and Experimental History

The animals to be used in this study will be obtained from an approved vendor. The name, address, and telephone number of the animal source will be included in the permanent animal records, and the source will be specified in the study file.

## 3. Starting Age and Weight Range

Animals selected for use in this study will be as uniform in age and weight as possible. Records of the dates of birth for the animals used in this study will be retained in the ASC archives, and the weight range at the time of group assignment will be specified in the study file.

## 4. Identification per SOP ASC-AC-001 (Animal and Room Identification)

Animals will be identified by an ear tag placed by the vendor and by the cage label.

## 5. Housing and Environmental Controls per SOP ASC-HU-002 (Environmental Monitoring and Control of Animal and Procedure Areas)

The animals will be housed in individual cages and within the same room(s) as per ASC SOPs. Primary enclosures will be as specified in the USDA Animal Welfare Act (9 CFR, Parts 1, 2, and 3) and as described in the Guide for Care Use of Laboratory Animals (ILAR publication, 2011, National Academy Press). The room(s) in which the animals will be kept will be documented in the study records.

No other species will be housed in the same room(s). The rooms will be well ventilated (greater than 10 air changes per hour) with at least 60% fresh air. A 12-hour light/12-hour dark photoperiod will be maintained, except when rooms must be illuminated during the dark cycle to accommodate necessary study procedures. Room temperature and humidity will be recorded once daily as per ASC SOPs.

## 6. Food and Water per SOP ASC-HU-012: (Husbandry, Rabbits)

Animals will have *ad libitum* access to species specific chow. No contaminants are known to be present in the diet at levels that would interfere with the results of this study.

Municipal tap water will be available *ad libitum* to each animal. No contaminants are known to be present in the water at levels that would interfere with the results of this study. Records of annual water quality testing are maintained in the ASC archives and may be shared with the Sponsor.

## 7. Acclimatization per SOP ASC-HU-003 (Animal Ordering, Receiving and Acclimation Procedures)

All study animals will be acclimated to their designated housing for at least 5 days prior to test article administration.

## 8. Humane Care and Use of Animals

The study site is an AAALAC International-accredited site. During the study, the care and use of animals will be conducted in accordance with the regulations of the USDA Animal Welfare Act (i.e., relevant sections of Section 9, Parts 1, 2, and 3, of the Code of Federal Regulations) and in compliance with ASC's Animal Welfare Assurance (D16-00645 [A4282-01]) filed with the Office of Laboratory Animal Welfare (OLAW) at the National Institutes of Health (NIH), as applicable. Treatment of the animals will be in accordance with ASI SOPs and the conditions specified in the Guide for Care Use of Laboratory Animals (ILAR publication, 2011, National Academy Press).

This protocol and any amendments or procedures involving the care or use of animals in this study will be reviewed and approved by ASC's Institutional Animal Care and Use Committee (IACUC) prior to the initiation of such procedures (i.e., prior to the start of the study for most protocol-specified procedures).

The study animals will be observed at least daily for signs of illness or distress, and any such observations will be promptly reported to the Attending Veterinarian and Study Director. The Attending Veterinarian may make initial recommendations about treatment of the animal(s) and/or alteration of study procedures, which must be approved by the Study Director and the Sponsor.

All such actions will be properly documented in the study records and, when appropriate, by protocol amendment. If the condition of the animal(s) warrants significant therapeutic intervention or alterations in study procedures, the Sponsor Representative will be contacted via phone and email during normal working hours to discuss appropriate action. If the condition of the animal(s) is such that emergency measures must be taken, the Attending Veterinarian will attempt to consult with the Study Director and Sponsor Representative prior to responding to the medical crisis, but the veterinary staff has authority to act immediately at their discretion to alleviate suffering. The Sponsor Representative will be fully informed of any such events. In the event that an animal dies or is euthanized during the study, terminal procedures will be conducted as described above.

### 9. Final Disposition

Carcasses of deceased animals will be discarded following post mortem examination in accordance with applicable regulations on biological waste.

### 10. Unscheduled Treatment

Any unscheduled treatment of the animal(s) will be approved by the Study Director and the Sponsor. If the condition of the animal(s) is such that emergency measures must be taken, the Attending Veterinarian will attempt to consult with the Study Director and Sponsor prior to responding to the medical crisis, but the veterinary staff has authority to act immediately at their discretion to alleviate suffering. All unscheduled treatments will be recorded.

### Records and Reports

### A. Records

The following will be collected and retained as part of the study file or in ASC's archives:

- Animal procurement records including existing prior health records and vendor
- Test article records
- Surgical procedure records
- Records of cageside and other clinical or health observations
- Body weight data
- Clinical ophthalmic examination scores
- Images
- Euthanasia and tissue collection records
- Shipping logs
- Protocol, amendments, and deviations
- Study file

All records and remaining tissue specimens that are not consumed during analysis or submitted to the Sponsor or Sponsor's designated laboratory will be retained and archived. For those items listed above, the storage location will be at the study site for a period of 1 year from submission of study data to the Sponsor, after which time the Sponsor has 90 days to collect records and specimens. Otherwise, records and specimens will be disposed of after 90 days. The Sponsor will be responsible for archival of records and tissue specimens that are derived from portions of the study conducted by the Sponsor (or a Sponsor-appointed subcontractor).

### B. Draft Report

The draft report will include test article information and description, materials and methods, results, and conclusions.

The Sponsor will provide comments based on a review of the summary and data. Report finalization will be conducted on a mutually-agreeable timetable. Upon finalization, a copy of the final report will be provided to the Sponsor as a PDF file.

### C. Corneal Haze Scoring

Haze grading is based on a scale used to grade post-PRK Haze, Arch. Ophthalmology (1992) (110): 1286-1291):

Clear (Grade 0): sporadic, peripheral faint haze, (CLEAR CENTER), not visible with diffuse slit lamp beam, minimally visible by oblique or slit beam. Vision is not affected.

Trace Haze (Grade 1): Trace haze covering mid-peripheral and center of inlay. Visible with difficulty using diffuse illumination, visible by broad tangential illumination. May present with myopic shift, reduced hear point, visual symptoms (glare and halo).

Mild (Grade 2)

Moderate (Grade 3)

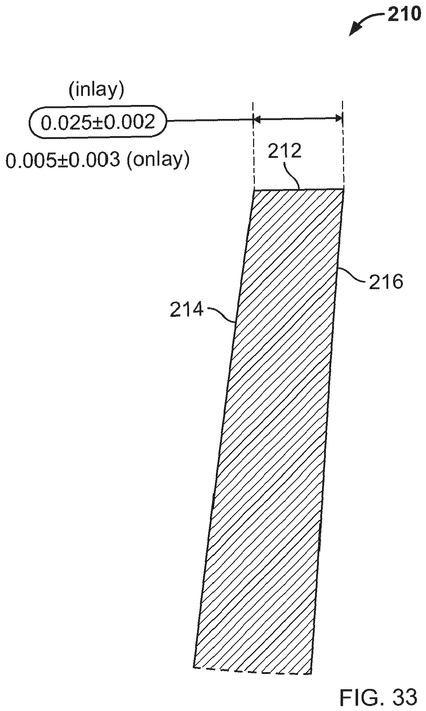

**FIGS. 33A-33E** illustrate the guideline haze grading schemes for corneal haze scoring to be used during testing of the inlays. The best clinical judgment to grade the level of haze based on slit lamp presentation and associated clinical findings will be used.

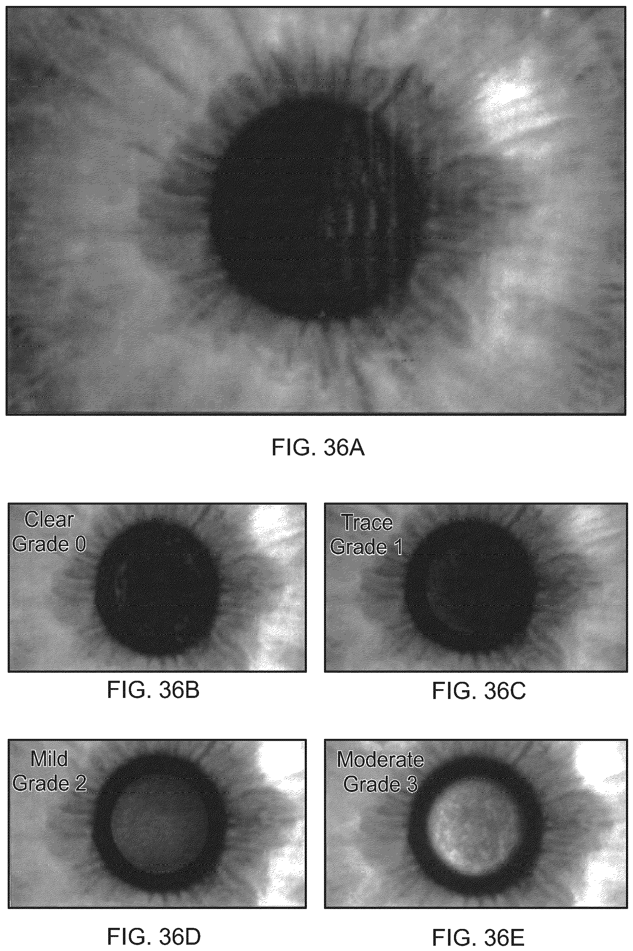

**FIGS. 36A and 36B** show a clear, Grade 0 corneal haze score, **FIG. 36C** shows a trace, Grade 1 corneal haze score, **FIG. 36D** shows a mild, Grade 2 corneal haze score, and **FIG. 36E** shows a moderate, Grade 3 corneal haze score.

With respect to **FIG. 36B,** the clear, Grade 0 score reflects a sporadic, peripheral faint haze (clear center), not visible with diffuse slit lamp beam, minimally visible by oblique or slit beam. Vision is not affected.

With respect to **FIG. 36C,** the trace haze, Grade 1 score reflects a trace haze covering mid-peripheral and center of inlay. Visible with difficulty using diffuse illumination, visible by broad tangential illumination. May present with myoptic shift, reduced near point, visual symptoms (e.g., glare and halos).

With respect to **FIG. 36D,** the mild haze, Grade 2 score reflects a relatively dense, granular confluent haze, covering the entire inlay and outer periphery. The haze is easily seen by diffuse and slit beam illumination. The haze presents with myoptic shift, greatly reduced near point, and a marked increase in visual symptoms (e.g., glare and halos).

With respect to **FIG. 36E,** the moderate haze, Grade 3 score reflects a dense. Reticular, confluent haze presentation. Iris details are obscured with more severe symptoms of Grade 2 possible.

**Appendix A. Modified McDonald-Shadduck Scoring System**

(T. McDonald and J. A. Shadduck, "Eye irritation," in Advances in Modern Toxicology: Dermatoxicology, F. Marzulli and H. I. Maibach, Eds., pp. 579-582, Hemisphere Publishing Corporation, Washington, DC, USA, 1977)

**Examination:**

Use the slit lamp to observe the following:

- Pupillary Response
- Conjunctival Discharge
- Conjunctival Congestion
- Conjunctival Swelling
- Cornea
- Surface Area of Cornea Involvement
- Pannus
- Aqueous Flare
- Aqueous Cell
- Iris Involvement
- Lens

Use the Indirect Ophthalmoscope for the following:

- Vitreous Flare
- Vitreous Cell
- Vitreal Hemorrhage
- Retinal Detachment
- Retinal Hemorrhage
- Choroidal/Retinal Inflammation

Prepare animal for observation by using one of three solutions to dilate the pupil. Usually two drops of ophthalmic preparations of atropine, tropicamide, or phenylephrine is sufficient. The choice of dilator will generally be outlined in the study protocol. Wait until pupil of animal appears to be dilated. It may take up to 60 minutes to achieve pupil dilation.

**Pupillary Response:** Check for any blockage or a sluggish response in the pupillary region. Scoring will be taken as follows:

0= Normal pupil response.

1= Sluggish or incomplete pupil response.

2= No pupil response.

3= No pupil response due to pharmacological blockage.

**Conjunctival Discharge:** Discharge is defined as a whitish gray precipitate from the eye. Scoring will be taken as follows:
0= Normal. No discharge.

1= Discharge above normal and present on the inner portion of the eye but not on the lids or hairs of the eyelids.

2= Discharge is abundant, easily observed and has collected on the lids and hairs of the eyelids.

3= Discharge has been flowing over the eyelids so as to wet the hairs substantially on the skin around the eye.

**Conjunctival Congestion:** Congestion causes the blood vessels of the eye to become enlarged. Scoring will be taken as follows:
0= Normal. May appear blanched to reddish pink without perilimbal injection (except at the 12:00 and 6:00 positions) with vessels of the palpebral and bulbar conjunctiva easily observed.

1= A flushed, reddish color predominantly confined to the palpebral conjunctiva with some perilimbal injection but primarily confined to the lower and upper parts of the eye from the 4:00 to 7:00 and 11:00 to 1:00 positions.

2= Bright red color of the palpebral conjunctiva with accompanying perilimbal injection covering at least 75% of the circumference of the perilimbal region.

3= Dark, beefy red color with congestion of both the bulbar and palpebral conjunctiva along with pronounced perilimbal injection and the presence of petechia on the conjunctiva. The petechia generally predominates along the nictitating membrane and upper palpebral conjunctiva.

**Conjunctival Swelling** (meaning swelling of the conjunctiva). Scoring will be taken as follows:

0= Normal or no swelling of the conjunctival tissue

1= Swelling above normal without eversion of the eyelids (easily discerned by noting upper and lower eyelids are positioned as in the normal eye); swelling generally starts in the lower cul-de-sac near the inner canthus.

2= Swelling with misalignment of the normal approximation of the lower and upper eyelids; primarily confined to the upper eyelid so that in the initial stages, the misapproximation of the eyelids begins by partial eversion of the upper eyelid. In this stage the swelling is confined generally to the upper eyelid with some swelling in the lower cul-de-sac.

3= Swelling definite with partial eversion of the upper and lower eyelids essentially equivalent. This can be easily observed by looking at the animal head-on and noting the position of the eyelids; if the eye margins do not meet, eversion has occurred.

4= Eversion of the upper eyelid is pronounced with less pronounced eversion of the lower eyelid. It is difficult to retract the lids and observe the perilimbal region.

**Cornea:** Check the Cornea for any abnormalities. Scoring will be taken as follows:
0= Normal Cornea

1= Some loss of transparency. Only the epithelium and/or the anterior half of the stroma are involved. The underlying structures are clearly visible although some cloudiness may be readily apparent.

2= Involvement of the entire thickness of the stroma. With diffuse illumination, the underlying structures are just barely visible (can still observe flare, iris, pupil response, and lens).

3= Involvement of the entire thickness of the stroma. With diffuse illumination, the underlying structures cannot be seen.

**Surface Area of Cornea Involvement:** Check the eye for cloudiness in the stromal region. Scoring will be taken as follows:

0= Normal

1= 1-25% area of stromal cloudiness.

2= 26-50% area of stromal cloudiness.

3= 51-75% area of stromal cloudiness.

4= 76%-100% area of stromal cloudiness.

**Pannus:** Check for vascularization of Cornea. Scoring will be taken as follows:

0= No pannus (vascularization of the cornea)

1= Vascularization present but vessels have not invaded the entire cornea circumference.

2= Vessels have invaded 2 mm or more around entire corneal surface.

**Aqueous Flare:** Breakdown of the blood-aqueous barrier. Field size is a 1 mm x 1 mm slit beam. Scoring will be taken as follows (based on Jabs DA et al., 2005):

0=None

1= Faint

2= Moderate (iris and lens details clear)

3= Marked (iris and lens details hazy)

4= Intense (fibrin or plastic aqueous)

**Aqueous Cell:** Cellular observation in the aqueous humor. Field size is a 1 mm x 1 mm slit beam. Scoring will be taken as follows (based on Jabs DA et al., 2005):

0=None

0.5= Trace (1-5)

1=6-15

2=16-25

3=26-50

4=>50

**Iris Involvement:** Check the iris for hyperemia of the blood vessels. Scoring will be taken as follows:

0= Normal iris without any hyperemia of the blood vessels.

1= Minimal injection of the secondary vessels but not tertiary vessels. Generally uniform but may be of greater intensity at the 12:00 to 1:00 or 6:00 position. If confined to this area, the tertiary vessels must be substantially hyperemic.

2= Minimal injection of tertiary vessels and minimal to moderate injection of the secondary vessels.

3= Moderate injection of the secondary and tertiary vessels with slight swelling of the iris stroma (the iris surface appears slightly rugose, usually most predominant near the 3:00 and 9:00 positions).

4=Marked injection of the secondary and tertiary vessels with marked swelling of the iris stroma. The iris appears rugose; may be accompanied by hemorrhage (hyphema) in the anterior chamber.

**Lens:** Observe the lens for any cataracts. Scoring will be taken as follows:

0= Lens clear.

1= Anterior (cortical/capsular).

2= Nuclear.

3= Posterior (cortical/optical).

4= Equatorial.

**Vitreous Flare:** Opacity or fogginess of the vitreous humor. Scoring will be taken as follows (based on Opremcak EM, 2012):

0=None (nerve fiber layer [NFL] clearly visible)

1=Faint (optic nerve and vessels clear, NFL hazy)

2=Moderate (optic nerve and vessels hazy)

3=Marked (optic nerve only visible)

4=Intense (no optic nerve visible)

**Vitreous Cell:** Cellular observation in the vitreous humor. Scoring will be taken as follows (based on Opremcak EM, 2012):

0= Trace (0-10)

1=11-20

2=21-30

3=31-100

4=>100

**Vitreal Hemorrhage:** Observe the vitreous for any hemorrhage. Scoring will be taken as follows:

0=None

1=1-25%

2=26-50%

3=51-75%

4=76-100%

**Retinal Detachment:** During a retinal detachment, bleeding from small retinal blood vessels may cloud the interior of the eye, which is normally filled with vitreous fluid. Scoring will be taken as follows:
0=None

1= Rhegmatogenous (retinal detachment occurs when subretinal fluid accumulates in the potential space between the neurosensory retina and the underlying retinal pigment epithelium).

2= Exudative (occurs due to inflammation, injury, or vascular abnormalities that results in fluid accumulating underneath the retina without the presence of a hole, tear, or break).

3= Tractional (occurs when fibrous or fibrovascular tissue, caused by an injury, inflammation, or neovascularization that pulls the sensory retina from the retinal pigment epithelium).

**Retinal Hemorrhage:** Abnormal bleeding of the blood vessels in the retina. Scoring will be taken as follows:
0=None

1=1-25%

2=26-50%

3=51-75%

4=76-100%

**Choroidal/Retinal Inflammation:** Inflammation of the retina and/or choroid. Scoring will be taken as follows:

0 = None

1=Mild

2 = Moderate

3 = Severe

References:

Jabs DA, Nussenblatt RB, Rosenbaum JT, Standardization of Uveitis Nomenclature (SUN) Working Group (2005). Standardization of uveitis nomenclature for reporting clinical data. Results of the First International Workshop. American Journal of Ophthalmology 140(3): 509-516.

Opremcak EM (2012). Uveitis: A Clinical Manual for Ocular Inflammation. New York: Springer Science + Business Media.

**Embodiments - Implant for Treating Ectatic Corneal Disease**

As discussed herein, keratoconus has a prevalence of about 1.39 per 1,000 people worldwide [Hashemi, H. et al. The Prevalence and Risk Factors for Keratoconus: A Systematic Review and Meta-Analysis. Cornea. (2020) Feb;39(2):263-270; doi: 10.1097/ICO.0000000000002150. PMID: 31498247], about 0.15% in the U.S. (approximately 495,000) [Munir, S.Z. et al. Estimated Prevalence of Keratoconus in the United States From a Large Vision Insurance Database. Eye & Contact Lens. (2021 July) DOI: 10.1097/icl.0000000000000812], about $2.3 \pm 0.2\%$ in rural India [Jonas, J.B. et al. Prevalence and associations of keratoconus in rural maharashtra in central India: the central India eye and medical study. Am J Ophthalmol. (2009 November) 148(5):760-5. doi: 10.1016/j.ajo.2009.06.024. Epub 2009 Aug 11.

PMID: 19674732], and about 0.9±0.2% in northern China [Xu, L. et al. Prevalence and associations of steep cornea/keratoconus in Greater Beijing. The Beijing Eye Study. PLoS ONE 7, e39313. https://doi.org/10.1371/journal.pone.0039313 (2012)]. These figures do not include corneal ectatic diseases resulting from refractive surgeries, such as LASIK, which is the world's most popular elective procedure, with more than 28 million LASIK procedures having been performed worldwide. Annie Stuart, A look at LASIK Past, Present and Future. [EyeNet Magazine July 2021, article from June 2009. https://www.aao.org/eyenet/article/look-at-lasik-past-present-future]. Current treatments include corneal transplants, corneal cross-linking, eyeglasses, hard contact lenses, and intacs.

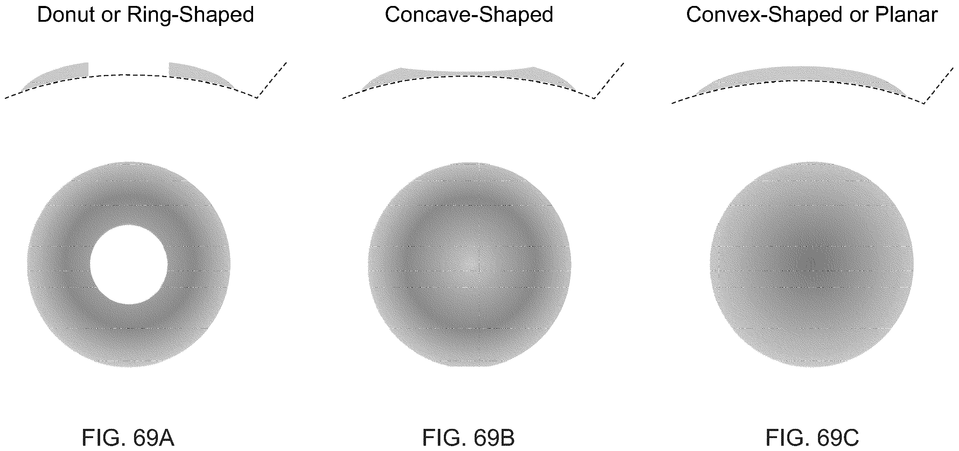

The exemplary hydrogel corneal implant can be used to treat corneal ectatic diseases, including keratoconus and corneal ectasias after LASIK, small incision lenticule extraction (SMILE), and photorefractive keratectomy (PRK). **FIG. 69A, 69B and 69C** are diagrammatic views of the corneal implant lenticule configurations for treatment of corneal ectatic diseases, including a donut or ring-shaped configuration (**FIG. 69A**), a concave-shaped or crater-shaped configuration (**FIG. 69B**), and a convex-shaped, meniscus-shaped (e.g., 120 $\mu$m) or planar configuration (**FIG. 69C**).

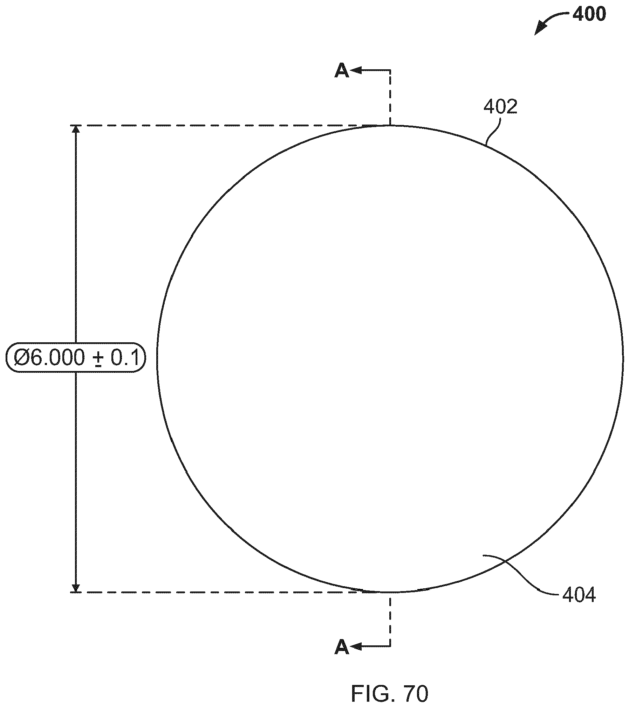

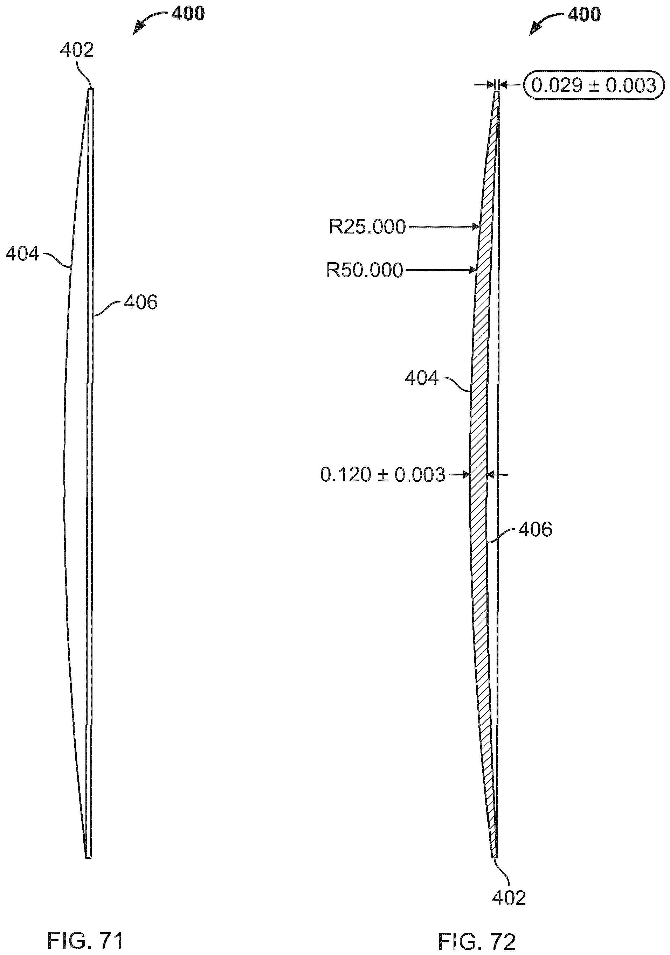

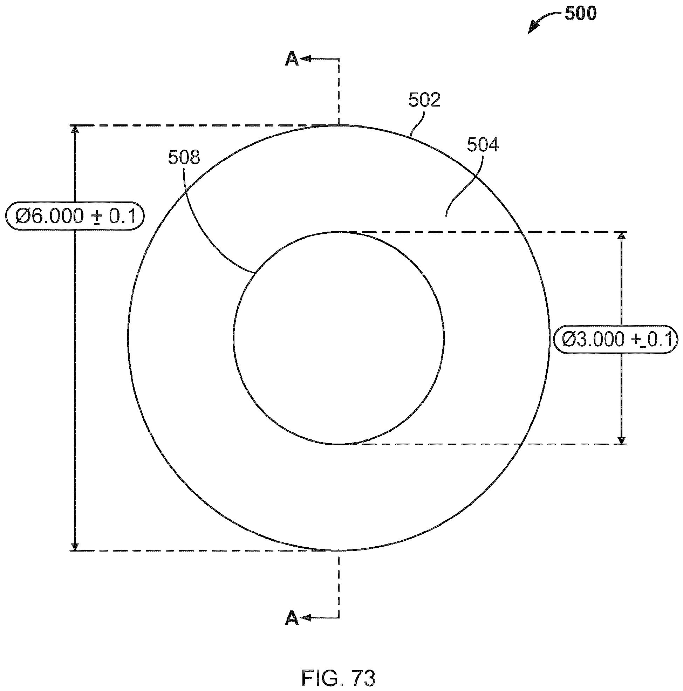

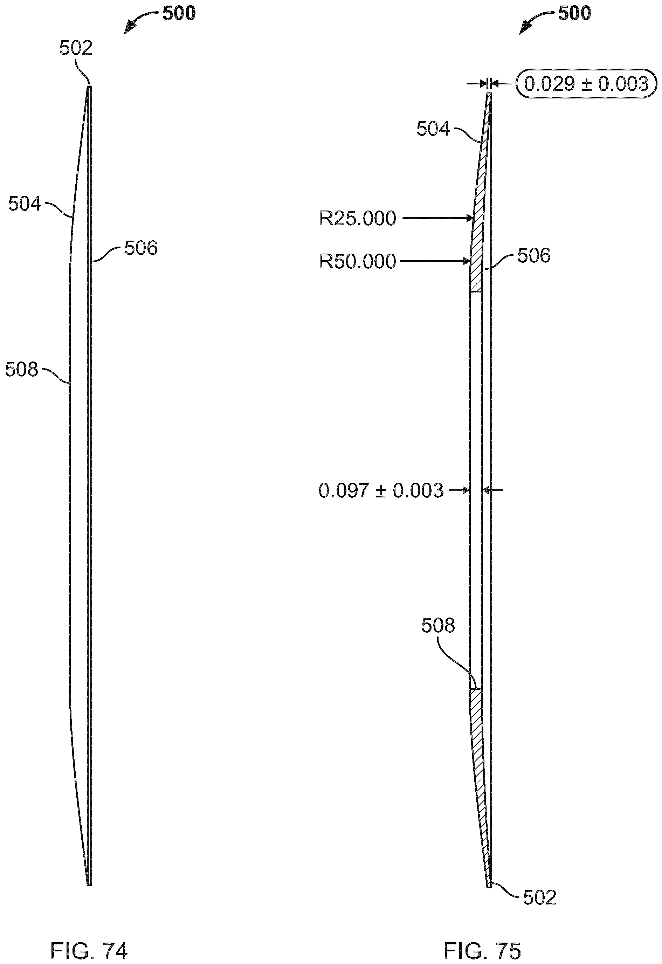

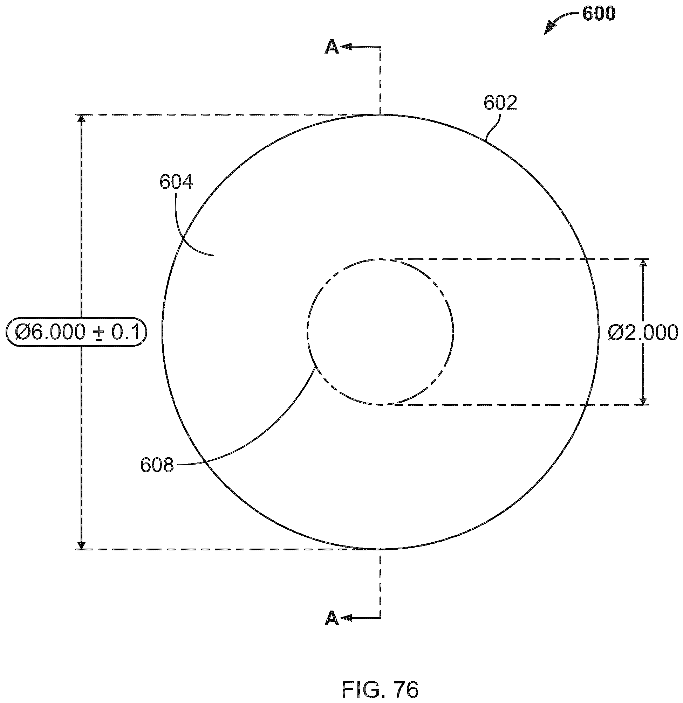

Substantially similar mold assemblies to those discussed herein can be used for formation of the exemplary hydrogel corneal implant for treatment of corneal ectatic diseases. **FIGS. 70-72** are top, side and cross-sectional views of an corneal implant device 400 having a concave shape, **FIGS. 73-75** are top, side and cross-sectional views of an corneal implant device 500 having a donut or ring shape, and **FIGS. 76-78** are top, side and cross-sectional views of an corneal implant device 600 having a convex shape.

With reference to **FIGS. 70-72,** the device 400 can define a substantially circular shape of a diameter of about, e.g., 5.5-9 mm inclusive, 5.5-8.9 mm inclusive, 5.5-8.8 mm inclusive, 5.5-8.7 mm inclusive, 5.5-8.6 mm inclusive, 5.5-8.5 mm inclusive, 5.5-8.4 mm inclusive, 5.5-5.8 mm inclusive, 5.5-8.3 mm inclusive, 5.5-8.2 mm, 5.5-8.1 mm, 5.5-8.0 mm; 5.5-7.9 mm; 5.5-7.8 mm; 5.5-7.7 mm; 5.5-7.6 mm; 5.5-5.5 mm; 5.5-7.4 mm; 5.5-7.3 mm; 5.5-7.2 mm; 5.5-7.1 mm; 5.5-7.0 mm; 5.5-6.9 mm; 5.5-6.8 mm; 5.5-6.7 mm; 5.5-6.4 mm; 5.5-6.3 mm; 5.5-6.2 mm; 5.5-6.1 mm; 5.5-6.0 mm; 5.5-5.9 mm; 5.5- 5.8 mm; 5.5-5.7 mm; 5.5-5.6 mm, inclusive, about 5.5 mm, about 5.6 mm, about 5.7 mm, about 5.8 mm, about 5.9 mm, about 6.0 mm, about 6.1 mm, about 6.2 mm, about 6.3 mm, about 6.4 mm, about 6.5 mm, about 6.6 mm, about 6.7 mm, about 6.8 mm, about 6.9 mm, about 7.0 mm, about 7.1 mm, about 7.2 mm, about 7.3 mm, about 7.4 mm, about 7.5 mm, about 7.6 mm, about 7.7 mm, about 7.8 mm, about 7.9 mm, about 8.0 mm, about 8.1 mm, about 8.2 mm, about 8.3 mm, about 8.4 mm, about 8.5 mm, about 8.6 mm, about 8.7 mm, about 8.8 mm, about 8.9 mm, or about 9 mm or the like. The device 400 can define an edge 402 thickness of about, e.g., 0.023-0.033 mm inclusive, 0.023-0.032 mm inclusive, 0.023-0.031 mm inclusive, 0.023-0.030 mm inclusive, 0.023-0.029 mm inclusive, 0.023-0.028 mm inclusive, 0.023-0.027 mm inclusive, 0.023-0.026 mm inclusive, 0.023-0.025 mm inclusive, 0.023-0.024 mm inclusive, 0.024-0.032 mm inclusive, 0.025-0.032 mm inclusive, 0.026-0.032 mm inclusive, 0.027-0.032 mm inclusive, 0.028-0.032 mm inclusive, 0.029-0.032 mm inclusive, 0.030-0.032 mm inclusive, 0.031-0.032 mm inclusive, 0.023 mm, 0.024 mm, 0.025 mm, 0.026 mm, 0.027 mm, 0.028 mm, 0.029 mm, 0.030 mm, 0.031 mm, 0.032, 0.033 mm, or the like. The device 400 can have a center thickness of about, e.g., 0.115-0.290 mm inclusive, 0.115-0.289 mm inclusive, 0.115-0.288 mm inclusive, 0.115-0.287 mm inclusive, 0.115-0.286 mm inclusive, 0.115-0.285 mm; 0.115-284 mm; 0.115-283 mm; 0.115-282 mm; 0.115-281 mm; 0.115-281 mm; 0.115-0.280 mm; 0.115-0.279 mm; 0.115-0.278 mm; 0.115-0.277 mm; 0.115-0.276 mm; 0.115-0.275 mm; 0.115-0.275 mm; 0.115-0.273 mm; 0.115-0.272 mm; 0.115-0.271 mm; 0.115-0.270 mm; 0.115-0.269 mm; 0.115-0.268 mm; 0.115-0.267 mm; 0.115-0.266 mm; 0.115-0.265 mm; 0.115-0.264 mm; 0.115-0.263 mm; 0.115-0.262 mm; 0.115-0.261 mm; 0.115-0.260 mm; 0.115-0.269 mm; 0.115-0.268 mm; 0.115-0.267 mm; 0.115-0.266 mm; 0.115-0.265 mm; 0.115-0.264 mm; 0.115-0.263 mm; 0.115-0.262 mm; 0.115-0.261 mm; 0.115-0.260 mm 0.115-0.259 mm; 0.115-0.258 mm; 0.115-0.257 mm; 0.115-0.256 mm; 0.115-0.255 mm; 0.115-0.254 mm; 0.115-0.253 mm; 0.115-0.252 mm; 0.115-0.251 mm; 0.115-0.250 mm 0.115-0.249 mm; 0.115-0.248 mm; 0.115-0.247 mm; 0.115-0.246 mm; 0.115-0.245 mm; 0.115-0.244 mm; 0.115-0.243 mm; 0.115-0.242 mm; 0.115-0.241 mm; 0.115-0.240 mm 0.115-0.239 mm; 0.115-0.238 mm; 0.115-0.237 mm; 0.115-0.236 mm; 0.115-0.235 mm; 0.115-0.234 mm; 0.115-0.233 mm; 0.115-0.232 mm; 0.115-0.231 mm; 0.115-0.230 mm 0.115-0.229 mm; 0.115-0.228 mm; 0.115-0.227 mm; 0.115-0.226 mm; 0.115-0.225 mm; 0.115-0.224 mm; 0.115-0.223 mm; 0.115-0.222 mm; 0.115-0.221 mm; 0.115-0.220 mm 0.115-0.219 mm; 0.115-0.218 mm; 0.115-0.217 mm; 0.115-0.216 mm; 0.115-0.215 mm; 0.115-0.214 mm; 0.115-0.213 mm; 0.115-0.212 mm; 0.115-0.211 mm; 0.115-0.210 mm 0.115-0.209 mm; 0.115-0.208 mm; 0.115-0.207 mm; 0.115-0.206 mm; 0.115-0.205 mm; 0.115-0.204 mm; 0.115-0.203 mm; 0.115-0.202 mm; 0.115-0.201 mm; 0.115-0.200 mm 0.115-0.199 mm; 0.115-0.198 mm; 0.115-0.197 mm; 0.115-0.196 mm; 0.115-0.195 mm; 0.115-0.194 mm; 0.115-0.193 mm; 0.115-0.192 mm; 0.115-0.191 mm; 0.115-0.190 mm 0.115-0.189 mm; 0.115-0.188 mm; 0.115-0.187 mm; 0.115-0.186 mm; 0.115-0.185 mm; 0.115-0.184 mm; 0.115-0.183 mm; 0.115-0.182 mm; 0.115-0.181 mm; 0.115-0.180 mm 0.115-0.179 mm; 0.115-0. 178 mm; 0.115-0. 177 mm; 0.115-0. 176 mm; 0.115-0. 175 mm; 0.115-0. 174 mm; 0.115-0. 173 mm; 0.115-0. 172 mm; 0.115-0. 171 mm; 0.115-0. 170 mm 0.115-0.169 mm; 0.115-0.168 mm; 0.115-0.167 mm; 0.115-0.166 mm; 0.115-0.165 mm; 0.115-0.164 mm; 0.115-0.163 mm; 0.115-0.162 mm; 0.115-0.161 mm; 0.115-0.160 mm 0.115-0.159 mm; 0.115-0. 158 mm; 0.115-0. 157 mm; 0.115-0. 156 mm; 0.115-0. 155 mm; 0.115-0. 154 mm; 0.115-0. 153 mm; 0.115-0. 152 mm; 0.115-0. 151 mm; 0.115-0. 150 mm 0.115-0.149 mm; 0.115-0. 148 mm; 0.115-0. 147 mm; 0.115-0. 146 mm; 0.115-0. 145 mm; 0.115-0. 144 mm; 0.115-0. 143 mm; 0.115-0.

142 mm; 0.115-0. 141 mm; 0.115-0. 140 mm 0.115-0.139 mm; 0.115-0. 138 mm; 0.115-0. 137 mm; 0.115-0. 136 mm; 0.115-0. 135 mm; 0.115-0. 134 mm; 0.115-0.133 mm; 0.115-0. 132 mm; 0.115-0. 131 mm; 0.115-0.120 mm 0.115-0. 129 mm; 0.115-0. 128 mm; 0.115-0. 127 mm; 0.115-0. 126 mm; 0.115-0. 125 mm; 0.115-0. 124 mm; 0.115-0.123 mm; 0.115-0.122 mm; 0.115-0. 121 mm; 0.115-0. 120 mm; 129 mm; 0.115-0. 118 mm; 0.115-0. 117 mm; 0.115-0. 116 inclusive,, 0.115 mm, 0.116 mm, 0.117 mm, 0.118 mm, 0.119 mm, 0.120 mm, 0.121 mm, 0.122 mm, 0.123 mm, 0.124 mm, 0.125 mm, 0.126 mm, 0.127 mm, 0.128 mm; 0.129 mm, 0.130 mm, 0.131 mm; 0.132 mm; 0.133 mm, 0.134 mm, 0.135 mm; 0.136 mm; 0.137 mm; 0.13 mm 8; 0.139 mm; 0.140 mm, 0.141 mm, 0.142 mm, 0.143 mm; 0.144 mm, 0.145 mm, 0.146 mm, 0.147 mm, 0.14 mm 8, 0.149 mm, 0.150, 0.151 mm; 0.152; 0.153 mm; 0.154 mm, 0.155 mm, 0.156 mm, 0.157 mm, 0.158 mm, 0.159 mm, 0.160 mm, 0.161 mm, 0162 mm, 0.163 mm, 0.164 mm, 0.165; mm 0.166 mm, 0.167 mm, 0.168 mm, 0.169 mm, 0.170 mm, 0.171 mm, 0.172, 0.173, 0.174, 0.175 mm, 0.176 mm, 0.17 mm 7, 0.178 mm, 0.179 mm, 0.180 mm, 0.181 mm, 0.182 mm, 0.183 mm, 0.184 mm, 0.185 mm, 0.186 mm, 0.187 mm, 0.188 mm, 0.189 mm, 0.190 mm, 0.191 mm, 0.192 mm, 0.193 mm, 0.194 mm, 0.195 mm, 0.196 mm, 0.197 mm, 0.198 mm, 0.199 mm, or about 200 - 290 $\mu$m inclusive or the like.

The device 400 includes a substantially flat perimeter edge 402, a convex top surface 404, and a concave bottom surface 406. The radius of the top surface 404 can be about, e.g., 20-30 inclusive, 20-29 inclusive, 20-28 inclusive, 20-27 inclusive, 20-26 inclusive, 20-25 inclusive, 20-24 inclusive, 20-23 inclusive, 20-22 inclusive, 20-21 inclusive, 21-30 inclusive, 22-30 inclusive, 23-30 inclusive, 24-30 inclusive, 25-30 inclusive, 26-30 inclusive, 27-30 inclusive, 28-30 inclusive, 29-30 inclusive, 20, 21, 22, 23, 24, 25, 26, 27, 28, 29, 30, or the like. The radius of the bottom surface 406 can be about, e.g., 45-55 inclusive, 45-54 inclusive, 45-53 inclusive, 45-52 inclusive, 45-51 inclusive, 45-50 inclusive, 45-49 inclusive, 45-48 inclusive, 45-47 inclusive, 45-46 inclusive, 46-55 inclusive, 47-55 inclusive, 48-55 inclusive, 49-55 inclusive, 50-55 inclusive, 51-55 inclusive, 52-55 inclusive, 53-55 inclusive, 54-55 inclusive, 45, 46, 47, 48, 49, 50, 51, 52, 53, 54, 55, or the like. The body of the device 400 is solidly formed from the hydrogel (e.g., no openings or hollow cavities), with the device 400 tapering from the thickest area at the central longitudinal axis to the thinnest area at the perimeter edge 402

With reference to **FIGS. 73-75,** the device 500 can define a substantially circular shape of an outer diameter of about, e.g., 5.5-7.5 mm inclusive, 5.5-7.4 mm inclusive, 5.5-7.3 mm inclusive, 5.5-7.2 mm inclusive, 5.5-7.1 mm inclusive, 5.5-7.0 mm 5.5-5.5-6.9 mm inclusive, 5.5-6.8 mm inclusive, 5.5-6.7 mm inclusive, 5.5-6.6 mm; 5.5-6.5 mm inclusive, 5.5-6.5 mm inclusive, 5.5-6.3 mm inclusive, 5.5-6.2 mm inclusive, 5.5-6.1 mm inclusive, 5.5-6.0 mm inclusive, 5.5-5.9 mm inclusive, 5.5-5.8 mm inclusive, 5.5-5.7 mm inclusive, 5.5-5.6 mm inclusive, 5.6-6.5 mm inclusive, 5.7-6.5 mm inclusive, 5.8-6.5 mm inclusive, 5.9-6.5 mm inclusive, 6.0-6.5 mm inclusive, 6.1-6.5 mm inclusive, 6.2-6.5 mm inclusive, 6.3-6.5 mm inclusive, 6.4-6.5 mm inclusive, 5.5 mm, 5.6 mm, 5.7 mm, 5.8 mm, 5.9 mm, 6.0 mm, 6.1 mm, 6.2 mm, 6.3 mm, 6.4 mm, 6.5 mm, or the like. The device 500 can define an outermost perimeter edge 502 thickness of about, e.g., 0.023-0.033 mm inclusive, 0.023-0.032 mm inclusive, 0.023-0.031 mm inclusive, 0.023-0.030 mm inclusive, 0.023-0.029 mm inclusive, 0.023-0.028 mm inclusive, 0.023-0.027 mm inclusive, 0.023-0.026 mm inclusive, 0.023-0.025 mm inclusive, 0.023-0.024 mm inclusive, 0.024-0.032 mm inclusive, 0.025-0.032 mm inclusive, 0.026-0.032 mm inclusive, 0.027-0.032 mm inclusive, 0.028-0.032 mm inclusive, 0.029-0.032 mm inclusive, 0.030-0.032 mm inclusive, 0.031-0.032 mm inclusive, 0.023 mm, 0.024 mm, 0.025 mm, 0.026 mm, 0.027 mm, 0.028 mm, 0.029 mm, 0.030 mm, 0.031 mm, 0.032, 0.033 mm, or the like. The device 500 can includes a substantially flat perimeter edge 502, a convex top surface 504, and a concave bottom surface 506.

In some embodiments, the device 500 can define a donut shape with outer and inner diameters and include a central circular opening 508 extending through the body of the device 500 to create the donut or ring shape of the device 500 with an outer diameter of about, e.g., 5.5-7.5 mm inclusive, 5.5-7.4 mm inclusive, 5.5-7.3 mm inclusive, 5.5-7.2 mm inclusive, 5.5-7.1 mm inclusive, 5.5-7.0 mm 5.5-5.5-6.9 mm inclusive, 5.5-6.8 mm inclusive, 5.5-6.7 mm inclusive, 5.5-6.6 mm; 5.5-6.5 mm inclusive, 5.5-6.5 mm inclusive, 5.5-6.3 mm inclusive, 5.5-6.2 mm inclusive, 5.5-6.1 mm inclusive, 5.5-6.0 mm inclusive, 5.5-5.9 mm inclusive, 5.5-5.8 mm inclusive, 5.5-5.7 mm inclusive, 5.5-5.6 mm inclusive, 5.6-6.5 mm inclusive, 5.7-6.5 mm inclusive, 5.8-6.5 mm inclusive, 5.9-6.5 mm inclusive, 6.0-6.5 mm inclusive, 6.1-6.5 mm inclusive, 6.2-6.5 mm inclusive, 6.3-6.5 mm inclusive, 6.4-6.5 mm inclusive, 5.5 mm, 5.6 mm, 5.7 mm, 5.8 mm, 5.9 mm, 6.0 mm, 6.1 mm, 6.2 mm, 6.3 mm, 6.4 mm, 6.5 mm, or the like, and an inner diameter of 3 to 6.7 mm, 3 to 6.6 mm, 3 to 6.5 mm, 3-6.4 mm, 3-6.3 mm, 3-6.2 mm, 3-6.1 mm, 3-6.0 mm, 3-5.9 mm, 3-5.8 mm, 3-5.7 mm, 3 to 5.6 mm, 3-5.5 mm, 3-5.4 mm, 3-5.3 mm, 3-5.2 mm, 3-5.1 mm, 3.50-mm, 3-4.9 mm, 3-4.8 mm, 3-4.7 mm, 3 to 4.6 mm, 3-4.5 mm, 3-4.4 mm, 3-4.3 mm, 3-4.2 mm, 3-4.1 mm, 3-4.0 mm; 3-3.9 mm, 3-3.8 mm, 3-3.7 mm, 3 to 3.6 mm, 3-3.5 mm, 3-3.4 mm, 3-3.3 mm, 3-3.2 mm, 3-3.1 mm, inclusive

The device 500 can have a center thickness (as measured at the inner edge of the opening 508) of about, e.g., 0.090-0.104 mm inclusive, 0.090-0.103 mm inclusive, 0.090-0.102 mm inclusive, 0.090-0.101 mm inclusive, 0.090-0.100 mm inclusive, 0.090-0.099 mm inclusive, 0.090-0.098 mm inclusive, 0.090-0.097 mm inclusive, 0.090-0.096 mm inclusive, 0.090-0.095 mm inclusive, 0.090-0.094 mm inclusive, 0.090-0.093 mm inclusive, 0.090-0.092 mm inclusive, 0.090-0.091 mm inclusive, 0.091-0.104 mm inclusive, 0.092-0.104 mm inclusive, 0.093-0.104 mm inclusive, 0.094-0.104 mm inclusive, 0.095-0.104 mm inclusive, 0.096-0.104 mm inclusive, 0.097-0.104 mm inclusive, 0.098-0.104 mm inclusive, 0.099-0.104 mm inclusive, 0.100-0.104 mm inclusive, 0.101-0.104 mm inclusive, 0.102-0.104 mm inclusive, 0.103-0.104 mm inclusive, 0.090 mm, 0.091 mm, 0.092 mm, 0.093 mm, 0.094 mm, 0.095 mm, 0.096 mm, 0.097 mm, 0.098 mm, 0.099 mm, 0.100 mm, 0.101 mm, 0.102 mm, 0.103 mm, 0.104 mm, or the like. A diameter of the opening 508 can be about, e.g., 2.5-3.5 mm inclusive, 2.5-3.4 mm inclusive, 2.5-3.3 mm inclusive, 2.5-3.2 mm inclusive, 2.5-3.1 mm inclusive, 2.5-3.0 mm inclusive, 2.5-2.9 mm inclusive, 2.5-2.8 mm inclusive, 2.5-2.7 mm inclusive, 2.5-2.6 mm inclusive, 2.6-3.5 mm inclusive, 2.7-3.5 mm inclusive, 2.8-3.5 mm inclusive, 2.9-3.5 mm inclusive, 3.0-3.5 mm inclusive, 3.1-3.5 mm inclusive, 3.2-3.5 mm inclusive, 3.3-3.5 mm inclusive, 3.4-3.5 mm inclusive, 2.5 mm, 2.6 mm, 2.7 mm, 2.8 mm, 2.9 mm, 3.0 mm, 3.1 mm, 3.2 mm, 3.3 mm, 3.4 mm, 3.5 mm, or the like. The radius of the top surface 504 can be about, e.g., 20-30 inclusive, 20-29 inclusive, 20-28 inclusive, 20-27 inclusive, 20-26 inclusive, 20-25 inclusive, 20-24 inclusive, 20-23 inclusive, 20-22 inclusive, 20-21 inclusive, 21-30 inclusive, 22-30 inclusive, 23-30 inclusive, 24-30 inclusive, 25-30 inclusive, 26-30 inclusive, 27-30 inclusive, 28-30 inclusive, 29-30 inclusive, 20, 21, 22, 23, 24, 25, 26, 27, 28, 29, 30, or the like. The radius of the bottom surface 506 can be about, e.g., 45-55 inclusive, 45-54 inclusive, 45-53 inclusive, 45-52 inclusive, 45-51 inclusive, 45-50 inclusive, 45-49 inclusive, 45-48 inclusive, 45-47 inclusive, 45-46 inclusive, 46-55 inclusive, 47-55 inclusive, 48-55 inclusive, 49-55 inclusive, 50-55 inclusive, 51-55 inclusive, 52-55 inclusive, 53-55 inclusive, 54-55 inclusive, 45, 46, 47, 48, 49, 50, 51, 52, 53, 54, 55, or the like. The body of the device 500 around the opening 508 is solidly formed from the hydrogel (e.g., no openings or hollow cavities), with the device 500 tapering from the thickest area at the inner perimeter edge defining the opening 508 to the thinnest area at the perimeter edge 502.

With reference to **FIGS. 76-78,** the device 600 can define a substantially circular shape of a diameter of about, e.g., 5.5-6.5 mm inclusive, 5.5-6.4 mm inclusive, 5.5-6.3 mm inclusive, 5.5-6.2 mm inclusive, 5.5-6.1 mm inclusive, 5.5-6.0 mm inclusive, 5.5-5.9 mm inclusive, 5.5-5.8 mm inclusive, 5.5-5.7 mm inclusive, 5.5-5.6 mm inclusive, 5.6-6.5 mm inclusive, 5.7-6.5 mm inclusive, 5.8-6.5 mm inclusive, 5.9-6.5 mm inclusive, 6.0-6.5 mm inclusive, 6.1-6.5 mm inclusive, 6.2-6.5 mm inclusive, 6.3-6.5 mm inclusive, 6.4-6.5 mm inclusive, 5.5 mm, 5.6 mm, 5.7 mm, 5.8 mm, 5.9 mm, 6.0 mm, 6.1 mm, 6.2 mm, 6.3 mm, 6.4 mm, 6.5 mm, or the like. The device 600 can define an edge 602 thickness of about, e.g., 0.023-0.033 mm inclusive, 0.023-0.032 mm inclusive, 0.023-0.031 mm inclusive, 0.023-0.030 mm inclusive, 0.023-0.029 mm inclusive, 0.023-0.028 mm inclusive, 0.023-0.027 mm inclusive, 0.023-0.026 mm inclusive, 0.023-0.025 mm inclusive, 0.023-0.024 mm inclusive, 0.024-0.032 mm inclusive, 0.025-0.032 mm inclusive, 0.026-0.032 mm inclusive, 0.027-0.032 mm inclusive, 0.028-0.032 mm inclusive, 0.029-0.032 mm inclusive, 0.030-0.032 mm inclusive, 0.031-0.032 mm inclusive, 0.023 mm, 0.024 mm, 0.025 mm, 0.026 mm, 0.027 mm, 0.028 mm, 0.029 mm, 0.030 mm, 0.031 mm, 0.032, 0.033 mm, or the like. In some embodiments, the device 400 can have a center thickness ranging from 20 μm (peripheral thickness 116 μm) to about 350 μm (peripheral thickness 400 μm), inclusive. In some embodiments, the center thickness is about, e.g., 0.015-0.025 mm inclusive, 0.015-0.024 mm inclusive, 0.015-0.023 mm inclusive, 0.015-0.022 mm inclusive, 0.015-0.021 mm inclusive, 0.015-0.020 mm inclusive, 0.015-0.019 mm inclusive, 0.015-0.018 mm inclusive, 0.015-0.017 mm inclusive, 0.015-0.016 mm inclusive, 0.016-0.025 mm inclusive, 0.017-0.025 mm inclusive, 0.018-0.025 mm inclusive, 0.019-0.025 mm inclusive, 0.020-0.025 mm inclusive, 0.021-0.025 mm inclusive, 0.022-0.025 mm inclusive, 0.023-0.025 mm inclusive, 0.024-0.025 mm inclusive, 0.015 mm, 0.016 mm, 0.017 mm, 0.018 mm, 0.019 mm, 0.020 mm, 0.021 mm, 0.022 mm, 0.023 mm, 0.024 mm, 0.025 mm, or the like.

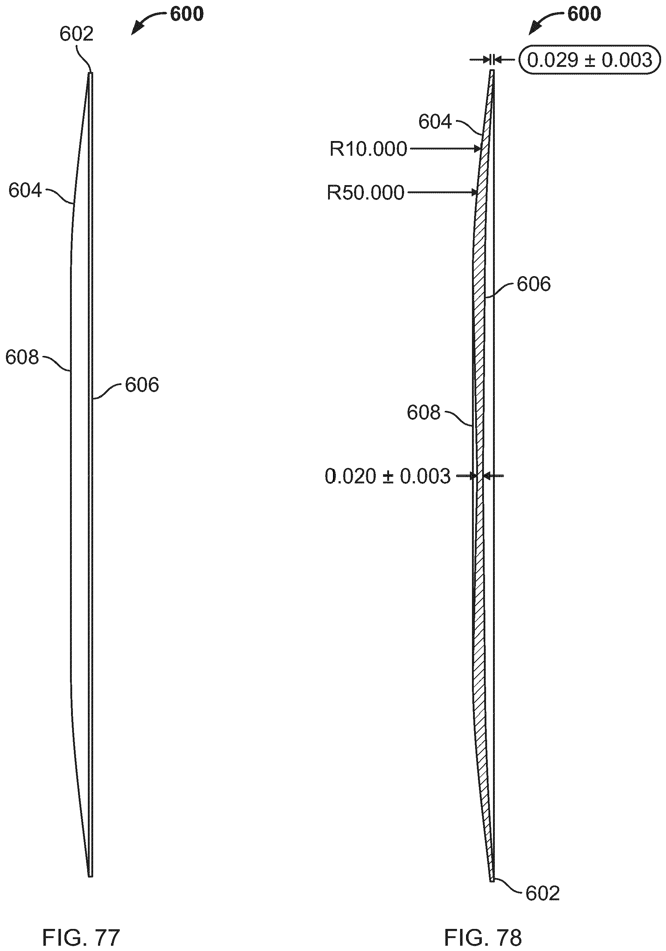

The device 600 includes a substantially flat perimeter edge 602, a convex top surface 604, and a concave bottom surface 606. The top surface 604 includes a central convex area 608 that gradually tapers or angles inwardly to define a thinner central section of the device 600. In the side view of **FIG. 77,** the convex area 608 can appear substantially planar, and from the cross-sectional view of **FIG. 78,** the convex area 608 can appear convex. The diameter of the convex area 608 can be about, e.g., 1.5-2.5 mm inclusive, 1.5-2.4 mm inclusive, 1.5-2.3 mm inclusive, 1.5-2.2 mm inclusive, 1.5-2.1 mm inclusive, 1.5-2.0 mm inclusive, 1.5-1.9 mm inclusive, 1.5-1.8 mm inclusive, 1.5-1.7 mm inclusive, 1.5-1.6 mm inclusive, 1.6-2.5 mm inclusive, 1.7-2.5 mm inclusive, 1.8 -2.5 mm inclusive, 1.9-2.5 mm inclusive, 2.0-2.5 mm inclusive, 2.1-2.5 mm inclusive, 2.2-2.5 mm inclusive, 2.3-2.5 mm inclusive, 2.4-2.5 mm inclusive, 1.5 mm, 1.6 mm, 1.7 mm, 1.8 mm, 1.9 mm, 2.0 mm, 2.1 mm, 2.2 mm, 2.3 mm, 2.4 mm, 2.5 mm, or the like. The radius of the top surface 604 can be about, e.g., 5-15 inclusive, 5-14 inclusive, 5-13 inclusive, 5-12 inclusive, 5-11 inclusive, 5-10 inclusive, 5-9 inclusive, 5-8 inclusive, 5-7 inclusive, 5-6 inclusive, 6-15 inclusive, 7-15 inclusive, 8-15 inclusive, 9-15 inclusive, 10-15 inclusive, 11-15 inclusive, 12-15 inclusive, 13-15 inclusive, 14-15 inclusive, 5, 6, 7, 8, 9, 10, 11, 12, 13, 14, 15, or the like. The radius of the bottom surface 606 can be about, e.g., 45-55 inclusive, 45-54 inclusive, 45-53 inclusive, 45-52 inclusive, 45-51 inclusive, 45-50 inclusive, 45-49 inclusive, 45-48 inclusive, 45-47 inclusive, 45-46 inclusive, 46-55 inclusive, 47-55 inclusive, 48-55 inclusive, 49-55 inclusive, 50-55 inclusive, 51-55 inclusive, 52-55 inclusive, 53-55 inclusive, 54-55 inclusive, 45, 46, 47, 48, 49, 50, 51, 52, 53, 54, 55, or the like. The body of the device 600 is solidly formed from the hydrogel (e.g., no openings or hollow cavities), with the device 600 tapering from the thinnest area at the central longitudinal axis of the convex area 608 to the thickest area at the concave top surface 604, and tapering again to a thin perimeter edge 602

In some embodiments, the corneal implant devices discussed herein can be implanted in the cornea at a depth of about, e.g., 0.100-0.250 mm inclusive, 0.100-0.240 mm inclusive, 0.100-0.230 mm inclusive, 0.100-0.220 mm inclusive, 0.100-0.210 mm inclusive, 0.100-0.200 mm inclusive, 0.100-0.190 mm inclusive, 0.100-0.180 mm inclusive, 0.100-0.170 mm inclusive, 0.100-0.160 mm inclusive, 0.100-0.150 mm inclusive, 0.100-0.140 mm inclusive, 0.100-0.130 mm inclusive, 0.100-0.120 mm inclusive, 0.100-0.110 mm inclusive, 0.110-0.250 mm inclusive, 0.120-0.250 mm inclusive, 0.130-0.250 mm inclusive, 0.140-0.150 mm inclusive, 0.160-0.250 mm inclusive, 0.170-0.250 mm inclusive, 0.180-0.250 mm inclusive, 0.190 -0.250 mm inclusive, 0.200-0.250 mm inclusive, 0.210-0.250 mm inclusive, 0.220-0.250 mm inclusive, 0.230-0.250 mm inclusive, 0.240-0.250 mm inclusive, 0.100 mm, 0.110 mm, 0.120 mm, 0.130 mm, 0.140 mm, 0.150 mm, 0.160 mm, 0.170 mm, 0.180 mm, 0.190 mm, 0.200 mm, 0.210 mm, 0.220 mm, 0.230 mm, 0.240 mm, 0.250 mm, or the like.

In some embodiments, the corneal implant devices discussed herein can have a thickness of about, e.g., 0.0115-0.290 mm inclusive, 0.0155-0.280 mm inclusive, 0.0155-0.270 mm inclusive, 0.0155-0.260 mm inclusive, 0.0155-0.250 mm inclusive, 0.0155-0.240 mm inclusive, 0.0155-0.230 mm inclusive, 0.0155-0.220 mm inclusive, 0.0155-0.210 mm inclusive, 0.0155-0.200 mm inclusive, 0.0155-0.190 mm inclusive, 0.0155-0.180 mm inclusive, 0.0155-0.170 mm inclusive, 0.0155-0.160 mm inclusive, 0.0155-0.150 mm inclusive, 0.0155-0.140 mm inclusive, 0.0155-0.130 mm inclusive, 0.0155-0.120 mm inclusive, 0.0155-0.110 mm inclusive, 0.0155-0.100 mm inclusive, 0.0155-0.090 mm inclusive, 0.0155-0.080 mm inclusive, 0.0155-0.070 mm inclusive, 0.0155-0.060 mm inclusive, 0.0155-0.050 mm inclusive, 0.0155-0.040 mm inclusive, 0.0155-0.030 mm inclusive, 0.0155-0.020 mm inclusive, 0.020-0.290 mm inclusive, 0.030-0.290 mm inclusive, 0.040-0.290 mm inclusive, 0.050-0.290 mm inclusive, 0.060-0.290 mm inclusive, 0.070-0.290 mm inclusive, 0.080-0.290 mm inclusive, 0.090-0.290 mm inclusive, 0.100-0.290 mm inclusive, 0.110-0.290 mm inclusive, 0.120-0.290 mm inclusive, 0.130-0.290 mm inclusive, 0.140-0.290 mm inclusive, 0.150-0.290 mm inclusive, 0.160-0.290 mm inclusive, 0.170-0.290 mm inclusive, 0.180-0.290 mm inclusive, 0.190-0.290 mm inclusive, 0.200-0.290 mm inclusive, 0.210-0.290 mm inclusive, 0.220-0.290 mm inclusive, 0.230-0.290 mm inclusive, 0.240-0.290 mm inclusive, 0.250-0.290 mm inclusive, 0.260-0.290 mm inclusive, 0.270-0.290 mm inclusive, 0.280-0.290 mm inclusive, 0.0155 mm, 0.020 mm, 0.030 mm, 0.040 mm, 0.050 mm, 0.060 mm, 0.070 mm, 0.080 mm, 0.090 mm, 0.100 mm, 0.110 mm, 0.120 mm, 0.130 mm, 0.140 mm, 0.0150 mm, 0.160 mm, 0.170 mm, 0.180 mm, 0.190 mm, 0.200 mm, 0.210 mm, 0.220 mm, 0.230 mm, 0.240 mm, 0.250 mm, 0.260 mm, 0.270 mm, 0.280 mm, 0.290 mm, or the like.

The devices discussed herein can be used as an implant to correct corneal damage caused by ectatic corneal disease. According to some embodiments, the device (e.g., the inlay) can be implanted to support the corneal structure weakened by the ectatic corneal disease. According to some embodiments, the corneal implant device can be implanted to change the anterior curvature of the cornea to correct vision of the subject with ectatic corneal disease. As previously noted, ectatic corneal disease can result in asymmetric corneal complications, with changes to the right and left eyes being different. The devices discussed herein can therefore be initially formed in a mold, and subsequently modified to accommodate the specific corneal topography on a patient-specific basis. As an example, a femtosecond laser can be used to modify the device after molding prior to insertion into a pocket or flap of a cornea of the recipient, or prior to positioning of the onlay device over the cornea of the recipient.

While the present invention has been described with reference to the specific embodiments thereof it should be understood by those skilled in the art that various changes may be made and equivalents may be substituted without departing from the true spirit and scope of the invention. In addition, many modifications may be made to adopt a particular situation, material, composition of matter, process, process step or steps, to the objective spirit and scope of the present invention. All such modifications are intended to be within the scope of the claims appended hereto.

**Claims**

1. A method of treating ectatic corneal disease, comprising:

   implanting in or on a cornea of a mammalian subject a corneal device of water content of between 78%-92%% (w/w), the corneal device comprising a thickness, a diameter, a base surface and a top surface,
   wherein the corneal device is effective to support a corneal structure weakened by the ectatic corneal disease, and to improve vision of an eye of the mammalian subject.

2. The method of claim 1, wherein the corneal device is a corneal inlay device implanted in the cornea.

3. The method of claim 1, wherein the corneal device is implanted on an anterior surface of the cornea.

4. The method of claim 1, wherein the base surface defines a convex configuration.

5. The method of claim 1, wherein the top surface defines a concave configuration.

6. The method of claim 1, wherein the top surface of the corneal device extends along an entire central area of the corneal device.

7. The method of claim 1, wherein the corneal device comprises an opening extending through the corneal device to define a ring or donut shape.

8. The method of claim 1, wherein the corneal device comprises a convex section in the top surface.

9. The method of claim 1, wherein the implanting of the corneal device is by cutting a flap in the cornea and positioning the corneal device beneath the flap.

10. The method of claim 1, wherein the implanting of the corneal device is by positioning the corneal device within a pocket formed in the cornea.

11. The method of claim 1, wherein the implanting of the corneal device is in the cornea at a depth of about 100 microns to about 250 microns, inclusive.

12. The method of claim 1, wherein the implanting of the corneal device is in the cornea at a depth of about 130 microns to about 160 microns, inclusive.

13. The method of claim 1, wherein the thickness of the corneal device ranges from at least 11.5 microns to about 290 microns, inclusive.

14. The method of claim 1, wherein a diameter of the corneal device is from about 5.5 mm to about 7.5 mm, inclusive.

15. The method of claim 1, wherein the corneal device is molded from a hydrogel.

16. The method of claim 1, wherein the corneal device comprises water, a natural polymer, and two synthetic polymers, the two synthetic polymers forming an interpenetrating polymer network, wherein the synthetic polymers and the natural polymer are at least partially interlaced on a molecular scale but not covalently bonded to each other and cannot be separated.

17. The method of claim 16, wherein the natural polymer is a collagen.

18. The method of claim 17, wherein the collagen is a porcine collagen.

19. The method of claim 16, wherein a hydrogel for fabricating the corneal device comprise at least 1%, at least 2%, at least 3%, at least 4%, or at least 5% by weight of the natural polymer.

20. The method of claim 16, wherein one of the two synthetic polymers is poly(2-methacryloyloxyethyl phosphorylcholine) (MPC).

21. The method of claim 16, wherein one of the two synthetic polymers is Poly(ethylene glycol) diacrylate (PEGDA).

22. The method of claim 1, wherein the water content of the corneal device ranges from about 78% to about 90% inclusive.

23. The method of claim 22, wherein the water content of the corneal device ranges from about 78% to about 88%, inclusive.

24. The method of claim 22, wherein the water content of the corneal device ranges from about 78% to about 84%, inclusive.

25. The method of claim 1, wherein the corneal device is optically transparent, biocompatible, permeable and refractive.

26. The method of claim 1, wherein the ectatic corneal disease is naturally occurring or derived from refractive surgery.

27. The method of claim 26, wherein the naturally occurring ectatic corneal disease is one or more of keratoconus, forme fruste keratoconus; keratoglobus, or pellucid marginal degeneration.

28. The method of claim 26, wherein the ectatic corneal disease derived from refractive surgery is derived from LASIK, small incision lenticule extraction (SMILE), or photorefractive keratectomy (PRK).

29. A method of treating ectatic corneal disease, comprising:

implanting in or on a cornea of a mammalian subject a corneal device of water content of between 78%-92%% (w/w), the corneal device comprising a thickness, a diameter, a base surface and a top surface, wherein the corneal device is effective to change an anterior curvature of the cornea to improve vision of an eye of the mammalian subject.

30. The method of claim 29, wherein the ectatic corneal disease is naturally occurring or derived from refractive surgery.

31. The method of claim 30, wherein the naturally occurring ectatic corneal disease is one or more of keratoconus, forme fruste keratoconus; keratoglobus, or pellucid marginal degeneration.

32. The method of claim 30, wherein the ectatic corneal disease derived from refractive surgery is derived from LASIK, small incision lenticule extraction (SMILE), or photorefractive keratectomy (PRK).

33. Use of a corneal device with low water content to correct vision of a mammalian subject with corneal structure weakened by ectatic corneal disease, the corneal device comprising a thickness, a diameter, a base surface, and a top surface, wherein the corneal device when placed in or on the cornea is effective to support the corneal structure weakened by the ectatic corneal disease or change an anterior curvature of the cornea to improve vision of the eye of the mammalian subject.

34. A corneal device for treating ectatic corneal disease, comprising:
a body defining a thickness, a diameter, a base surface, and a top surface, wherein the corneal device is fabricated from a hydrogel composition comprising an interpenetrating polymer network of two polymers at least partially interlaced on a molecular scale but not covalently bonded to each other that cannot be separated, comprising a water content ranging from 78%-92%, inclusive.

35. The corneal device for treating ectatic corneal disease according to claim 34, wherein the ectatic corneal disease is naturally occurring or derived from refractive surgery.

36. The corneal device for treating ectatic corneal disease according to claim 35, wherein the naturally occurring ectatic corneal disease is one or more of keratoconus, forme fruste keratoconus; keratoglobus, or pellucid marginal degeneration.

37. The corneal device for treating ectatic corneal disease according to claim 35, wherein the ectatic corneal disease derived from refractive surgery is derived from LASIK, small incision lenticule extraction (SMILE), or photorefractive keratectomy (PRK).

**Eye Anatomy**

conjunctiva

ciliary body

choroid

retina

iris

lens

vitreous body

macula

anterior chamber

pupil

cornea

conjunctiva

optic nerve

sclera

optic disc

FIG. 1
(Prior Art)

FIG. 2
(Prior Art)

**Presbyopia**

Stiffened Lens
Due to Age

Cornea

Light

Healthy Eye

Light is Focused
Behind the Retina

Retina

FIG. 3
(Prior Art)

EP 4 137 167 A1

Iris

Lens

Keratoconus Cornea

Anterior Chamber

Normal Cornea

FIG. 4
(Prior Art)

Comeal Inlay
10

Thickness 12

Diameter 14

FIG. 5

EP 4 137 167 A1

Cornea
20

New Anterior Shape
after Corneal Inlay
32

Pre-operative Anterior
Corneal Surface
30

Flap
28

Corneal Flap

Corneal Bed
Under Flap
26
Stomal Bed

Posterior Cornea

Posterior
Surface
24

Corneal Anterior
Inlay Surface
22

Corneal
Inlay
10

FIG. 6

FIG. 7

Emmetropes: Derived from wavefront measurements

Change in Anterior Corneal
Surface Height (microns)

Induced Add Power
(diopters)

*Inlay Radius

Radium from Center of Onlay (mm)

FIG. 8

EP 4 137 167 A1

Preoperative OCT

Postoperative OCT

70

Corneal Inlay Location

FIG. 9

FIG. 10

FIG. 11

EP 4 137 167 A1

FIG. 12

100

102

104

FIG. 13

106

102

A

104

FIG. 14

FIG. 15

FIG. 16

EP 4 137 167 A1

FIG. 17

95

FIG. 18

FIG. 19

FIG. 20

FIG. 21

FIG. 22

FIG. 23

FIG. 24

FIG. 25

FIG. 26

200

A

202

Ø1.800±0.1

A

FIG. 27

**200**

202

206

204

FIG. 28

**200**

202

A

206

204

(inlay)
0.040±.0.003
0.015-
0.030±0.003
(onlay)

FIG. 29

**200**

(inlay)

0.015±0.003

0.005±0.003
(onlay)

202

206

204

FIG. 30

**210**

212

214

FIG. 31

**210**

212

B

216

214

R5.773

R10

(inlay)-
0.055±0.002

0.015-
0.030±0.003
(onlay)

FIG. 32

**210**

(inlay)

0.025±0.002

0.005±0.003 (onlay)

212

216

214

FIG. 33

100

FIG. 34

Control

Material

FIG. 35

FIG. 36A

FIG. 36B

FIG. 36C

FIG. 36D

FIG. 36E

FIG. 37

FIG. 38

FIG. 39

FIG. 40A

FIG. 40B

FIG. 40C

FIG. 40D

FIG. 40E

FIG. 40F

FIG. 40G

EP 4 137 167 A1

FIG. 41A

FIG. 41B    FIG. 41C    FIG. 41D    FIG. 41E

FIG. 41F        FIG. 41G

EP 4 137 167 A1

Cells seeded in
4mL of media

Material (6-10mm disc)

FIG. 42

FIG. 43

FIG. 44A

FIG. 44B        FIG. 44C        FIG. 44D        FIG. 44E        FIG. 44F

FIG. 44G        FIG. 44H        FIG. 44I

FIG. 45A

FIG. 45B

FIG. 45C

FIG. 45D

FIG. 45E

FIG. 45F

FIG. 45G

FIG. 45H

FIG. 45I

yellow
MTT

mitochondrial
dehydrogenase

purple
formazan

FIG. 46

FIG. 47A

FIG. 47B

FIG. 47C

FIG. 47D

FIG. 47E

FIG. 47F

FIG. 48A

FIG. 48B

FIG. 48C

FIG. 48D

FIG. 48E

FIG. 48F

FIG. 48G

FIG. 48H

FIG. 48I

FIG. 48J

FIG. 49

FIG. 50

Cells seeded in
100 µL at edge of plate

Material (6-10mm disc)

FIG. 51

FIG. 52A

FIG. 52B

FIG. 53

FIG. 54A

FIG. 54B

FIG. 55B

FIG. 55A

FIG. 56

300

302

304

FIG. 57

306

300

302

304

FIG. 58

FIG. 59

316

302

308

312

318

Ø 16.670

FIG. 60

302

318

0.500

0.552

Ø 17.546

312

10.500

10.000

314

Ø 18.650

FIG. 61

0.500

318

308

302

A

322

314

Ø 16.670

FIG. 62

302

0.250

0.500

100°

308

318

FIG. 63

FIG. 64

FIG. 65

FIG. 66

FIG. 67

FIG. 68

Donut or Ring-Shaped

FIG. 69A

Concave-Shaped

FIG. 69B

Convex-Shaped or Planar

FIG. 69C

FIG. 70

FIG. 71

FIG. 72

500

A

502

508

504

Ø6.000 ± 0.1

Ø3.000 + 0.1

A

FIG. 73

**500**

502

504

506

508

**FIG. 74**

**500**

0.029 ± 0.003

504

R25.000

R50.000

506

0.097 ± 0.003

508

502

**FIG. 75**

**600**

A

602

604

Ø6.000 ± 0.1

Ø2.000

608

A

FIG. 76

**600**

602

604

608

606

FIG. 77

**600**

(0.029 ± 0.003)

604

R10.000

R50.000

606

608

0.020 ± 0.003

602

FIG. 78

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RAFAT MEHRDAD ET AL: "Composite core-and-skirt collagen hydrogels with differential degradation for corneal therapeutic applications", BIOMATERIALS, ELSEVIER, AMSTERDAM, NL, vol. 83, 4 January 2016 (2016-01-04), pages 142-155, XP029416056, ISSN: 0142-9612, DOI: 10.1016/J.BIOMATERIALS.2016.01.004 * 2.1 and 2.6; figure 6 * | 1,2, 4-26,29, 30,33-37 | INV. A61L27/26 A61L27/52 |
| X | US 2012/321585 A1 (GRIFFITH MAY [CA] ET AL) 20 December 2012 (2012-12-20) * paragraphs [0019], [0232], [0235]; claims 19,30; tables 25,27 * | 1-37 | |
| X | LIU W ET AL: "Collagen-phosphorylcholine interpenetrating network hydrogels as corneal substitutes", BIOMATERIALS, ELSEVIER, AMSTERDAM, NL, vol. 30, no. 8, 1 March 2009 (2009-03-01), pages 1551-1559, XP025876351, ISSN: 0142-9612, DOI: 10.1016/J.BIOMATERIALS.2008.11.022 [retrieved on 2009-01-19] * 2.1-22; table 1 * | 34-37 | |

|  |
|---|
| TECHNICAL FIELDS SEARCHED (IPC) |
| A61L |

The present search report has been drawn up for all claims

| Place of search | Date of completion of the search | Examiner |
|---|---|---|
| The Hague | 13 January 2023 | Siebum, Bastiaan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 18 9379

This annex lists the patent family members relating to the patent documents cited in the above-mentioned European search report.
The members are as contained in the European Patent Office EDP file on
The European Patent Office is in no way liable for these particulars which are merely given for the purpose of information.

13-01-2023

| Patent document cited in search report | | Publication date | Patent family member(s) | | Publication date |
|---|---|---|---|---|---|
| US 2012321585 | A1 | 20-12-2012 | CA | 2775670 A1 | 07-04-2011 |
| | | | EP | 2483332 A1 | 08-08-2012 |
| | | | US | 2012321585 A1 | 20-12-2012 |
| | | | WO | 2011038485 A1 | 07-04-2011 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- US 63231138


### Non-patent literature cited in the description

- **SRIDHAR, M.S.** Anatomy of cornea and ocular surface. *Indian J. Ophthalmol.,* 2018, vol. 66 (2), 190-194
- **G. WESTHEIMER.** Directional sensitivity of the retina: 75 years of Stiles-Crawford effect. *Proc. R. Soc. B.,* 2008, vol. 275 (1653), 2777-86
- **FRIEDLANDER, M.** Fibrosis and diseases of the eye. *J. Clin. Invest.,* 2007, vol. 117 (3), 576-86
- **LJUBIMOV , AV ; SAGHIZADEH, M.** Progress in corneal wound healing. *Prog. Retin. Eye Res.,* 2015, vol. 49, 17-45
- **KUWABARA T et al.** Sliding of the epithelium in experimental corneal wounds. *Invest. Ophthalmol.,* 1976, vol. 15, 4-14
- **CROSSON, CE et al.** Epithelial wound closure in the rabbit cornea. A biphasic process. *Invest. Ophthalmol. Vis. Sci.,* 1986, vol. 27, 464-473
- **ANDERSON, SC et al.** Rho and Rho-kinase (ROCK) signaling in adherens and gap junction assembly in corneal epithelium. *Invest. Ophthalmol. Vis. Sci.,* 2002, vol. 43, 978-986
- **LEE, A et al.** Hypoxia-induced changes in Ca2+ mobilization and protein phosphorylation implicated in impaired wound healing. *Am. J. Physiol. Cell. Physiol.,* 2014, vol. 306, C972-985
- **WEINGER, I et al.** Tri-nucleotide receptors play a critical role in epithelial cell wound repair. *Purinerg. Signal.,* 2005, vol. 1, 281-292
- Hypoxia-induced changes in Ca2+ mobilization and protein phosphorylation implicated in impaired wound healing. *Am. J. Physiol. Cell. Physiol.,* 2014, vol. 306, C972-985
- **LEE, A. et al.** Hypoxia-induced changes in Ca2+ mobilization and protein phosphorylation implicated in impaired wound healing. *Am. J. Physiol. Cell. Physiol.,* 2014, vol. 306, C972-985
- **ESLANI, M et al.** The role of toll-like receptor 4 in corneal epithelial wound healing. *Invest. Ophthalmol. Vis. Sci.,* 2014, vol. 55, 6108-6115
- **PEARLMAN, E et al.** Toll-like receptors at the ocular surface. *Ocul. Surf.,* 2008, vol. 6, 108-116

- **KOSTARNOY, AV et al.** Topical bacterial lipopolysaccharide application affects inflammatory response and promotes wound healing. *J. Interferon Cytokine Res.,* 2013, vol. 33, 514-522
- **WILSON SE et al.** Stromal-epithelial interactions in the cornea. *Prog. Retin. Eye Res.,* 1999, vol. 18, 293-309
- *Exp. Eye Res.,* 2001, vol. 72, 631-641
- **OAKDALE, Y.** Expression of fos family and jun family proto-oncogenes during corneal epithelial wound healing. *Curr. Eye Res.,* 1996, vol. 15, 824-832
- **WILSON SE et al.** The corneal wound healing response: cytokine mediated interaction of the epithelium, stroma, and inflammatory cells. *Prog. Retin. Eye Res.,* 2001, vol. 20
- **LYU J.** Transactivation of EGFR mediates insulin-stimulated ERK1/2 activation and enhanced cell migration in human corneal epithelial cells. *Mol. Vis.,* 2006, vol. 12, 1403-1410
- **SPIX JK et al.** Hepatocyte growth factor induces epithelial cell motility through transactivation of the epidermal growth factor receptor. *Exp. Cell Res.,* 2007, vol. 313, 3319-3325
- **SHARMA GD ; HE J ; BAZAN HE.** p38 and ERK1/2 coordinate cellular migration and proliferation in epithelial wound healing: evidence of cross-talk activation between MAP kinase cascades. *J. Biol. Chem.,* 2003, vol. 278, 21989-21997
- **HO TC et al.** PEDF promotes self-renewal of limbal stem cell and accelerates corneal epithelial wound healing. *Stem Cells,* 2013, vol. 31, 1775-1784
- **TUFT SJ et al.** Photorefractive keratectomy: implications of corneal wound healing. *Br. J. Ophthalmol.,* 1993, vol. 77, 243-247
- **WEINGER I et al.** Tri-nucleotide receptors play a critical role in epithelial cell wound repair. *Purinerg. Signal.,* 2005, vol. 1, 281-292
- **BOUCHER I.** Injury and nucleotides induce phosphorylation of epidermal growth factor receptor: MMP and HB-EGF dependent pathway. *Exp. Eye Res.,* 2007, vol. 85, 130-141

- **YIN J ; XU K ; ZHANG J ; KUMAR A ; YU FS.** Wound-induced ATP release and EGF receptor activation in epithelial cells. *J. Cell Sci.,* 2007, vol. 120, 815-825
- **LEE A et al.** Hypoxia-induced changes in Ca2+ mobilization and protein phosphorylation implicated in impaired wound healing. *Am. J. Physiol. Cell. Physiol.,* 2014, vol. 306, C972-985
- **SCHALLER, MD.** Paxillin: a focal adhesion associated adaptor protein. *Oncogene,* 2001, vol. 20, 6459-72
- **KIMURA K et al.** Role of JNK-dependent serine phosphorylation of paxillin in migration of corneal epithelial cells during wound closure. *Invest. Ophthalmol. Vis. Sci.,* 2008, vol. 49, 125-132
- **MAYO C et al.** Regulation by P2X7: epithelial migration and stromal organization in the cornea. *Invest. Ophthalmol. Vis. Sci.,* 2008, vol. 49, 4384-4391
- **LEE JG ; KAY EP.** FGF-2-induced wound healing in corneal endothelial cells requires Cdc42 activation and Rho inactivation through the phosphatidylinositol 3-kinase pathway. *Invest. Ophthalmol. Vis. Sci.,* 2006, vol. 47, 1376-1386
- **SEOMUN Y ; JOO CK.** Lumican induces human corneal epithelial cell migration and integrin expression via ERK 1/2 signaling. *Biochem. Biophys. Res. Commun.,* 2008, vol. 372, 221-225
- **SALANI B et al.** IGF-I induced rapid recruitment of integrin β1 to lipid rafts is caveolin-1 dependent. *Biochem. Biophys. Res. Commun.,* 2009, vol. 380, 489-492
- **AZAR DT et al.** Altered epithelial-basement membrane interactions in diabetic corneas. *Arch. Ophthalmol.,* 1992, vol. 110, 537-40
- **KURPAKUS MA et al.** The role of the basement membrane in differential expression of keratin proteins in epithelial cells. *Dev. Biol.,* 1992, vol. 150, 243-255
- **ZIESKE JD et al.** Basement membrane assembly and differentiation of cultured corneal cells: importance of culture environment and endothelial cell interaction. *Exp. Cell Res.,* 1994, vol. 214, 621-633
- **LJUBIMOV AV et al.** Extracellular matrix alterations in human corneas with bullous keratopathy. *Invest. Ophthalmol. Vis. Sci.,* 1996, vol. 37, 997-1007
- **LJUBIMOV AV et al.** Basement membrane abnormalities in human eyes with diabetic retinopathy. *J Histochem Cytochem,* 1996, vol. 44, 1469-1479
- **SUZUKI K et al.** Cell-matrix and cell-cell interactions during corneal epithelial wound healing. *Prog. Retin. Eye Res.,* 2003, vol. 22, 113-133
- **NAKAYASU K et al.** Distribution of types I, II, III, IV and V collagen in normal and keratoconus corneas. *Ophthalmic Res.,* 1986, vol. 18, 1-10
- **MARTIN GR ; TIMPL R.** Laminin and other basement membrane components. *Annu. Rev. Cell Biol.,* 1987, vol. 3, 57-85
- **LJUBIMOV AV et al.** Human corneal basement membrane heterogeneity: topographical differences in the expression of type IV collagen and laminin isoforms. *Lab Invest.,* 1995, vol. 72, 461-473
- **TUORI A et al.** The immunohistochemical composition of the human corneal basement membrane. *Cornea,* 1996, vol. 15, 286-294
- **KABOSOVA A et al.** Human diabetic corneas preserve wound healing, basement membrane, integrin and MMP-10 differences from normal corneas in organ culture. *Exp. Eye Res.,* 2003, vol. 77, 211-217
- **SCHLÖTZER-SCHREHARDT U et al.** Characterization of extracellular matrix components in the limbal epithelial stem cell compartment. *Exp. Eye Res.,* 2007, vol. 85, 845-60
- **KABOSOVA A et al.** Compositional differences between infant and adult human corneal basement membranes. *Invest. Ophthalmol. Vis. Sci.,* 2007, vol. 48, 4989-4999
- **SCHREHARDT U et al.** Characterization of extracellular matrix components in the limbal epithelial stem cell compartment. *Exp. Eye Res.,* 2007, vol. 85, 845-60
- **DIETRICH-NTOUKAS T et al.** Comparative analysis of the basement membrane composition of the human limbus epithelium and amniotic membrane epithelium. *Cornea,* 2012, vol. 31, 564-569
- **LI Z et al.** Lymphocyte function-associated antigen-1-dependent inhibition of corneal wound healing. *Am. J. Pathol.,* 2006, vol. 169, 1590-600
- **LI Z et al.** Platelet response to corneal abrasion is necessary for acute inflammation and efficient re-epithelialization. *Invest. Ophthalmol. Vis. Sci.,* 2006, vol. 47, 4794-4802
- **STAPLETON WM et al.** Topical interleukin-1 receptor antagonist inhibits inflammatory cell infiltration into the cornea. *Exp. Eye Res.,* 2008, vol. 86, 753-757
- **LI SD ; HUANG L.** Non-viral is superior to viral gene delivery. *J. Control Release.,* 2007, vol. 123, 181-183
- **YAMAGAMI S et al.** CCR5 chemokine receptor mediates recruitment of MHC class II-positive Langerhans cells in the mouse corneal epithelium. *Invest. Ophthalmol. Vis. Sci.,* 2005, vol. 46, 1201-1207
- **JIN Y et al.** The chemokine receptor CCR7 mediates corneal antigen-presenting cell trafficking. *Mol. Vis.,* 2007, vol. 13, 626-634
- **GAO N et al.** Dendritic cell-epithelium interplay is a determinant factor for corneal epithelial wound repair. *Am. J. Pathol.,* 2011, vol. 179, 2243-53

- **LI SD ; HUANG L.** Non-viral is superior to viral gene delivery. *J. Control Release,* 2007, vol. 123, 181-183
- **LAM FW et al.** Platelets enhance neutrophil transendothelial migration via P-selectin glycoprotein ligand-1. *Am. J Physiol. Heart Circ. Physiol.,* 2011, vol. 300, H468-H475
- **TORRICELLI, AAM et al.** The Corneal Epithelial Basement Membrane: Structure, Function and Disease. *Invest. Ophthalmol. Vis. Sci.,* 2013, vol. 54, 6390-6400
- **FUJIKAWA LS et al.** Basement membrane components in healing rabbit corneal epithelial wounds: immunofluorescence and ultrastructural studies. *J Cell Biol.,* 1984, vol. 98, 128-138
- **STA IGLESIA DD ; STEPP MA.** Disruption of the basement membrane after corneal debridement. *Invest Ophthalmol Vis Sci.,* 2000, vol. 41, 1045-1053
- **CHI C.** Trinkaus-Randall V. New insights in wound response and repair of epithelium. *J Cell Physiol.,* 2013, vol. 228, 925-929
- **PAL-GHOSH S et al.** Removal of the basement membrane enhances corneal wound healing. *Exp Eye Res.,* 2011, vol. 93, 927-936
- **TORRICELLI AA et al.** Transmission electron microscopy analysis of epithelial basement membrane repair in rabbit corneas with haze. *Invest Ophthalmol Vis Sci,* 2013, vol. 54, 4026-4033
- **JESTER JV et al.** Transforming growth factor (beta)-mediated corneal myofibroblast differentiation requires actin and fibronectin assembly. *Invest Ophthalmol Vis Sci,* 1999, vol. 40, 1959-1967
- **MASUR SK et al.** Myofibroblasts differentiate from fibroblasts when plated at low density. *Proc Natl Acad Sci U S A.,* 1996, vol. 93, 4219-4223
- **WILSON SE et al.** Stromal-epithelial interactions in the cornea. *Prog Retin Eye Res,* 1999, vol. 18, 293-309
- **SINGH V et al.** Stromal fibroblast-bone marrow-derived cell interactions: implications for myofibroblast development in the cornea. *Exp Eye Res,* 2012, vol. 98, 1-8
- **SINGH V et al.** Effect of TGFbeta and PDGF-B blockade on corneal myofibroblast development in mice. *Exp Eye Res,* 2011, vol. 93, 810-817
- **FINI ME ; STRAMER BM.** How the cornea heals: cornea-specific repair mechanisms affecting surgical outcomes. *Cornea,* 2005, vol. 24, S2-S11
- **STRAMER BM et al.** Molecular mechanisms controlling the fibrotic repair phenotype in cornea: implications for surgical outcomes. *Invest Ophthalmol Vis Sci,* 2003, vol. 44, 4237-4246
- **WILSON SE et al.** Hepatocyte growth factor, keratinocyte growth factor, their receptors, fibroblast growth factor receptor-2, and the cells of the cornea. *Invest Ophthalmol Vis Sci.,* 1993, vol. 34, 2544-2561
- **WILSON SE et al.** Effect of epidermal growth factor, hepatocyte growth factor, and keratinocyte growth factor, on proliferation, motility and differentiation of human corneal epithelial cells. *Exp Eye Res,* 1994, vol. 59, 665-678
- **LATVALA T et al.** Distribution of alpha 6 and beta 4 integrins following epithelial abrasion in the rabbit cornea. *Acta Ophthalmol Scand,* 1996, vol. 74, 21-25
- **STEPP MA et al.** Changes in beta 4 integrin expression and localization in vivo in response to corneal epithelial injury. *Invest Ophthalmol Vis Sci,* 1996, vol. 37
- **NAKAYASU K.** Stromal changes following removal of epithelium in rat cornea. *Jpn. J. Ophthalmol.,* 1988, vol. 32, 113-125
- **SZERENYI KD et al.** Keratocyte loss and repopulation of anterior corneal stroma after de-epithelialization. *Arch. Ophthalmol.,* 1994, vol. 112, 973-976
- **WILSON SE et al.** Epithelial injury induces keratocyte apoptosis: hypothesized role for the interleukin-1 system in the modulation of corneal tissue organization and wound healing. *Exp. Eye Res.,* 1996, vol. 62
- **WILSON SE et al.** The corneal wound healing response: cytokine mediated interaction of the epithelium, stroma, and inflammatory cells. *Prog. Retin. Eye Res.,* 2001, vol. 20, 625-637
- **WILSON SE et al.** Apoptosis in the initiation, modulation and termination of the corneal wound healing response. *Exp. Eye Res.,* 2007, vol. 85, 305-311
- **MAYCOCK NJ.** Marshall J. Genomics of corneal wound healing: a review of the literature. *Acta Ophthalmol,* 2014, vol. 92, e170-84
- **ZIESKE JD et al.** Activation of epidermal growth factor receptor during corneal epithelial migration. *Invest. Ophthalmol. Vis. Sci.,* 2000, vol. 41, 1346-1355
- **STRAMER BM et al.** Molecular mechanisms controlling the fibrotic repair phenotype in cornea: implications for surgical outcomes. *Invest. Ophthalmol. Vis. Sci.,* 2003, vol. 44, 4237-4246
- **FINI ME ; STRAMER BM.** How the cornea heals: cornea-specific repair mechanisms affecting surgical outcomes. *Cornea,* 2005, vol. 24 (1), S2-S11
- **WEST-MAYS JA ; DWIVEDI DJ.** The keratocyte: corneal stromal cell with variable repair phenotypes. *Int. J. Biochem. Cell Biol.,* 2006, vol. 38, 1625-1631
- **MAYS JA ; DWIVEDI DJ.** The keratocyte: corneal stromal cell with variable repair phenotypes. *Int. J. Biochem. Cell Biol.,* 2006, vol. 38, 1625-1631

- **JESTER JV ; HO-CHANG J.** Modulation of cultured corneal keratocyte phenotype by growth factors/cytokines control in vitro contractility and extracellular matrix contraction. *Exp. Eye Res.,* 2003, vol. 77, 581-592
- **CHEN J et al.** Rho-mediated regulation of TGF-β1- and FGF-2-induced activation of corneal stromal keratocytes. *Invest. Ophthalmol. Vis. Sci.,* 2009, vol. 50, 3662-3670
- **FINI ME.** Keratocyte and fibroblast phenotypes in the repairing cornea. *Prog. Retin. Eye Res.,* 1999, vol. 18, 529-551
- **JESTER JV et al.** Corneal stromal wound healing in refractive surgery: the role of myofibroblasts. *Prog. Retin. Eye Res.,* 1999, vol. 18, 311-356
- **CARLSON EC et al.** Altered KSPG expression by keratocytes following corneal injury. *Mol. Vis.,* 2003, vol. 9, 615-6223
- **CHAURASIA SS et al.** Dynamics of the expression of intermediate filaments vimentin and desmin during myofibroblast differentiation after corneal injury. *Exp. Eye Res.,* 2009, vol. 89, 590-59
- **TERVO K et al.** Expression of tenascin and cellular fibronectin in the rabbit cornea after anterior keratectomy. Immunohistochemical study of wound healing dynamics. *Invest. Ophthalmol. Vis. Sci.,* 1991, vol. 32, 2912-2918
- **JESTER JV et al.** Expression of alpha-smooth muscle (a-SM) actin during corneal stromal wound healing. *Invest. Ophthalmol. Vis. Sci.,* 1995, vol. 36, 809-819
- **BARBOSA FL et al.** Corneal myofibroblast generation from bone marrow-derived cells. *Exp. Eye Res.,* 2010, vol. 91, 92-96
- **KAUR H et al.** Corneal stroma PDGF blockade and myofibroblast development. *Exp Eye Res.,* 2009, vol. 88, 960-965
- **SINGH V et al.** Transforming growth factor β and platelet-derived growth factor modulation of myofibroblast development from corneal fibroblasts in vitro. *Exp. Eye Res.,* 2014, vol. 120, 152-160
- **KARAMICHOS D et al.** Reversal of fibrosis by TGF-β2 in a 3D in vitro model. *Exp. Eye Res.,* 2014, vol. 124, 31-36
- **SINGH V et al.** Mouse strain variation in SMA(+) myofibroblast development after corneal injury. *Exp. Eye Res.,* 2013, vol. 115, 27-30
- **MATSUBA M et al.** Localization of thrombospondin-1 and myofibroblasts during corneal wound repair. *Exp. Eye Res.,* 2011, vol. 93, 534-540
- **RUIZ-EDERRA J ; VERKMAN AS.** Aquaporin-1-facilitated keratocyte migration in cell culture and in vivo corneal wound healing models. *Exp. Eye Res.,* 2009, vol. 89, 159-165
- **GAN L et al.** Effect of leukocytes on corneal cellular proliferation and wound healing. *Invest. Ophthalmol. Vis. Sci.,* 1999, vol. 40, 575-581
- **WILSON SE et al.** RANK, RANKL, OPG, and M-CSF expression in stromal cells during corneal wound healing. *Invest. Ophthalmol. Vis. Sci.,* 2004, vol. 45, 2201-2211
- **LIU Q et al.** NK Cells Modulate the Inflammatory Response to Corneal Epithelial Abrasion and Thereby Support Wound Healing. *J. Pathol.,* 2012, vol. 181, 452-462
- **LI S et al.** Macrophage depletion impairs corneal wound healing after autologous transplantation in mice. *PLoS One,* 2013, vol. 8, e61799
- **HAYASHI Y et al.** Lumican is required for neutrophil extravasation following corneal injury and wound healing. *J. Cell Sci.,* 2010, vol. 123, 2987-2995
- **ZIESKE JD et al.** Kinetics of keratocyte proliferation in response to epithelial debridement. *Exp. Eye Res.,* 2001, vol. 72, 33-39
- **TORRICELLI AA ; WILSON SE.** Cellular and extracellular matrix modulation of corneal stromal opacity. *Exp. Eye Res.,* 2014, vol. 129, 151-160
- **ISHIZAKI M et al.** Expression of collagen I, smooth muscle alpha-actin, and vimentin during the healing of alkali-burned and lacerated corneas. *Invest. Ophthalmol. Vis. Sci.,* 1993, vol. 34, 3320-3328
- **ISHIZAKI M et al.** Stromal fibroblasts are associated with collagen IV in scar tissues of alkali-burned and lacerated corneas. *Curr. Eye Res.,* 1997, vol. 16, 339-348
- **MAGUEN E et al.** Alterations of corneal extracellular matrix after multiple refractive procedures: a clinical and immunohistochemical study. *Cornea,* 1997, vol. 16, 675-682
- **LJUBIMOV AV et al.** Extracellular matrix changes in human corneas after radial keratotomy. *Exp. Eye Res.,* 1998, vol. 67, 265-272
- **MAGUEN E et al.** Extracellular matrix and matrix metalloproteinase changes in human corneas after complicated laser-assisted in situ keratomileusis (LASIK). *Cornea,* 2002, vol. 21, 95-100
- **MAGUEN E et al.** Immunohistochemical evaluation of two corneal buttons with post-LASIK keratectasia. *Cornea,* 2007, vol. 26, 983-991
- **KATO T et al.** Expression of type XVIII collagen during healing of corneal incisions and keratectomy wounds. *Invest. Ophthalmol. Vis. Sci.,* 2003, vol. 44, 78-85
- **KAMMA-LORGER CS et al.** Collagen ultrastructural changes during stromal wound healing in organ cultured bovine corneas. *Exp. Eye Res.,* 2009, vol. 88, 953-959
- **LATVALA T et al.** Expression of cellular fibronectin and tenascin in the rabbit cornea after excimer laser photorefractive keratectomy: a 12 month study. *Br. J. Ophthalmol.,* 1995, vol. 79, 65-69
- **MAGUEN E et al.** Extracellular matrix and matrix metalloproteinase changes in human corneas after complicated laser-assisted in situ keratomileusis (LASIK). *Cornea,* 2002, vol. 21, 95-10

- **MAGUEN E et al.** Alterations of extracellular matrix components and proteinases in human corneal buttons with INTACS for post-laser in situ keratomileusis keratectasia and keratoconus. *Cornea,* 2008, vol. 27, 565-573
- **SAIKA S et al.** Epithelial basement membrane in alkali-burned corneas in rats. Immunohistochemical study. *Cornea,* 1993, vol. 12, 383-390
- **MELLES GR et al.** Immunohistochemical analysis of unsutured and sutured corneal wound healing. *Curr. Eye Res.,* 1995, vol. 14, 809-817
- **NICKELEIT V et al.** Healing corneas express embryonic fibronectin isoforms in the epithelium, subepithelial stroma, and endothelium. *Am. J. Pathol.,* 1996, vol. 149, 549-558
- **MAGUEN E et al.** xtracellular matrix and matrix metalloproteinase changes in human corneas after complicated laser-assisted in situ keratomileusis (LASIK). *Cornea,* 2002, vol. 21, 95-10
- **JAVIER JA et al.** Basement membrane and collagen deposition after laser subepithelial keratomileusis and photorefractive keratectomy in the leghorn chick eye. *Arch. Ophthalmol.,* 2006, vol. 124, 703-709
- **CHAURASIA SS et al.** Hevin plays a pivotal role in corneal wound healing. *PLoS One,* 2013, vol. 8, e81544
- **SAIKA S et al.** Wakayama symposium: modulation of wound healing response in the corneal stroma by osteopontin and tenascin-C. *Ocul. Surf.,* 2013, vol. 11, 12-15
- *Intl. J. Biochem. Cell Biol.,* 2004, vol. 38 (6), 991-96
- **CITING STERN ME et al.** Effect of platelet-derived growth factor on rabbit corneal wound healing. *Wound Repair Regen,* 1995, vol. 3, 59-65
- **BALDWIN HC ; MARSHALL J.** Growth factors in corneal wound healing following refractive surgery: A review. *Acta. Ophthalmol. Scand.,* 2002, vol. 80, 238-247
- **CARRINGTON LM ; BOULTON M.** Hepatocyte growth factor and keratinocyte growth factor regulation of epithelial and stromal corneal wound healing. *J. Cataract Refract. Surg.,* 2005, vol. 31, 412-423
- **BALDWIN HC.** Marshall J. Growth factors in corneal wound healing following refractive surgery: A review. *Acta. Ophthalmol. Scand.,* 2002, vol. 80, 238-247
- **KAKAZU A et al.** Lipoxin A inhibits platelet-activating factor inflammatory response and stimulates corneal wound healing of injuries that compromise the stroma. *Exp. Eye Res.,* 2012, vol. 103, 9-16
- **MALECAZE F et al.** Upregulation of bone morphogenetic protein-1/mammalian tolloid and procollagen C-proteinase enhancer-1 in corneal scarring. *Invest. Ophthalmol. Vis. Sci.,* 2014, vol. 55

- **IZUMI K et al.** Involvement of insulin-like growth factor-I and insulin-like growth factor binding protein-3 in corneal fibroblasts during corneal wound healing. *Invest. Ophthalmol. Vis. Sci.,* 2006, vol. 47, 591-598
- **SHI L et al.** Activation of JNK signaling mediates connective tissue growth factor expression and scar formation in corneal wound healing. *PLoS One,* 2012, vol. 7, e32128
- **KARAMICHOS D et al.** Reversal of fibrosis by TGF-β3 in a 3D in vitro model. *Exp. Eye Res.,* 2014, vol. 124, 31-36
- **HUXLIN KR et al.** Topical rosiglitazone is an effective anti-scarring agent in the cornea. *PLoS One,* 2013, vol. 8, e70785
- **MØLLER-PEDERSEN T et al.** Neutralizing antibody to TGFβ modulates stromal fibrosis but not regression of photoablative effect following PRK. *Curr. Eye Res.,* 1998, vol. 17, 736-747
- **JUNG JC et al.** Constitutive collagenase-1 synthesis through MAPK pathways is mediated, in part, by endogenous IL-1α during fibrotic repair in corneal stroma. *J. Cell Biochem.,* 2007, vol. 102, 453-462
- **HUH MI et al.** Distribution of TGF-β isoforms and signaling intermediates in corneal fibrotic wound repair. *J. Cell Biochem.,* 2009, vol. 108, 476-488
- **MILANI BY et al.** Rapamycin inhibits the production of myofibroblasts and reduces corneal scarring after photorefractive keratectomy. *Invest. Ophthalmol. Vis. Sci.,* 2013, vol. 54, 7424-7430
- **LEE SH et al.** Bevacizumab accelerates corneal wound healing by inhibiting TGF-β2 expression in alkali-burned mouse cornea. *BMB Rep,* 2009, vol. 42, 800-805
- **ZHAO B et al.** An investigation into corneal alkali burns using an organ culture model. *Cornea,* 2009, vol. 28, 541-546
- **MELLES GR et al.** Descemet membrane endothelial keratoplasty (DMEK). *Cornea,* 2006, vol. 25, 987-990
- **PRICE MO ; PRICE FW.** Descemet's stripping endothelial keratoplasty. *Curr. Opin. Ophthalmol.,* 2007, vol. 18, 290-294
- **CALDWELL MC et al.** The histology of graft adhesion in Descemet stripping with endothelial keratoplasty. *Am. J. Ophthalmol.,* 2007, vol. 148, 277-281
- **DIRISAMER M et al.** Patterns of corneal endothelialization and corneal clearance after Descemet membrane endothelial keratoplasty for Fuchs endothelial dystrophy. *Am. J. Ophthalmol.,* 2011, vol. 152, 543-555
- **MIMURA T et al.** Corneal endothelial regeneration and tissue engineering. *Prog. Retin. Eye Res.,* 2013, vol. 35, 1-17
- **JOYCE NC et al.** In vitro pharmacologic separation of corneal endothelial migration and spreading responses. *Invest. Ophthalmol. Vis. Sci.,* 1990, vol. 31, 1816-1826

- **ICHIJIMA H et al.** Actin filament organization during endothelial wound healing in the rabbit cornea: comparison between transcorneal freeze and mechanical scrape injuries. *Invest. Ophthalmol. Vis. Sci.,* 1993, vol. 34, 2803-28012
- **GORDON SR.** Cytological and immunocytochemical approaches to the study of corneal endothelial wound repair. *Prog. Histochem. Cytochem.,* 1994, vol. 28, 1-64
- **LEE JG ; KAY EP.** FGF-2-induced wound healing in corneal endothelial cells requires Cdc42 activation and Rho inactivation through the phosphatidylinositol 3-kinase pathway. *Invest. Ophthalmol. Vis. Sci.,* 2006, vol. 47 (1), 376-1386
- **LANDSHMAN N et al.** Cell division in the healing of the corneal endothelium of cats. *Arch. Ophthalmol.,* 1989, vol. 107, 1804-1808
- **LEE HT et al.** FGF-2 induced by interleukin-1 beta through the action of phosphatidylinositol 3-kinase mediates endothelial mesenchymal transformation in corneal endothelial cells. *J. Biol. Chem.,* 2004, vol. 279, 32325-32332
- **MIYAMOTO T et al.** Endothelial mesenchymal transition: a therapeutic target in retrocorneal membrane. *Cornea,* 2010, vol. 29 (1), S52-56
- **PETROLL WM et al.** ZO-1 reorganization and myofibroblast transformation of corneal endothelial cells after freeze injury in the cat. *Exp. Eye Res.,* 1997, vol. 64, 257-267
- **LEE JG et al.** Endothelial mesenchymal transformation mediated by IL-1β-induced FGF-2 in corneal endothelial cells. *Exp. Eye Res.,* 2012, vol. 95, 35-39
- **SUMIOKA T et al.** Inhibitory effect of blocking TGF-β/Smad signal on injury-induced fibrosis of corneal endothelium. *Mol. Vis.,* 2008, vol. 14, 2272-2281
- **ICHIJIMA H et al.** In vivo confocal microscopic studies of endothelial wound healing in rabbit cornea. *Cornea,* 1993, vol. 12, 369-378
- **NAKANO Y et al.** Connexin 43 knockdown accelerates wound healing but inhibits mesenchymal transition after corneal endothelial injury in vivo. *Invest. Ophthalmol. Vis. Sci.,* 2008, vol. 49, 93-104
- **OKUMURA N et al.** Inhibition of TGF-β signaling enables human corneal endothelial cell expansion in vitro for use in regenerative medicine. *PLoS One,* 2013, vol. 8, e58000
- **MUNJAL ID et al.** Thrombospondin: biosynthesis, distribution, and changes associated with wound repair in corneal endothelium. *Eur. J. Cell Biol.,* 1990, vol. 52, 252-263
- **GUNDOROVA RA et al.** Stimulation of penetrating corneal wound healing by exogenous fibronectin. *Eur. J. Ophthalmol.,* 1994, vol. 4, 202-210
- *Investig. Ophthalmol. Vis. Sci.,* 1997, vol. 38, 121-129
- **MATHEW, JH et al.** Lamellar changes in the keratoconic cornea. *Acta Ophthalmol,* 2015, vol. 93, 767-773
- **DOWNIE, L. ; LINDSAY, RG.** Contact lens management of keratoconus. *Clin. Exp. Optom.,* 2015, vol. 98, 299-311
- **JINABHAI, A. et al.** Visual performance and optical quality with soft lenses in heratoconus patients. *Ophthalmic Physiol. Opt.,* 2012, vol. 32, 100-16
- **GRIFFITHS, M. et al.** Masking of irregular corneal topography with contact lenses. *CLAO J,* 1998, vol. 24, 76-81
- **MCMAHON, T. et al.** A new method for grading the severity of keratoconus: The keratoconus severity score (KSS). *Cornea,* 2006, vol. 25, 794-800
- **PERRY, HD et al.** Round and oval cones in keratoconus. *Ophthalmology,* 1980, vol. 87, 905-9
- **PERRY, HD et al.** round and oval cones in kertoconus. *Ophthalmology,* 1980, vol. 87, 905-9
- **JIMENEZ, M. et al.** Keratoconus: a review. *Cont. Lens Anterior Eye,* 2010, vol. 33, 157-66
- **MCKAY, TB et al.** Mechanisms of Collagen crosslinking in Diabetes and Keratoconus. *Cells,* 2019, vol. 8, 1239
- **KAGAN H.M. ; LI W.** Lysyl oxidase: Properties, specificity, and biological roles inside and outside of the cell. *J. Cell. Biochem.,* 2003, vol. 88, 660-672
- **TAKAOKA. A. et al.** An Evaluation of Lysyl Oxidase-Derived Cross-Linking in Keratoconus by Liquid Chromatography/Mass Spectrometry. *Investig. Ophthalmol. Vis. Sci.,* 2016, vol. 57, 126-136
- **YAMAUCHI, M. et al.** Cross-linking and the molecular packing of corneal collagen. *Biochem. Biophys. Res. Commun.,* 1996, vol. 219, 311-315
- **ERLER, JT et al.** Lysyl oxidase is essential for hypoxia-induced metastasis. *Nature,* 2006, vol. 440, 1222
- **LEVANTAL, KR et al.** Matrix Crosslinking Forces Tumor Progression by Enhancing Integrin Signaling. *Cell,* 2009, vol. 139, 891-906
- **AKIRI, G. et al.** Lysyl oxidase-related protein-1 promotes tumor fibrosis and tumor progression in vivo. *Cancer Res.,* 2003, vol. 63, 1657-1666
- **KAGAN H.M. ; TRACKMAN P.C.** Properties and function of lysyl oxidase. *Am. J. Respir. Cell Mol. Biol.,* 1991, vol. 5, 206-210
- **MURRAY J.C.** Keloids and hypertrophic scars. *Clin. Dermatol.,* 1994, vol. 12, 27-37
- **ROBINS S.P.** Biochemistry and functional significance of collagen cross-linking. *Biochem. Soc. Trans.,* 2007, vol. 35, 849-852
- **MCCALL, AS et al.** Mechanisms of Corneal Tissue Cross-linking in Response to Treatment with Topical Riboflavin and Long-Wavelength Ultraviolet Radiation (UVA) Investig. *Ophthalmol. Vis. Sci.,* 2010, vol. 51, 129-138

- **RAISKUP F. ; SPOERL E.** Corneal crosslinking with riboflavin and ultraviolet. *A. I. Principles. Ocul. Surf.,* 2013, vol. 11, 65-74
- **RAISKUP F. ; SPOERL E.** Corneal crosslinking with riboflavin and ultraviolet A. *I. Principles. Ocul. Surf.,* 2013, vol. 11, 65-74
- **ZHANG, Y. et al.** Effects of ultraviolet-A and riboflavin on the interaction of collagen and proteoglycans during corneal cross-linking. *J. Biol. Chem.,* 2011, vol. 286, 13011-13022
- **KATO, Y. et al.** Aggregation of collagen exposed to UVA in the presence of riboflavin: A plausible role of tyrosine modification. *Photochem. Photobiol.,* 1994, vol. 59, 343-349
- **WOLLENSAK, G. et al.** Collagen fiber diameter in the rabbit cornea after collagen crosslinking by riboflavin/UVA. *Cornea,* 2004, vol. 23, 503-507
- **WOLLENSAK, G. et al.** Stress-strain measurements of human and porcine corneas after riboflavin-ultraviolet-A-induced cross-linking. *J. Cataract. Refract. Surg.,* 2003, vol. 29, 1780-1785
- **WOLLENSAK, G. ; IOMDINA, E.** Long-term biomechanical properties of rabbit cornea after photodynamic collagen crosslinking. *Acta Ophthalmol,* 2009, vol. 87, 48-51
- **BRADFORD, SM et al.** Collagen fiber crimping following in vivo UVA-induced corneal crosslinking. *Exp. Eye Res.,* 2018, vol. 177, 173-180
- Collagen fiber crimping following in vivo UVA-induced corneal crosslinking. *Exp. Eye Res.,* 2018, vol. 177, 173-180
- **HERSH, PS et al.** United States Multicenter Clinical Trial of Corneal Collagen Crosslinking for Keratoconus Treatment. *Ophthalmology,* 2017, vol. 124, 1259-1270
- **HERSH, PS et al.** Multicenter Clinical Trial of Corneal Collagen Crosslinking for Treatment of Corneal Ectasia after Refractive Surgery. *Ophthalmology,* 2017, vol. 124, 1475-1484
- **WOLLENSAK, G. et al.** Keratocyte cytotoxicity of riboflavin/UVA-treatment in vitro. *Eye,* 2004, vol. 18, 718-722
- **MAZZOTTA, C. et al.** In Vivo Confocal Microscopy after Corneal Collagen Crosslinking. *Ocul. Surf.,* 2015, vol. 13, 298-314
- **JORDAN, C. et al.** In vivo confocal microscopy analyses of corneal microstructural changes in a prospective study of collagen cross-linking in keratoconus. *Ophthalmology,* 2014, vol. 121, 469-474
- **WOLLENSAK, G. et al.** Corneal endothelial cytotoxicity of riboflavin/UVA treatment in vitro. *Ophthalmic Res,* 2003, vol. 35, 324-328
- **SHARIF, R. et al.** Human in vitro Model Reveals the Effects of Collagen Cross-linking on Keratoconus Pathogenesis. *Sci. Rep.,* 2017, vol. 7, 12517
- **SHHHARIF, R. et al.** Human in vitro Model Reveals the Effects of Collagen Cross-linking on Keratoconus Pathogenesis. *Sci. Rep.,* 2017, vol. 7, 12517
- **SHARIF, R. et al.** Collagen cross-linking impact on keratoconus extracellular matrix. *PLoS ONE,* 2018, vol. 13, e0200704
- **KOBAYASHI, H. ; IKACIA, Y.** *Current Eye Res.,* 2009, vol. 10 (10), 899-908
- **ROSTRON, CK et al.** *Arch. Ophthalmol.,* 1988, vol. 106, 1103-6
- **NAM, S. ; MOONEY, D.** *Chem. Rev.,* 2021
- **NAM, S. ; MOONEY, D.** *Chem. Rev.,* 2020
- **GOMEZ-GUILLEN, MC et al.** *Food Hydrocolloids,* 2011, vol. 25, 1813-27
- **YUE, K. et al.** *Biomaterial,* 2015, vol. 73, 254-71
- **FULLER, C. J.** *Cardiothorac. Surg.,* 2013, vol. 8
- **DUARTE, AP et al.** *Prog. Polym. Sci.,* 2012, vol. 37, 1031-50
- **SEYEDNEJAD, H. et al.** *Br. J. Surg.,* 2008, vol. 95, 1197-1225
- **MEHDIZADEH, M. ; YANG, J.** *Macromol. Biosci.,* 2013, vol. 13, 271-88
- **PREUL, MC et al.** *J. Neurosurg. Spin,* 2010, vol. 12, 381-90
- **FRANSEN, P.** *Spine J.,* 2010, vol. 10, 751-61
- **KIM, K. D et al.** *Global Spine J.,* 2019, vol. 9, 272-8
- **CHEN, CX. et al.** *Proc. Natl Acad. Sci. USA,* 2020, vol. 117, 15497-15503
- **REDMOND, ROBERT W. et al.** *Photochemistry and Photobiology,* 2019, vol. 95, 1097-1115
- **VOTE, BJ et al.** *Clin Exp Ophthalmol.,* 2000, vol. 28 (6), 437-42
- *Good Laboratory Practice for Nonclinical Laboratory Studies,* 20 June 1979
- American Veterinary Medical Association (AVMA) Guidelines for Euthanasia of Animals. 2020
- Guide for Care Use of Laboratory Animals. ILAR. National Academy Press, 2011
- **HAZE.** *Arch. Ophthalmology,* 1992, vol. 110, 1286-1291
- Eye irritation. **T. MCDONALD ; J. A. SHADDUCK.** Advances in Modern Toxicology: Dermatoxicology. Hemisphere Publishing Corporation, 1977, 579-582
- **JABS DA ; NUSSENBLATT RB ; ROSENBAUM JT.** Standardization of Uveitis Nomenclature (SUN) Working Group. *Standardization of uveitis nomenclature for reporting clinical data. Results of the First International Workshop. American Journal of Ophthalmology,* 2005, vol. 140 (3), 509-516
- **OPREMCAK EM.** Uveitis: A Clinical Manual for Ocular Inflammation. Springer Science + Business Media, 2012
- **HASHEMI, H. et al.** The Prevalence and Risk Factors for Keratoconus: A Systematic Review and Meta-Analysis. *Cornea.,* 2020, vol. 39 (2), 263-270

- **MUNIR, S.Z. et al.** Estimated Prevalence of Keratoconus in the United States From a Large Vision Insurance Database. *Eye & Contact Lens.,* July 2021
- **JONAS, J.B. et al.** Prevalence and associations of keratoconus in rural maharashtra in central India: the central India eye and medical study. *Am J Ophthalmol.,* 11 August 2009, vol. 148 (5), 760-5

- **XU, L. et al.** Prevalence and associations of steep cornea/keratoconus in Greater Beijing. The Beijing Eye Study. *PLoS ONE,* 2012, vol. 7, e39313, https://doi.org/10.1371/journal.pone.0039313
- **ANNIE STUART.** A look at LASIK Past, Present and Future. *EyeNet Magazine,* June 2009, https://www.aao.org/eyenet/article/look-at-lasik-past-present-future